(19) Europäisches Patentamt
European Patent Office
Office européen des brevets

(11) **EP 2 998 401 A2**

(12) **EUROPEAN PATENT APPLICATION**

(43) Date of publication:
**23.03.2016 Bulletin 2016/12**

(51) Int Cl.:
**C12N 15/82** (2006.01)   **C12N 1/21** (2006.01)
**A01H 5/00** (2006.01)

(21) Application number: **15186345.3**

(22) Date of filing: **21.06.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2010   US 358028 P**
**16.07.2010   US 364824 P**
**10.11.2010   US 411972 P**

(62) Document number(s) of the earlier application(s) in accordance with Art. 76 EPC:
**11797709.0 / 2 585 604**

(71) Applicant: **BASF Plant Science Company GmbH**
**67056 Ludwigshafen (DE)**

(72) Inventors:
• **Reuzeau, Christophe**
**24350 La Chapelle Gonaguet (FR)**
• **Vandenabeele, Steven**
**9700 Oudenaarde (BE)**
• **Frankard, Valerie**
**1410 Waterloo (BE)**

(74) Representative: **Mistry, Meeta et al**
**Greaves Brewster LLP**
**Copa House**
**Station Road**
**Cheddar, BS27 3AH (GB)**

Remarks:
•The complete document including Reference Tables and the Sequence Listing can be downloaded from the EPO website
•This application was filed on 22-09-2015 as a divisional application to the application mentioned under INID code 62.

(54) **PLANTS HAVING ENHANCED YIELD-RELATED TRAITS AND A METHOD FOR MAKING THE SAME**

(57)   The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding an OsRSZ33 RRM polypeptide or a growth-related protein (GRP) having at least 25% amino acid sequence identity to SEQ ID NO: 251 or a ZPR polypeptide. The present invention also concerns plants having modulated expression of a nucleic acid encoding an OsRSZ33 RRM polypeptide or a growth-related polypeptide as defined herein or a ZPR polypeptide, which plants have enhanced yield-related traits relative to control plants. The invention also provides hitherto unknown OsRSZ33 RRM-encoding nucleic acids or GRP-encoding nucleic acids or a ZPR polypeptide, and constructs comprising the same, useful in performing the methods of the invention.

EP 2 998 401 A2

**Description**

The present invention relates generally to the field of molecular biology and concerns a method for enhancing various economically important yield-related traits in plants by modulating expression in a plant of a nucleic acid encoding i) an OsRSZ33 RRM polypeptide, an orthologue of atRSZ33 or ii) a growth-related protein (GRP) having at least 25% amino acid sequence identity to SEQ ID NO: 251 or iii) a LITTLE ZIPPER (ZPR) polypeptide. The present invention also concerns plants having modulated expression of a nucleic acid encoding an OsRSZ33 RRM polypeptide or a growth-related protein (GRP) having at least 25% amino acid sequence identity to SEQ ID NO: 251 or a ZPR polypeptide, which plants have enhanced yield-related traits relative to corresponding wild type plants or other control plants. The invention also provides constructs useful in the methods of the invention or hitherto unknown GRP-encoding nucleic acids or ZPR-encoding nucleic acids, and constructs comprising the same, useful in performing the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta 218, 1-14, 2003). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various yield-related traits may be improved in plants by modulating expression in a plant of a nucleic acid encoding an OsRSZ33 RRM polypeptide as defined herein or a growth-related polypeptide as defined herein or a ZPR polypeptide as defined herein.

**Background**

Pre-mRNA processing to mRNA requires, in addition to the spliceosome, the activity of a large number of proteins. Many of these proteins, known as SR proteins and SR-related proteins, contain so called RS-domains rich in alternating Arg and Ser residues. Some of these proteins are involved in RNA splicing, others are needed for 3' end formation of mRNA or for export. Splicing (and alternative splicing) is a common step in the formation in mRNA and allows regulation of gene expression by selection of alternative splice sites in the pre-mRNA, thereby generating multiple protein isoforms or variants. An overview of SR proteins and their activities in plants is given in Barta et al. (Curr. Top. Microbiol. Immunol. 326, 83-102, 2008).

Some of the SR proteins are unique to plants, for example several of the 19 SR proteins encoded in the Arabidopsis genome have no orthologues in metazoan (Kalyna et al., Nucleic Acids Res. 34, 4395-4405, 2006). One of these, atRSZ33, acts as a splicing factor for atSRp30 and atSRp34/SR1 (Kalyna et al., Mol Biol. Cell, 14, 3565-3577, 2003). atRSZ33 comprises an RNA recognition motif (RRM), two zinc knuckles embedded in a basic RS region and an acidic C-terminal domain (Lopato et al., J. Biol. Chem. 277, 39989-39998, 2002). It was postulated that its main activity is in spliceosome assembly, and that both zinc knuckles and part of the RS and the RRM domain are needed for protein-protein interaction (Lopato et al. 2002).

Expression of atRSZ33 cDNA under control of the 35S promoter in Arabidopsis caused pleiotropic changes in plant development resulting in increased cell expansion and changed polarisation of cell elongation and division resulting in abnormal and sterile plants. Expression of rice RS proteins in rice reportedly did not influence plant growth in general (Isshiki et al., Plant Cell 18, 146-148, 2006).

In accordance with the present invention, it has been found that modulating the expression of genes that are involved in stress responses in plants may lead to plants having enhanced yield-related traits, in particular plants having increased yield such as but not limited to increased seed yield and increased biomass, relative to control plants.

Stress conditions trigger a wide range of plant responses, including altered gene expression and changes of cellular metabolism. A stress response is usually initiated when plants recognizes stress at the cellular level. Stress recognition activates signal transduction pathways that transmit information within the individual cell and throughout the plant.

Chakrabarty et al. (2009, Chemosphere 74: 688-702) for instance describe comparative transcriptome analysis of arsenate and arsenite stresses in rice seedlings. The authors studied the effect of arsenic (As) exposure on genome-wide expression was examined in rice (Oryza sativa L., ssp. Indica). A group of defense and stress-responsive genes, transporters, heat-shock proteins, metallothioneins, sulfate-metabolizing proteins, and regulatory genes showed differential expression in rice seedlings challenged with arsenate (AsV) and arsenite (AsIII). AsV stress led to upregulation or downregulation of an additional set of genes in comparison to AsIII.

Reactive oxygen species (ROS) are key players in the regulation of plant development, stress responses, and programmed cell death. Previous studies indicated that depending on the type of ROS (hydrogen peroxide, superoxide, or singlet oxygen) or its subcellular production site (plastidic, cytosolic, peroxisomal, or apoplastic), a different physiological, biochemical, and molecular response is provoked. Plants are especially susceptible to oxidative damage, since the production of ROS is increased during stresses imposed by the environment such as extremes of temperature, suboptimal water availability and pollution. Plants have, therefore, evolved a dynamic network of antioxidant defenses that serve to reduce the accumulation of ROS, to detoxify ROS, and to detoxify and repair oxidized molecules. Studies on the role of ROS signalling in stress responses and plant responses thereon are ongoing.

For instance, Gadjev et al. (2006, Plant Physiology, 141:436-445) used transcriptome data generated from ROS-related microarray experiments to assess the specificity of ROS-driven transcript expression. Data sets obtained by exogenous application of oxidative stress-causing agents (methyl viologen, Alternaria alternata toxin, 3-aminotriazole, and ozone) and from a mutant (fluorescent) and transgenic plants, in which the activity of an individual antioxidant enzyme was perturbed (catalase, cytosolic ascorbate peroxidase, and copper/zinc superoxide dismutase), were compared. In total, the abundance of nearly 26,000 transcripts of Arabidopsis (Arabidopsis thaliana) was monitored in response to different ROS. Marker transcripts that were specifically regulated by hydrogen peroxide, superoxide, or singlet oxygen, several transcripts were identified as general oxidative stress response markers because their steady-state levels were at least 5-fold elevated in most experiments. For instance, one transcript, encoding a protein of unknown function (At2g21640), was at least 5-fold elevated in all experiments, except in the KO-Apx1 plants. The authors also inferred new candidate regulatory transcripts that could be responsible for orchestrating the specific transcriptomic signatures triggered by different ROS.

Sweetlove et al. (2002; Plant Journal, 32:891:904) have studied the impact of oxidative stress on Arabidopsis mitochondria and conclude that differences between stress treatments have implications for the nature of the integration of stress responses between plant cell compartments. The authors present a survey of the impact of oxidative stress on the protein composition and function of plant mitochondria. They showed that stress has a significant effect on the mitochondrial proteome leading to the degradation of a number of key proteins. Using 2D gel electrophoresis in combination with MS/MS the authors were also able to identify new, inducible components of the mitochondrial antioxidant system, amongst which stress-responsive genes, At2g21640. They also demonstrated that mitochondrial function is negatively affected by oxidative stress in a manner that will have implications across the cell, and that plant mitochondria utilize a number of antioxidant enzymes to scavenge and detoxify ROS to ameliorate this oxidative damage.

Ho et al. (Plant Physiology, 147:1858-1873, 2008) have studied transcript abundance and promoters of genes encoding mitochondrial proteins to identify signaling pathways that regulate stress-induced gene expression. In their study they used Arabidopsis alternative oxidase AOX1a, external NADP H-dehydrogenase NDB2, and two additional highly stress-responsive genes, At2g21640 and BCS1. Analysis of transcript abundance of these genes in a variety of defense signaling mutants confirmed that BCS1 expression is regulated in a different manner compared to AOX1a, NDB2, and At2g21640. According to the authors, at least three distinctive pathways regulate mitochondrial stress response at a transcriptional level, an SA-dependent pathway represented by BCS1, a second pathway that represents a convergence point for signals generated by H(2)O(2) and rotenone on multiple CAREs, and a third pathway that acts via EDS1 and PAD4 regulating only AOX1a.

In *Arabidopsis,* according to Jun et al. (Plant Signaling & Behavior 3:9, 615-617; 2008) ZPR proteins were identified by two independent groups as a group of genes encoding proteins with small molecular sizes that are composed of 67-100 residues in Arabidopsis (Wenkel et al. The Plant Cell, Vol. 19: 3379-3390, 2007; and , Kim et al. The Plant Cell, Vol. 20: 920-933, 2008).

Wenkel et al. (2007) described this small family of plant-specific LITTLE ZIPPER (ZPR) genes that encode small proteins (67 to 105 amino acids long) and that include a stretch of leucine zipper motifs. This stretch of leucine zipper is approximately six heptads in length and is similar to the leucine zipper found in the class III homeodomain-leucine zipper (HD-ZIPIII) proteins. Class III homeodomain-leucine zipper (HD-ZIPIII) proteins are conserved plant proteins that act as potent regulators of ad/abaxial polarity in *Arabidosis.* HD-ZIPIII protein activity promotes the development of adaxial leaf fates and meristem formation; in its absence, lower abaxial leaf fates develop and meristems fail to form.

Wenkel et al. (2007) reported that LITTLE ZIPPER (ZPR) genes are transcriptionally upregulated by HD-ZIPIII activity. Wenkel et al. (2007) further described that the structural features of the ZPR proteins, and their induction by HD-ZIPIII activity, suggest a model for the action of the ZPR genes as part of a negative feedback loop on HD-ZIPIII function. In the regulatory module proposed by Wenkel et al. (2007), HD-ZIPIII proteins activate transcription of the ZPR genes. The ZPR proteins then form heterodimers with the HD-ZIPIII proteins, preventing or altering their DNA binding.

Kim et al. (2008) demonstrated that a small ZIP protein, ZPR3, and its functionally redundant homolog, ZPR4, negatively regulate the HD-ZIP III activity in Shoot apical meristem (SAM) development. ZPR3 directly interacts with PHABULOSA (PHB) and other HD-ZIP III proteins via the ZIP motifs and forms nonfunctional heterodimers. In view thereof, Kim et al. (2008) proposed that HD-ZIPIII activity in regulating SAM is modulated by, among other things, a feedback loop involving the competitive inhibitors ZPR3 and ZPR4.

Transgenic *Arabidopsis* plants overexpressing ZPR genes have been described in literature (see e.g. Wenkel et al. 2007; Kim et al. 2008). For instance, Wenkel et al. (2007) described transgenic *Arabidopsis* plants that overexpress the *Arabidopsis* ZPR1 gene or the *Arabidopsis* ZPR3 gene. The authors of this paper showed that when overexpressed, the ZPR1 and ZPR3 proteins cause abaxialization consistent with inhibition of HD-ZIPIII function. ZPR3 causes a stronger phenotype, in some cases resulting in the production of a rod-shaped leaf that terminates the shoot apical meristem.

Phylogenetic analysis revealed that ZPR proteins can be classified into two distinct groups: one including ZPR1 and ZPR2 and the other including ZPR3 and ZPR4 (Wenkel et al., 2007). The two groups are distinct in their molecular sizes and the location of the ZIP motifs within the proteins. ZPR1 and ZPR2 (93 and 105 residues, respectively) are larger than ZPR3 and ZPR4 (67 and 72 residues, respectively) mainly because ZPR1 and ZPR2 have N-terminal extensions. Kim et al. (2008) described that this structural distinction, together with the phenotypes of the mutant and transgenic Arabidopsis plants with altered ZPR expressions (Wenkel et al., 2007; Kim et al. 2008), support the idea that, whereas ZPR3 and ZPR4 play a role principally in the SAM development, ZPR1 and ZPR2 are more closely linked to the patterning of leaf polarity.

## Summary

Surprisingly, it has now been found that modulating expression of a nucleic acid encoding OsRSZ33 RRM, an orthologue of atRSZ33 (Arabidopsis thaliana RS-containing Zinc knuckle protein with a molecular mass of 33 kDa, Lopato et al. 2002) and also known as RSZ36 (Isshiki et al., 2006) or a growth-related polypeptide (GRP) or a ZPR polypeptide as defined herein, gives plants having enhanced yield-related traits, in particular increased yield relative to control plants, such as but not limited to increased seed yield and increased biomass relative to control plants.

According one embodiment, there is provided a method for improving yield-related traits in plants relative to control plants, in particular yield, and more particularly seed yield in plants relative to control plants, comprising modulating expression in a plant of a nucleic acid encoding an OsRSZ33 RRM polypeptide or a growth-related polypeptide (GRP) or ZPR polypeptide as defined herein.

The section captions and headings in this specification are for convenience and reference purpose only and should not affect in any way the meaning or interpretation of this specification.

**Definitions**

The following definitions will be used throughout the present specification.

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide and may range from 1 to 10 amino acids; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---------|---------------------------|---------|---------------------------|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |

(continued)

| Residue | Conservative Substitutions | Residue | Conservative Substitutions |
|---------|----------------------------|---------|----------------------------|
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain, Motif/Consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Tables A, F or J of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 \times \log_{10}[Na^+]^a + 0.41 \times \%[G/C^b] - 500 \times [L^c]^{-1} - 0.61 \times \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (ln)$
For 20-35 nucleotides: $T_m = 22 + 1.46 (ln)$

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = $2 \times$(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. $1 \times SSC$ is 0.15M NaCl and 15mM sodium citrate; the hybridisation solution and wash solutions may additionally include 5x Denhardt's reagent, 0.5-1.0% SDS, 100 $\mu$g/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.
The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.
For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |

(continued)

| Gene Source | Reference |
|---|---|
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |

(continued)

| Gene Source | Reference |
|---|---|
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |

(continued)

| Gene source | Reference |
|---|---|
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117 putative rice 40S ribosomal protein | WO 2004/070039 |
| PRO0136 rice alanine aminotransferase | unpublished |
| PRO0147 trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |

(continued)

| Gene source | Reference |
|---|---|
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|---|---|---|
| rice OSH1 | Shoot apical meristem, from embryo globular stage to seedling stage | Sato *et al.* (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not present in, or originating from, the genome of said plant; or are present in the genome of said plant but not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

It shall further be noted that in the context of the present invention, the term "isolated nucleic acid" or "isolated polypeptide" may in some instances be considered as a synonym for a "recombinant nucleic acid" or a "recombinant polypeptide", respectively and refers to a nucleic acid or polypeptide that is not located in its natural genetic environment and/or that has been modified by recombinant methods.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. For the purposes of this invention, the original unmodulated expression may also be absence of any expression. The term "modulating the activity" shall mean any change of the expression of the inventive nucleic acid sequences or encoded proteins, which leads to increased yield and/or increased growth of the plants. The expression can increase from zero (absence of or immeasurable expression) to a certain amount, or can decrease from a certain amount to immeasurable small amounts or zero.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level. For the purposes of this invention, the original wild-type expression level might also be zero (absence of or immeasurable expression).

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Mol Gen Genet 208:1-9; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Traits

Yield related traits are traits or features which are related to plant yield. Yield related traits may comprise one or more of the following non-limitative list of features: early flowering time,yield, biomass, seed yield, early vigour, greenness index, increased growth rate, improved agronomic traits (such as increased tolerance to submergence (which leads to increased yield in rice), improved Water Use Efficiency (WUE), Nitrogen Use Efficiency (NUE), etc.).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters.

The terms

"yield" of a plant or "plant yield" are used interchangeably herein may relate to vegetative biomass (root and/or shoot biomass), to reproductive organs, and/or to propagules (such as seeds) of that plant.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, panicle length, number of spikelets per panicle, number of flowers (florets) per panicle, increase in the seed filling rate (which is the number of filled seeds divided by the total number of seeds and multiplied by 100), increase in thousand kernel weight, among others. In rice, submergence tolerance may also result in increased yield.

Flowers in maize are unisexual; male inflorescences (tassels) originate from the apical stem and female inflorescences (ears) arise from axillary bud apices. The female inflorescence produces pairs of spikelets on the surface of a central axis (cob). Each of the female spikelets encloses two fertile florets, one of them will usually mature into a maize kernel once fertilized. Hence a yield increase in maize may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate, which is the number of filled florets (i.e. florets containing seed) divided by the total number of florets and multiplied by 100), among others.

Inflorescences in rice plants are named panicles. The panicle bears spikelets, which are the basic units of the panicles, and which consist of a pedicel and a floret. The floret is borne on the pedicel and includes a flower that is covered by two protective glumes: a larger glume (the lemma) and a shorter glume (the palea). Hence, taking rice as an example, a yield increase may manifest itself as an increase in one or more of the following: number of plants per square meter, number of panicles per plant, panicle length, number of spikelets per panicle, number of flowers (or florets) per panicle; an increase in the seed filling rate which is the number of filled florets (i.e. florets containing seeds) divided by the total number of florets and multiplied by 100; an increase in thousand kernel weight, among others.

Early flowering time

Plants having an "early flowering time" as used herein are plants which start to flower earlier than control plants. Hence this term refers to plants that show an earlier start of flowering. Flowering time of plants can be assessed by counting the number of days ("time to flower") between sowing and the emergence of a first inflorescence. The "flowering time" of a plant can for instance be determined using the method as described in WO 2007/093444.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures.
"Biotic stresses" are typically those stresses caused by pathogens, such as bacteria, viruses, fungi, nematodes and insects.
The abiotic stress may be an osmotic stress caused by a water stress (particularly due to drought), salt stress, oxidative stress, an ionic stress or freezing stress. Abiotic stress may also be an oxidative stress or a cold stress. "Freezing stress" is intended to refer to stress due to freezing temperatures, i.e. temperatures at which available water molecules freeze and turn into ice. "Cold stress", also called "chilling stress", is intended to refer to cold temperatures, e.g. temperatures below 10°, or preferably below 5°C, but at which water molecules do not freeze. Biotic stresses are typically those stresses caused by pathogens, such as bacteria, viruses, fungi, nematodes and insects.
As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

In particular, the methods of the present invention may be performed under non-stress conditions. In an example, the methods of the present invention may be performed under non-stress conditions such as mild drought to give plants having increased yield relative to control plants.

In another embodiment, the methods of the present invention may be performed under stress conditions.

In an example, the methods of the present invention may be performed under stress conditions such as drought to give plants having increased yield relative to control plants.

In another example, the methods of the present invention may be performed under stress conditions such as nutrient deficiency to give plants having increased yield relative to control plants.

Nutrient deficiency may result from a lack of nutrients such as nitrogen, phosphates and other phosphorous-containing compounds, potassium, calcium, magnesium, manganese, iron and boron, amongst others.

In yet another example, the methods of the present invention may be performed under stress conditions such as salt stress to give plants having increased yield relative to control plants. The term salt stress is not restricted to common salt (NaCl), but may be any one or more of: NaCl, KCl, LiCl, $MgCl_2$, $CaCl_2$, amongst others.

In yet another example, the methods of the present invention may be performed under stress conditions such as cold stress or freezing stress to give plants having increased yield relative to control plants.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following:

   a) an increase in seed biomass (total seed weight) which may be on an individual seed basis and/or per plant and/or per square meter;
   b) increased number of flowers per plant;
   c) increased number of seeds and/or increased number of (filled) seeds;
   d) increased seed filling rate (which is expressed as the ratio between the number of filled seeds divided by the total number of seeds);
   e) increased harvest index, which is expressed as a ratio of the yield of harvestable parts, such as seeds, divided by the total biomass; and
   f) increased thousand kernel weight (TKW), which is extrapolated from the number of filled seeds counted and their total weight. An increased TKW may result from an increased seed size and/or seed weight, and may also result from an increase in embryo and/or endosperm size.

The terms "filled florets" and "filled seeds" may be considered synonyms.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Biomass

The term "biomass" as used herein is intended to refer to the total weight of a plant. Within the definition of biomass, a distinction may be made between the biomass of one or more parts of a plant, which may include any one or more of:

- aboveground (harvestable) parts such as but not limited to shoot biomass, seed biomass, leaf biomass, etc.;
- (harvestable) parts below ground, such as but not limited to root biomass, tubers, bulbs, etc. ;
- vegetative biomass such as root biomass, shoot biomass, etc.;
- reproductive organs;
- propagules such as seed.

Marker assisted breeding

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Use as probes in (gene mapping)

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossipium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

**Detailed description of the invention**

Surprisingly, it has now been found that modulating expression in a plant of a nucleic acid encoding an OsRSZ33 RRM polypeptide or a growth-related polypeptide or a ZPR polypeptide as described hereunder gives plants having enhanced yield-related traits, in particular increased yield, and more particularly increased seed yield relative to control plants.

According to a first embodiment, the present invention provides a method for enhancing yield-related traits in plants relative to control plants, comprising modulating expression in a plant of a nucleic acid encoding an OsRSZ33 RRM polypeptide or a growth-related polypeptide as defined herein or a ZPR polypeptide and optionally selecting for plants having enhanced yield-related traits.

According to another embodiment, the present invention provides a method for producing plants having enhanced yield-related traits relative to control plants, wherein said method comprises the steps of modulating expression in said plant of a nucleic acid encoding an OsRSZ33 RRM polypeptide, or a growth-related polypeptide or a ZPR polypeptide as described herein and optionally selecting for plants having enhanced yield-related traits.

A preferred method for modulating, preferably increasing, expression of a nucleic acid encoding an OsRSZ33 RRM polypeptide or a growth-related polypeptide as described herein or a ZPR polypeptide is by introducing and expressing in a plant a nucleic acid encoding an OsRSZ33 RRM polypeptide or a growth-related polypeptide as provided herein or a ZPR polypeptide, respectively.

Concerning "OsRSZ33 RRM polypeptides" any reference hereinafter to a "protein useful in the methods of the invention" is taken to mean an OsRSZ33 RRM polypeptide as defined herein. Any reference hereinafter to a "nucleic acid useful in the methods of the invention" is taken to mean a nucleic acid capable of encoding such an OsRSZ33 RRM polypeptide. The isolated nucleic acid to be introduced into a plant (and therefore useful in performing the methods of the invention) is any nucleic acid encoding the type of protein which will now be described, hereafter also named *"OsRSZ33 RRM* nucleic acid" or *"OsRSZ33 RRM* gene". Preferably the isolated *OsRSZ33 RRM* nucleic acid to be introduced into a host cell or plant is substantially similar to the endogenous *OsRSZ33 RRM* gene of that host cell or host plant. In one preferred embodiment, the nucleic acid encoding the OsRSZ33 RRM polypeptide is a genomic sequence, in another preferred embodiment, the nucleic acid encoding the OsRSZ33 RRM polypeptide is a cDNA sequence.

Concerning "growth-related proteins", also denoted as GRP, any reference hereinafter to a "protein useful in the methods of the invention" is taken to mean a growth-related polypeptide as defined herein. Any reference hereinafter to a "nucleic acid useful in the methods of the invention" is taken to mean a nucleic acid capable of encoding a growth-related polypeptide as defined herein. The nucleic acid to be introduced into a plant, and therefore useful in performing the methods of the invention, is any nucleic acid encoding the type of protein which will now be described, hereafter also named "growth-related nucleic acid" or "growth-related gene".

Concerning ZPR polypeptides, any reference hereinafter to a "protein useful in the methods of the invention" is taken to mean a "ZPR polypeptide", also named "ZPR protein" as defined herein. ZPR stands for "Little Zipper Protein. Any reference hereinafter to a "nucleic acid useful in the methods of the invention" is taken to mean a nucleic acid capable of encoding such a ZPR polypeptide. The nucleic acid to be introduced into a plant (and therefore useful in performing the methods of the invention) is any nucleic acid encoding the type of protein which will now be described, hereafter also named "ZPR nucleic acid" or "ZPR gene".

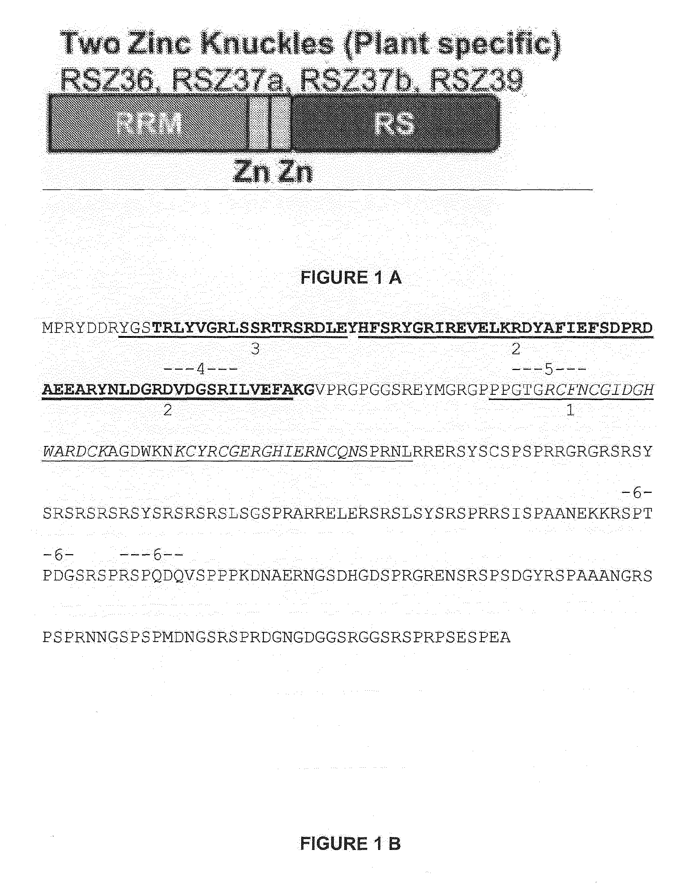

An "OsRSZ33 RRM polypeptide" as defined herein refers to any polypeptide comprising from N- to C-terminus an RRM domain, two Zinc Knuckles and an RS domain (Figure 1). Preferably, the RRM domain corresponds to the RRM_1 domain of Pfam entry PF00076 (SMART SM00360, ProfileScan PS50102) and the Zn knuckles to the zf-CCHC domain of Pfam entry PF00098 (SMART SM00343, ProfileScan PS50158, FPrintScan PR00939). Preferably, the C-terminal domain starting from the last conserved Cys residue of the second Znc finger domain (which C-terminal domain comprises the RS domain), has an Arg+Ser content of more than 13%, preferably at least 14, 15, 16, 17, 18, 19, 20%, more preferably at least 21, 22, 23, 24, 25, 26, 27, 28, 29%, most preferably it has 30%, 31%, 32, 33%, 34%, 35% or more of the amino acids are Arg and/or Ser.

Alternatively and/or additionally, the OsRSZ33 RRM polypeptide comprises one or more of the following motifs:

Motif 1 (SEQ ID NO: 241): PPPG[ST]GRCFNCG[IL]DGHWARDCKAGDWKNKCYRCG ERGHIERNC[QK]NSP[KR][KN]L

Motif 2 (SEQ ID NO: 242): [AHL]F[SG][RK]YGR[VI]R[GDE]V[DE][ML]K[NRH]D[YF]AF[VI] [DE]FSDPRDA[DE][DE]ARY[NS]L[DN]GRD[VF]DGSRI[ILV]VEFA

Motif3 (SEQ ID NO: 243): YG[NGS]TRLYVG[RH]L[SA]SRTR[ST]RDLE

Motifs 1 to 3 were derived using the MEME algorithm (Bailey and Elkan, Proceedings of the Second International Conference on Intelligent Systems for Molecular Biology, pp. 28-36, AAAI Press, Menlo Park, California, 1994). At each position within a MEME motif, the residues are shown that are present in the query set of sequences with a frequency higher than 0.2. Residues within square brackets represent alternatives.

Alternatively and or additionally, OsRSZ33 RRM polypeptides comprise one or more of the following signature sequences:

Motif 4 (SEQ ID NO: 244): RLYVGRL

Motif 5 (SEQ ID NO: 245): RDYAFIE

Motif 6 (SEQ ID NO: 246): RSYSRS

Motifs 4 and 5 are related to respectively the RNP 2 and RNP1 submotifs in the RRM domain (Lopato et al., 2002).

More preferably, the OsRSZ33 RRM polypeptide comprises in increasing order of preference, at least 2, at least 3, at least 4, at least 5, or all 6 motifs.

Additionally or alternatively, the homologue of an OsRSZ33 RRM protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 2, provided that the homologous protein comprises any one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in an OsRSZ33 RRM polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 241 to SEQ ID NO: 246 (Motifs 1 to 6).

The terms "domain", "signature" and "motif" are defined in the "definitions" section herein.

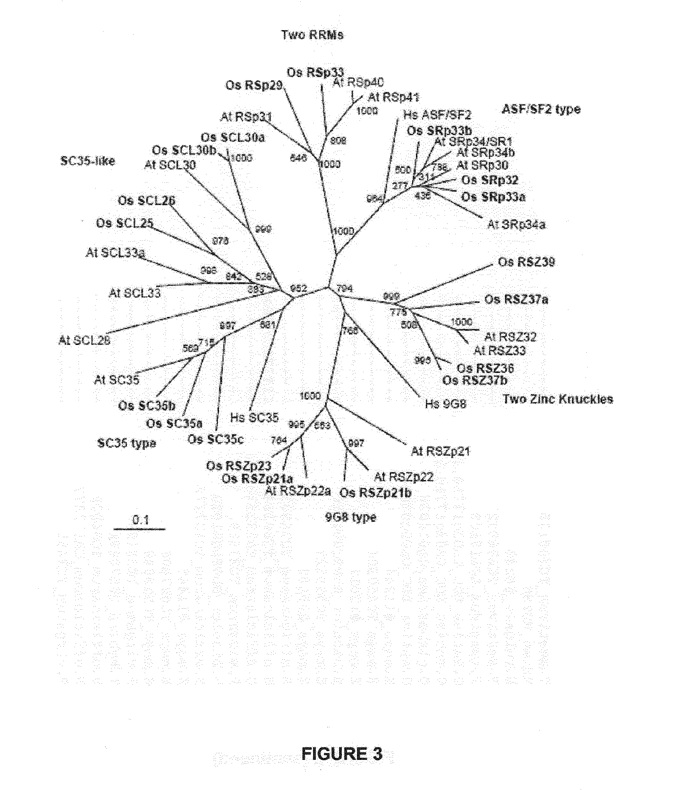

Preferably, the polypeptide sequence which when used in the construction of a phylogenetic tree, such as the one depicted in Figure 3, clusters with the group of SR proteins with two Zinc Knuckles, polypeptides comprising the amino acid sequence represented by SEQ ID NO: 2 (OsRSZ36), rather than with any other group.

Furthermore, OsRSZ33 RRM polypeptides (at least in their native form) typically interact with other splicing factors (Lopato et al., 2002). Tools and techniques for measuring protein-protein interactions, such as yeast two-hybrid screens, are well known in the art. Further details are provided in Example 6.

In addition, OsRSZ33 RRM polypeptides, when expressed in rice according to the methods of the present invention as outlined in Examples 7 and 8, give plants having increased yield related traits, in particular one or more of increased biomass, seed yield and/or early vigour.

The present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any OsRSZ33 RRM-encoding nucleic acid or OsRSZ33 RRM polypeptide as defined herein.

Examples of nucleic acids encoding OsRSZ33 RRM polypeptides are given in Table A of the Examples section herein. Such nucleic acids are useful in performing the methods of the invention. The amino acid sequences given in Table A of the Examples section are example sequences of orthologues and paralogues of the OsRSZ33 RRM polypeptide represented by SEQ ID NO: 2, the terms "orthologues" and "paralogues" being as defined herein. Further orthologues and paralogues may readily be identified by performing a so-called reciprocal blast search as described in the definitions section; where the query sequence is SEQ ID NO: 1 or SEQ ID NO: 2, the second BLAST (back-BLAST) would be against rice sequences.

The invention also provides hitherto unknown OsRSZ33 RRM-encoding nucleic acids and OsRSZ33 RRM polypeptides useful for conferring enhanced yield-related traits in plants relative to control plants.

According to a further embodiment of the present invention, there is therefore provided an isolated nucleic acid molecule selected from:

(i) a nucleic acid represented by any one of SEQ ID NO: 109, 135, 221, 115, 165, 225, 197, 211, or 237 (B.napus_BN06MC04617_42261920@4606#1; G.max_GM06MC33050_sl61g01@32287#1; H.vulgare_c62741371hv270303@6248#1; V.vinifera_GSVIVT00023097001#1; V.vinifera_GSVIVT00029498001#1; Z.mays_c67261821gm030403@11244#1; Z.mays_c68511709gm030403@11445#1; Z.mays_ZM07MC13041_BFb0138E15@13012#1; Z.mays_ZM07MC35249_BFb0381l21@35142#1);

(ii) the complement of any of the nucleic acid represented by any one of SEQ ID NO: 109, 135, 221, 115, 165, 225, 197, 211, or 237;

(iii) a nucleic acid encoding an OsRSZ33 RRM polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any of the amino acid sequences represented by SEQ ID NO: 110, 136, 222, 116, 166, 226, 198, 212, or 238 (B.napus_BN06MC04617_42261920@4606#1; >G.max_GM06MC33050_sl61g01@32287#1; >H.vulgare_c62741371hv270303@6248#1; >V.vinifera_GSVIVT00023097001#1;

>V.vinifera_GSVIVT00029498001#1;                    >Z.mays_c67261821gm030403@11244#1;
>Z.mays_c68511709gm030403@11445#1;                 >Z.mays_ZM07MC13041_BFb0138E15@13012#1;
>Z.mays_ZM07MC35249_BFb0381I21@35142#1), and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs given in SEQ ID NO: 241 to SEQ ID NO: 246, and further preferably conferring enhanced yield-related traits relative to control plants.
(iv) a nucleic acid molecule which hybridizes with a nucleic acid molecule of (i) to (iii) under high stringency hybridization conditions and preferably confers enhanced yield-related traits relative to control plants.

According to a further embodiment of the present invention, there is also provided an isolated polypeptide selected from:

(i) an amino acid sequence represented by any of SEQ ID NO: 110, 136, 222, 116, 166, 226, 198, 212, or 238 (B.napus_BN06MC04617_42261920@4606#1;                    >G.max_GM06MC33050_sl61g01@32287#1;
>H.vulgare_c62741371hv270303@6248#1;                    >V.vinifera_GSVIVT00023097001#1;
>V.vinifera_GSVIVT00029498001#1;                    >Z.mays_c67261821gm030403@11244#1;
>Z.mays_c68511709gm030403@11445#1;                 >Z.mays_ZM07MC13041_BFb0138E15@13012#1;
>Z.mays_ZM07MC35249_BFb0381I21 @35142#1);
(ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 110, 136, 222, 116, 166, 226, 198, 212, or 238 (B.napus_BN06MC04617_42261920@4606#1;
>G.max_GM06MC33050_sl61g01@32287#1;                    >H.vulgare_c62741371hv270303@6248#1;
>V.vinifera_GSVIVT00023097001#1;                    >V.vinifera_GSVIVT00029498001#1;
>Z.mays_c67261821gm030403@11244#1;                    >Z.mays_c68511709gm030403@11445#1;
>Z.mays_ZM07MC13041_BFb0138E15@13012#1; >Z.mays_ZM07MC35249_BFb0381I21@35142#1), and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs given in SEQ ID NO: 241 to SEQ ID NO: 246, and further preferably conferring enhanced yield-related traits relative to control plants;
(iii) derivatives of any of the amino acid sequences given in (i) or (ii) above.

Nucleic acid variants may also be useful in practising the methods of the invention. Examples of such variants include nucleic acids encoding homologues and derivatives of any one of the amino acid sequences given in Table A of the Examples section, the terms "homologue" and "derivative" being as defined herein. Also useful in the methods of the invention are nucleic acids encoding homologues and derivatives of orthologues or paralogues of any one of the amino acid sequences given in Table A of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding OsRSZ33 RRM polypeptides, nucleic acids hybridising to nucleic acids encoding OsRSZ33 RRM polypeptides, splice variants of nucleic acids encoding OsRSZ33 RRM polypeptides, allelic variants of nucleic acids encoding OsRSZ33 RRM polypeptides and variants of nucleic acids encoding OsRSZ33 RRM polypeptides obtained by gene shuffling. The terms hybridising sequence, splice variant, allelic variant and gene shuffling are as described herein.

Nucleic acids encoding OsRSZ33 RRM polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A of the Examples section, or a portion of a nucleic acid encoding an orthologue, paralogue or homologue of any of the amino acid sequences given in Table A of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode an OsRSZ33 RRM polypeptide as defined herein, and have substantially the same biological activity as the amino acid sequences given in Table A of the Examples section.

Preferably, the portion is a portion of any one of the nucleic acids given in Table A of the Examples section, or is a portion of a nucleic acid encoding an orthologue or paralogue of any one of the amino acid sequences given in Table A of the Examples section. Preferably the portion is at least 400, 450, 500, 550, 600, 650, 700, 750, 800, 850, 900, 950, 1000, 1050, 1100 consecutive nucleotides in length, the consecutive nucleotides being of any one of the nucleic acid sequences given in Table A of the Examples section, or of a nucleic acid encoding an orthologue or paralogue of any one of the amino acid sequences given in Table A of the Examples section. Most preferably the portion is a portion of the nucleic acid of SEQ ID NO: 1. Preferably, the portion encodes a fragment of an amino acid sequence which, when used in the construction of a phylogenetic tree, such as the one depicted in Figure 3, clusters with the group of SR proteins with two Zinc Knuckles, polypeptides comprising the amino acid sequence represented by SEQ ID NO: 2 (OsRSZ36), rather than with any other group and/or comprises an RRM domain, two Zinc knuckle domains and preferably also one or more of motifs 1 to 6.

Another nucleic acid variant useful in the methods of the invention is a nucleic acid capable of hybridising, under reduced stringency conditions, preferably under stringent conditions, with a nucleic acid encoding an OsRSZ33 RRM polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A of the Examples section, or comprising introducing and expressing in a plant a nucleic acid capable of hybridising to a nucleic acid encoding an orthologue, paralogue or homologue of any of the nucleic acid sequences given in Table A of the Examples section.

Hybridising sequences useful in the methods of the invention encode an OsRSZ33 RRM polypeptide as defined herein, having substantially the same biological activity as the amino acid sequences given in Table A of the Examples section. Preferably, the hybridising sequence is capable of hybridising to the complement of any one of the nucleic acids given in Table A of the Examples section, or to a portion of any of these sequences, a portion being as defined above, or the hybridising sequence is capable of hybridising to the complement of a nucleic acid encoding an orthologue or paralogue of any one of the amino acid sequences given in Table A of the Examples section. Most preferably, the hybridising sequence is capable of hybridising to the complement of a nucleic acid as represented by SEQ ID NO: 1 or to a portion thereof.

Preferably, the hybridising sequence encodes a polypeptide with an amino acid sequence which, when full-length and used in the construction of a phylogenetic tree, such as the one depicted in Figure 3, clusters with the group of SR proteins with two Zinc Knuckles, polypeptides comprising the amino acid sequence represented by SEQ ID NO: 2 (OsRSZ36), rather than with any other group and/or comprises an RRM domain, two Zinc knuckle domains and preferably also one or more of motifs 1 to 6.

Another nucleic acid variant useful in the methods of the invention is a splice variant encoding an OsRSZ33 RRM polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A of the Examples section, or a splice variant of a nucleic acid encoding an orthologue, paralogue or homologue of any of the amino acid sequences given in Table A of the Examples section.

Preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant, when used in the construction of a phylogenetic tree, such as the one depicted in Figure 3, clusters with the group of SR proteins with two Zinc Knuckles, polypeptides comprising the amino acid sequence represented by SEQ ID NO: 2 (OsRSZ36), rather than with any other group and/or comprises an RRM domain, two Zinc knuckle domains and preferably also one or more of motifs 1 to 6.

Another nucleic acid variant useful in performing the methods of the invention is an allelic variant of a nucleic acid encoding an OsRSZ33 RRM polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A of the Examples section, or comprising introducing and expressing in a plant an allelic variant of a nucleic acid encoding an orthologue, paralogue or homologue of any of the amino acid sequences given in Table A of the Examples section.

The polypeptides encoded by allelic variants useful in the methods of the present invention have substantially the same biological activity as the OsRSZ33 RRM polypeptide of SEQ ID NO: 2 and any of the amino acids depicted in Table A of the Examples section. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant, when used in the construction of a phylogenetic tree, such as the one depicted in Figure 3, clusters with the group of SR proteins with two Zinc Knuckles, polypeptides comprising the amino acid sequence represented by SEQ ID NO: 2 (OsRSZ36), rather than with any other group and/or comprises an RRM domain, two Zinc knuckle domains and preferably also one or more of motifs 1 to 6.

Gene shuffling or directed evolution may also be used to generate variants of nucleic acids encoding OsRSZ33 RRM polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A of the Examples section, or comprising introducing and expressing in a plant a variant of a nucleic acid encoding an orthologue, paralogue or homologue of any of the amino acid sequences given in Table A of the Examples section, which variant nucleic acid is obtained by gene shuffling.

Preferably, the amino acid sequence encoded by the variant nucleic acid obtained by gene shuffling, when used in the construction of a phylogenetic tree such as the one depicted in Figure 3, clusters with the group of SR proteins with two Zinc Knuckles, polypeptides comprising the amino acid sequence represented by SEQ ID NO: 2 (OsRSZ36), rather than with any other group and/or comprises an RRM domain, two Zinc knuckle domains and preferably also one or more of motifs 1 to 6.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding OsRSZ33 RRM polypeptides may be derived from any natural or artificial source. The nucleic acid may be modified from its native form in composition and/or genomic environment through deliberate human manipulation. Preferably the OsRSZ33 RRM polypeptide-encoding nucleic acid is from a plant, further preferably from a monocotyledonous plant, more preferably from the family Poaceae, most preferably the nucleic acid is from *Oryza sativa.*

Performance of the methods of the invention gives plants having enhanced yield-related traits. In particular performance of the methods of the invention gives plants having increased early vigour and/or increased yield, especially increased seed yield and/or increased biomass relative to control plants. The terms "early vigour", "yield" and "seed yield" are described in more detail in the "definitions" section herein.

Reference herein to enhanced yield-related traits is taken to mean an increase early vigour and/or in biomass (weight) of one or more parts of a plant, which may include aboveground (harvestable) parts and/or (harvestable) parts below ground. In particular, such harvestable parts are vegetative biomass (roots and/or shoots) and seeds, and performance of the methods of the invention results in plants having increased yield (increased biomass and/or seed yield) and/or increased early vigour, relative to the yield of control plants.

The present invention provides a method for increasing yield-related traits, especially early vigour, biomass and/ore seed yield of plants, relative to control plants, which method comprises modulating expression in a plant of a nucleic acid encoding an OsRSZ33 RRM polypeptide as defined herein.

Since the transgenic plants according to the present invention have increased yield-related traits, it is likely that these plants exhibit an increased growth rate (during at least part of their life cycle), relative to the growth rate of control plants at a corresponding stage in their life cycle.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding an OsRSZ33 RRM polypeptide as defined herein.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding an OsRSZ33 RRM polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding an OsRSZ33 RRM polypeptide.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding an OsRSZ33 RRM polypeptide.

The invention also provides genetic constructs and vectors to facilitate introduction and/or expression in plants of nucleic acids encoding OsRSZ33 RRM polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding an OsRSZ33 RRM polypeptide as defined above;

(b) one or more control sequences capable of driving expression of the nucleic acid sequence of (a); and optionally

(c) a transcription termination sequence.

Preferably, the nucleic acid encoding an OsRSZ33 RRM polypeptide is as defined above. The term "control sequence" and "termination sequence" are as defined herein.

The invention furthermore provides plants transformed with a construct as described above. In particular, the invention provides plants transformed with a construct as described above, which plants have increased yield-related traits as described herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a ubiquitous constitutive promoter of medium strength. See the "Definitions" section herein for definitions of the various promoter types.

It should be clear that the applicability of the present invention is not restricted to the OsRSZ33 RRM polypeptide-encoding nucleic acid represented by SEQ ID NO: 1, nor is the applicability of the invention restricted to expression of an OsRSZ33 RRM polypeptide-encoding nucleic acid when driven by a constitutive promoter.

The constitutive promoter is preferably a medium strength promoter. More preferably it is a plant derived promoter, such as a GOS2 promoter or a promoter of substantially the same strength and having substantially the same expression pattern (a functionally equivalent promoter), more preferably the promoter is the promoter GOS2 promoter from rice. Further preferably the constitutive promoter is represented by a nucleic acid sequence substantially similar to SEQ ID NO: 247, most preferably the constitutive promoter is as represented by SEQ ID NO: 247. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 247, and the nucleic acid encoding the OsRSZ33 RRM polypeptide. Furthermore, one or more sequences encoding selectable markers may be present on the construct introduced into a plant.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding an OsRSZ33 RRM polypeptide is by introducing and expressing in a plant a nucleic acid encoding an OsRSZ33 RRM polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The invention also provides a method for the production of transgenic plants having enhanced yield-related traits relative to control plants, comprising introduction and expression in a plant of any nucleic acid encoding an OsRSZ33 RRM polypeptide as defined hereinabove.

More specifically, the present invention provides a method for the production of transgenic plants having enhanced yield-related traits, particularly increased yield and/or increased early vigour, which method comprises:

(i) introducing and expressing in a plant or plant cell an OsRSZ33 RRM polypeptide-encoding nucleic acid or a genetic construct comprising an OsRSZ33 RRM polypeptide-encoding nucleic acid; and

(ii) cultivating the plant cell under conditions promoting plant growth and development.

The nucleic acid of (i) may be any of the nucleic acids capable of encoding an OsRSZ33 RRM polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The present invention clearly extends to any plant cell or plant produced by any of the methods described herein, and to all plant parts and propagules thereof. The present invention encompasses plants or parts thereof (including seeds) obtainable by the methods according to the present invention. The plants or parts thereof comprise a nucleic acid transgene encoding an OsRSZ33 RRM polypeptide as defined above. The present invention extends further to

EP 2 998 401 A2

encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding an OsRSZ33 RRM polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, chicory, carrot, cassava, trefoil, beet, sugar beet, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding an OsRSZ33 RRM polypeptide. The invention furthermore relates to products derived, preferably directly derived, from a harvestable part of such a plant, such as dry pellets or powders, oil, fat and fatty acids, starch or proteins.

The present invention also encompasses use of nucleic acids encoding OsRSZ33 RRM polypeptides as described herein and use of these OsRSZ33 RRM polypeptides in enhancing any of the aforementioned yield-related traits in plants. For example, nucleic acids encoding OsRSZ33 RRM polypeptide described herein, or the OsRSZ33 RRM polypeptides themselves, may find use in breeding programmes in which a DNA marker is identified which may be genetically linked to an OsRSZ33 RRM polypeptide-encoding gene. The nucleic acids/genes, or the OsRSZ33 RRM polypeptides themselves may be used to define a molecular marker. This DNA or protein marker may then be used in breeding programmes to select plants having enhanced yield-related traits as defined hereinabove in the methods of the invention. Furthermore, allelic variants of an OsRSZ33 RRM polypeptide-encoding nucleic acid/gene may find use in marker-assisted breeding programmes. Nucleic acids encoding OsRSZ33 RRM polypeptides may also be used as probes for genetically and physically mapping the genes that they are a part of, and as markers for traits linked to those genes. Such information may be useful in plant breeding in order to develop lines with desired phenotypes.

The terms "growth-related polypeptide", "growth-related protein" or "GRP", as given herein are all intended to include a polypeptide as represented by SEQ ID NO: 251 and homologues thereof. In an embodiment, a homologue of a GRP has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 251.

In an embodiment of the invention a method as provided herein is presented wherein said method comprises the modulation of expression in a plant of a nucleic acid encoding a growth-related protein (GRP) having at least 25% sequence identity to the amino acid sequence represented by SEQ ID NO: 251. In another embodiment, the present method comprises the modulation of expression in a plant of a nucleic acid encoding a growth-related protein (GRP) having at least 25%, and for instance at least 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 251.

In another embodiment, a growth-related polypeptide according to the present invention comprises a conserved motif. The terms "domain", "signature" and "motif" are defined in the "definitions" section herein.

In particular, in a preferred embodiment, a growth-related polypeptide according to the present invention comprises a motif which has at least 70% sequence identity to motif 7: [NHPS][SC][CR][RK]K[NK][VST][PD][GD][TAV][ST]F[VL][SE]DL[RK]DH[IM][HD]EFI[NH]AS[ ASM]DEH[MRK][TH]CF[ TK][KN]T[IL][KQ][KR]MF[GD]MS[KM][TAV]V[AT] (SEQ ID NO: 742). Motif 7 was derived using the MEME algorithm (Bailey and Elkan, Proceedings of the Second International Conference on Intelligent Systems for Molecular Biology, pp. 28-36, AAAI Press, Menlo Park, California, 1994). At each position within a MEME motif, the residues are shown that are present in the query set of sequences with a frequency higher than 0.2. Residues within square brackets represent alternatives. In a preferred embodiment, a growth-related polypeptide according to the present invention comprises a motif which has in increasing order of preference at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the motif represented by SEQ ID NO: 742 (Motif 7).

Additionally or alternatively, the homologue of a GRP has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 251, provided that the homologous protein comprises a motif which has at least 70% sequence identity to conserved motif 7 as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motif in a GRP has at least 70%, and for instance at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the motif represented by SEQ ID NO: 742 (Motif 7).

In an example, a growth-related polypeptide according to the present invention has a motif which has at least 70%, and for instance at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% sequence identity to motif X, or corresponds to motif X, wherein motif X is represented by: SCRKKKSDDATFLEDLKDHIDEFIHASMDEHKHCFK NTIQKMFGMSKVVA (SEQ ID NO: 743).

In other words, in another embodiment a method is provided wherein said GRP comprises a motif which has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to, or corresponds to a conserved domain of amino acid coordinates 23 to 72 of SEQ ID NO:251.

In addition, growth-related polypeptides according to the invention, when expressed transgenic plants such as e.g. rice according to the methods of the present invention as outlined in the example section, give plants having increased yield-related traits, and preferably increased yield relative to control plants, and even more preferably wherein said increased yield-related traits is selected from the group comprising or consisting of increased seed yield, increased biomass and early vigour.

The present invention is illustrated by transforming plants with the nucleic acid sequence represented by SEQ ID NO: 250, encoding the polypeptide sequence of SEQ ID NO: 251. However, performance of the invention is not restricted to these sequences; the methods of the invention may advantageously be performed using any GRP-encoding nucleic acid or GRP polypeptide as defined herein.

Examples of nucleic acids encoding GRP polypeptides are given in Table F of the Examples section. Any of the sequences listed in Table F is suitable for performing the methods according to the invention. The amino acid sequences given in Table F of the Examples section are exemplary sequences of orthologues and paralogues of the polypeptide represented by SEQ ID NO: 251, the terms "orthologues" and "paralogues" being as defined herein. Further orthologues and paralogues may readily be identified by performing a so-called reciprocal blast search as described in the definitions section; where the query sequence is SEQ ID NO: 250 or SEQ ID NO: 251, the second BLAST (back-BLAST) would be against rice sequences.

The invention also provides hitherto unknown GRP-encoding nucleic acids and growth-related proteins useful for conferring enhanced yield-related traits in plants relative to control plants.

According to an embodiment of the present invention, there is therefore provided an isolated nucleic acid selected from:

> (i) a nucleic acid represented by any one of SEQ ID NO: 266, 286, 316, 374, 376, 380, 424, 456, 458, 460, 462, 648, 650, 670, and 736;
> (ii) the complement of a nucleic acid represented by any one of SEQ ID NO: 266, 286, 316, 374, 376, 380, 424, 456, 458, 460, 462, 648, 650, 670, and 736;
> (iii) a nucleic acid encoding the polypeptide as represented by any one of SEQ ID NO: 267, 287, 317, 375, 377, 381, 425, 457, 459, 461, 463, 649, 651, 671 and 737, preferably as a result of the degeneracy of the genetic code, said isolated nucleic acid can be derived from a polypeptide sequence as represented by any one of SEQ ID NO: 267, 287, 317, 375, 377, 381, 425, 457, 459, 461, 463, 649, 651, 671 and 737 and further preferably confers enhanced yield-related traits relative to control plants;
> (iv) a nucleic acid having, in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences represented by any one of SEQ ID NO: 266, 286, 316, 374, 376, 380, 424, 456, 458, 460, 462, 648, 650, 670, and 736; and any of the other nucleic acid sequences in Table F and further preferably conferring enhanced yield-related traits relative to control plants;

(v) a nucleic acid molecule which hybridizes with a nucleic acid molecule of (i) to (iv) under stringent hybridization conditions and preferably confers enhanced yield-related traits relative to control plants;

(vi) a nucleic acid encoding a polypeptide having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 267, 287, 317, 375, 377, 381, 425, 457, 459, 461, 463, 649, 651, 671 and 737 and any of the other amino acid sequences in Table F and preferably conferring enhanced yield-related traits relative to control plants.

According to another embodiment of the present invention, there is also provided an isolated polypeptide selected from:

(i) an amino acid sequence represented by any one of SEQ ID NO: 267, 287, 317, 375, 377, 381, 425, 457, 459, 461, 463, 649, 651, 671 and 737;

(ii) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%,50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 267, 287, 317, 375, 377, 381, 425, 457, 459, 461, 463, 649, 651, 671 and 737, and any of the other amino acid sequences in Table F and preferably conferring enhanced yield-related traits relative to control plants, and

(iii) derivatives of any of the amino acid sequences given in (i) or (ii) above.

The invention hence also relates to the use of an isolated nucleic acid as provided above, or of an isolated polypeptide as provided above for enhancing yield-related traits in plants, and preferably wherein said yield-related traits are as defined herein, relative to control plants. Preferably said yield-related traits are as defined in herein relative to control plants. Further preferably said use involves a step of introducing in a plant, plant part or plant cell of such isolated nucleic acid or such isolated polypeptide as defined herein.

Nucleic acid variants may also be useful in practising the methods of the invention. Examples of such variants include nucleic acids encoding homologues and derivatives of any one of the amino acid sequences given in Table F of the Examples section, the terms "homologue" and "derivative" being as defined herein. Also useful in the methods of the invention are nucleic acids encoding homologues and derivatives of orthologues or paralogues of any one of the amino acid sequences given in Table F of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding growth-related polypeptides, nucleic acids hybridising to nucleic acids encoding growth-related polypeptides, splice variants of nucleic acids encoding growth-related polypeptides, allelic variants of nucleic acids encoding growth-related polypeptides and variants of nucleic acids encoding growth-related polypeptides obtained by gene shuffling. The terms hybridising sequence, splice variant, allelic variant and gene shuffling are as described herein.

Nucleic acids encoding growth-related polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table F of the Examples section, or a portion of a nucleic acid encoding an orthologue, paralogue or homologue of any of the amino acid sequences given in Table F of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a growth-related polypeptide as defined herein. Preferably, the portion is a portion of any one of the nucleic acids given in Table F of the Examples section, or is a portion of a nucleic acid encoding an orthologue or paralogue of any one of the amino acid sequences given in Table F of the Examples section. Preferably the portion is at least 150, 200, 250, 300, 350, 400, 450, 500, 550, 600, 650, 700, consecutive nucleotides in length, the consecutive nucleotides being of any one of the nucleic acid sequences given in Table F of the Examples section, or of a nucleic acid encoding an orthologue or paralogue of any one of the amino acid sequences given in Table F of the Examples section. Most preferably the portion is a portion of the nucleic acid of SEQ ID NO: 250. Preferably, the portion encodes a fragment of an amino acid sequence which has a motif having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to motif 7 and/or which has at least 25%, 26%, 27%, 28%, 29%, 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 251.

Another nucleic acid variant useful in the methods of the invention is a nucleic acid capable of hybridising, under reduced stringency conditions, preferably under stringent conditions, with a nucleic acid encoding a GRP as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table F of the Examples section, or comprising introducing and expressing in a plant a nucleic acid capable of hybridising to a nucleic acid encoding an orthologue, paralogue or homologue of any of the nucleic acid sequences given in Table F of the Examples section.

Hybridising sequences useful in the methods of the invention encode a GRP as defined herein, having substantially the same biological activity as the amino acid sequences given in Table F of the Examples section. Preferably, the hybridising sequence is capable of hybridising to the complement of any one of the nucleic acids given in Table F of the Examples section, or to a portion of any of these sequences, a portion being as defined above, or the hybridising sequence is capable of hybridising to the complement of a nucleic acid encoding an orthologue or paralogue of any one of the amino acid sequences given in Table F of the Examples section. Most preferably, the hybridising sequence is capable of hybridising to the complement of a nucleic acid as represented by SEQ ID NO: 250 or to a portion thereof.

Preferably, the hybridising sequence encodes a polypeptide with an amino acid sequence which has a motif having at least 70%, and for instance at least 75%, 80%, 85%, 90%, 95%, or 99% sequence identity to motif 7 as herein defined and/or which has at least 25%, and for instance at least 50%, 75%, 90%, or 95% sequence identity to SEQ ID NO: 251.

Another nucleic acid variant useful in the methods of the invention is a splice variant encoding a GRP as defined hereinabove, a splice variant being as defined herein.

According to the present invention, there is provided a method for enhancing yield-related traits in plants, comprising introducing and expressing in a plant a splice variant of any one of the nucleic acid sequences given in Table F of the Examples section, or a splice variant of a nucleic acid encoding an orthologue, paralogue or homologue of any of the amino acid sequences given in Table F of the Examples section.

Preferred splice variants are splice variants of a nucleic acid represented by SEQ ID NO: 250, or a splice variant of a nucleic acid encoding an orthologue or paralogue of SEQ ID NO: 251. Preferably, the amino acid sequence encoded by the splice variant has a motif having at least 70%, and for instance at least 75%, 80%, 85%, 90%, 95%, or 99% sequence identity to motif 7 as herein defined and/or has at least 25%, and for instance at least 50%, 75%, 90%, or 95% sequence identity to SEQ ID NO: 251.

Another nucleic acid variant useful in performing the methods of the invention is an allelic variant of a nucleic acid encoding a GRP as defined hereinabove, an allelic variant being as defined herein.

According to the present invention, there is provided a method for enhancing yield-related traits in plants, comprising introducing and expressing in a plant an allelic variant of any one of the nucleic acids given in Table F of the Examples section, or comprising introducing and expressing in a plant an allelic variant of a nucleic acid encoding an orthologue, paralogue or homologue of any of the amino acid sequences given in Table F of the Examples section.

The polypeptides encoded by allelic variants useful in the methods of the present invention have substantially the same biological activity as the GRP of SEQ ID NO: 251 and any of the amino acids depicted in Table F of the Examples section. Allelic variants exist in nature, and encompassed within the methods of the present invention is the use of these natural alleles. Preferably, the allelic variant is an allelic variant of SEQ ID NO: 250 or an allelic variant of a nucleic acid encoding an orthologue or paralogue of SEQ ID NO: 251. Preferably, the amino acid sequence encoded by the allelic variant has a motif having at least 70%, and for instance at least 75%, 80%, 85%, 90%, 95%, or 99% sequence identity to motif 7 as herein defined and/or has at least 25%, and for instance at least 50%, 75%, 90%, or 95% sequence identity to SEQ ID NO: 251.

Gene shuffling or directed evolution may also be used to generate variants of nucleic acids encoding GRP as defined above; the term "gene shuffling" being as defined herein.

According to the present invention, there is provided a method for enhancing yield-related traits in plants, comprising introducing and expressing in a plant a variant of any one of the nucleic acid sequences given in Table F of the Examples section, or comprising introducing and expressing in a plant a variant of a nucleic acid encoding an orthologue, paralogue or homologue of any of the amino acid sequences given in Table F of the Examples section, which variant nucleic acid is obtained by gene shuffling.

Preferably, the amino acid sequence encoded by the variant nucleic acid obtained by gene shuffling has a motif having at least 70%, and for instance at least 75%, 80%, 85%, 90%, 95%, or 99% sequence identity to motif 7 as herein defined and/or has at least 25%, and for instance at least 50%, 75%, 90%, or 95% sequence identity to SEQ ID NO: 251.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding GRP may be derived from any natural or artificial source. The nucleic acid may be modified from its native form in composition and/or genomic environment through deliberate human manipulation.

In an embodiment, the GRP-encoding nucleic acid is from a plant, further preferably from a monocotyledonous plant, more preferably from the family *Poaceae,* most preferably the nucleic acid is from *Oryza sativa.* In a preferred embodiment, said GRP-encoding nucleic acid encodes the polypeptide as represented by SEQ ID NO: 251.

In another embodiment, the GRP-encoding nucleic acid is from a plant, further preferably from a dicotyledonous plant, more preferably from the family *Brassicaceae,* most preferably the nucleic acid is from *Arabidopsis thaliana.* In another preferred embodiment, said GRP-encoding nucleic acid encodes a polypeptide as represented by any of SEQ ID NO: 275, 277 or 279, and preferably as represented by SEQ ID NO: 275.

Performance of the methods of the invention gives plants having enhanced yield-related traits. In one embodiment, reference herein to enhanced yield-related traits is taken to mean an increase in early vigour. Hence, in an embodiment, performance of the methods of the invention gives plants having early vigour relative to control plants.

In another embodiment, reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

In another embodiment, reference herein to enhanced yield-related traits is taken to mean an increase in yield, especially increased seed yield relative to control plants. The terms "yield" and "seed yield" are described in more detail in the "definitions" section herein. In a preferred embodiment, said increased seed yield is selected from any one or more of:

(i) increased harvest index;
(ii) increased total seed weight;
(iii) increased number of filled seeds;
(iv) number of flowers per panicle; and
(v) increased thousand kernel weight.

The present invention provides a method for increasing yield, especially seed yield of plants, relative to control plants, which method comprises modulating expression in a plant of a nucleic acid encoding a GRP as defined herein. In another embodiment, the present invention also provides a method for increasing biomass of plants, relative to control plants, which method comprises modulating expression in a plant of a nucleic acid encoding a GRP as defined herein. In yet another embodiment, the present invention also provides a method for increasing early vigour of plants, relative to control plants, which method comprises modulating expression in a plant of a nucleic acid encoding a GRP as defined herein. In another embodiment, according to the present invention, there is also provided a method for increasing the growth rate of plants, which method comprises modulating expression in a plant of a nucleic acid encoding a GRP polypeptide as defined herein.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding a GRP as defined herein.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding a GRP as defined herein.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding a GRP as defined herein.

The invention also provides genetic constructs and vectors to facilitate introduction and/or expression in plants of nucleic acids encoding growth related polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a GRP as defined above;
(b) one or more control sequences capable of driving expression of the nucleic acid sequence of (a); and optionally
(c) a transcription termination sequence.

Preferably, the nucleic acid encoding a GRP is as defined above. The term "control sequence" and "termination sequence" are as defined herein.

The invention furthermore provides plants transformed with a construct as described above. In particular, the invention provides plants transformed with a construct as described above, which plants have increased yield-related traits as described herein.

In another embodiment, the invention also relates to the use of a construct as given herein in a method for making plants having enhanced yield-related traits, preferably yield-related traits as defined herein, relative to control plants. Preferably said use involves a step of introducing in a plant, plant part or plant cell plant of a construct as given above.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences, e.g. at least to a promoter.

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a ubiquitous constitutive promoter of medium strength. See the "Definitions" section herein for definitions of the various promoter types.

It should be clear that the applicability of the present invention is not restricted to a GRP-encoding nucleic acid represented by SEQ ID NO: 250, nor is the applicability of the invention restricted to expression of a GRP-encoding nucleic acid when driven by a constitutive promoter.

The constitutive promoter is preferably a medium strength promoter. More preferably it is a plant derived promoter, such as a GOS2 promoter or a promoter of substantially the same strength and having substantially the same expression pattern (a functionally equivalent promoter). More preferably the promoter is the GOS2 promoter from rice. Further preferably the constitutive promoter is represented by a nucleic acid sequence substantially similar to SEQ ID NO: 744, most preferably the constitutive promoter is as represented by SEQ ID NO: 744. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 744, and the nucleic acid encoding the GRP. More preferably, the expression cassette comprises the sequence represented by SEQ ID NO: 745 (pGOS2::GRP:terminator). Furthermore, one or more sequences encoding selectable markers may be present on the construct introduced into a plant.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a GRP as given herein is by introducing and expressing in a plant a nucleic acid encoding a GRP as given herein; however the effects of performing the method, i.e. enhancing yield-related traits may also be achieved using other well known techniques, including but not limited to T-DNA activation tagging, TILLING, homologous recombination. A description of these techniques is provided in the definitions section.

The invention also provides a method for the production of transgenic plants having enhanced yield-related traits relative to control plants, comprising introduction and expression in a plant of any nucleic acid encoding a GRP as defined hereinabove.

More specifically, the present invention provides a method for the production of transgenic plants having enhanced yield-related traits, preferably such as those mentioned above, which method comprises:

(i) introducing and expressing in a plant or plant cell a GRP-encoding nucleic acid or a genetic construct comprising a GRP-encoding nucleic acid; and

(ii) cultivating the plant cell under conditions promoting plant growth and development.

Cultivating the plant cell under conditions promoting plant growth and development, may or may not include regeneration and or growth to maturity.

The nucleic acid of (i) may be any of the nucleic acids capable of encoding a GRP as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The present invention clearly extends to any plant cell or plant produced by any of the methods described herein, and to all plant parts and propagules thereof. The present invention encompasses plants or parts thereof including seeds obtainable by the methods according to the present invention. The plants or parts thereof comprise a nucleic acid transgene encoding a GRP as defined above. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a GRP as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

In a preferred embodiment a growth-related polypeptide encoded by a nucleic acid according to the invention that has been introduced and expressed in a transgenic plant cell, plant part or plant when applying methods as described herein, may be present in all compartments of the plant cell including the cytosol. In a preferred embodiment, said growth-related polypeptide is located mitochondria or in chloroplast.

The methods of the invention are advantageously applicable to any plant, in particular to any plant as defined herein. Plants that are particularly useful in the methods of the invention include all plants which belong to the superfamily Viridiplantae, in particular monocotyledonous and dicotyledonous plants including fodder or forage legumes, ornamental plants, food crops, trees or shrubs.

According to an embodiment of the present invention, the plant is a crop plant. Examples of crop plants include but are not limited to chicory, carrot, cassava, trefoil, soybean, beet, sugar beet, sunflower, canola, alfalfa, rapeseed, linseed, cotton, tomato, potato and tobacco.

According to another embodiment of the present invention, the plant is a monocotyledonous plant. Examples of monocotyledonous plants include sugarcane.

According to another embodiment of the present invention, the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding a GRP. The invention furthermore relates to products derived, preferably directly derived, from a harvestable part of such a plant, such as dry pellets or powders, oil, fat and fatty acids, starch or proteins.

The present invention also encompasses use of nucleic acids encoding growth-related polypeptides as described herein and use of these polypeptides in enhancing any of the aforementioned yield-related traits in plants. For example, nucleic acids encoding a GRP described herein, or the GRP themselves, may find use in breeding programmes in which a DNA marker is identified which may be genetically linked to a GRP polypeptide-encoding gene. The nucleic acids/genes, or the GRP polypeptides themselves may be used to define a molecular marker. This DNA or protein marker may then be used in breeding programmes to select plants having enhanced yield-related traits as defined hereinabove in the methods of the invention. Furthermore, allelic variants of a GRP-encoding nucleic acid/gene may find use in marker-assisted breeding programmes. Nucleic acids encoding GRP may also be used as probes for genetically and physically mapping the genes that they are a part of, and as markers for traits linked to those genes. Such information may be useful in plant breeding in order to develop lines with desired phenotypes.

In another embodiment the invention relates to the use of a nucleic acid encoding a polypeptide as defined herein for enhancing yield-related traits in plants and preferably wherein said yield-related traits are as defined in herein relative to control plants. Preferably said use involves a step of introducing in a plant, plant part or plant cell of such nucleic acid as defined herein.

A "ZPR polypeptide" as defined herein refers to any polypeptide comprising a Leucine Zipper (ZIP) domain, and preferably comprises a Leucine Zipper (ZIP) domain that is six heptads in length.

The terms "domain", "signature" and "motif" are defined in the "definitions" section herein.

ZPR proteins have high amino acid sequence similarities to the class III homeodomain-leucin zipper transcription factors (HD-ZIP IIls). However, the small proteins, designated little zippers (ZPRs), are structurally unique from the HD-ZIP IIls and other known transcription factors in that they have only the ZIP motif, which mediates protein-protein interactions, but lack the DNA-binding basic sequence region and the C-terminal activation domain (Wenkel et al. 2007). It is therefore unlikely that the ZPR proteins are functional transcription factors belonging to the HD-ZIP III subfamily.

In particular, in an embodiment, a ZPR protein as defined herein comprise a Leucine Zipper domain, herein also named ZIP or L-ZIP motif, similar to the leucine zipper domain present in HD-ZIPIII proteins (Wenkel et al. 2007). According to another embodiment, a ZPR protein as defined herein comprises a Leucine Zipper (ZIP) domain which is six heptads in length.

A leucine zipper is a common three-dimensional structural motif in proteins. The leucine zipper is a super-secondary structure that functions as a dimerization domain, and its presence generates adhesion forces in parallel alpha helices. (Landschulz et al., 1988, Science 240: 1759-1764). Leucine zipper domains are coiled-coil domains consisting of heptad repeats with a Leu residue in the "d" position (Landschulz et al., 1988). A single leucine zipper consists of multiple leucine residues at approximately 7-residue intervals, which forms an amphipathic alpha helix with a hydrophobic region running along one side. This hydrophobic region provides an area for dimerization, allowing the motifs to "zip" together. The main feature of the leucine zipper domain is the predominance of the common amino acid leucine at the d position of the heptad repeat. Each half of a leucine zipper consists of a short alpha-helix with a leucine residue at every seventh position. The standard 3.6-residues-per-turn alpha-helix structure changes slightly to become a 3.5-residues-per-turn alpha-helix. Known also as the heptad repeat, one leucine comes in direct contact with another leucine on the other strand every second turn.

The terms "heptad" and "heptad repeat" are used herein as synonyms and both refer to a structural motif that consists of a repeating pattern of seven amino acids. A heptad repeat can be schematically represented as follows: HPPHCPC, where H represents hydrophobic residues; C represents, typically, charged residues, and P represents polar and, therefore, hydrophilic residues. The positions of the heptad repeat are commonly denoted by the lowercase letters a through g. As indicated above, leucine zipper motifs predominantly have leucine in the d position of the heptad repeat (Landschulz et al., 1988).

In another embodiment, said leucine zipper domain comprises an amino acid sequence having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% overall sequence identity to the amino acid sequence represented by SEQ ID NO: 936. In other words, in another embodiment a method is provided wherein said ZPR polypeptide comprises a conserved domain (or motif) with at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to the conserved domain starting with amino acid 59 up to and including amino acid 100 in SEQ ID NO:747.

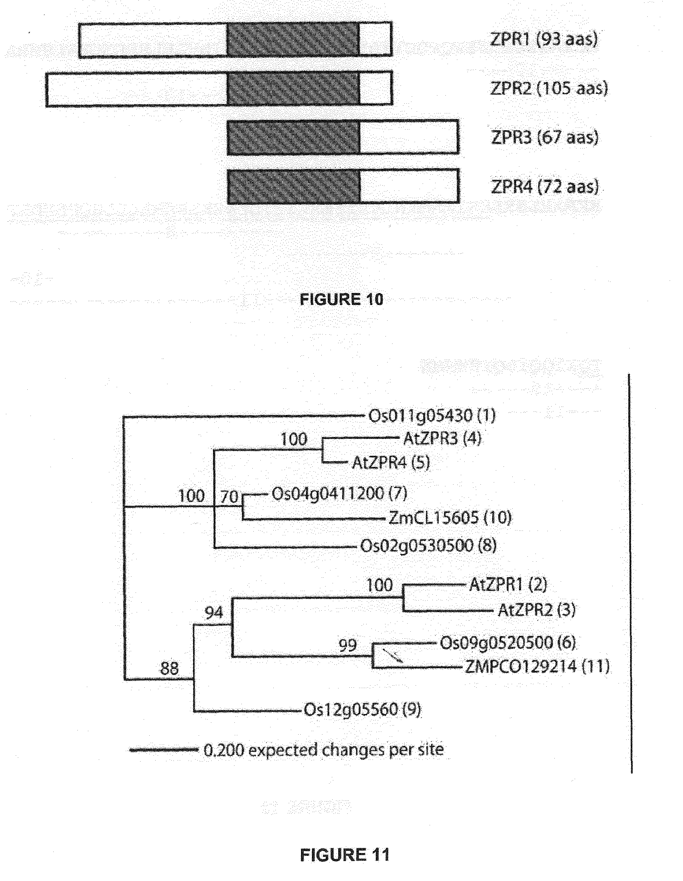

ZPR proteins are structurally similar in that they possess a leucine zipper domain as described above. However ZPR proteins vary in the placement of the leucine zipper domain within the protein. For instance, in *Arabidopsis,* phylogenetic analysis (see Figure 11 taken from Wenkel et al. 2007) revealed that ZPR proteins can be classified into two distinct groups. Class A proteins are ZPR1/2 like proteins and contain the leucine zipper domain centrally to C-terminally. This group includes ZPR1 and ZPR2. Class B proteins are ZPR3/4 like proteins and includes ZPR3 and ZPR4; they comprises the leucine zipper domain located in the N-terminal regions of ZPR3 and ZPR4 (see Figure 10 taken from Wenkel et al. 2007). Moreover, both types of ZPR proteins, ZPR1/ZPR2 types and ZPR3/ZPR4 types, are present in rice and maize as well as in Arabidopsis, indicating that the two types of ZPR genes diverged prior to the split between monocots and eudicots (Wenkel et al. 2007).

In a preferred embodiment, the ZPR polypeptide sequence which when used in the construction of a phylogenetic tree such as the one shown in Figure 11, clusters with the group ZPR1/ZPR2 types of ZPR polypeptides that comprise said leucine zipper domain in the central to C-terminal region of said ZPR polypeptides. In addition, preferably said ZPR polypeptide sequence clusters with the group of ZPR polypeptides that comprises the amino acid sequence represented by SEQ ID NO: 747 rather than with any other group.

In an embodiment, a ZPR polypeptide according to the invention comprises one or more of the following motifs:

> (i) Motif 8: II[EK]ENE[KR]LR[KE][KR]A[LS][LA]L[HR][QR]EN (SEQ ID NO: 929),
> (ii) Motif 9: [EI]M[EK][MI]KNLKLY[EML]EN[KQ][SCI] (SEQ ID NO: 930),
> (iii) Motif 10: [QKL][AD]L[LF][ST][QE][LI][QI][KQ][KQ][LI]SxPx (SEQ ID NO: 931)

Motifs 8 to 10 can be found in ZPR polypeptides belonging to both of the above-mentioned phylogenic groups.

In another embodiment, a ZPR polypeptide according to the invention further comprises one or more of the following motifs:

(i) Motif 11: K[VL][KA]K[EI]M[EK]MKNLKLY[MEL]EN[QKR][SIC]I[IL]EENE[KR]LR[KE][KRQ]A[LS][LA]L[HR][QR]EN[LKQ][AD]L[LF][STQ][QI][LI][QI][KQN][KQ][IF]S (SEQ ID NO: 932),

(ii) Motif 12: MC[HS][AG][SI]Sx[HS][LS][ES]S (SEQ ID NO: 933),

(iii) Motif 13: [VI][HS][RV]L[NK][LR]RR (SEQ ID NO:934)

Motifs 11 to 13 can be found in ZPR polypeptides belonging to the above-mentioned phylogenic group of ZPR1/ZPR2 types of ZPR polypeptides.

Motifs 8 to 13 as provided herein are based on and were derived using the MEME algorithm (Bailey and Elkan, Proceedings of the Second International Conference on Intelligent Systems for Molecular Biology, pp. 28-36, AAAI Press, Menlo Park, California, 1994). At each position within a MEME motif, the residues are shown that are present in the query set of sequences with a frequency higher than 0.2. Residues within square brackets represent alternatives.

More preferably, the ZPR polypeptide comprises in increasing order of preference, at least 2, at least 3, at least 4, at least 5, or all 6 motifs.

The term "ZPR" or "ZPR polypeptide" or "ZPR protein" as used herein also intends to include homologues as defined hereunder of "ZPR polypeptide".

Additionally or alternatively, the homologue of a ZPR protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 747, provided that the homologous protein comprises any one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in a ZPR polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 929 to SEQ ID NO: 934 (Motifs 8 to 13).

Homeodomain-leucine zipper (HD-ZIPIII) proteins activate transcription of the ZPR genes. The ZPR proteins then form heterodimers with the HD-ZIPIII proteins (Wenkel et al. 2007). Tools and techniques for measuring or determining activation of ZPR transcription are well known in the art. Further details are provided in Example 26.

In addition, ZPR polypeptides, when expressed in rice according to the methods of the present invention as outlined in Examples 27 and 28, give plants having increased yield related traits, in particular increased yield, and more particularly increased seed yield. In an embodiment, said increased seed yield relative to control plants comprises any one or more of increased fill rate, increased total seed weight, increased harvest index and increased number of filled seeds.

The present invention is illustrated by transforming plants with the nucleic acid sequence represented by SEQ ID NO: 746, encoding the polypeptide sequence of SEQ ID NO: 747. However, performance of the invention is not restricted to these sequences; the methods of the invention may advantageously be performed using any ZPR-encoding nucleic acid or ZPR polypeptide as defined herein.

Examples of nucleic acids encoding ZPR polypeptides are given in Table J of the Examples section herein. Such nucleic acids are useful in performing the methods of the invention. The amino acid sequences given in Table J of the Examples section includes example sequences of orthologues and paralogues of the ZPR polypeptide represented by SEQ ID NO: 747, the terms "orthologues" and "paralogues" being as defined herein. Further orthologues and paralogues may readily be identified by performing a so-called reciprocal blast search as described in the definitions section; where the query sequence is SEQ ID NO: 746 or SEQ ID NO: 747, the second BLAST (back-BLAST) would be against tomato sequences.

The invention also provides hitherto unknown ZPR-encoding nucleic acids and ZPR polypeptides useful for conferring enhanced yield-related traits as defined herein in plants relative to control plants.

According to one further embodiment of the present invention, there is therefore provided an isolated nucleic acid molecule selected from:

(i) a nucleic acid represented by any one of SEQ ID NO: 748, 790, 792, 862, 864, 870, 878, 902, 912, and 918;

(ii) the complement of a nucleic acid represented by any one of SEQ ID NO: 748, 790, 792, 862, 864, 870, 878, 902, 912, and 918;

(iii) a nucleic acid encoding a ZPR polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented any one of SEQ ID NO: 749, 791, 793, 863, 865, 871, 879, 903, 913, and 919; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 13 as given in SEQ ID NO: 929 to SEQ ID NO: 934, and further preferably conferring enhanced yield-related traits relative to control plants;

(iv) a nucleic acid encoding a ZPR polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented any one of SEQ ID NO: 878, 902, 912, and 918; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 10 as given in SEQ ID NO: 929 to SEQ ID NO: 931, and further preferably conferring enhanced yield-related traits relative to control plants.

(v) a nucleic acid molecule which hybridizes with a nucleic acid molecule of (i) to (iv) under high stringency hybridization conditions and preferably confers enhanced yield-related traits relative to control plants.

According to another further embodiment of the present invention, there is also provided an isolated polypeptide selected from:

(i) an amino acid sequence represented by any one of SEQ ID NO: 749, 791, 793, 863, 865, 871, 879, 903, 913, and 919;

(ii) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 749, 791, 793, 863, 865, and 871; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 13 given in SEQ ID NO: 929 to SEQ ID NO: 934, and further preferably conferring enhanced yield-related traits relative to control plants;

(iii) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 879, 903, 913, and 919; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 10 given in SEQ ID NO: 929 to SEQ ID NO: 931 and further preferably conferring enhanced yield-related traits relative to control plants;

(iv) derivatives of any of the amino acid sequences given in (i) to (iii) above.

Nucleic acid variants may also be useful in practicing the methods of the invention. Examples of such variants include nucleic acids encoding homologues and derivatives of any one of the amino acid sequences given in Table J of the Examples section, the terms "homologue" and "derivative" being as defined herein. Also useful in the methods of the invention are nucleic acids encoding homologues and derivatives of orthologues or paralogues of any one of the amino acid sequences given in Table J of the Examples section. Homologues and derivatives useful in the methods of the present invention have substantially the same biological and functional activity as the unmodified protein from which they are derived. Further variants useful in practicing the methods of the invention are variants in which codon usage is optimised or in which miRNA target sites are removed. Further nucleic acid variants useful in practicing the methods of the invention include portions of nucleic acids encoding ZPR polypeptides, nucleic acids hybridising to nucleic acids encoding ZPR polypeptides, splice variants of nucleic acids encoding ZPR polypeptides, allelic variants of nucleic acids encoding ZPR polypeptides and variants of nucleic acids encoding ZPR polypeptides obtained by gene shuffling. The terms hybridising sequence, splice variant, allelic variant and gene shuffling are as described herein.

Nucleic acids encoding ZPR polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table J of the Examples section, or a portion of a nucleic acid encoding an orthologue, paralogue or homologue of any of the amino acid sequences given in Table J of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a ZPR polypeptide as defined herein, and have substantially the same biological activity as the amino acid sequences given in Table J of the Examples section. Preferably, the portion is a portion of any one of the nucleic acids given in Table J of the Examples section, or is a portion of a nucleic acid encoding an orthologue or paralogue of any one of the amino acid sequences given in Table J of the Examples section. Preferably the portion is at least 150, 175, 200, 225, 250, 275, 300, 325, 350, 375, 400, 425, 450, 475, 500, 525, 530, or 537 consecutive nucleotides in length, the consecutive nucleotides being of any one of the nucleic acid sequences given in Table J of the Examples section, or of a nucleic acid encoding an orthologue or paralogue of any one of the amino acid sequences given in Table J of the Examples section. Most preferably the portion is a portion of the nucleic acid of SEQ ID NO: 746.

Preferably, the portion encodes a fragment of an amino acid sequence which has one or more of the following characteristics:

- when used in the construction of a phylogenetic tree, such as the one depicted in Figure 11, clusters with the group of ZPR1/2 like polypeptides comprising the amino acid sequence represented by SEQ ID NO: 747 rather than with any other group;
- comprises any one or more of the motifs 8 to 13 as provided herein,
- has HD-ZIPIII-suppressing activity, and
- has at least 25% sequence identity to SEQ ID NO: 747.

Another nucleic acid variant useful in the methods of the invention is a nucleic acid capable of hybridising, under reduced stringency conditions, preferably under stringent conditions, with a nucleic acid encoding a POI polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table J of the Examples section, or comprising introducing and expressing in a plant a nucleic acid capable of hybridising to a nucleic acid encoding an orthologue, paralogue or homologue of any of the nucleic acid sequences given in Table J of the Examples section.

Hybridising sequences useful in the methods of the invention encode a ZPR polypeptide as defined herein, having substantially the same biological activity as the amino acid sequences given in Table J of the Examples section. Preferably, the hybridising sequence is capable of hybridising to the complement of any one of the nucleic acids given in Table J of the Examples section, or to a portion of any of these sequences, a portion being as defined above, or the hybridising sequence is capable of hybridising to the complement of a nucleic acid encoding an orthologue or paralogue of any one of the amino acid sequences given in Table J of the Examples section. Most preferably, the hybridising sequence is capable of hybridising to the complement of a nucleic acid as represented by SEQ ID NO: 746 or to a portion thereof.

Preferably, the hybridising sequence encodes a polypeptide with an amino acid sequence which has one or more of the following characteristics:

- when used in the construction of a phylogenetic tree, such as the one depicted in Figure 11, clusters with the group of ZPR1/2 like polypeptides comprising the amino acid sequence represented by SEQ ID NO: 747 rather than with any other group;
- comprises any one or more of the motifs 8 to 13 as provided herein,
- has HD-ZIPIII-suppressing activity, and
- has at least 25% sequence identity to SEQ ID NO: 747.

Another nucleic acid variant useful in the methods of the invention is a splice variant encoding a ZPRI polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table J of the Examples section, or a splice variant of a nucleic acid encoding an orthologue, paralogue or homologue of any of the amino acid sequences given in Table J of the Examples section.

Preferred splice variants are splice variants of a nucleic acid represented by SEQ ID NO: 746, or a splice variant of a nucleic acid encoding an orthologue or paralogue of SEQ ID NO: 747. Preferably, the amino acid sequence encoded by the splice variant, has one or more of the following characteristics:

- when used in the construction of a phylogenetic tree, such as the one depicted in Figure 11, clusters with the group of ZPR1/2 like polypeptides comprising the amino acid sequence represented by SEQ ID NO: 747 rather than with any other group;
- comprises any one or more of the motifs 8 to 13 as provided herein,
- has HD-ZIPIII-suppressing activity, and
- has at least 25% sequence identity to SEQ ID NO: 747.

Another nucleic acid variant useful in performing the methods of the invention is an allelic variant of a nucleic acid encoding a ZPR polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table J of the Examples section, or comprising introducing and expressing in a plant an allelic variant of a nucleic acid encoding an orthologue, paralogue or homologue of any of the amino acid sequences given in Table J of the Examples section.

The polypeptides encoded by allelic variants useful in the methods of the present invention have substantially the same biological activity as the ZPR polypeptide of SEQ ID NO: 747 and any of the amino acids depicted in Table J of the Examples section. Allelic variants exist in nature, and encompassed within the methods of the present invention is the use of these natural alleles. Preferably, the allelic variant is an allelic variant of SEQ ID NO: 746 or an allelic variant of a nucleic acid encoding an orthologue or paralogue of SEQ ID NO: 747. Preferably, the amino acid sequence encoded by the allelic variant, has one or more of the following characteristics:

- when used in the construction of a phylogenetic tree, such as the one depicted in Figure 11, clusters with the group of ZPR1/2 like polypeptides comprising the amino acid sequence represented by SEQ ID NO: 747 rather than with any other group;
- comprises any one or more of the motifs 8 to 13 as provided herein,
- has HD-ZIPIII-suppressing activity, and
- has at least 25% sequence identity to SEQ ID NO: 747.

Gene shuffling or directed evolution may also be used to generate variants of nucleic acids encoding ZPR polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table J of the Examples section, or comprising introducing and expressing in a plant a variant of a nucleic acid encoding an orthologue, paralogue or homologue of any of the amino acid sequences given in Table J of the Examples section, which variant nucleic acid is obtained by gene shuffling.

Preferably, the amino acid sequence encoded by the variant nucleic acid obtained by gene shuffling, has one or more of the following characteristics:

- when used in the construction of a phylogenetic tree, such as the one depicted in Figure 11, clusters with the group of ZPR1/2 like polypeptides comprising the amino acid sequence represented by SEQ ID NO: 747 rather than with any other group;
- comprises any one or more of the motifs 8 to 13 as provided herein,
- has HD-ZIPIII-suppressing activity, and
- has at least 25% sequence identity to SEQ ID NO: 747.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding ZPR polypeptides may be derived from any natural or artificial source. The nucleic acid may be modified from its native form in composition and/or genomic environment through deliberate human manipulation.

Preferably the ZPR polypeptide-encoding nucleic acid is from a plant, further preferably from a dicotyledonous plant, more preferably from the family *Solanaceae.* In a particularly preferred embodiment, said ZPR polypeptide-encoding nucleic acid is from the genus *Solanum,* most preferably from *Solanum lycopersicum* (synonym: *Lycopersicum esculentum).*

In another preferred embodiment, said ZPR polypeptide-encoding nucleic acid is from a plant, further preferably from a dicotyledonous plant, more preferably from the family *Salicaceae,* preferably from the genus *Populus,* most preferably from *Populus trichocarpa.*

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include (i) aboveground parts and preferably aboveground harvestable parts and/or (ii) parts below ground and preferably harvestable below ground. In particular, such harvestable parts are seeds, and performance of the methods of the invention results in plants having increased seed yield relative to the seed yield of control plants.

The present invention provides a method for increasing yield-related traits, particularly yield, more particularly seed yield of plants, relative to control plants, which method comprises modulating expression in a plant of a nucleic acid encoding a ZPR polypeptide as defined herein.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding a ZPR polypeptide as defined herein.

The methods of the present invention may be performed under non-stress conditions or under stress conditions as defined above.

In an embodiment, the methods of the present invention are performed under non-stress conditions.

For instance, performance of the methods of the invention gives plants grown under non-stress conditions or under mild drought conditions increased yield relative to control plants grown under comparable conditions. Therefore, according to the present invention, there is provided a method for increasing yield, preferably seed yield, in plants grown under non-stress conditions or under mild drought conditions, which method comprises modulating expression in a plant of a nucleic acid encoding a ZPR polypeptide.

In another embodiment, the methods of the present invention are performed under stress conditions.

In an example, the methods of the present invention are performed under stress conditions such as drought. Performance of the methods of the invention gives plants that are grown under drought conditions increased yield-related traits as provided herein relative to control plants grown under comparable conditions. Therefore, according to the present invention, there is provided a method for increasing yield-related traits in plants grown under stress conditions, and in particular grown under drought conditions, which method comprises modulating expression in a plant of a nucleic acid encoding a ZPR polypeptide as defined herein.

In another example, performance of the methods of the invention gives plants grown under conditions of nutrient deficiency, particularly under conditions of nitrogen deficiency, increased yield-related traits as provided herein relative to control plants grown under comparable conditions. Therefore, according to the present invention, there is provided a method for increasing yield-related traits as provided herein in plants grown under conditions of nutrient deficiency, which method comprises modulating expression in a plant of a nucleic acid encoding a ZPR polypeptide.

In yet another example, performance of the methods of the invention gives plants grown under conditions of salt stress, increased yield-related traits as provided herein relative to control plants grown under comparable conditions. Therefore, according to the present invention, there is provided a method for increasing yield-related traits as provided herein in plants grown under conditions of salt stress, which method comprises modulating expression in a plant of a nucleic acid encoding a ZPR polypeptide.

The invention also provides genetic constructs and vectors to facilitate introduction and/or expression in plants of nucleic acids encoding ZPR polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a ZPR polypeptide as defined above;
(b) one or more control sequences capable of driving expression of the nucleic acid sequence of (a); and optionally
(c) a transcription termination sequence.

Preferably, the nucleic acid encoding a ZPR polypeptide is as defined above. The term "control sequence" and "termination sequence" are as defined herein.

The invention furthermore provides plants transformed with a construct as described above. In particular, the invention provides plants transformed with a construct as described above, which plants have increased yield-related traits as described herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a ubiquitous constitutive promoter of medium strength. See the "Definitions" section herein for definitions of the various promoter types.

It should be clear that the applicability of the present invention is not restricted to the ZPR polypeptide-encoding nucleic acid represented by SEQ ID NO: 746, nor is the applicability of the invention restricted to expression of a ZPR polypeptide-encoding nucleic acid when driven by a constitutive promoter.

In a preferred embodiment, the constitutive promoter is preferably a medium strength promoter. Preferably it is a plant derived promoter, more preferably it is a GOS2 promoter or a promoter of substantially the same strength and having substantially the same expression pattern (a functionally equivalent promoter). Even more preferably the promoter is the GOS2 promoter from rice. Further preferably the constitutive promoter is represented by a nucleic acid sequence substantially similar to SEQ ID NO: 928, most preferably the constitutive promoter is as represented by SEQ ID NO: 928. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 928, operably linked to the nucleic acid encoding the ZPR polypeptide. In on example, said expression cassette comprises the sequence that is at least 95% and for instance 96, 97, 98, 99 or 100% identical to the sequence represented by SEQ ID NO: 935 (pGOS2::ZPR::t-zein sequence). In another example, said expression cassette comprises a sequence that is at least 95% and for instance 96, 97, 98, 99 or 100% identical to the sequence represented by SEQ ID NO:937 (pGOS2::ZPR::terminator). Furthermore, one or more sequences encoding selectable markers may be present on the construct introduced into a plant.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a ZPR polypeptide is by introducing and expressing in a plant a nucleic acid encoding a ZPR polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The invention also provides a method for the production of transgenic plants having enhanced yield-related traits as provided herein relative to control plants, comprising introduction and expression in a plant of any nucleic acid encoding a ZPR polypeptide as defined hereinabove.

More specifically, the present invention provides a method for the production of transgenic plants having enhanced yield-related traits relative to control plants, preferably increased yield relative to control plants, and more preferably increased seed yield relative to control plants, which method comprises:

> (i) introducing and expressing in a plant or plant cell a ZPR polypeptide-encoding nucleic acid or a genetic construct comprising a ZPR polypeptide-encoding nucleic acid; and
> (ii) cultivating the plant cell under conditions promoting plant growth and development.

Cultivating the plant cell under conditions promoting plant growth and development, may or may not include regeneration and or growth to maturity.

The nucleic acid of (i) may be any of the nucleic acids capable of encoding a ZPR polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The present invention clearly extends to any plant cell or plant produced by any of the methods described herein, and to all plant parts and propagules thereof. The present invention encompasses plants or parts thereof (including seeds) obtainable by the methods according to the present invention. The plants or parts thereof comprise a nucleic acid transgene encoding a ZPR polypeptide as defined above. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a ZPR polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant, in particular to any plant as defined herein. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. Food crops may include for instance crop plants or cereals such as those provided herein.

According to an embodiment of the present invention, the plant is a crop plant. Examples of crop plants include but are not limited to chicory, carrot, cassava, trefoil, soybean, beet, sugar beet, sunflower, canola, alfalfa, rapeseed, linseed, cotton, tomato, potato and tobacco.

According to another embodiment of the present invention, the plant is a monocotyledonous plant. Examples of monocotyledonous plants include sugarcane.

According to another embodiment of the present invention, the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding a ZPR polypeptide. The invention furthermore relates to products derived, preferably directly derived, from a harvestable part of such a plant, such as dry pellets or powders, oil, fat and fatty acids, starch or proteins.

The present invention also encompasses use of nucleic acids encoding ZPRI polypeptides as described herein and use of these ZPR polypeptides in enhancing any of the aforementioned yield-related traits in plants. For example, nucleic acids encoding ZPR polypeptide described herein, or the ZPR polypeptides themselves, may find use in breeding programs in which a DNA marker is identified which may be genetically linked to a ZPR polypeptide-encoding gene. The nucleic acids/genes, or the ZPR polypeptides themselves may be used to define a molecular marker. This DNA or protein marker may then be used in breeding programs to select plants having enhanced yield-related traits as defined hereinabove in the methods of the invention. Furthermore, allelic variants of a ZPR polypeptide-encoding nucleic acid/gene may find use in marker-assisted breeding programs. Nucleic acids encoding ZPR polypeptides may also be used as probes for genetically and physically mapping the genes that they are a part of, and as markers for traits linked to those genes. Such information may be useful in plant breeding in order to develop lines with desired phenotypes.

**Items**

The invention is further characterised by one or more of the following items:

1. A method for enhancing yield-related traits in plants relative to control plants, comprising modulating expression in a plant of a nucleic acid encoding an OsRSZ33 RRM polypeptide, wherein said OsRSZ33 RRM polypeptide comprises from N- to C-terminus an RRM domain, two Zinc Knuckles.

2. Method according to item 1, wherein said OsRSZ33 RRM polypeptide comprises one or more of the motifs 1 to 3 (SEQ ID NO: 241 to 246).

3. Method according to item 1 or 2, wherein said modulated expression is effected by introducing and expressing in a plant a nucleic acid encoding an OsRSZ33 RRM polypeptide.

4. Method according to any one of items 1 to 3, wherein said nucleic acid encoding an OsRSZ33 RRM polypeptide encodes any one of the proteins listed in Table A or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A.

6. Method according to any preceding item, wherein said enhanced yield-related traits comprise increased yield and/or early vigour, relative to control plants.

7. Method according to any one of items 1 to 6, wherein said enhanced yield-related traits are obtained under non-stress conditions.

8. Method according to any one of items 3 to 7, wherein said nucleic acid is operably linked to a constitutive promoter, preferably to a GOS2 promoter, most preferably to a GOS2 promoter from rice.

9. Method according to any one of items 1 to 8, wherein said nucleic acid encoding an OsRSZ33 RRM polypeptide is of plant origin, preferably from a dicotyledonous plant, further preferably from the family Poaceae, more preferably from the genus Oryza, most preferably from Oryza sativa.

10. Plant or part thereof, including seeds, obtainable by a method according to any one of items 1 to 9, wherein said plant or part thereof comprises a recombinant nucleic acid encoding an OsRSZ33 RRM polypeptide.

11. Construct comprising:

(i) nucleic acid encoding an OsRSZ33 RRM polypeptide as defined in items 1 or 2;
(ii) one or more control sequences capable of driving expression of the nucleic acid sequence of (a); and optionally
(iii) a transcription termination sequence.

12. Construct according to item 11, wherein one of said control sequences is a constitutive promoter, preferably a GOS2 promoter, most preferably a GOS2 promoter from rice.

13. Use of a construct according to item 11 or 12 in a method for making plants having increased yield, particularly increased biomass and/or increased seed yield relative to control plants.

14. Plant, plant part or plant cell transformed with a construct according to item 11 or 12.

15. Method for the production of a transgenic plant having increased yield, particularly increased biomass and/or increased seed yield relative to control plants, comprising:

(i) introducing and expressing in a plant a nucleic acid encoding an OsRSZ33 RRM polypeptide as defined in item 1 or 2; and
(ii) cultivating the plant cell under conditions promoting plant growth and development.

16. Transgenic plant having increased yield, particularly increased biomass and/or increased seed yield, relative to control plants, resulting from modulated expression of a nucleic acid encoding an OsRSZ33 RRM polypeptide as defined in item 1 or 2, or a transgenic plant cell derived from said transgenic plant.

17. Transgenic plant according to item 10, 14 or 16, or a transgenic plant cell derived thereof, wherein said plant is a crop plant, such as beet or sugarbeet or alfalfa, or a monocot such as sugarcane, or a cereal, such as rice, maize, wheat, barley, millet, rye, triticale, sorghum emmer, spelt, secale, einkorn, teff, milo and oats.

18. Harvestable parts of a plant according to item 17, wherein said harvestable parts are preferably shoot biomass and/or seeds.

19. Products derived from a plant according to item 17 and/or from harvestable parts of a plant according to item 18.

20. Use of a nucleic acid encoding an OsRSZ33 RRM polypeptide in increasing yield, particularly in increasing seed yield and/or shoot biomass in plants, relative to control plants.

21. A method for enhancing yield-related traits in plants relative to control plants, comprising modulating expression in a plant of a nucleic acid encoding a growth-related protein (GRP) having at least 25% sequence identity to the amino acid sequence represented by SEQ ID NO: 251.

22. Method according to item 21, wherein said modulated expression is effected by introducing and expressing said nucleic acid in a plant.

23. Method according to item 21 or 22, wherein said enhanced yield-related trait comprises increased yield relative to control plants.

24. Method according to item 23, wherein said increased yield comprises increased seed yield relative to control plants, and preferably said increased seed yield is selected from any one or more of :

> (i) increased harvest index;
> (ii) increased total seed weight;
> (iii) increased number of filled seeds; and
> (iv) increased thousand kernel weight.

25. Method according to any of items 21 to 24, wherein said enhanced yield-related trait comprises increased biomass, and preferably increased aboveground biomass, relative to control plants.

26. Method according to any one of items 21 to 25, wherein said enhanced yield-related traits are obtained under non-stress conditions.

27. Method according to any one of items 21 to 25, wherein said enhanced yield-related traits are obtained under conditions of drought stress, salt stress or nitrogen deficiency.

28. Method according to any of items 21 to 27, wherein said growth-related protein has a motif having at least 70% sequence identity to motif 7: [NHPS][SC][CR][RK]K[NK] [VST][PD][GD][TAV][ST]F[VL][SE]DL[RK]DH[IM][HD]EFI[NH]AS[ASM]DEH[MRK][TH]C F[TK][KN]T[IL][KQ][KR]MF[GD]MS[KM][TAV]V[AT] (SEQ ID NO: 742).

29. Method according to any one of items 21 to 28, wherein said nucleic acid encoding said polypeptide is of plant origin, preferably from a monocotyledonous plant, further preferably from the family Poaceae, more preferably from the genus Oryza, most preferably from Oryza sativa.

30. Method according to any one of items 21 to 29, wherein said nucleic acid encodes any one of the polypeptides listed in Table F or is a portion of such a nucleic acid, or a nucleic acid capable of hybridising with such a nucleic acid.

31. Method according to any one of items 21 to 30, wherein said nucleic acid encodes an orthologue or paralogue of any of the polypeptides given in Table F.

32. Method according to any one of items 21 to 31, wherein said nucleic acid encodes the polypeptide as represented by SEQ ID NO: 251

33. Method according to any one of items 21 to 32, wherein said nucleic acid is operably linked to a constitutive promoter, preferably to a medium strength constitutive promoter, preferably to a plant promoter, more preferably to a GOS2 promoter, most preferably to a GOS2 promoter from rice.

34. Plant, plant part thereof, including seeds, or plant cell, obtainable by a method according to any one of items 21 to 33, wherein said plant, plant part or plant cell comprises a recombinant nucleic acid encoding a polypeptide as defined in any of items 21 and 28 to 32.

35. Construct comprising:

> (i) a nucleic acid encoding a polypeptide as defined in any of items 21 and 28 to 32,
> (ii) one or more control sequences capable of driving expression of the nucleic acid sequence of (i); and optionally
> (iii) a transcription termination sequence.

36. Construct according to item 35 wherein one of said control sequences is a constitutive promoter, preferably a medium strength constitutive promoter, preferably a plant promoter, more preferably a GOS2 promoter, most preferably a GOS2 promoter from rice.

37. Use of a construct according to item 35 or 36 in a method for making plants having enhanced yield-related traits.

38. Plant, plant part or plant cell transformed with a construct according to item 35 or 36.

39. Method for the production of a transgenic plant having enhanced yield-related traits relative to control plants, comprising:

(i) introducing and expressing in a plant cell or plant a nucleic acid encoding a polypeptide as defined in any of items 21 and 28 to 32; and
(ii) cultivating said plant cell or plant under conditions promoting plant growth and development.

40. Method according to item 39, wherein said enhanced yield-related traits are as defined in any of items 23 to 27.

41. Transgenic plant having enhanced yield-related traits relative to control plants, preferably as defined in any of items 23 to 25 resulting from modulated expression of a nucleic acid encoding a polypeptide as defined in any of items 21 and 28 to 32 or a transgenic plant cell derived from said transgenic plant.

42. Transgenic plant according to item 34, 38 or 41, or a transgenic plant cell derived therefrom, wherein said plant is a crop plant, such as beet, sugarbeet or alfalfa; or a monocotyledonous plant such as sugarcane; or a cereal, such as rice, maize, wheat, barley, millet, rye, triticale, sorghum, emmer, spelt, secale, einkorn, teff, milo or oats.

43. Harvestable parts of a plant according to item 41 or 42 wherein said harvestable parts are preferably shoot biomass and/or seeds.

44. Products derived from a plant according to item 41 or 42 and/or from harvestable parts of a plant according to item 43.

45. Use of a nucleic acid encoding a polypeptide as defined in any of items 21 and 28 to 32 for enhancing yield-related traits in plants, and preferably wherein said yield-related traits are as defined in any of items 23 to 27, relative to control plants.

46. An isolated nucleic acid selected from:

(i) a nucleic acid represented by any one of SEQ ID NO: 266, 286, 316, 374, 376, 380, 424, 456, 458, 460, 462, 648, 650, 670, and 736;
(ii) the complement of a nucleic acid represented by any one of SEQ ID NO: 266, 286, 316, 374, 376, 380, 424, 456, 458, 460, 462, 648, 650, 670, and 736;
(iii) a nucleic acid encoding the polypeptide as represented by any one of SEQ ID NO: 267, 287, 317, 375, 377, 381, 425, 457, 459, 461, 463, 649, 651, 671 and 737, preferably as a result of the degeneracy of the genetic code, said isolated nucleic acid can be derived from a polypeptide sequence as represented by any one of SEQ ID NO: 267, 287, 317, 375, 377, 381, 425, 457, 459, 461, 463, 649, 651, 671 and 737 and further preferably confers enhanced yield-related traits relative to control plants;
(iv) a nucleic acid having, in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences represented by any one of SEQ ID NO: 266, 286, 316, 374, 376, 380, 424, 456, 458, 460, 462, 648, 650, 670, and 736; and any of the other nucleic acid sequences in Table F and further preferably conferring enhanced yield-related traits relative to control plants;
(v) a nucleic acid molecule which hybridizes with a nucleic acid molecule of (i) to (iv) under stringent hybridization conditions and preferably confers enhanced yield-related traits relative to control plants;
(vi) a nucleic acid encoding a polypeptide having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 267, 287, 317, 375, 377, 381, 425, 457, 549, 461, 463, 649, 651, 671 and 737 and any of the other amino acid sequences in Table F and preferably conferring enhanced yield-related traits relative to control plants.

47. An isolated polypeptide selected from:

(i) an amino acid sequence represented by any one of SEQ ID NO: 267, 287, 317, 375, 377, 381, 425, 457, 459, 461, 463, 649, 651, 671 and 737;

(ii) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 267, 287, 317, 375, 377, 381, 425, 457, 459, 461, 463, 649, 651, 671 and 737, and any of the other amino acid sequences in Table F and preferably conferring enhanced yield-related traits relative to control plants, and

(iii) derivatives of any of the amino acid sequences given in (i) or (ii) above.

48. Use of an isolated nucleic acid according to item 46, or of an isolated polypeptide according to item 47 for enhancing yield-related traits in plants, and preferably wherein said yield-related traits are as defined in any of items 23 to 27, relative to control plants.

49. A method for enhancing yield-related traits in plants relative to control plants, comprising modulating expression in a plant of a nucleic acid encoding a ZPR polypeptide, wherein said ZPR polypeptide comprises a Leucine Zipper (ZIP) domain that is six heptads in length.

50. Method according item 49, wherein said ZPR polypeptide comprises one or more of the following motifs:

(i) Motif 8: II[EK]ENE[KR]LR[KE][KR]A[LS][LA]L[HR][QR]EN (SEQ ID NO: 929),
(ii) Motif 9: [EI]M[EK][MI]KNLKLY[EML]EN[KQ][SCI] (SEQ ID NO: 930),
(iii) Motif 10: [QKL][AD]L[LF][ST][QE][LI][QI][KQ][KQ][LI]SxPx (SEQ ID NO: 931)

51. Method according to item 49 or 50, wherein said Leucine Zipper domain is located in the central to C-terminal region of said ZPR polypeptide.

52. Method according to any of items 49 to 51, wherein said ZPR polypeptide further comprises one or more of the following motifs:

(i) Motif 11: K[VL][KA]K[EI]M[EK]MKNLKLY[MEL]EN[QKR][SIC]I[IL]EENE[KR]LR[KE][KRQ]A[LS][LA]L[HR][QR]EN[LKQ][AD]L[LF][STQ][QI][LI][QI][KQN][KQ][IF]S (SEQ ID NO: 932),
(ii) Motif 12: MC[HS][AG][SI]Sx[HS][LS][ES]S (SEQ ID NO: 933),
(iii) Motif 13: [VI][HS][RV]L[NK][LR]RR (SEQ ID NO: 934)

53. Method according to any one of items 49 to 52, wherein said nucleic acid encoding a ZPR polypeptide is of plant origin, and preferably from a dicotyledonous plant.

54. Method according to any one of items 49 to 53, wherein said nucleic acid encoding a ZPR polypeptide is from the family Solanaceae, more preferably from the genus Solanum, most preferably from Solanum lycopersicum.

55. Method according to any one of items 49 to 54, wherein said nucleic acid encoding a ZPR polypeptide encodes any one of the polypeptides listed in Table J or is a portion of such a nucleic acid, or a nucleic acid capable of hybridising with such a nucleic acid.

56. Method according to any one of items 49 to 55, wherein said nucleic acid sequence encoding a ZPR polypeptide encodes an orthologue or paralogue of any of the polypeptides given in Table J.

57. Method according to any one of items 49 to 56, wherein said nucleic acid encodes the polypeptide represented by SEQ ID NO: 747 or a homologue thereof.

58. Method according to any of items 49 to 57, wherein said modulated expression is effected by introducing and expressing in a plant said nucleic acid encoding said ZPR polypeptide.

59. Method according to any one of items 49 to 58, wherein said enhanced yield-related traits comprise increased yield relative to control plants, and preferably increased seed yield relative to control plants.

60. Method according to any one of items 49 to 59, wherein said enhanced yield-related traits are obtained under non-stress conditions.

61. Method according to any one of items 49 to 59, wherein said enhanced yield-related traits are obtained under conditions of drought stress, salt stress or nitrogen deficiency.

62. Method according to any one of items 49 to 61, wherein said nucleic acid is operably linked to a constitutive promoter, preferably to a medium strength constitutive promoter, preferably to a plant promoter, more preferably to a GOS2 promoter, most preferably to a GOS2 promoter from rice.

63. Plant, plant part thereof, including seeds, or plant cell, obtainable by a method according to any one of items 49 to 62, wherein said plant, plant part or plant cell comprises a recombinant nucleic acid encoding a ZPR polypeptide as defined in any of items 49 to 57.

64. Construct comprising:

   (i) nucleic acid encoding a ZPR as defined in any of items 49 to 57;
   (ii) one or more control sequences capable of driving expression of the nucleic acid sequence of (i); and optionally
   (iii) a transcription termination sequence.

65. Construct according to item 64, wherein one of said control sequences is a constitutive promoter, preferably a medium strength constitutive promoter, more preferably a plant promoter

66. Construct according to item 64 or 65, wherein one of said control sequences is a GOS2 promoter, preferably a GOS2 promoter from rice.

67. Use of a construct according to any of items 64 to 66 in a method for making plants having enhanced yield-related traits, preferably increased yield relative to control plants, and more preferably increased seed yield relative to control plants.

68. Plant, plant part or plant cell transformed with a construct according to any of items 64 to 66.

69. Method for the production of a transgenic plant having enhanced yield-related traits relative to control plants, preferably increased yield relative to control plants, and more preferably increased seed yield relative to control plants, comprising:

   (i) introducing and expressing in a plant cell or plant a nucleic acid encoding a ZPR polypeptide as defined in any of items 49 to 57; or a genetic construct comprising a ZPR polypeptide-encoding nucleic acid as defined in any of items 64 to 66; and
   (ii) cultivating the plant cell under conditions promoting plant growth and development.

70. Transgenic plant having enhanced yield-related traits relative to control plants, preferably increased yield relative to control plants, and more preferably increased seed yield relative to control plants, resulting from modulated expression of a nucleic acid encoding a ZPR polypeptide as defined in any of items 49 to 57 or a transgenic plant cell derived from said transgenic plant.

71.Transgenic plant according to item 63, 68 or 70, or a transgenic plant cell derived therefrom, wherein said plant is a crop plant, such as beet, sugarbeet or alfalfa; or a monocotyledonous plant such as sugarcane; or a cereal, such as rice, maize, wheat, barley, millet, rye, triticale, sorghum, emmer, spelt, secale, einkorn, teff, milo or oats.

72. Harvestable parts of a plant according to any of items 63, 68, 70 and 71, wherein said harvestable parts are seeds.

73. Products derived from a plant according to any of items 63, 68, 70 and 71, and/or from harvestable parts of a plant according to item 72.

74. An isolated nucleic molecule selected from:

   (i) a nucleic acid represented by any one of SEQ ID NO: 748, 790, 792, 862, 864, 870, 878, 902, 912, and 918;
   (ii) the complement of a nucleic acid represented by any one of SEQ ID NO: 748, 790, 792, 862, 864, 870, 878, 902, 912, and 918;

(iii) a nucleic acid encoding a ZPR polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented any one of SEQ ID NO: 749, 791, 793, 863, 865, and 871; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 1 to 6 as given in SEQ ID NO: 929 to SEQ ID NO: 934, and further preferably conferring enhanced yield-related traits relative to control plants;

(iv) a nucleic acid encoding a ZPR polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented any one of SEQ ID NO: 878, 902, 912, and 918; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 10 as given in SEQ ID NO: 929 to SEQ ID NO: 931, and further preferably conferring enhanced yield-related traits relative to control plants.

(v) a nucleic acid molecule which hybridizes with a nucleic acid molecule of (i) to (iv) under high stringency hybridization conditions and preferably confers enhanced yield-related traits relative to control plants.

75. An isolated polypeptide selected from:

(i) an amino acid sequence represented by any one of SEQ ID NO: 749, 791, 793, 863, 865, 871, 879, 903, 913, and 919;

(ii) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 749, 791, 793, 863, 865, and 871; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 13 given in SEQ ID NO: 929 to SEQ ID NO: 934, and further preferably conferring enhanced yield-related traits relative to control plants;

(iii) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 879, 903, 913, and 919; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 10 given in SEQ ID NO: 929 to SEQ ID NO: 931, and further preferably conferring enhanced yield-related traits relative to control plants;

(iv) derivatives of any of the amino acid sequences given in (i) to (iii) above.

76. Use of a nucleic acid as defined in any of items 49 to 57 and 74 or of a nucleic acid encoding a ZPR polypeptide as defined in any of items 49 to 57 and 75 for enhancing yield-related traits in plants relative to control plants, preferably for increasing yield in plants relative to control plants, and more preferably for increasing seed yield in plants relative to control plants.

**Description of figures**

The present invention will now be described with reference to the following figures in which:

**Fig. 1** (a) represents a graphical overview of an OsRSZ33 RRM protein (Isshiki et al., 2006); (b) represents the domain structure of SEQ ID NO: 2 with the RRM domain in bold and the two Zinc knuckle domains in italics. The conserved motifs 1 to 3 are underlined and numbered. The position of the signature sequences are indicated by a dashed line.

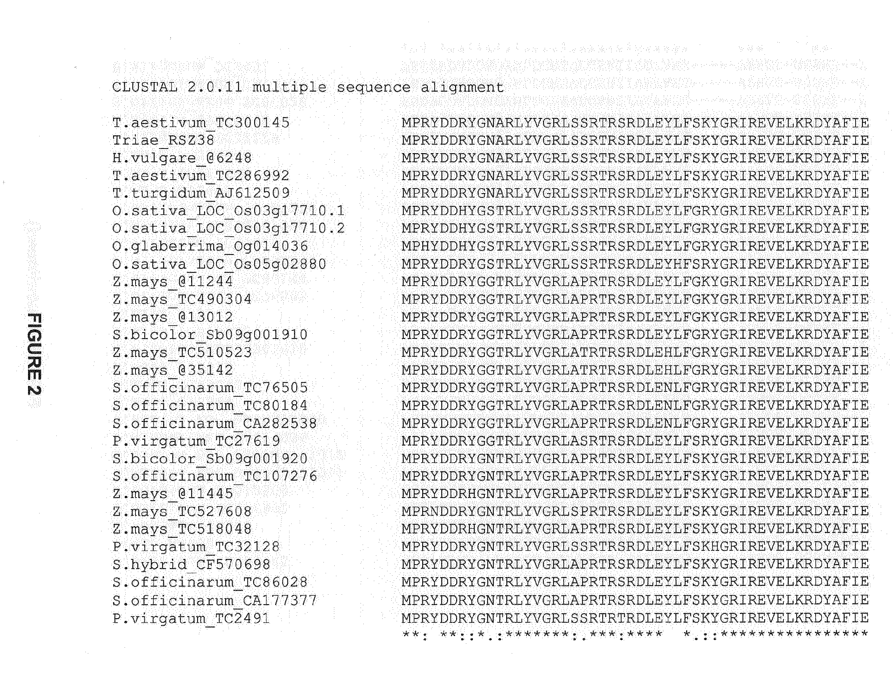

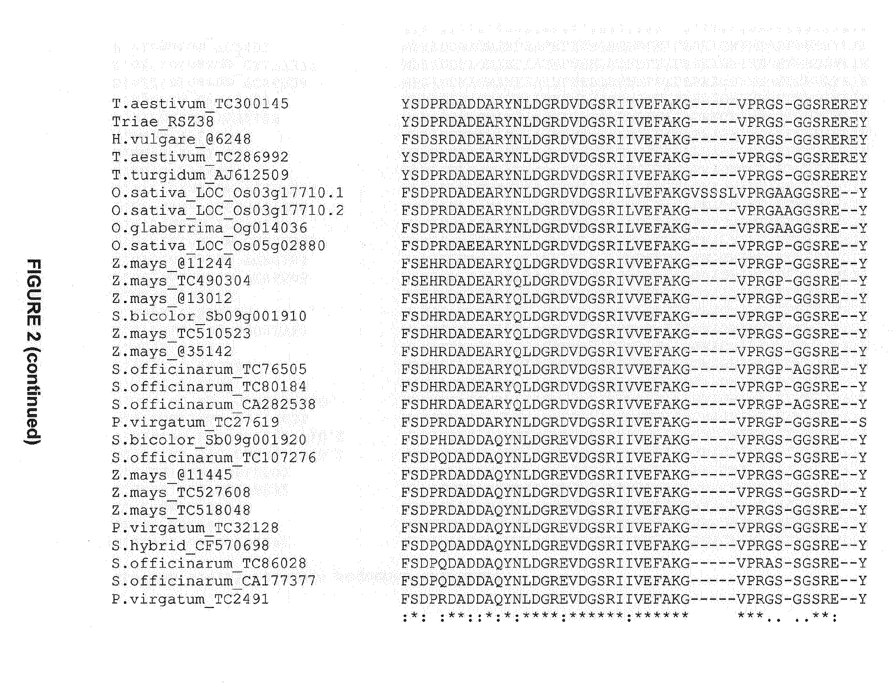

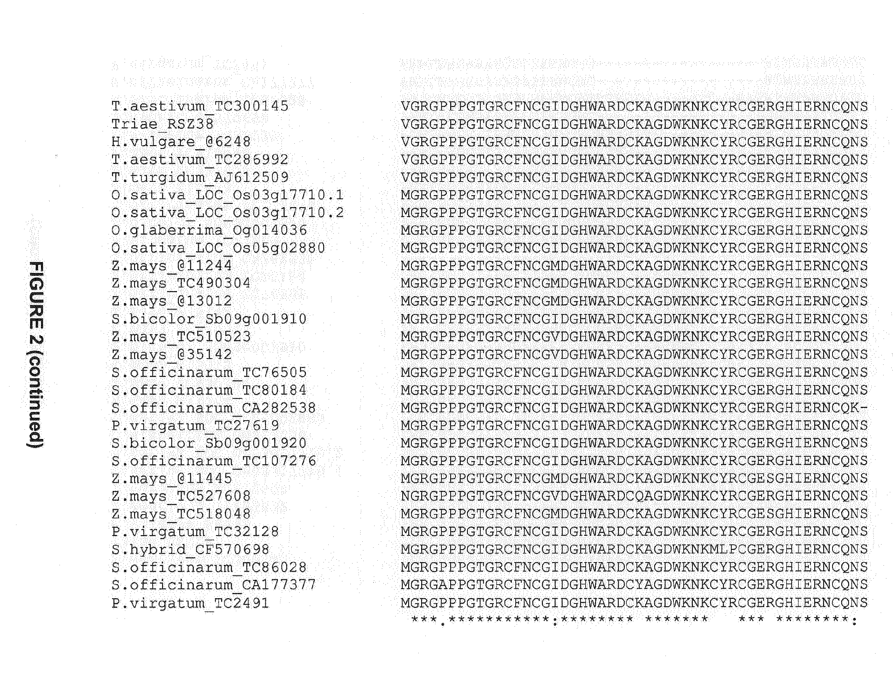

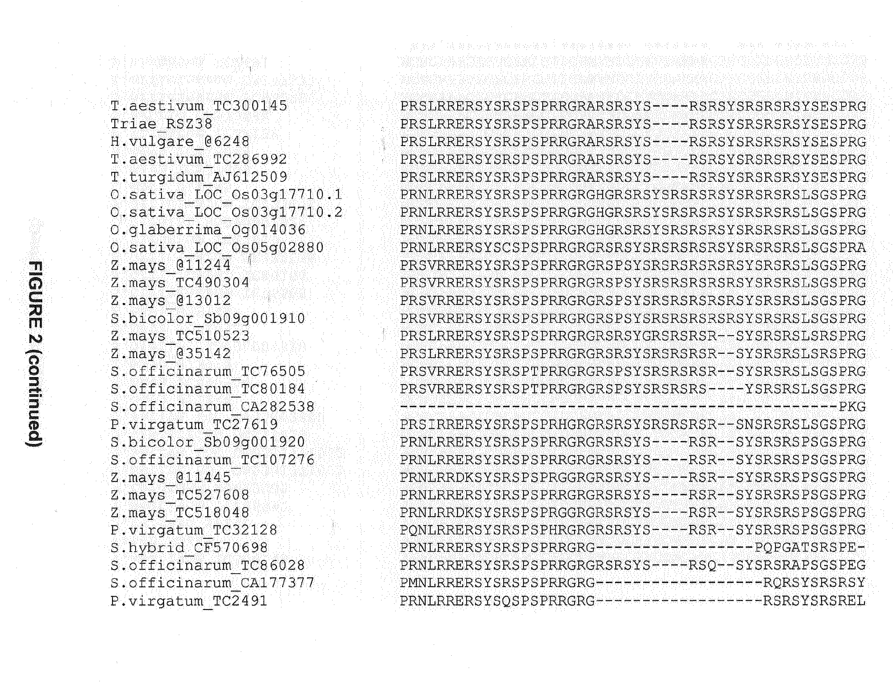

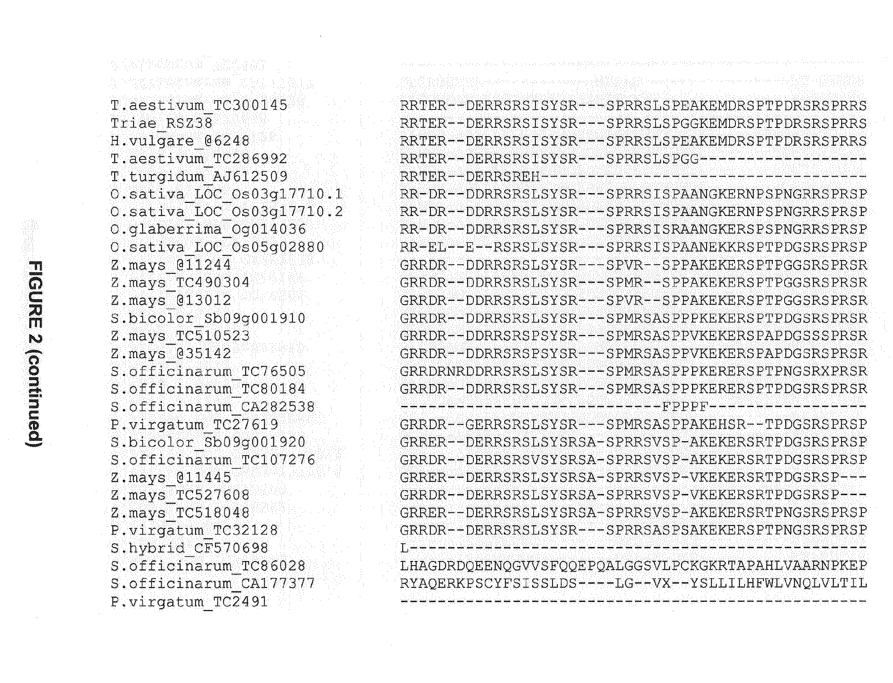

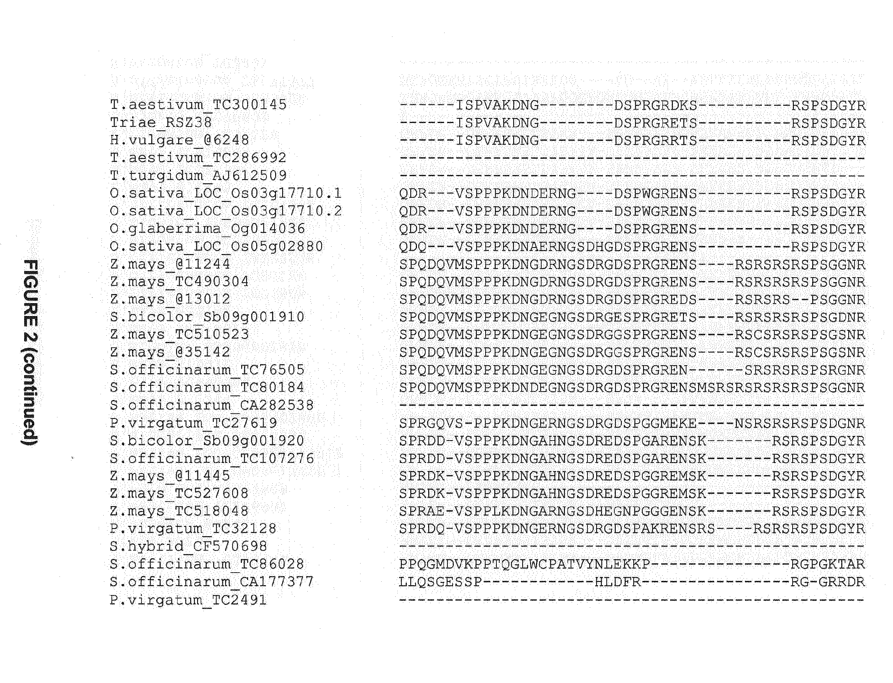

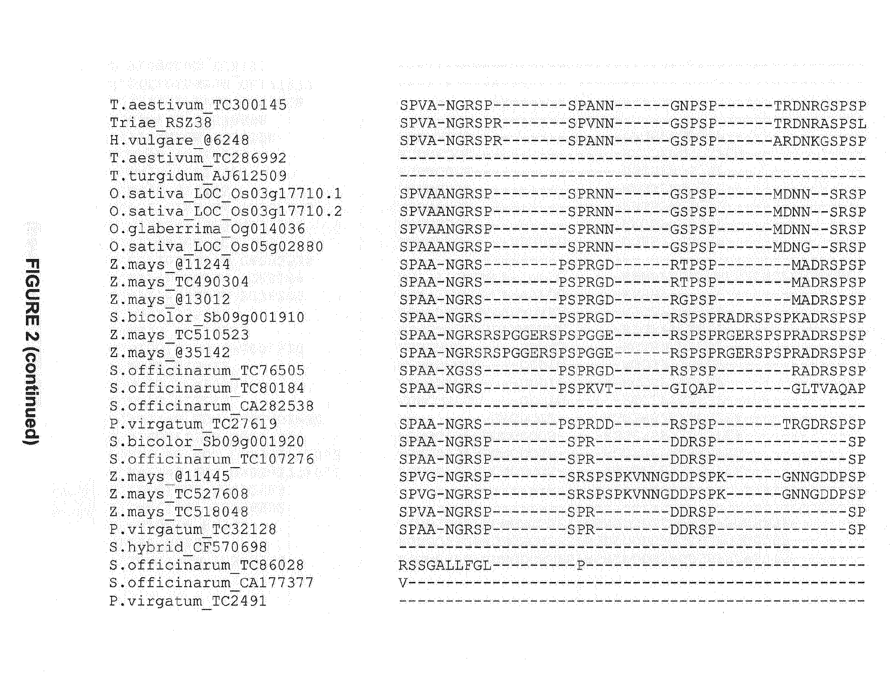

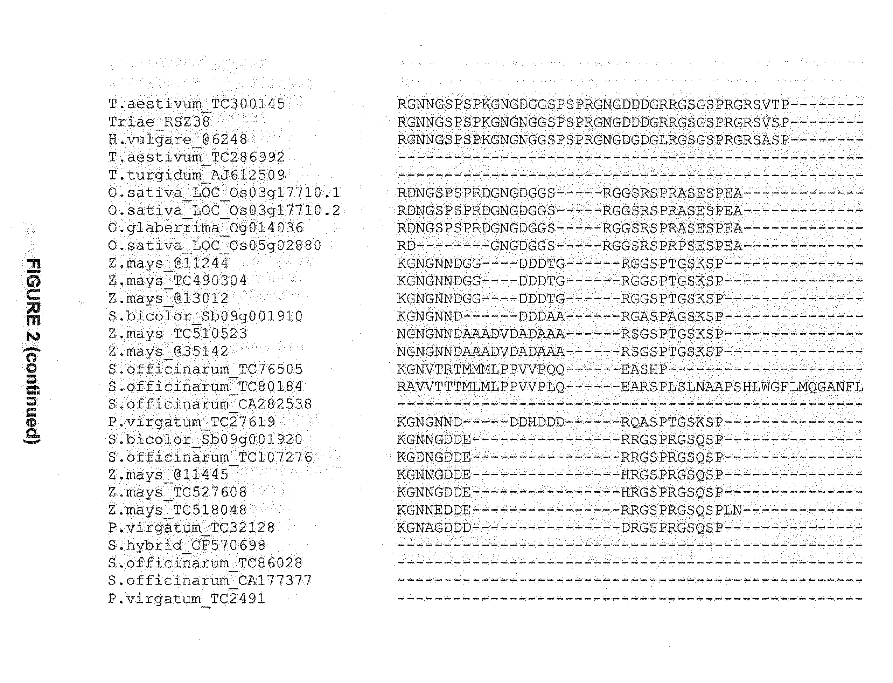

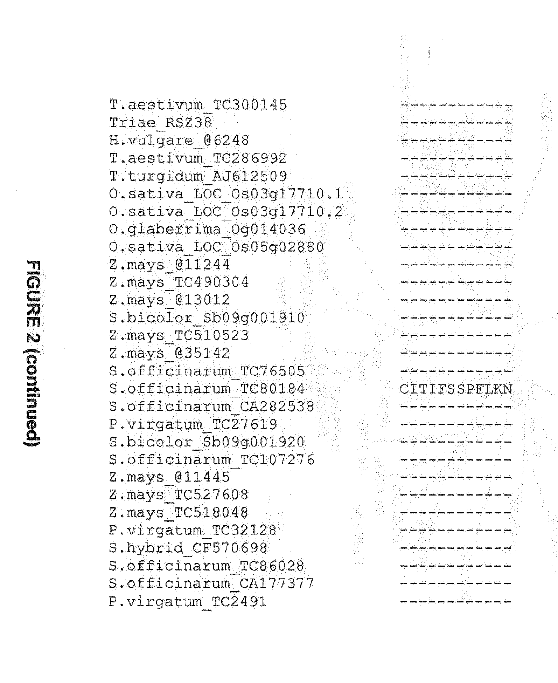

**Fig. 2** represents a multiple alignment of various OsRSZ33 RRM polypeptides. The asterisks indicate identical amino acids among the various protein sequences, colons represent highly conserved amino acid substitutions, and the dots represent less conserved amino acid substitution; on other positions there is no sequence conservation. These alignments can be used for defining further motifs, when using conserved amino acids.

**Fig. 3** shows phylogenetic tree of OsRSZ33 RRM polypeptides and other SR proteins (Isshiki et al., 2006, supplemental figure 2).

The amino acid sequences of rice, Arabidopsis, and human SR proteins are aligned with ClustalW software. The accession numbers of the SR proteins shown are as follows: At SRp34/SR1, 022315; At SRp30, NP_172386; At SRp34a, NP_567235; At SRp34b, NP_190512; At RSp31, P92964; At RSp40, P92965; At RSp41, P92966; At RSZp21, NP_973901; At RSZp22, NP_194886; At RSZp22a, NP_180035; At RSZ32, NP_190918 At RSZ33, NP_973620; At SC35, NP_201225; At SCL33, NP_564685; At SCL30, NP_567021; At SCL30a, NP_187966; At SCL28, NP_197382; Hs ASF/SF2, Q07955; Hs SC35, Q01130 and Hs 9G8, Q16629.

**Fig. 4** represents the binary vector used for increased expression in Oryza sativa of an OsRSZ33 RRM-encoding nucleic acid under the control of a rice GOS2 promoter (pGOS2)

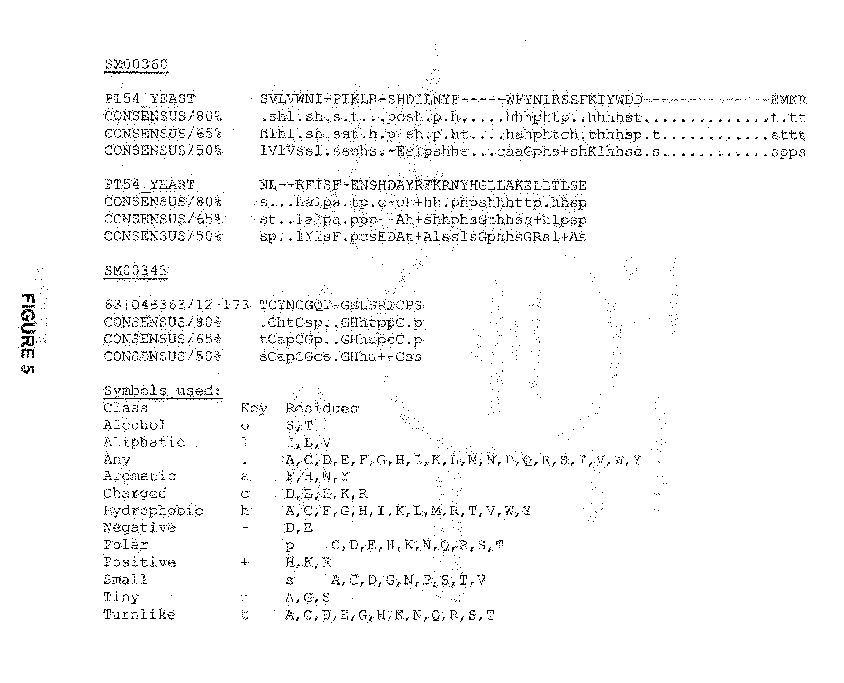

**Fig. 5** details the domain consensus sequence for SM00360 and SM00340 as given in SMART.

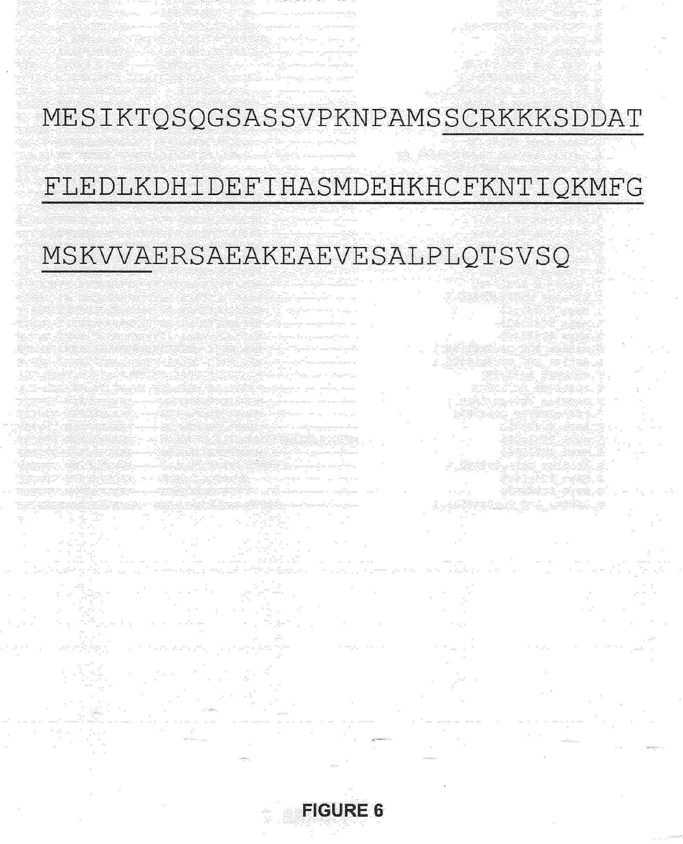

**Fig. 6** represents SEQ ID NO: 251 with indication of motif 7 (underlined).

**Fig. 7** represents a multiple alignment of a representative list of growth-related polypeptides according to the invention. The asterisks indicate identical amino acids among the various protein sequences, colons represent highly conserved amino acid substitutions, and the dots represent less conserved amino acid substitution; on other positions there is no sequence conservation. These alignments can be used for defining further motifs, when using conserved amino acids.

**Fig. 8** shows the MATGAT table for a representative list of growth-related polypeptides according to the invention (Example 14)

**Fig. 9** represents the binary vector used for increased expression in *Oryza sativa* of a nucleic acid encoding a polypeptide according to the invention under the control of a rice GOS2 promoter (pGOS2).

**Fig. 10** corresponds to Figure 12(A) provided in Wenkel et al. (2007) and shows the placement of the leucine zipper domain (grey zone) in each of the four Arabidopsis ZPR proteins.

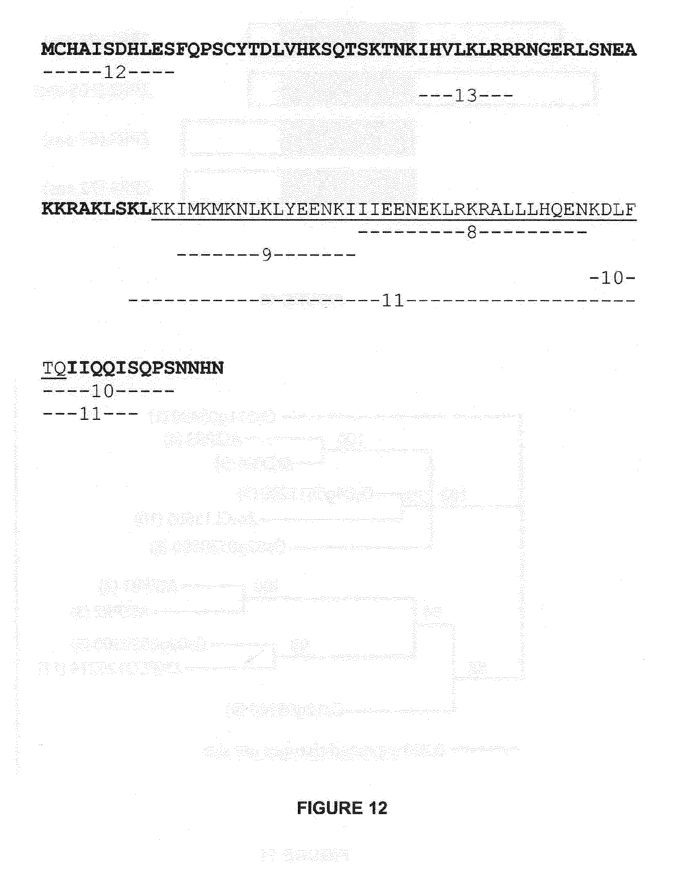

**Fig. 11** corresponds to Figure 12(D) provided in Wenkel et al. (2007) and shows a phylogenetic tree of all four Arabidopsis, five rice, and two maize ZPR proteins. Alignment of the ZPR proteins was performed using the ClustalW algorithm. Bayesian phylogenetic analysis was conducted on the aligned protein data set using MrBayes version 3.1.2 (Huelsenbeck and Ronquist, 2001 Bioinformatics 17: 754-755.). Default settings were used, and the program was allowed to run for 100,000 generations. Trees were summarized after discarding the first 25,000 generations. The number above each branch corresponds to the posterior probability for that node.

**Fig. 12** represents the domain structure of SEQ ID NO: 747 with conserved motifs 8 to 13 and with indication of the Leucine Zipper domain.

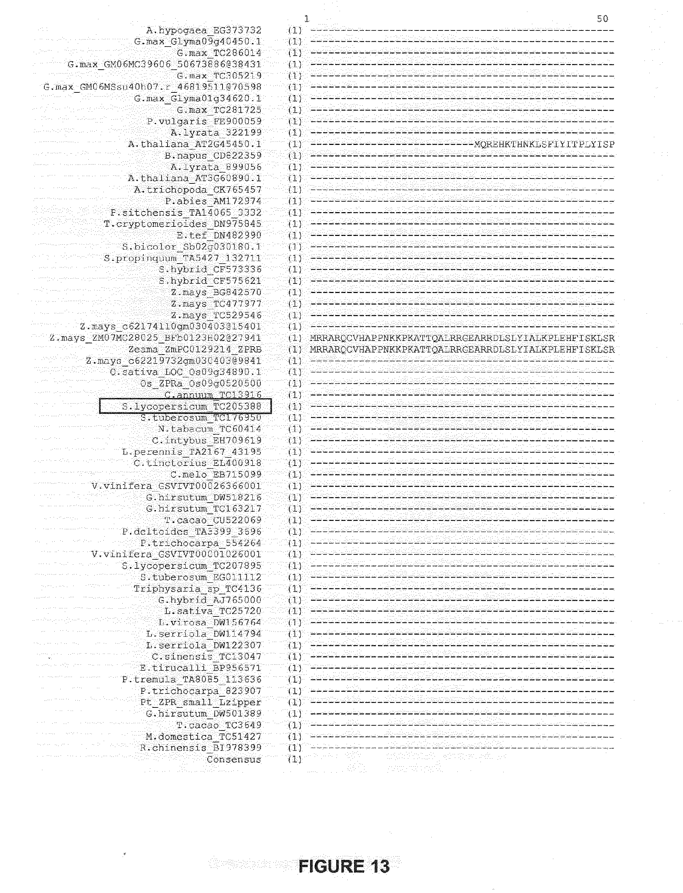

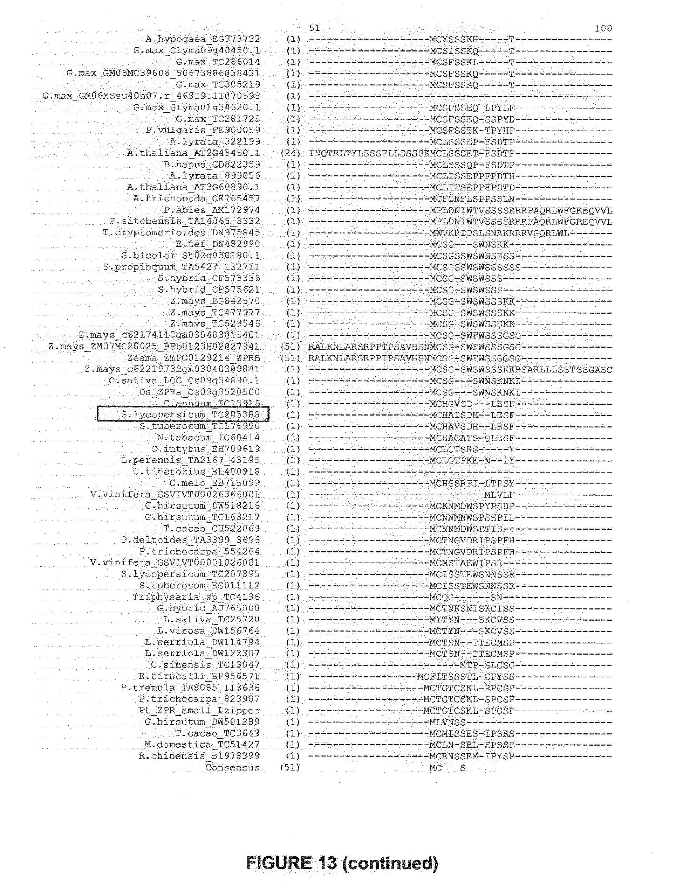

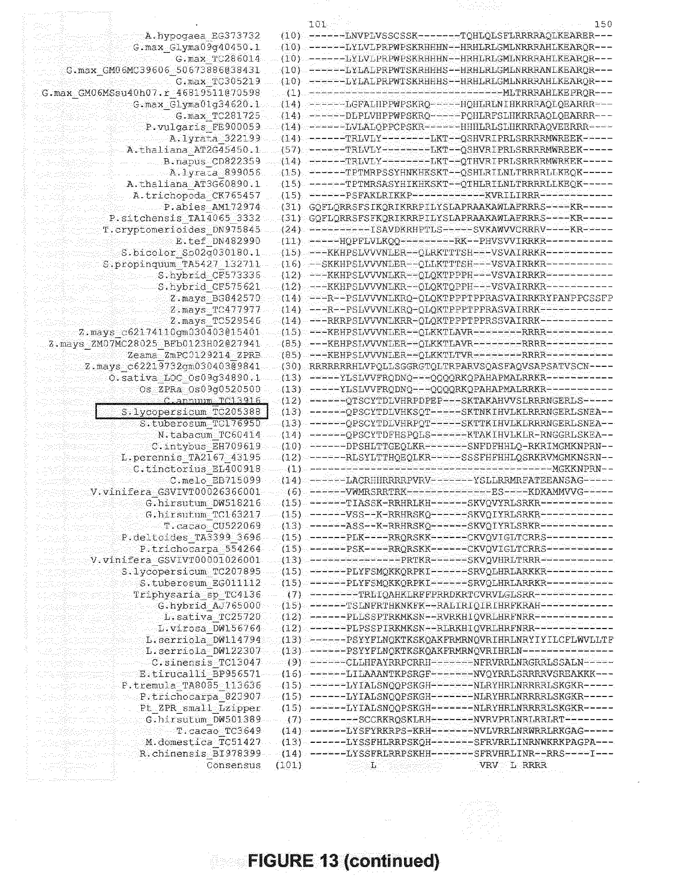

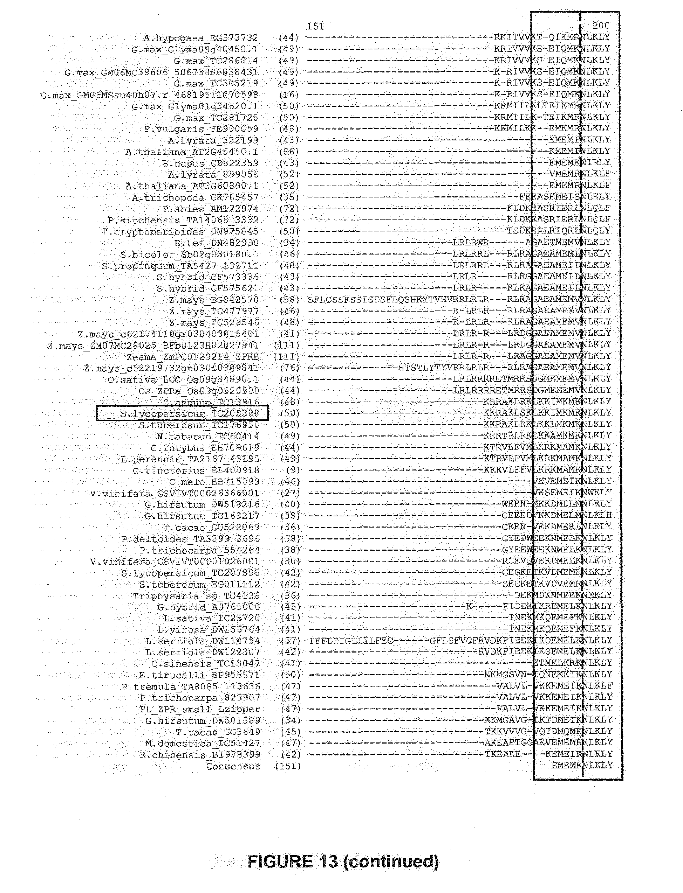

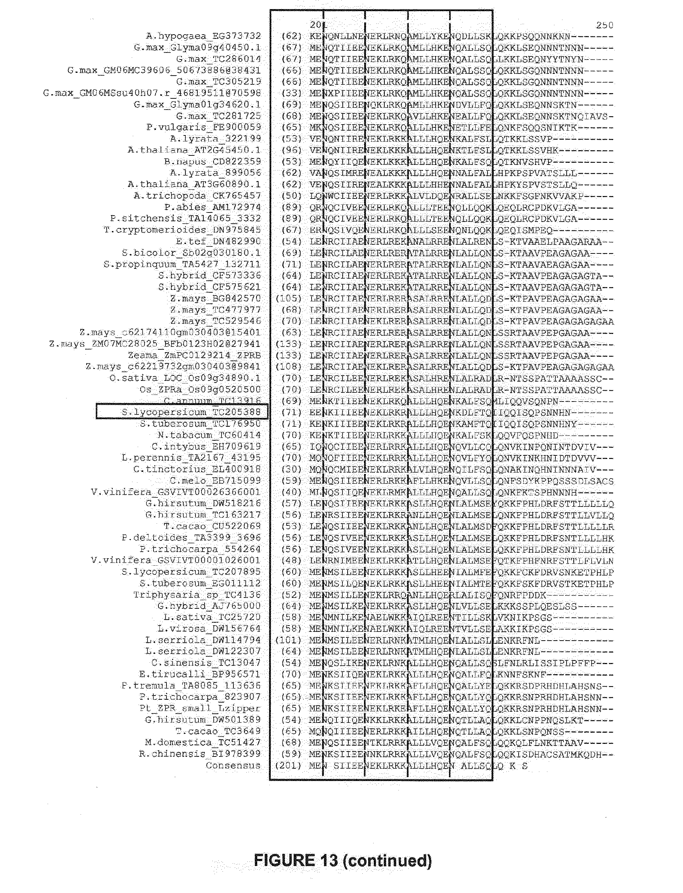

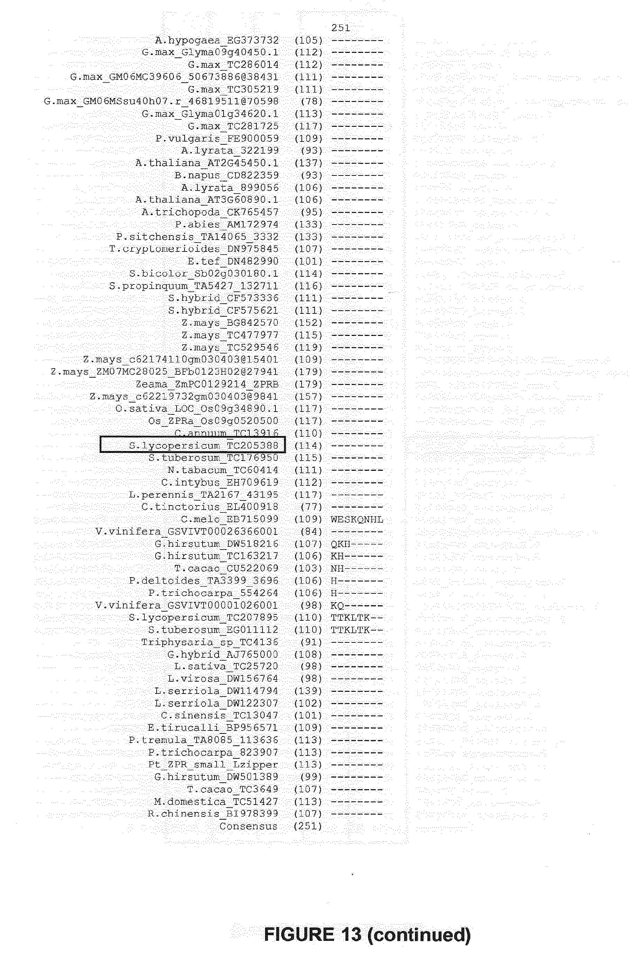

**Fig. 13** represents a multiple alignment of a representative number of ZPR polypeptides. In particular, the represented ZPR polypeptides are characterized in that the Leucine Zipper domain is located in the central to C-terminal region of said ZPR polypeptide. The ZPR polypeptide represented by SEQ ID NO:747 is indicated with a box. The Leucine Zipper domain is located between the amino acids at position 59 and 100 in this protein and is indicated with a box. The six different heptads are separated with black dotted lines. These alignments can be used for defining further motifs, when using conserved amino acids.

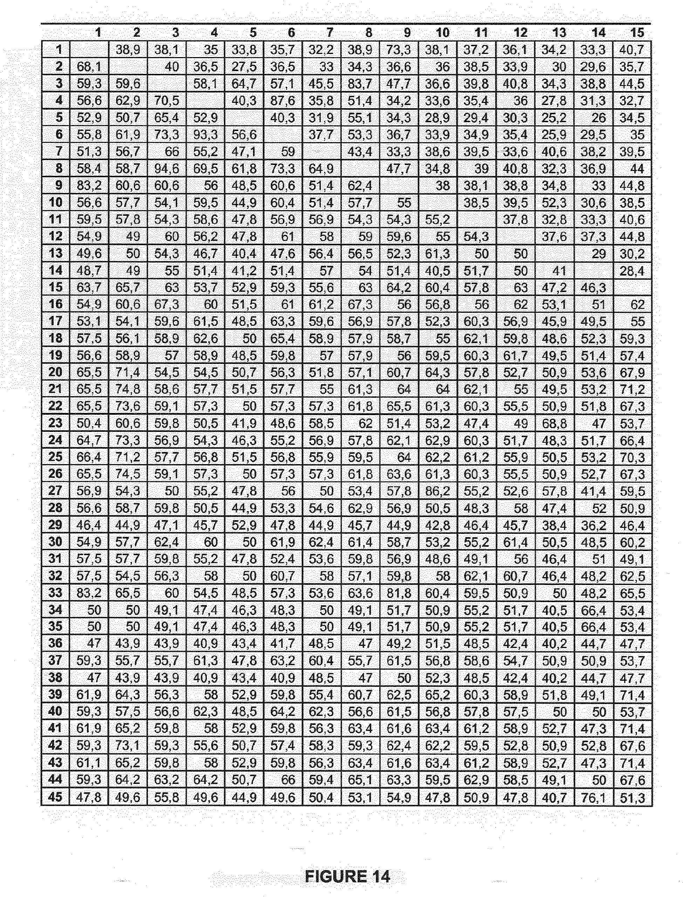

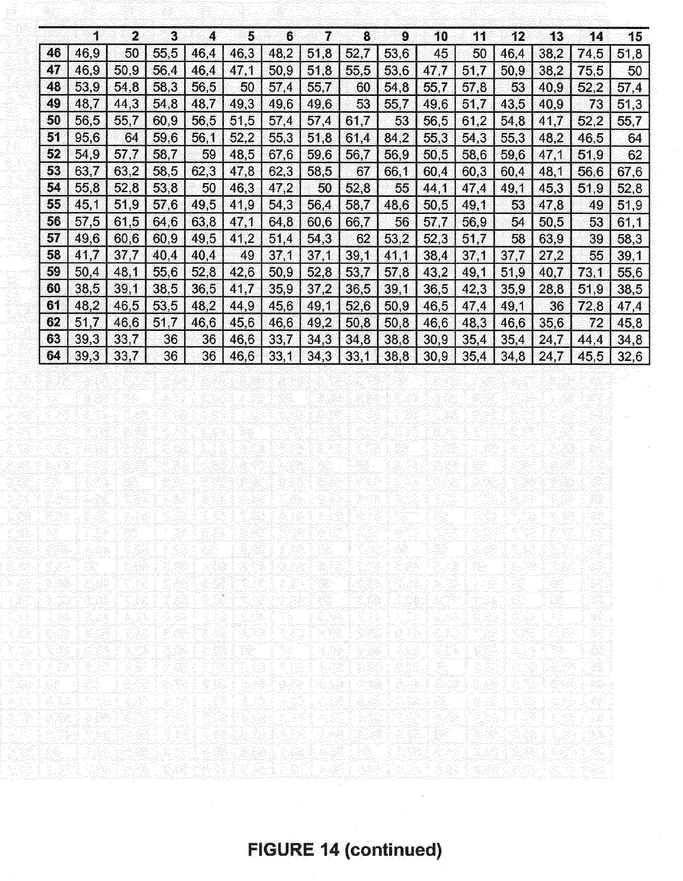

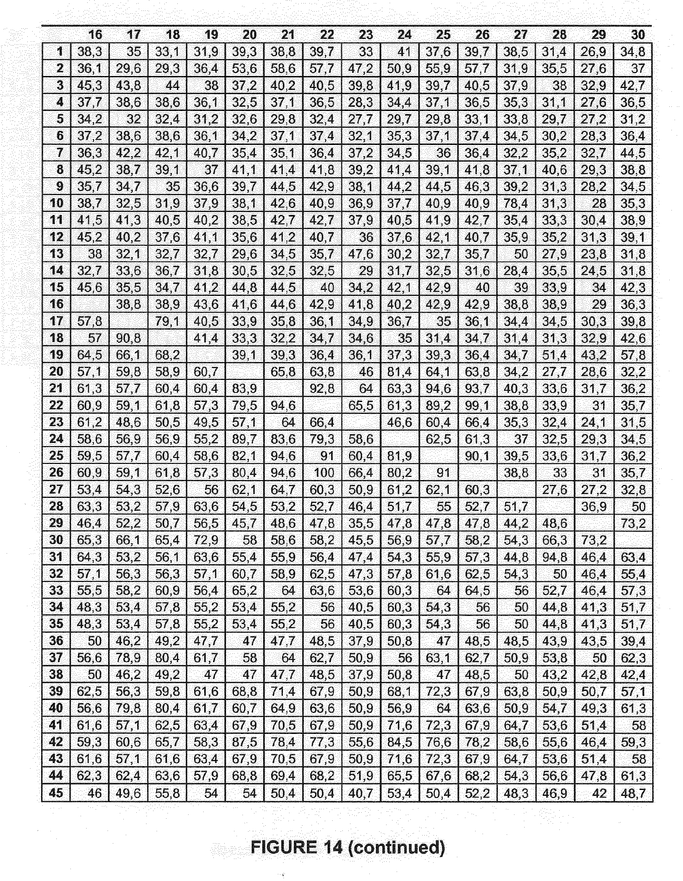

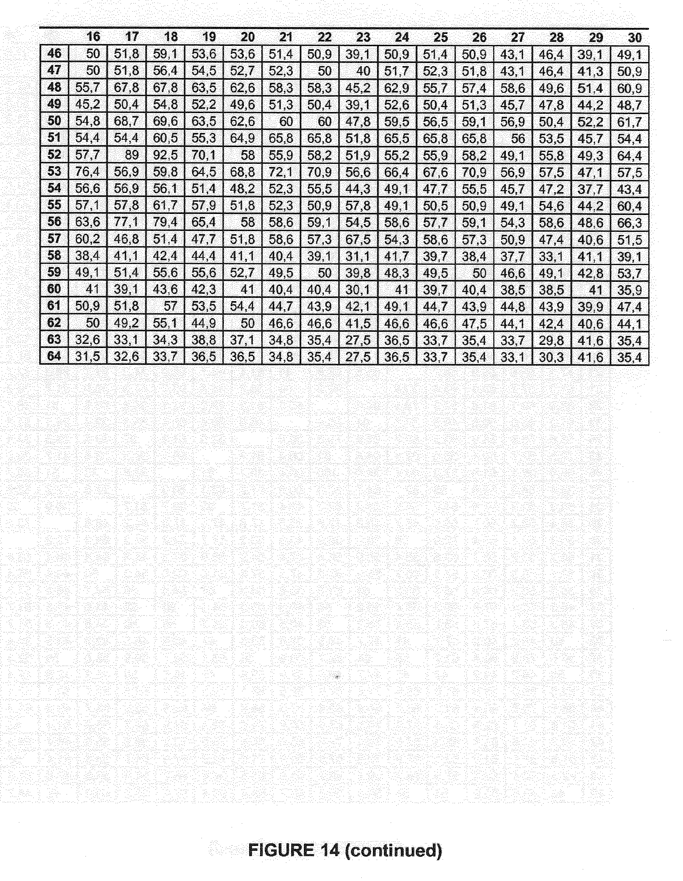

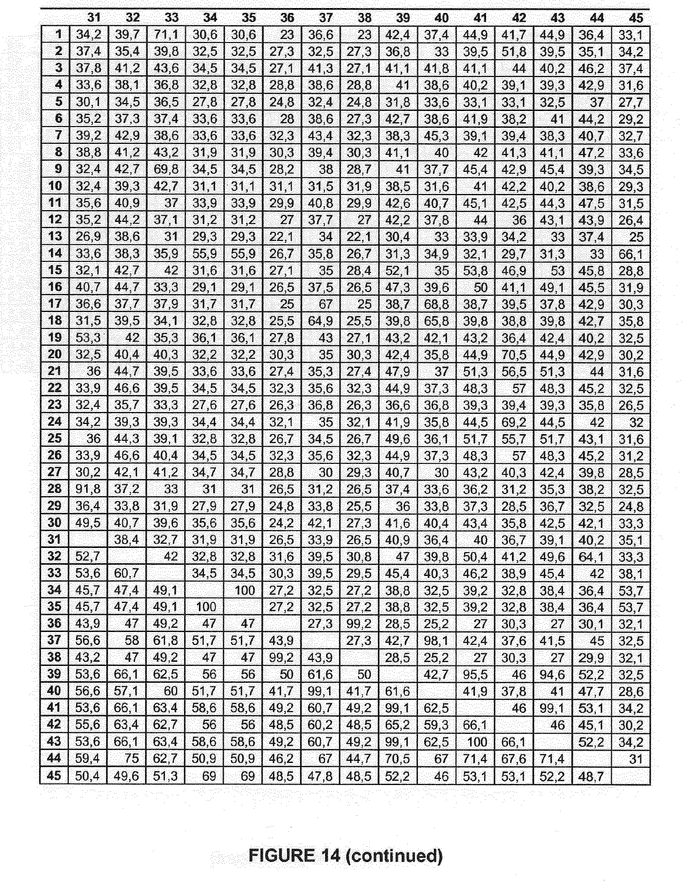

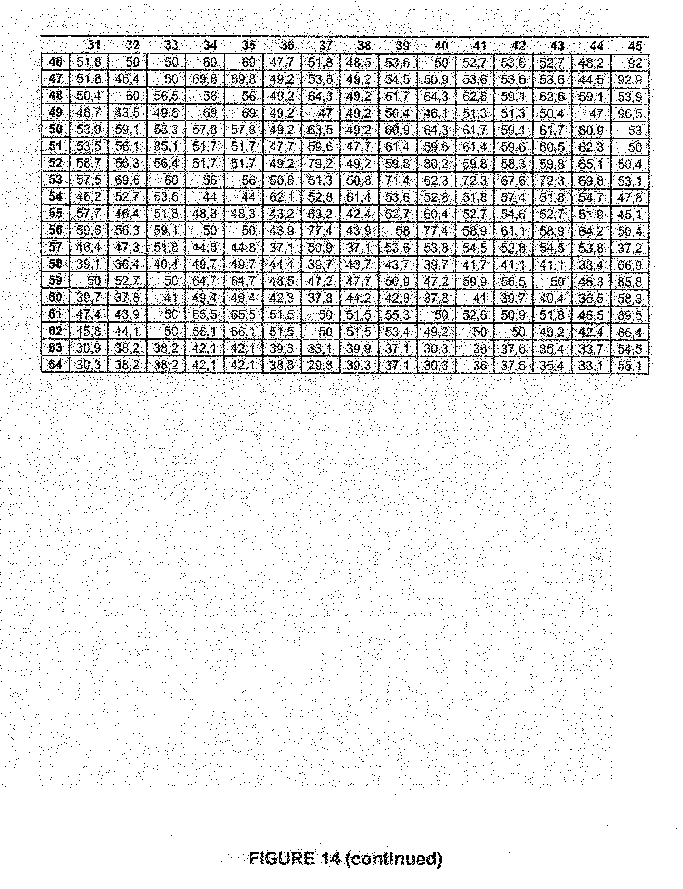

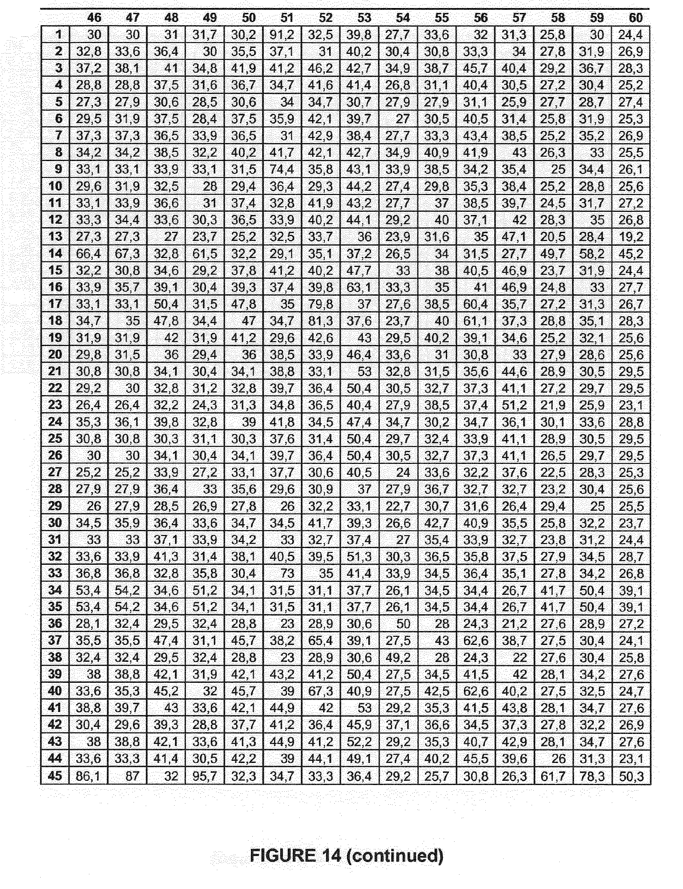

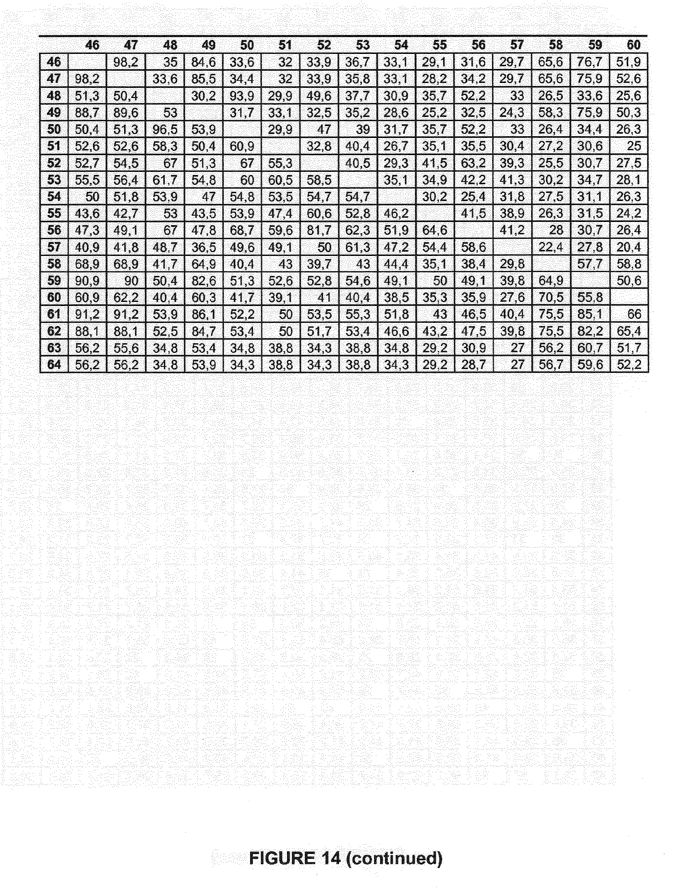

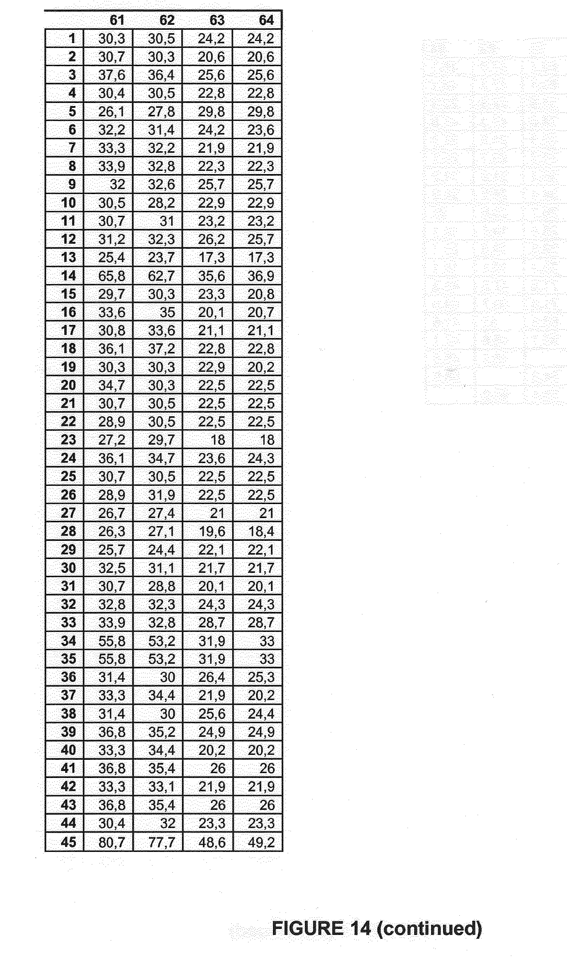

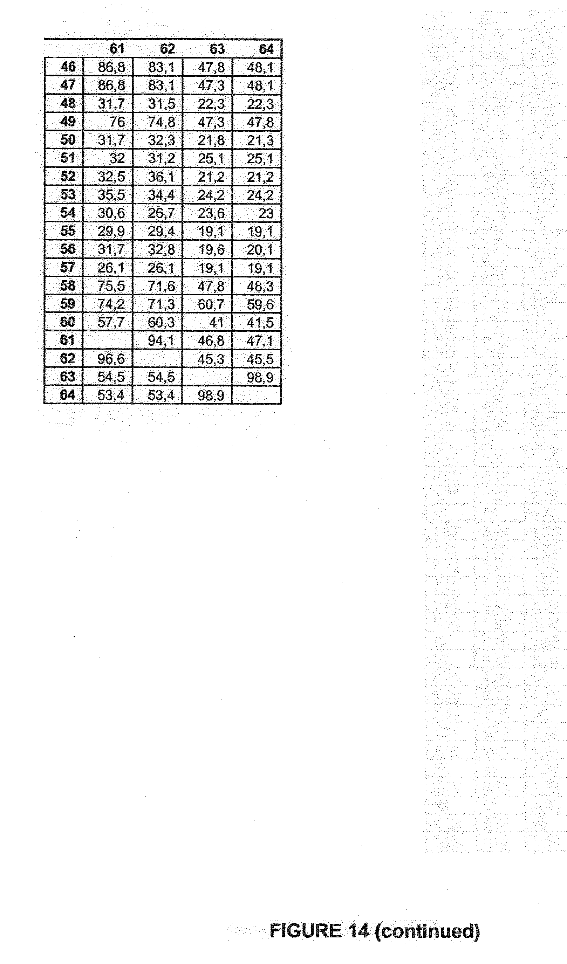

**Fig. 14** shows the MATGAT table of Example 23 for a representative number of ZPR polypeptides. The represented ZPR polypeptides are indicated by the following numbering: 1. S.lycopersicum_TC205388, 2. A.hypogaea_EG373732, 3. A.lyrata_322199, 4. A.lyrata_899056, 5. A.thaliana_AT2G45450.1, 6. A.thaliana_AT3G60890.1, 7. A.trichopoda_CK765457, 8. B.napus_CD822359, 9. C.annuum_TC13916, 10. C.intybus_EH709619, 11. C.melo_EB715099, 12. C.sinensis_TC13047, 13. C.tinctorius_EL400918, 14. E.tef_DN482990, 15. E.tirucalli_BP956571, 16. G.hirsutum_DW501389, 17. G.hirsutum_DW518216, 18. G.hirsutum_TC163217, 19. G.hybrid_AJ765000, 20. G.max_Glyma01g34620.1, 21. G.max-Glyma09g40450.1, 22. G.max_GM06MC39606_50673886@38431, 23. G.max_GM06MSsu40h07.r_46819511@70598, 24. G.max_TC281725, 25. G.max_TC286014, 26. G.max_TC305219, 27. L.perennis_TA2167_43195, 28. L.sativa_TC25720, 29. L.serriola_DW114794, 30. L.serriola_DW122307, 31. L.virosa_DW156764, 32. M.domestica_TC51427, 33. N.tabacum_TC60414, 34. O.sativa_LOC_Os09g34890.1, 35. Os_ZPRa_Os09g0520500, 36. P.abies_AM172974, 37. P.deltoides_TA3399_3696, 38. P.sitchensis_TA14065_3332, 39. P.tremula_TA8085_113636, 40. P.trichocarpa_554264, 41. P.trichocarpa_823907, 42. P.vulgaris_FE900059, 43. Pt_ZPR_small_Lzipper, 44. R.chinensis_BI978399, 45.

S.bicolor_Sb02g030180.1, 46. S.hybrid_CF573336, 47. S.hybrid_CF575621, 48. S.lycopersicum_TC207895, 49. S.propinquum_TA5427_132711, 50. S.tuberosum_EG011112, 51. S.tuberosum_TC176950, 52. T.cacao_CU522069, 53. T.cacao_TC3649, 54. T.cryptomerioides_DN975845, 55. Triphysaria_sp_TC4136, 56. V.vinifera_GSVIVT00001026001, 57. V.vinifera_GSVIVT00026366001, 58. Z.mays_BG842570, 59. Z.mays_c62174110gm030403@15401, 60. Z.mays_c62219732gm030403@9841, 61. Z.mays_TC477977, 62. Z.mays_TC529546, 63. Z.mays_ZM07MC28025_BFb0123H02@27941, 64. Zeama_ZmPC0129214_ZPRB

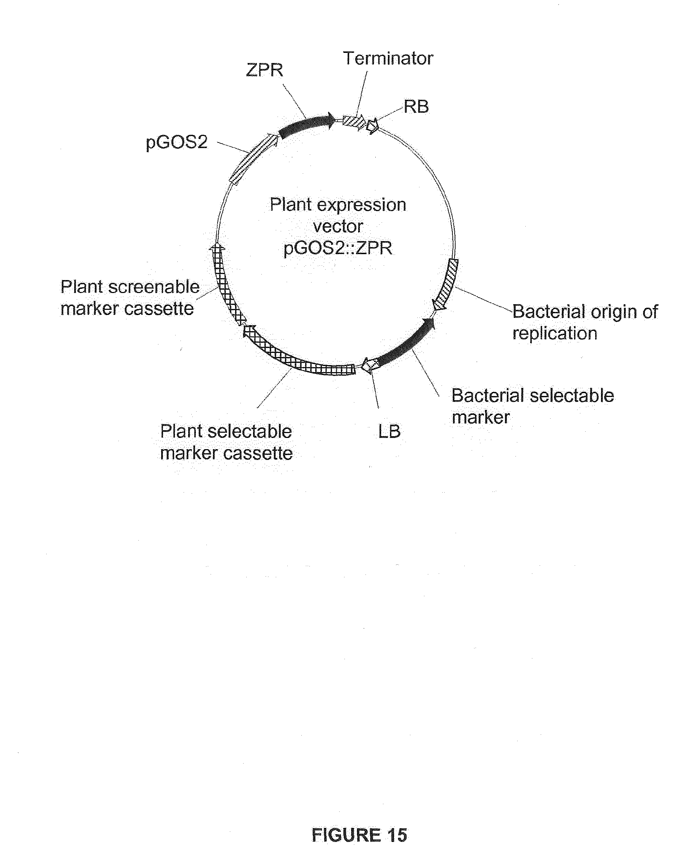

**Fig. 15** represents the binary vector used for increased expression in Oryza sativa of a ZPR-encoding nucleic acid under the control of a rice GOS2 promoter (pGOS2).

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

Example 1: Identification of sequences related to SEQ ID NO: 1 and SEQ ID NO: 2

Sequences (full length cDNA, ESTs or genomic) related to SEQ ID NO: 1 and SEQ ID NO: 2 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 1 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to SEQ ID NO: 1 and SEQ ID NO: 2.

**Table A:** Examples of OsRSZ33 RRM nucleic acids and polypeptides:

| Name | Nucleic acid SEQ ID NO: | Polypeptide SEQ ID NO: |
|---|---|---|
| O.sativa_LOC_Os05g02880.1#1 | 1 | 2 |
| S.officinarum_CA282538#1 | 3 | 4 |
| T.aestivum_TC361049#1 | 5 | 6 |
| G.hirsutum_ES798395#1 | 7 | 8 |
| P.virgatum_TC2491#1 | 9 | 10 |
| S.hybrid_CF570698#1 | 11 | 12 |
| G.max_Glyma19g35980.2#1 | 13 | 14 |
| G.max_Glyma19g35980.3#1 | 15 | 16 |
| P.trichocarpa_653760#1 | 17 | 18 |
| G.soja_BF598591#1 | 19 | 20 |
| N.tabacum_TC66751#1 | 21 | 22 |

(continued)

| Name | Nucleic acid SEQ ID NO: | Polypeptide SEQ ID NO: |
|---|---|---|
| A.cepa_TC5720#1 | 23 | 24 |
| P.patens_TC43147#1 | 25 | 26 |
| T.turgidum_AJ612509#1 | 27 | 28 |
| B.rapa_DY010361#1 | 29 | 30 |
| O.sativa_LOC_Os01g06290.4#1 | 31 | 32 |
| T.aestivum_TC286992#1 | 33 | 34 |
| T.aestivum_TC350935#1 | 35 | 36 |
| H.annuus_TC43928#1 | 37 | 38 |
| P.persica_TC9991#1 | 39 | 40 |
| E.esula_TC6405#1 | 41 | 42 |
| B.napus_TC102852#1 | 43 | 44 |
| S.officinarum_CA177377#1 | 45 | 46 |
| S.tuberosum_TC163945#1 | 47 | 48 |
| L.sativa_DY982195#1 | 49 | 50 |
| C.sinensis_TC6225#1 | 51 | 52 |
| G.hirsutum_TC144987#1 | 53 | 54 |
| L.sativa_TC21129#1 | 55 | 56 |
| G.max_Glyma19g35980.4#1 | 57 | 58 |
| P.virgatum_TC49604#1 | 59 | 60 |
| B.napus_TC90802#1 | 61 | 62 |
| B.napus_TC87766#1 | 63 | 64 |
| C.clementina_TC16391#1 | 65 | 66 |
| N.tabacum_TC43449#1 | 67 | 68 |
| T.aestivum_TC316707#1 | 69 | 70 |
| N.benthamiana_TC15005#1 | 71 | 72 |
| H.vulgare_TC162354#1 | 73 | 74 |
| S.officinarum_TC86028#1 | 75 | 76 |
| T.aestivum_TC316886#1 | 77 | 78 |
| B.napus_TC84291#1 | 79 | 80 |
| A.lyrata_323893#1 | 81 | 82 |
| A.thaliana_AT3G53500.2#1 | 83 | 84 |
| G.hirsutum_TC137472#1 | 85 | 86 |
| C.clementina_TC23768#1 | 87 | 88 |
| F.vesca_TA9870_57918#1 | 89 | 90 |
| G.hirsutum_TC160567#1 | 91 | 92 |
| G.max_Glyma19g35980.5#1 | 93 | 94 |
| G.max_Glyma03g33260.1#1 | 95 | 96 |
| G.max_Glyma19g35980.1#1 | 97 | 98 |

(continued)

| Name | Nucleic acid SEQ ID NO: | Polypeptide SEQ ID NO: |
|---|---|---|
| G.max_TC317123#1 | 99 | 100 |
| T.aestivum_TC347767#1 | 101 | 102 |
| A.thaliana_AT2G37340.1#1 | 103 | 104 |
| A.hypogaea_EG029774#1 | 105 | 106 |
| B.napus_TC73509#1 | 107 | 108 |
| B.napus_BN06MC04617_42261920@4606#1 | 109 | 110 |
| P.virgatum_TC14454#1 | 111 | 112 |
| Poptr_ATRSZ33 like | 113 | 114 |
| V.vinifera_GSVIVT00023097001#1 | 115 | 116 |
| C.clementina_TC39204#1 | 117 | 118 |
| B.napus_TC68381#1 | 119 | 120 |
| B.rapa_TA6039_3711#1 | 121 | 122 |
| C.clementina_TC34178#1 | 123 | 124 |
| P.vulgaris_TC8698#1 | 125 | 126 |
| S.bicolor_Sb03g005500.1#1 | 127 | 128 |
| C.canephora_TC3361#1 | 129 | 130 |
| G.max_Glyma19g35990.1#1 | 131 | 132 |
| G.max_TC324192#1 | 133 | 134 |
| G.max_GM06MC33050_sl61g01@32287#1 | 135 | 136 |
| G.max_TC321743#1 | 137 | 138 |
| I.batatas_TA3421_4120#1 | 139 | 140 |
| A.comosus_DT336917#1 | 141 | 142 |
| L.japonicus_TC42099#1 | 143 | 144 |
| N.tabacum_TC40467#1 | 145 | 146 |
| G.raimondii_TC4400#1 | 147 | 148 |
| A.officinalis_TA1321_4686#1 | 149 | 150 |
| G.hirsutum_TC163142#1 | 151 | 152 |
| S.tuberosum_TC164160#1 | 153 | 154 |
| T.cacao_TC5161#1 | 155 | 156 |
| G.hirsutum_TC134110#1 | 157 | 158 |
| F.vesca_TA9810_57918#1 | 159 | 160 |
| L.japonicus_TC37427#1 | 161 | 162 |
| Triphysaria_sp_TC3067#1 | 163 | 164 |
| V.vinifera_GSVIVT00029498001#1 | 165 | 166 |
| P.taeda_TA6650_3352#1 | 167 | 168 |
| P.trifoliata_TA5898_37690#1 | 169 | 170 |
| S.bicolor_Sb09g001920.1#1 | 171 | 172 |
| S.officinarum_TC107276#1 | 173 | 174 |

(continued)

| Name | Nucleic acid SEQ ID NO: | Polypeptide SEQ ID NO: |
|---|---|---|
| B.napus_TC84359#1 | 175 | 176 |
| Aquilegia_sp_TC24323#1 | 177 | 178 |
| P.pinaster_TA3625_71647#1 | 179 | 180 |
| P.virgatum_TC32128#1 | 181 | 182 |
| Z.mays_TC518048#1 | 183 | 184 |
| Aquilegia_sp_TC24824#1 | 185 | 186 |
| P.sitchensis_TA11267_3332#1 | 187 | 188 |
| N.tabacum_TC53470#1 | 189 | 190 |
| H.vulgare_TC154362#1 | 191 | 192 |
| T.aestivum_TC279628#1 | 193 | 194 |
| O.sativa_LOC_Os01g06290.1#1 | 195 | 196 |
| Z.mays_c68511709gm030403@11445#1 | 197 | 198 |
| Z.mays_TC527608#1 | 199 | 200 |
| O.glaberrima_Og014036.01#1 | 201 | 202 |
| O.sativa_LOC_Os03g17710.2#1 | 203 | 204 |
| P.virgatum_TC27619#1 | 205 | 206 |
| P.patens_TC31165#1 | 207 | 208 |
| S.officinarum_TC76505#1 | 209 | 210 |
| Z.mays_ZM07MC13041_BFb0138E15@13012#1 | 211 | 212 |
| M.crystallinum_TC10341#1 | 213 | 214 |
| T.aestivum_TC300145#1 | 215 | 216 |
| T.monococcum_TA2060_4568#1 | 217 | 218 |
| C.clementina_TC11431#1 | 219 | 220 |
| H.vulgare_c62741371hv270303@6248#1 | 221 | 222 |
| T.aestivum_RSZ38 | 223 | 224 |
| Z.mays_c67261821gm030403@11244#1 | 225 | 226 |
| Z.mays_TC490304#1 | 227 | 228 |
| O.sativa_LOC_Os03g17710.1#1 | 229 | 230 |
| O.sativa_LOC_Os05g07000.1#1 | 231 | 232 |
| S.bicolor_Sb09g001910.1#1 | 233 | 234 |
| Z.mays_TC510523#1 | 235 | 236 |
| Z.mays_ZM07MC35249_BFb0381I21@35142#1 | 237 | 238 |
| S.officinarum_TC80184#1 | 239 | 240 |

Sequences have been tentatively assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, such as by the Joint Genome Institute. Furthermore, access to proprietary databases, has allowed the identification of novel nucleic acid and polypeptide sequences.

Example 2: Alignment of OsRSZ33 RRM polypeptide sequences

An alignment of polypeptide sequences (Figure 2) was performed using the ClustalW (2.0) algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment. The alignment shows that the N-terminal half, up to the end of the Zn Knucle domains is highly conserved.

Combination of the information on the domains in figure 1 and the alignment can be used for identifying the various motifs in the homologues of SEQ ID NO: 2.

A phylogenetic tree of various SR polypeptides (Figure 3) was constructed using a ClustalW alignment (Isshiki et al., 2006). The OsRSZ33 RRM polypeptides useful in the methods of the present invention all belong to the cluster with two Zn Knuckle domains.

Example 3: Calculation of global percentage identity between polypeptide sequences

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were: Scoring matrix: Blosum62, First Gap: 12, Extending Gap: 2.

Results of the software analysis are shown in Table B for the global similarity and identity over the full length sequences from several OsRSZ33 RRM polypeptides from monocotyledonous plants. The sequence identity (in %) between the OsRSZ33 RRM polypeptide sequences useful in performing the methods of the invention can be as low as 41 %, but is generally higher than 50% compared to SEQ ID NO: 2.

**Table B:** MatGAT results for global similarity and identity over the full length of the polypeptide sequences.

| | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| 1. H.vulgare_@6248 | | 75.4 | 74.4 | 75.4 | 74.5 | 48.3 | 71.9 | 73.3 | 73.1 | 72.4 | 46.2 | 52.0 | 39.3 | 72.8 | 69.0 | 64.8 | 58.4 | 64.6 | 95.8 | 59.8 |
| 2. O.glaberrima_Og014036 | 83.5 | | 97.3 | 98.8 | 87.7 | 48.5 | 76.1 | 76.4 | 76.5 | 75.7 | 46.1 | 51.2 | 40.9 | 75.7 | 74.7 | 69.1 | 56.4 | 57.8 | 75.7 | 54.5 |
| 3. O.sativa_LOC_Os03g17710.1 | 83.3 | 97.6 | | 98.5 | 86.4 | 48.1 | 75.3 | 75.3 | 75.4 | 74.6 | 45.7 | 50.7 | 40.6 | 74.6 | 73.6 | 68.2 | 55.8 | 57.9 | 74.6 | 54.0 |
| 4. O.sativa_LOC_Os03g17710.2 | 83.8 | 99.1 | 98.5 | | 87.7 | 48.8 | 76.4 | 76.7 | 76.5 | 76.0 | 46.4 | 51.5 | 41.2 | 76.0 | 74.7 | 69.1 | 56.7 | 58.7 | 75.7 | 54.8 |
| 5. O.sativa_LOC_Os05g02880 | 81.1 | 90.9 | 89.9 | 91.2 | | 49.8 | 78.3 | 78.9 | 76.9 | 78.4 | 47.1 | 52.0 | 41.5 | 77.9 | 75.8 | 69.3 | 57.1 | 58.5 | 74.5 | 55.7 |
| 6. P.virgatum_TC2491 | 50.5 | 51.2 | 50.7 | 51.5 | 52.3 | | 48.2 | 52.5 | 45.7 | 53.2 | 90.3 | 67.6 | 73.0 | 52.9 | 46.2 | 42.1 | 57.8 | 74.2 | 48.8 | 79.8 |
| 7. P.virgatum_TC27619 | 80.2 | 82.4 | 81.8 | 83.0 | 83.3 | 50.6 | | 82.8 | 85.7 | 82.0 | 46.4 | 51.8 | 41.2 | 82.3 | 82.4 | 72.3 | 57.9 | 58.1 | 72.1 | 54.8 |
| 8. P.virgatum_TC32128 | 79.3 | 84.8 | 83.9 | 85.5 | 86.1 | 54.1 | 87.3 | | 80.1 | 91.5 | 50.0 | 55.7 | 41.1 | 91.1 | 78.1 | 70.5 | 62.1 | 60.4 | 73.5 | 56.6 |
| 9. S.bicolor_Sb09g001910 | 80.9 | 81.8 | 82.1 | 82.1 | 81.5 | 48.7 | 88.0 | 85.0 | | 77.8 | 44.9 | 49.6 | 41.9 | 77.3 | 86.9 | 78.9 | 55.1 | 54.8 | 71.9 | 51.6 |
| 10. S.bicolor_Sb09g001920 | 80.8 | 83.6 | 82.7 | 84.2 | 85.4 | 54.2 | 86.1 | 94.6 | 82.7 | | 52.6 | 58.3 | 42.3 | 98.1 | 75.7 | 69.0 | 64.8 | 61.4 | 74.0 | 57.9 |
| 11. S.hybrid_CF570698 | 48.6 | 49.1 | 48.7 | 49.4 | 50.2 | 93.7 | 48.2 | 51.6 | 47.2 | 52.9 | | 67.6 | 72.0 | 52.9 | 45.0 | 41.3 | 58.5 | 70.5 | 46.7 | 75.4 |
| 12. S.officinarum_CA177377 | 57.4 | 57.3 | 56.7 | 57.6 | 57.0 | 69.7 | 55.2 | 58.6 | 54.0 | 59.6 | 68.1 | | 54.2 | 59.3 | 50.5 | 45.4 | 63.1 | 69.6 | 52.7 | 68.9 |
| 13. S.officinarum_CA282538 | 40.5 | 41.8 | 41.5 | 42.1 | 42.4 | 77.6 | 42.1 | 43.9 | 42.2 | 44.2 | 76.6 | 57.1 | | 42.3 | 43.8 | 39.6 | 46.5 | 59.4 | 39.2 | 63.5 |
| 14. S.officinarum_TC107276 | 79.0 | 84.2 | 83.3 | 84.8 | 84.8 | 54.2 | 87.0 | 94.9 | 82.4 | 99.0 | 52.9 | 60.3 | 44.9 | | 76.0 | 68.8 | 65.7 | 60.8 | 73.1 | 57.0 |
| 15. S.officinarum_TC76505 | 79.0 | 81.3 | 80.6 | 81.6 | 81.0 | 49.5 | 85.8 | 83.7 | 88.9 | 81.6 | 48.6 | 55.3 | 44.1 | 82.2 | | 80.5 | 56.6 | 55.6 | 68.2 | 52.6 |
| 16. S.officinarum_TC80184 | 69.9 | 75.4 | 75.7 | 75.7 | 74.9 | 45.1 | 76.2 | 76.0 | 83.6 | 73.8 | 44.0 | 51.6 | 39.9 | 73.8 | 83.3 | | 51.5 | 50.8 | 64.1 | 47.3 |
| 17. S.officinarum_TC86028 | 66.1 | 66.1 | 65.4 | 66.4 | 66.3 | 59.6 | 65.5 | 69.7 | 64.2 | 70.2 | 58.9 | 65.6 | 49.3 | 70.8 | 66.2 | 60.1 | | 60.8 | 57.7 | 60.6 |
| 18. T.aestivum_TC286992 | 64.9 | 61.5 | 61.5 | 62.4 | 62.5 | 77.9 | 60.9 | 64.6 | 57.8 | 65.1 | 74.7 | 74.4 | 62.2 | 65.1 | 58.9 | 53.8 | 68.4 | | 65.1 | 92.6 |
| 19. T.aestivum_TC300145 | 97.6 | 85.2 | 84.8 | 85.5 | 82.5 | 50.9 | 81.0 | 82.5 | 81.5 | 82.5 | 49.1 | 57.2 | 41.0 | 82.5 | 77.7 | 69.9 | 64.5 | 65.4 | | 60.2 |
| 20. T.turgidum_AJ612509 | 60.1 | 57.6 | 57.0 | 57.9 | 58.5 | 83.7 | 57.3 | 60.5 | 54.3 | 60.9 | 79.8 | 73.9 | 66.5 | 60.6 | 55.6 | 50.5 | 66.3 | 92.6 | 60.5 | |
| 21. T.aestivum_RSZ38 | 96.7 | 84.1 | 83.9 | 84.4 | 82.0 | 50.8 | 80.2 | 81.7 | 80.6 | 81.7 | 48.9 | 58.0 | 40.8 | 81.7 | 76.9 | 70.5 | 67.0 | 65.2 | 97.9 | 60.4 |
| 22. Z.mays_@11244 | 80.5 | 84.4 | 84.2 | 84.7 | 84.7 | 49.8 | 88.6 | 86.8 | 94.1 | 85.3 | 48.9 | 56.2 | 43.2 | 85.6 | 89.8 | 80.6 | 62.8 | 59.8 | 82.0 | 55.6 |
| 23. Z.mays_@11445 | 82.9 | 83.9 | 83.0 | 84.5 | 84.3 | 51.7 | 85.2 | 88.9 | 83.3 | 91.7 | 50.8 | 57.2 | 42.5 | 91.4 | 81.0 | 73.5 | 66.8 | 62.8 | 84.6 | 58.5 |
| 24. Z.mays_TC490304 | 80.5 | 84.4 | 84.2 | 84.7 | 84.7 | 49.8 | 88.6 | 86.8 | 94.1 | 85.3 | 48.9 | 56.2 | 43.2 | 85.6 | 89.8 | 80.6 | 62.8 | 59.8 | 82.0 | 55.6 |
| 25. Z.mays_TC510523 | 77.9 | 78.8 | 79.0 | 79.0 | 77.6 | 47.0 | 83.0 | 80.5 | 90.1 | 78.5 | 45.9 | 51.8 | 40.5 | 78.5 | 83.9 | 80.3 | 61.5 | 56.7 | 78.2 | 53.0 |
| 26. Z.mays_TC518048 | 79.9 | 82.4 | 81.5 | 83.0 | 84.2 | 53.5 | 85.8 | 92.7 | 81.5 | 95.5 | 52.2 | 59.9 | 43.6 | 95.9 | 80.4 | 72.4 | 69.7 | 65.0 | 81.6 | 60.2 |
| 27. Z.mays_TC527608 | 82.9 | 83.9 | 83.0 | 84.5 | 83.4 | 51.7 | 84.8 | 88.9 | 83.3 | 91.7 | 50.8 | 57.8 | 42.2 | 91.4 | 81.0 | 73.5 | 66.8 | 62.8 | 84.6 | 58.5 |
| 28. Z.mays_@13012 | 80.5 | 84.6 | 83.9 | 84.9 | 84.9 | 50.2 | 89.4 | 86.7 | 93.3 | 85.8 | 49.2 | 56.8 | 43.5 | 86.1 | 90.0 | 80.1 | 63.7 | 60.1 | 82.2 | 55.9 |
| 29. Z.mays_@35142 | 78.5 | 79.3 | 79.6 | 79.6 | 78.2 | 47.3 | 83.6 | 80.7 | 90.7 | 78.8 | 45.9 | 52.7 | 40.5 | 78.8 | 84.4 | 80.6 | 61.8 | 56.9 | 78.8 | 53.3 |

| | 21 | 22 | 23 | 24 | 25 | 26 | 27 | 28 | 29 |
|---|---|---|---|---|---|---|---|---|---|
| 1. H.vulgare_@6248 | 96.1 | 72.6 | 74.2 | 72.6 | 71.6 | 69.7 | 75.1 | 72.1 | 72.2 |
| 2. O.glaberrima_Og014036 | 75.2 | 77.0 | 74.1 | 77.0 | 73.8 | 73.7 | 75.3 | 77.2 | 74.4 |
| 3. O.sativa_LOC_Os03g17710.1 | 74.1 | 75.9 | 73.1 | 75.9 | 72.8 | 72.6 | 74.2 | 76.1 | 73.4 |
| 4. O.sativa_LOC_Os03g17710.2 | 75.2 | 77.0 | 74.4 | 77.0 | 73.8 | 74.0 | 75.6 | 77.2 | 74.4 |
| 5. O.sativa_LOC_Os05g02880 | 73.8 | 78.6 | 74.9 | 78.6 | 73.0 | 75.4 | 75.8 | 78.7 | 73.6 |
| 6. P.virgatum_TC2491 | 48.3 | 46.5 | 49.5 | 46.5 | 43.6 | 51.3 | 49.8 | 46.8 | 43.9 |
| 7. P.virgatum_TC27619 | 72.2 | 85.0 | 77.6 | 85.3 | 79.9 | 79.1 | 77.9 | 85.0 | 80.5 |
| 8. P.virgatum_TC32128 | 73.0 | 82.1 | 82.8 | 82.1 | 75.4 | 87.4 | 83.4 | 81.2 | 75.6 |
| 9. S.bicolor_Sb09g001910 | 72.8 | 91.5 | 75.9 | 91.8 | 87.9 | 74.2 | 76.5 | 91.0 | 88.5 |
| 10. S.bicolor_Sb09g001920 | 73.8 | 80.4 | 89.3 | 80.4 | 74.1 | 93.3 | 88.7 | 80.5 | 74.4 |
| 11. S.hybrid_CF570698 | 46.2 | 45.6 | 49.2 | 45.6 | 42.8 | 50.3 | 48.9 | 45.9 | 42.8 |
| 12. S.officinarum_CA177377 | 52.6 | 50.8 | 54.2 | 50.8 | 48.4 | 57.0 | 54.8 | 51.1 | 48.4 |
| 13. S.officinarum_CA282538 | 39.3 | 42.0 | 40.0 | 42.0 | 39.7 | 41.4 | 39.4 | 42.3 | 39.7 |
| 14. S.officinarum_TC107276 | 72.9 | 80.4 | 87.8 | 80.4 | 73.8 | 92.4 | 87.8 | 80.5 | 74.1 |
| 15. S.officinarum_TC76505 | 67.6 | 86.1 | 71.8 | 86.4 | 80.8 | 73.9 | 72.4 | 86.3 | 81.4 |
| 16. S.officinarum_TC80184 | 64.8 | 76.2 | 67.1 | 76.5 | 76.4 | 65.5 | 67.7 | 75.4 | 76.6 |
| 17. S.officinarum_TC86028 | 57.6 | 56.0 | 59.8 | 56.0 | 53.9 | 61.4 | 59.8 | 57.5 | 53.1 |
| 18. T.aestivum_TC286992 | 65.2 | 56.1 | 58.1 | 56.1 | 53.5 | 60.1 | 59.0 | 56.5 | 53.8 |
| 19. T.aestivum_TC300145 | 96.4 | 71.6 | 74.7 | 71.6 | 70.4 | 70.9 | 75.6 | 72.0 | 71.0 |
| 20. T.turgidum_AJ612509 | 60.4 | 52.5 | 54.4 | 52.5 | 49.9 | 55.7 | 55.3 | 52.9 | 50.1 |
| 21. T.aestivum_RSZ38 | | 71.7 | 74.8 | 71.7 | 71.3 | 70.8 | 75.6 | 72.1 | 71.9 |
| 22. Z.mays_@11244 | 81.1 | | 76.0 | 99.7 | 84.2 | 76.9 | 76.3 | 98.8 | 84.8 |
| 23. Z.mays_@11445 | 83.8 | 84.1 | | 76.0 | 72.4 | 88.8 | 96.0 | 76.5 | 72.7 |
| 24. Z.mays_TC490304 | 81.1 | 100.0 | 84.1 | | 84.5 | 76.9 | 76.3 | 98.5 | 85.1 |
| 25. Z.mays_TC510523 | 77.9 | 87.3 | 77.9 | 87.3 | | 71.1 | 73.2 | 83.7 | 99.4 |
| 26. Z.mays_TC518048 | 81.1 | 83.8 | 91.4 | 83.8 | 77.6 | | 84.8 | 77.1 | 71.4 |
| 27. Z.mays_TC527608 | 83.8 | 84.1 | 98.8 | 84.1 | 77.9 | 90.2 | | 76.8 | 73.5 |
| 28. Z.mays_@13012 | 80.8 | 99.1 | 84.3 | 99.1 | 86.7 | 84.3 | 84.3 | | 84.2 |
| 29. Z.mays_@35142 | 78.5 | 87.8 | 78.2 | 87.8 | 99.4 | 77.9 | 78.2 | 87.3 | |

Example 4: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 2 are presented in Table C.

**Table C:** InterPro scan results (major accession numbers) of the polypeptide sequence as represented by SEQ ID NO: 2.

| Database | Number | Name | start | stop | p-value | Accession |
|---|---|---|---|---|---|---|
| ProfileScan | PS50158 | ZF_CCHC | 103 | 118 | 0,00E+00 | IPR001878 |
| ProfileScan | PS50158 | ZF_CCHC | 125 | 141 | 0,00E+00 | IPR001878 |
| Gene3D | G3DSA:3.30.70.330 | a_b_plait_nuc_bd | 9 | 119 | 6,60E-07 | IPR012677 |

(continued)

| Database | Number | Name | start | stop | p-value | Accession |
|---|---|---|---|---|---|---|
| superfamily | SSF54928 | SSF54928 | 3 | 103 | 2,40E-04 | NULL |
| HMMSmart | SM00343 | ZnF_C2HC | 103 | 119 | 6,00E+08 | IPR001878 |
| HMMSmart | SM00343 | ZnF_C2HC | 125 | 141 | 2,10E+08 | IPR001878 |
| HMMPanther | PTHR10548 | PTHR10548 | 10 | 281 | 7,10E-36 | NULL |
| HMMPanther | PTHR10548 | PTHR10548 | 10 | 281 | 7,10E-36 | NULL |
| superfamily | SSF57756 | SSF57756 | 90 | 145 | 4,30E+04 | NULL |
| ProfileScan | PS50102 | RRM | 11 | 81 | 0,00E+00 | IPR000504 |
| HMMPfam | PF00098 | zf-CCHC | 102 | 119 | 1,50E-03 | IPR001878 |
| HMMPfam | PF00098 | zf-CCHC | 124 | 141 | 1,30E-02 | IPR001878 |
| HMMPfam | PF00076 | RRM_1 | 13 | 75 | 7,60E+00 | IPR000504 |
| HMMSmart | SM00360 | RRM | 12 | 77 | 1,60E-06 | IPR000504 |

Example 5: Topology prediction of the OsRSZ33 RRM polypeptide sequences

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 2 are presented Table D. The "plant" organism group has been selected, no cutoffs defined, and the predicted length of the transit peptide requested. The subcellular localization of the polypeptide sequence as represented by SEQ ID NO: 2 is predicted to be the mitochondrion, no transit peptide is predicted.

**Table D:** TargetP 1.1 analysis of the polypeptide sequence as represented by SEQ ID NO: 2. Abbreviations: Len, Length; cTP, Chloroplastic transit peptide; mTP, Mitochondrial transit peptide, SP, Secretory pathway signal peptide, other, Other subcellular targeting, Loc, Predicted Location; RC, Reliability class; TPlen, Predicted transit peptide length.

| Name | Len | cTP | mTP | SP | other | Loc | RC | TPlen |
|---|---|---|---|---|---|---|---|---|
| OsRSZ33 | 323 | 0.020 | 0.914 | 0.012 | 0.270 | M | 2 | 46 |
| cutoff | | 0.000 | 0.000 | 0.000 | 0.000 | | | |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

Example 6: Functional assay for the OsRSZ33 RRM polypeptide

OsRSZ33 RRM proteins are postulated to interact with other SR proteins. Reference is made to the yeast two-hybrid assay with atRSZ33 as described in Lopato et al. 2002 and to the pull-down assay described by Lorkovic et al. (Exp. Cell Res. 314, 3175-3186, 2008).

Example 7: Cloning of the OsRSZ33 RRM encoding nucleic acid sequence

The nucleic acid sequence was amplified by PCR using as template a custom-made *Oryza sativa* seedlings cDNA library (in pCMV Sport 6.0; Invitrogen, Paisley, UK). PCR was performed using Hifi Taq DNA polymerase in standard conditions, using 200 ng of template in a 50 $\mu$l PCR mix. The primers used were prm15077 (SEQ ID NO: 248; sense, start codon in bold): 5'-ggggacaagtttgtacaaaaaagcaggcttaaaca**atg**ccgcgctatgatga-3' and prm15078 (SEQ ID NO: 249; reverse, complementary): 5'-ggggaccactttgtacaagaaagctgggtactgcgattaa atttcaggct-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pOsRSZ33 RRM. Plasmid pDONR201 was purchased from Invitrogen, as part of the Gateway® technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 247) for constitutive expression was located upstream of this Gateway cassette.

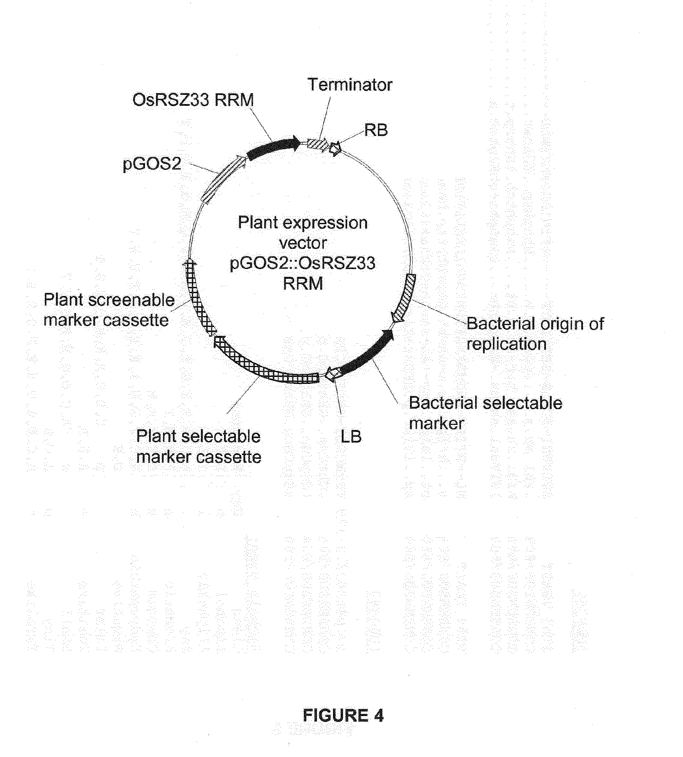

After the LR recombination step, the resulting expression vector pGOS2::OsRSZ33 RRM (Figure 4) was transformed into *Agrobacterium* strain LBA4044 according to methods well known in the art.

Example 8: Plant transformation

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% HgCl$_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD$_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Example 9: Transformation of other crops

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 $\mu$m acetosyrigninone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 $\mu$g/ml cefotaxime. The seeds are then transferred to SH-medium with 50$\mu$g/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 $\mu$g/ml MgCL2, and with 50 to 100 $\mu$g/ml cefotaxime and 400-500 $\mu$g/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

*Sugarbeet transformation*

Seeds of sugarbeet (Beta vulgaris L.) are sterilized in 70% ethanol for one minute followed by 20 min. shaking in 20% Hypochlorite bleach e.g. Clorox® regular bleach (commercially available from Clorox, 1221 Broadway, Oakland, CA 94612, USA). Seeds are rinsed with sterile water and air dried followed by plating onto germinating medium (Murashige and Skoog (MS) based medium (Murashige, T., and Skoog, ., 1962. Physiol. Plant, vol. 15, 473-497) including B5 vitamins (Gamborg et al.; Exp. Cell Res., vol. 50, 151-8.) supplemented with 10 g/l sucrose and 0,8% agar). Hypocotyl tissue is used essentially for the initiation of shoot cultures according to Hussey and Hepher (Hussey, G., and Hepher, A., 1978. Annals of Botany, 42, 477-9) and are maintained on MS based medium supplemented with 30g/l sucrose plus 0,25mg/l benzylamino purine and 0,75% agar, pH 5,8 at 23-25°C with a 16-hour photoperiod. Agrobacterium tumefaciens strain carrying a binary plasmid harbouring a selectable marker gene, for example nptII, is used in transformation experiments. One day before transformation, a liquid LB culture including antibiotics is grown on a shaker (28°C, 150rpm) until an optical density (O.D.) at 600 nm of ~1 is reached. Overnight-grown bacterial cultures are centrifuged and resuspended in inoculation medium (O.D. ~1) including Acetosyringone, pH 5,5. Shoot base tissue is cut into slices (1.0 cm x 1.0 cm x 2.0 mm approximately). Tissue is immersed for 30s in liquid bacterial inoculation medium. Excess liquid is removed by filter paper blotting. Co-cultivation occurred for 24-72 hours on MS based medium incl. 30g/l sucrose followed by a non-selective period including MS based medium, 30g/l sucrose with 1 mg/l BAP to induce shoot development and cefotaxim for eliminating the Agrobacterium. After 3-10 days explants are transferred to similar selective medium harbouring for example kanamycin or G418 (50-100 mg/l genotype dependent). Tissues are transferred to fresh medium every 2-3 weeks to maintain selection pressure. The very rapid initiation of shoots (after 3-4 days) indicates regeneration of existing meristems rather than organogenesis of newly developed transgenic meristems. Small shoots are transferred after several rounds of subculture to root induction medium containing 5 mg/l NAA and kanamycin or G418. Additional steps are taken to reduce the potential of generating transformed plants that are chimeric (partially transgenic). Tissue samples from regenerated shoots are used for DNA analysis. Other transformation methods for sugarbeet are known in the art, for example those by Linsey & Gallois (Linsey, K., and Gallois, P., 1990. Journal of Experimental Botany; vol. 41, No. 226; 529-36) or the methods published in the international application published as WO9623891A.

*Sugarcane transformation*

Spindles are isolated from 6-month-old field grown sugarcane plants (see Arencibia et al., 1998. Transgenic Research, vol. 7, 213-22; Enriquez-Obregon et al., 1998. Planta, vol. 206, 20-27). Material is sterilized by immersion in a 20% Hypochlorite bleach e.g. Clorox® regular bleach (commercially available from Clorox, 1221 Broadway, Oakland, CA 94612, USA) for 20 minutes. Transverse sections around 0,5cm are placed on the medium in the top-up direction. Plant material is cultivated for 4 weeks on MS (Murashige, T., and Skoog, ., 1962. Physiol. Plant, vol. 15, 473-497) based medium incl. B5 vitamins (Gamborg, O., et al., 1968. Exp. Cell Res., vol. 50, 151-8) supplemented with 20g/l sucrose, 500 mg/l casein hydrolysate, 0,8% agar and 5mg/l 2,4-D at 23°C in the dark. Cultures are transferred after 4 weeks onto identical fresh medium. Agrobacterium tumefaciens strain carrying a binary plasmid harbouring a selectable marker gene, for example hpt, is used in transformation experiments. One day before transformation, a liquid LB culture including antibiotics is grown on a shaker (28°C, 150rpm) until an optical density (O.D.) at 600 nm of ~0,6 is reached. Overnight-grown bacterial cultures are centrifuged and resuspended in MS based inoculation medium (O.D. ~0,4) including acetosyringone, pH 5,5. Sugarcane embryogenic callus pieces (2-4 mm) are isolated based on morphological characteristics as compact structure and yellow colour and dried for 20 min. in the flow hood followed by immersion in a liquid bacterial inoculation medium for 10-20 minutes. Excess liquid is removed by filter paper blotting. Co-cultivation occurred for 3-5 days in the dark on filter paper which is placed on top of MS based medium incl. B5 vitamins containing 1 mg/l 2,4-D. After co-cultivation calli are washed with sterile water followed by a non-selective cultivation period on similar medium containing 500 mg/l cefotaxime for eliminating remaining Agrobacterium cells. After 3-10 days explants are transferred to MS based selective medium incl. B5 vitamins containing 1 mg/l 2,4-D for another 3 weeks harbouring 25 mg/l of hygromycin (genotype dependent). All treatments are made at 23°C under dark conditions. Resistant calli are further cultivated on medium lacking 2,4-D including 1 mg/l BA and 25 mg/l hygromycin under 16 h light photoperiod resulting in the development of shoot structures. Shoots are isolated and cultivated on selective rooting medium (MS based including, 20g/l sucrose, 20 mg/l hygromycin and 500 mg/l cefotaxime). Tissue samples from regenerated shoots are used for DNA analysis. Other transformation methods for sugarcane are known in the art, for example from the international application published as WO2010/151634A and the granted European patent EP1831378.

Example 10: Phenotypic evaluation procedure

*10.1 Evaluation setup*

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development.

From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

*10.2 Statistical analysis: F test*

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

*10.3 Parameters measured*

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

Examples 11: Results of the phenotypic evaluation of the transgenic plants

Evaluation of transgenic T1 rice plants expressing the nucleic acid encoding the OsRSZ33 RRM protein of SEQ ID NO: 2 under control of the rice GOS2 promoter and grown under normal conditions, revealed that these plants had an improved early vigour and increased yield. In particular the plants showed increased biomass and had a higher seed yield. Details are presented in Table E. AreaMax is a parameter reflecting the above-ground biomass whereas GravityYMax is an indication of plant height, EmerVigor is early vigour, root biomass is reflected by the RootMax, RootSHind and the RootThickMax parameters, seed yield is reflected by totalwgseeds, nrtotalseed, harvestindex, nrfilledseed. The firstpan parameter represents the number of panicles in the first flush.

**Table E:** Data summary for transgenic rice plants; for each parameter, the percentage overall increase is shown; for each parameter the p-value is <0.05 and the increase is above 5%.

| Parameter | Overall increase |
| --- | --- |
| AreaMax | 8.5 |
| EmerVigor | 20.2 |
| RootMax | 11.3 |
| RootShInd | 5.8 |
| totalwgseeds | 27.9 |
| nrtotalseed | 19.7 |
| harvestindex | 18.8 |
| firstpan | 19.7 |
| nrfilledseed | 26.2 |
| GravityYMax | 6.9 |
| RootThickMax | 7.9 |

Example 12: Identification of sequences related to SEQ ID NO: 250 and SEQ ID NO: 251

Sequences (full length cDNA, ESTs or genomic) related to SEQ ID NO: 250 and SEQ ID NO: 251 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 250 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table F provides SEQ ID NO: 250 and SEQ ID NO: 251 and a list of nucleic acid sequences related to SEQ ID NO: 250 and SEQ ID NO: 251.

**Table F:**

| Name of gene from a plant source | Nucleic acid SEQ ID NO: | Protein SEQ ID NO: |
| --- | --- | --- |
| >O.sativa_LOC_Os03g51459.1 | 250 | 251 |
| >A.acetabulum_TA54_35845 | 252 | 253 |
| >A.capillus-veneris_BP912995 | 254 | 255 |
| >A.cepa_CF450030 | 256 | 257 |
| >A.hypogaea_EE126007 | 258 | 259 |
| >A.lyrata_490696 | 260 | 261 |

(continued)

| Name of gene from a plant source | Nucleic acid SEQ ID NO: | Protein SEQ ID NO: |
|---|---|---|
| >A.lyrata_899434 | 262 | 263 |
| >A.lyrata_925291 | 264 | 265 |
| >A.ostenfeldii_s_ao001019065r | 266 | 267 |
| >A.sativa_CN817242 | 268 | 269 |
| >A.stolonifera_DV867767 | 270 | 271 |
| >A.stolonifera_TA231_63632 | 272 | 273 |
| >A.thaliana_AT2G21640.1 | 274 | 275 |
| >A.thaliana_AT3G05570.1 | 276 | 277 |
| >A.thaliana_AT4G39235 | 278 | 279 |
| >A.trichopoda_CK760605 | 280 | 281 |
| >Aquilegia_sp_DR926960 | 282 | 283 |
| >B.distachyon_DV476865 | 284 | 285 |
| >B.napus_BN06MC17528_45500759 | 286 | 287 |
| B.napus_EV018535 | 288 | 289 |
| B.napus_TC83237 | 290 | 291 |
| B.napus_TC89650 | 292 | 293 |
| B.napus_TC90230 | 294 | 295 |
| B.napus_TC91334 | 296 | 297 |
| B.napus_TC97660 | 298 | 299 |
| B.pendula_AF124839 | 300 | 301 |
| B.pendula_X77601 | 302 | 303 |
| B.rapa_L37464 | 304 | 305 |
| C.annuum_GD122922 | 306 | 307 |
| C.annuum_TC18479 | 308 | 309 |
| C.annuum_TC26276 | 310 | 311 |
| C.canephora_DV681808 | 312 | 313 |
| C.canephora_DV695337 | 314 | 315 |
| C.cohnii_c_crco7281_t | 316 | 317 |
| C.intybus_TA3382_13427 | 318 | 319 |
| C.japonica_BP176677 | 320 | 321 |
| C.lanatus_DV737304 | 322 | 323 |
| C.maculosa_TA314_215693 | 324 | 325 |
| C.melo_DV634184 | 326 | 327 |
| C.obtusa_TA672_13415 | 328 | 329 |
| C.papaya_CP00062G01530 | 330 | 331 |
| C.persicum_AJ886827 | 332 | 333 |
| C.persicum_AJ887419 | 334 | 335 |
| C.richardii_BE641723 | 336 | 337 |

(continued)

| Name of gene from a plant source | Nucleic acid SEQ ID NO: | Protein SEQ ID NO: |
| --- | --- | --- |
| C.rumphii_CB089289 | 338 | 339 |
| C.rumphii_CB091964 | 340 | 341 |
| C.rumphii_TA1592_58031 | 342 | 343 |
| C.sativus_TA1216_3659 | 344 | 345 |
| C.sinensis_CB290321 | 346 | 347 |
| C.sinensis_EY668068 | 348 | 349 |
| C.sinensis_EY672891 | 350 | 351 |
| C.sinensis_EY727333 | 352 | 353 |
| C.solstitialis_EH773123 | 354 | 355 |
| C.solstitialis_EH786461 | 356 | 357 |
| C.solstitialis_TA4017_347529 | 358 | 359 |
| C.tetragonoloba_EG985118 | 360 | 361 |
| C.tinctorius_EL398763 | 362 | 363 |
| C.tinctorius_EL406768 | 364 | 365 |
| C.tinctorius_TA2172_4222 | 366 | 367 |
| D.sophia_BU238588 | 368 | 369 |
| E.esula_DV122995 | 370 | 371 |
| E.esula_DV150176 | 372 | 373 |
| E.gracilis_s_eg003003033r | 374 | 375 |
| E.gracilis_s_eg003043031r | 376 | 377 |
| E.grandis_CD668035 | 378 | 379 |
| E.lagascae_s_el01539_t | 380 | 381 |
| E.tereticornis_CD668461 | 382 | 383 |
| E.tereticornis_CD668945 | 384 | 385 |
| E.tirucalli_BP955531 | 386 | 387 |
| F.arundinacea_DT704699 | 388 | 389 |
| F.vesca_EX687128 | 390 | 391 |
| G.hirsutum_DR458244 | 392 | 393 |
| G.hirsutum_TC155046 | 394 | 395 |
| G.max_Glyma01g38260.1 | 396 | 397 |
| G.max_Glyma04g16400.1 | 398 | 399 |
| G.max_Glyma06g46580.1 | 400 | 401 |
| G.max_Glyma11g07320.1 | 402 | 403 |
| H.annuus_AJ318325 | 404 | 405 |
| H.annuus_DY919530 | 406 | 407 |
| H.annuus_DY923448 | 408 | 409 |
| H.annuus_TC50420 | 410 | 411 |
| H.argophyllus_EE609770 | 412 | 413 |

(continued)

| Name of gene from a plant source | Nucleic acid SEQ ID NO: | Protein SEQ ID NO: |
|---|---|---|
| H.argophyllus_EE616927 | 414 | 415 |
| H.centranthoides_CB088324 | 416 | 417 |
| H.exilis_EE660703 | 418 | 419 |
| H.tuberosus_EL467266 | 420 | 421 |
| H.vulgare_AJ473991 | 422 | 423 |
| H.vulgare_HV04MC01061_62590923 | 424 | 425 |
| H.vulgare_TC157023 | 426 | 427 |
| H.vulgare_TC173149 | 428 | 429 |
| H.vulgare_TC181924 | 430 | 431 |
| Helicosporidium_CX129083 | 432 | 433 |
| I.nil_TC8501 | 434 | 435 |
| L.chinensis_CN466203 | 436 | 437 |
| L.japonicus_BP063220 | 438 | 439 |
| L.japonicus_TC39016 | 440 | 441 |
| L.japonicus_TC42407 | 442 | 443 |
| L.saligna_DW062513 | 444 | 445 |
| L.saligna_DW070628 | 446 | 447 |
| L.saligna_TA4820_75948 | 448 | 449 |
| L.serriola_DW108864 | 450 | 451 |
| L.serriola_TC4935 | 452 | 453 |
| L.temulentum_TA732_34176 | 454 | 455 |
| L.usitatissimum_61837850.Ce04_2 | 456 | 457 |
| L.usitatissimum_c61576581 | 458 | 459 |
| L.usitatissimum_LU04MC07265_61837850 | 460 | 461 |
| L.usitatissimum_LU04MC10588_62332478 | 462 | 463 |
| L.virosa_DW169559 | 464 | 465 |
| L.virosa_DW172306 | 466 | 467 |
| M.acuminata_ES437129 | 468 | 469 |
| M.acuminata_TA641_4641 | 470 | 471 |
| M.crystallinum_BE034710 | 472 | 473 |
| M.crystallinum_BF479478 | 474 | 475 |
| M.domestica_TC40305 | 476 | 477 |
| M.esculenta_DV449414 | 478 | 479 |
| M.piperita_AW255467 | 480 | 481 |
| M.polymorpha_BJ867244 | 482 | 483 |
| M.sieboldii_CN915468 | 484 | 485 |
| M.truncatula_AC135566_19.5 | 486 | 487 |
| N.advena_CD474178 | 488 | 489 |

(continued)

| Name of gene from a plant source | Nucleic acid SEQ ID NO: | Protein SEQ ID NO: |
|---|---|---|
| N.benthamiana_TC16460 | 490 | 491 |
| N.nucifera_EH613403 | 492 | 493 |
| N.sylvestris_BP745520 | 494 | 495 |
| N.tabacum_TC51197 | 496 | 497 |
| N.tabacum_TC59871 | 498 | 499 |
| N.tabacum_TC76332 | 500 | 501 |
| Nicotiana_langsdorffii_x_sanderae_TA262_164110 | 502 | 503 |
| O.glaberrima_Og010279.01 | 504 | 505 |
| O.sativa_LOC_Os03g05700.1 | 506 | 507 |
| O.sativa_LOC-Os03g51470.1 | 508 | 509 |
| S.cereale_BQ160374 | 510 | 511 |
| O.sativa_LOC_Os08g10400.1 | 512 | 513 |
| O.sativa_LOC_Os10g40824.1 | 514 | 515 |
| P.americana_CV002279 | 516 | 517 |
| P.armeniaca_TA3954_36596 | 518 | 519 |
| P.axillaris_TA164_33119 | 520 | 521 |
| P.canadensis_CX183847 | 522 | 523 |
| P.cerasus_EE488481 | 524 | 525 |
| P.coccineus_CA911820 | 526 | 527 |
| P.dulcis_BU573498 | 528 | 529 |
| P.dulcis_BU574572 | 530 | 531 |
| P.equestris_CB033634 | 532 | 533 |
| P.euphratica_AJ773453 | 534 | 535 |
| P.ginseng_DV556099 | 536 | 537 |
| P.ginseng_DV556139 | 538 | 539 |
| P.glauca_C0479030 | 540 | 541 |
| P.glaucum_EB410943 | 542 | 543 |
| P.hybrida_TC1584 | 544 | 545 |
| P.menziesii_ES421929 | 546 | 547 |
| P.patens_TC54724 | 548 | 549 |
| P.pinaster_TA3303_71647 | 550 | 551 |
| P.sitchensis_TA13290_3332 | 552 | 553 |
| P.taeda_TA15984_3352 | 554 | 555 |
| P.tenuiflora_CN487508 | 556 | 557 |
| P.tremula_TA10333_113636 | 558 | 559 |
| P.tremula_TA7771_113636 | 560 | 561 |
| P.tremula_TA7772_113636 | 562 | 563 |
| P.trichocarpa_585933 | 564 | 565 |

(continued)

| Name of gene from a plant source | Nucleic acid SEQ ID NO: | Protein SEQ ID NO: |
|---|---|---|
| P.trichocarpa_643359 | 566 | 567 |
| P.trichocarpa_831254 | 568 | 569 |
| P.tricornutum_34756 | 570 | 571 |
| P.tricornutum_35319 | 572 | 573 |
| P.tricornutum_45377 | 574 | 575 |
| P.trifoliata_CX640083 | 576 | 577 |
| P.virgatum_FL844915 | 578 | 579 |
| P.virgatum_GD012331 | 580 | 581 |
| P.virgatum_TC15762 | 582 | 583 |
| P.virgatum_TC18672 | 584 | 585 |
| P.virgatum_TC19371 | 586 | 587 |
| P.virgatum_TC21333 | 588 | 589 |
| P.virgatum_TC35301 | 590 | 591 |
| P.virgatum_TC45394 | 592 | 593 |
| P.virgatum_TC49042 | 594 | 595 |
| P.vulgaris_TC16757 | 596 | 597 |
| P.wickerhamii_TA800_3111 | 598 | 599 |
| P.zeylanica_CB817807 | 600 | 601 |
| R.chinensis_BI978170 | 602 | 603 |
| R.chinensis_BI978786 | 604 | 605 |
| R.chinensis_TA261_74649 | 606 | 607 |
| R.hybrid_BQ104489 | 608 | 609 |
| S.alterniflora_EH277022 | 610 | 611 |
| S.alterniflora_EH277475 | 612 | 613 |
| S.bicolor_Sb01g009650.1 | 614 | 615 |
| S.bicolor_Sb01g009660.1 | 616 | 617 |
| S.bicolor_Sb01g029160.1 | 618 | 619 |
| S.bicolor_Sb01g035820.1 | 620 | 621 |
| S.henryi_DT591892 | 622 | 623 |
| S.hybrid_CF574777 | 624 | 625 |
| S.lycopersicum_DB694810 | 626 | 627 |
| S.lycopersicum_TC200119 | 628 | 629 |
| S.officinarum_CA100293 | 630 | 631 |
| S.officinarum_CA142694 | 632 | 633 |
| S.officinarum_CA202822 | 634 | 635 |
| S.officinarum_TC78965 | 636 | 637 |
| S.officinarum_TC81273 | 638 | 639 |
| S.salsa_AW982145 | 640 | 641 |

(continued)

| Name of gene from a plant source | Nucleic acid SEQ ID NO: | Protein SEQ ID NO: |
|---|---|---|
| S.tuberosum_TC181351 | 642 | 643 |
| T.aestivum_CA603660 | 644 | 645 |
| T.aestivum_CK151611 | 646 | 647 |
| T.aestivum_TA06MC03363_54495378 | 648 | 649 |
| T.aestivum_TA06MC09026_55236832 | 650 | 651 |
| T.aestivum_TC299614 | 652 | 653 |
| T.aestivum_TC300853 | 654 | 655 |
| T.aestivum_TC305290 | 656 | 657 |
| T.aestivum_TC307541 | 658 | 659 |
| T.aestivum_TC307720 | 660 | 661 |
| T.aestivum_TC311647 | 662 | 663 |
| T.aestivum_TC313329 | 664 | 665 |
| T.aestivum_TC335317 | 666 | 667 |
| T.androssowii_TA2143_189785 | 668 | 669 |
| T.erecta_SIN_01b-CS_Scarletade-9-C16.b1 | 670 | 671 |
| T.kok-saghyz_DR401105 | 672 | 673 |
| T.kok-saghyz_DR401469 | 674 | 675 |
| T.monococcum_BE492863 | 676 | 677 |
| T.monococcum_BQ802764 | 678 | 679 |
| T.officinale_DY838884 | 680 | 681 |
| T.officinale_DY839222 | 682 | 683 |
| T.officinale_TA1556_50225 | 684 | 685 |
| T.pseudonana_25031 | 686 | 687 |
| T.ruralis_CN200805 | 688 | 689 |
| T.versicolor_EY020157 | 690 | 691 |
| Triphysaria_sp_EY135750 | 692 | 693 |
| Triphysaria_sp_EY140347 | 694 | 695 |
| Triphysaria_sp_TC10995 | 696 | 697 |
| V.arizonica_x_rupestris_DN943112 | 698 | 699 |
| V.corymbosum_CV190443 | 700 | 701 |
| V.vinifera_GSVIVT00036379001 | 702 | 703 |
| Y.filamentosa_DT598701 | 704 | 705 |
| Z.aethiopica_TA1222_69721 | 706 | 707 |
| Z.elegans_AU288933 | 708 | 709 |
| Z.mays_AI649427 | 710 | 711 |
| Z.mays_FL334597 | 712 | 713 |
| Z.mays_TC477409 | 714 | 715 |
| Z.mays_TC479072 | 716 | 717 |

(continued)

| Name of gene from a plant source | Nucleic acid SEQ ID NO: | Protein SEQ ID NO: |
|---|---|---|
| Z.mays_TC486459 | 718 | 719 |
| Z.mays_TC487740 | 720 | 721 |
| Z.mays_TC490320 | 722 | 723 |
| Z.mays_TC502630 | 724 | 725 |
| Z.mays_TC506050 | 726 | 727 |
| Z.mays_TC514541 | 728 | 729 |
| Z.mays_TC522093 | 730 | 731 |
| Z.mays_TC522166 | 732 | 733 |
| Z.mays_TC535140 | 734 | 735 |
| Z.mays_ZM07MSbpsHQ_57582449.r01 | 736 | 737 |
| Z.officinale_TA5715_94328 | 738 | 739 |

Sequences have been tentatively assembled and publicly disclosed by research institutions, such as The Institute for Genomic Research (TIGR; beginning with TA). For instance, the Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, e.g. for certain prokaryotic organisms, such as by the Joint Genome Institute. Furthermore, access to proprietary databases, has allowed the identification of novel nucleic acid and polypeptide sequences.

Example 13: Alignment of GRP polypeptide sequences

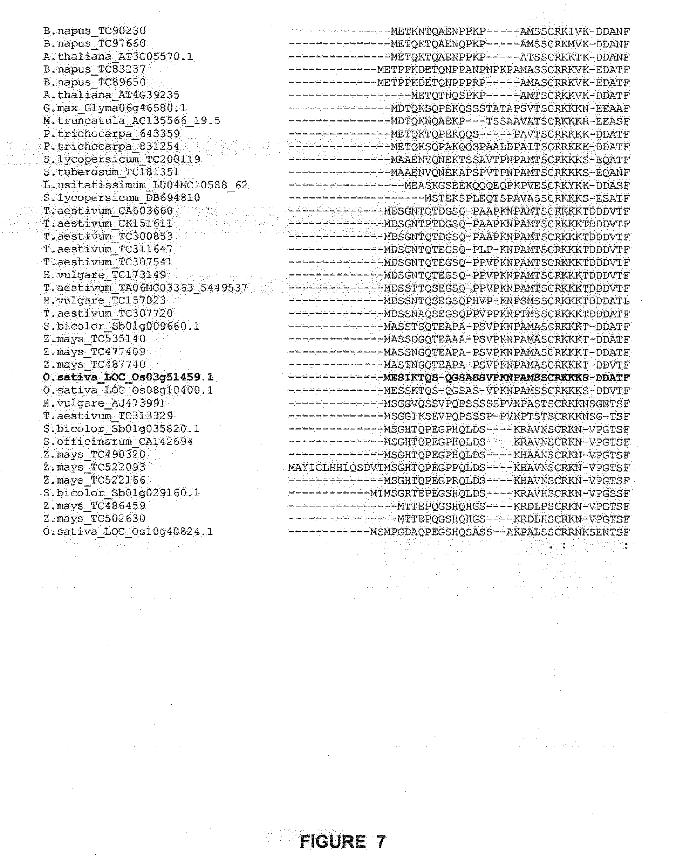

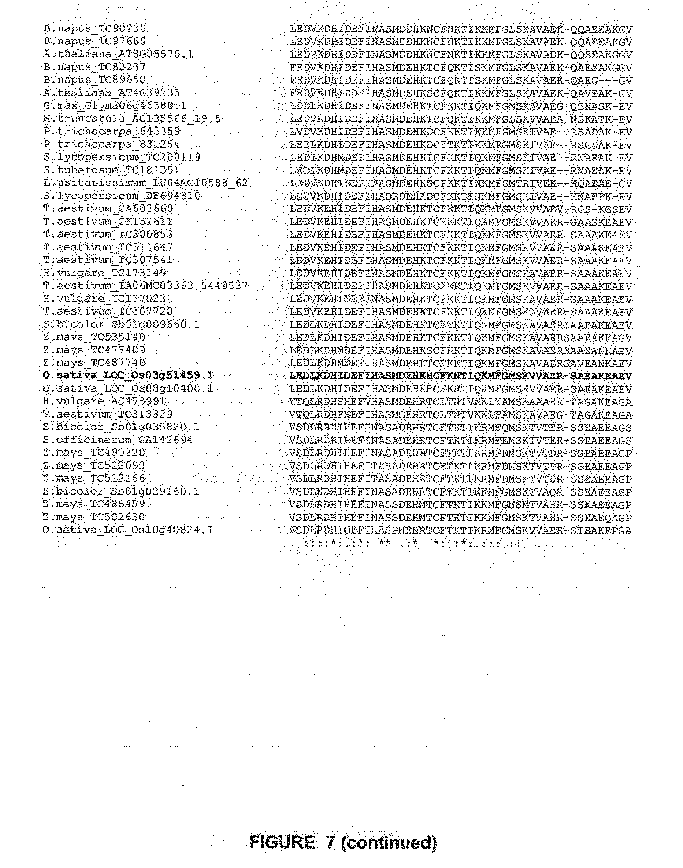

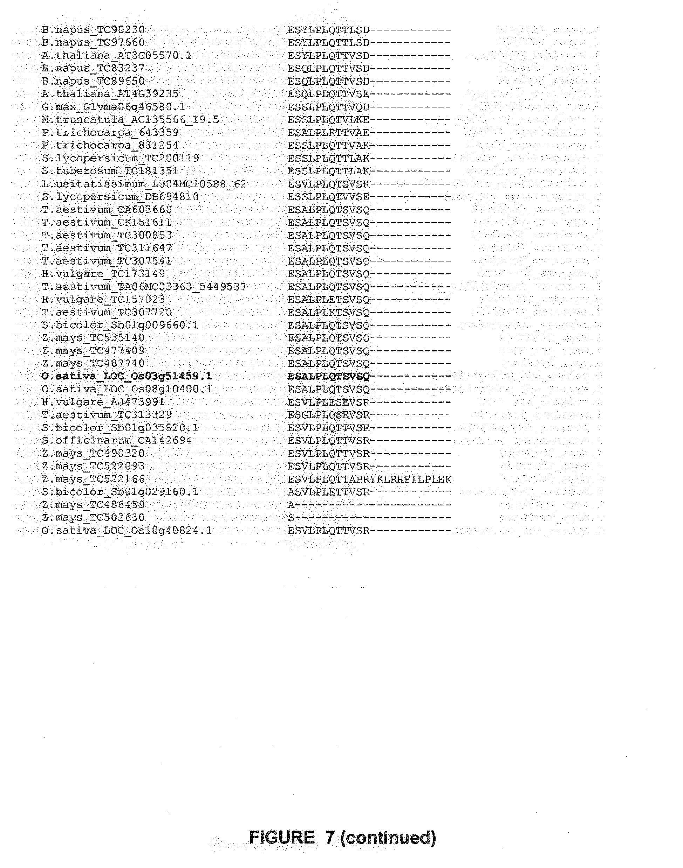

Alignment of a number of polypeptide sequences was performed using the ClustalW (1.8) algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment. These polypeptides are aligned in Figure 7.

Example 14: Calculation of global percentage identity between polypeptide sequences

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix.

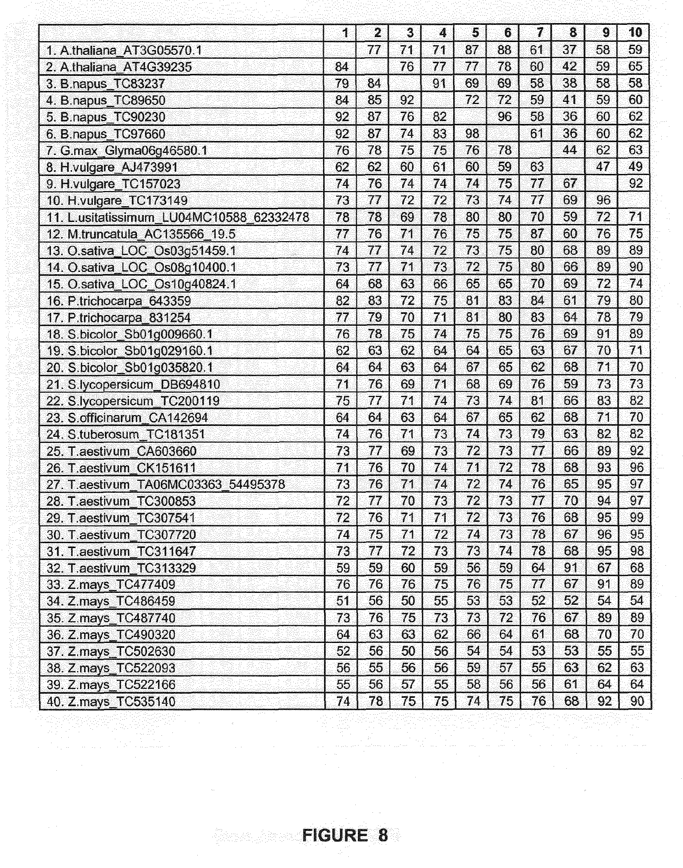

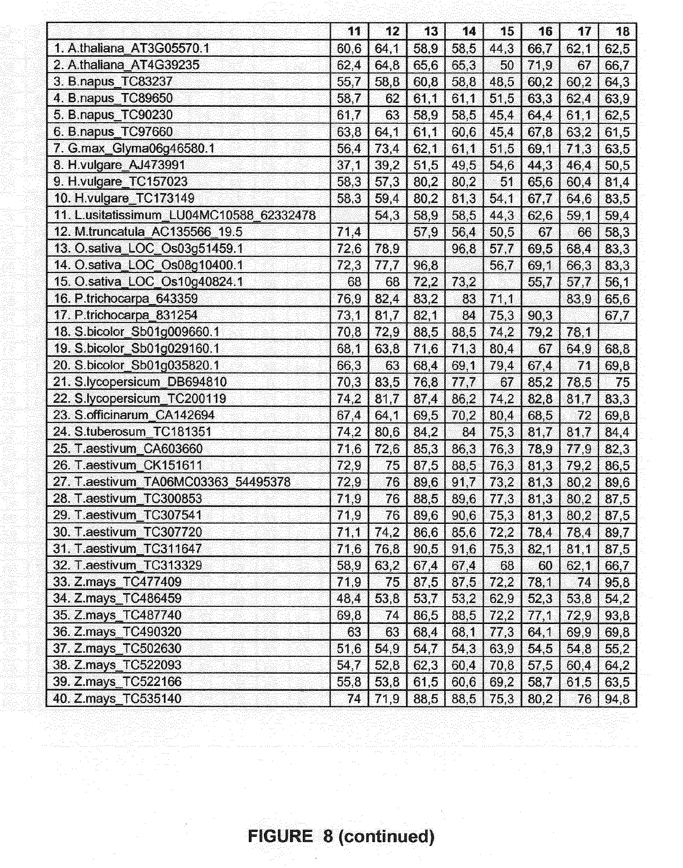

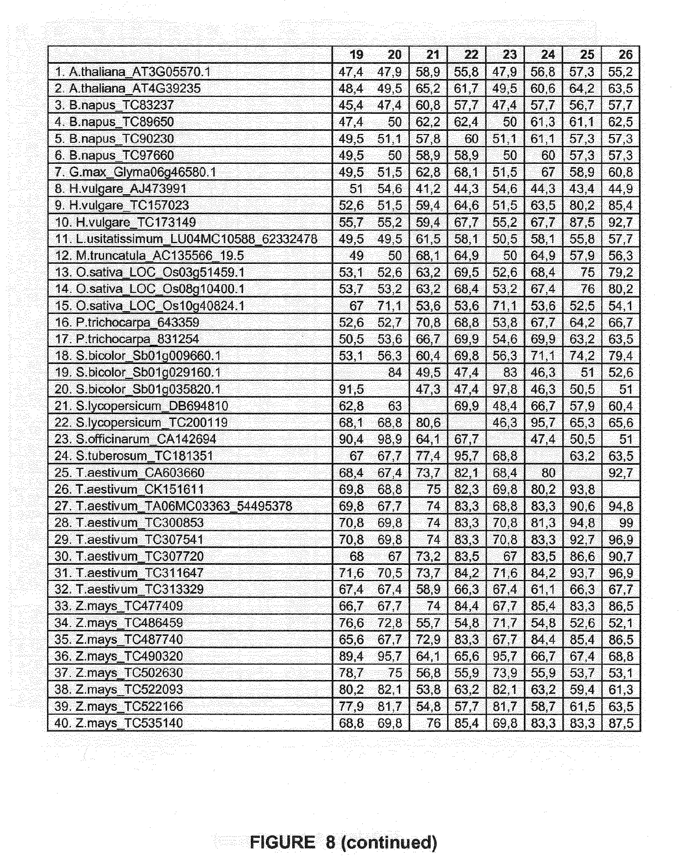

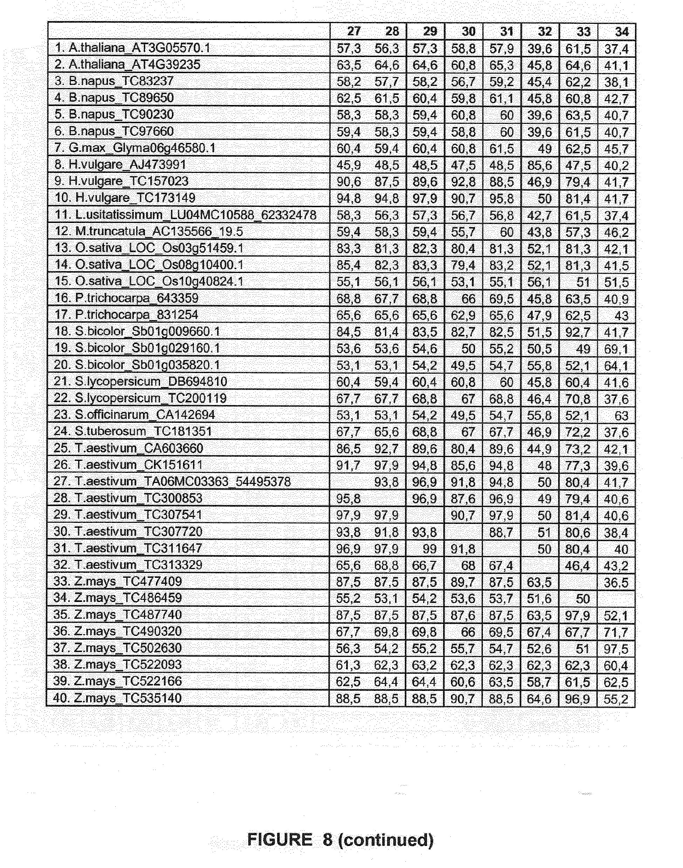

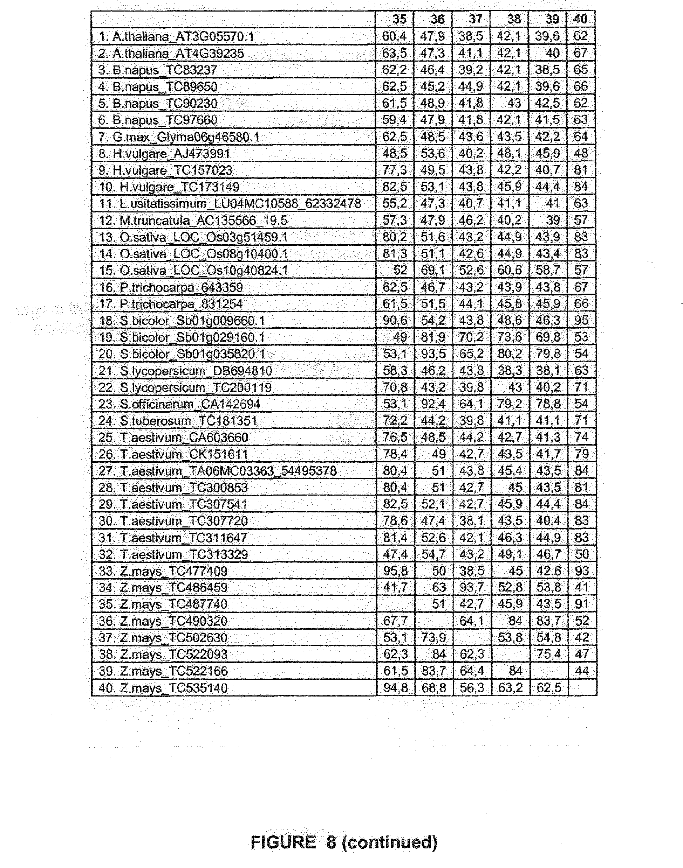

Results of the software analysis are shown in Figure 8 for the global similarity and identity over the full length of a representative list of polypeptide sequences. Sequence similarity is shown in the bottom half of the dividing line and sequence identity is shown in the top half of the diagonal dividing line. Parameters used in the comparison were: Scoring matrix: Blosum62, First Gap: 12, Extending Gap: 2. The sequence identity (in %) between the polypeptide sequences useful in performing the methods of the invention is generally higher than 20%) compared to SEQ ID NO: 251.

Example 15: Topology prediction of a polypeptide sequences according to the invention

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters can be selected, such as organism group (non-plant or plant), cutoff sets (none, predefined set of cutoffs, or user-specified set of cutoffs), and the calculation of prediction of cleavage sites (yes or no). In the present case, the "plant" organism group has been selected, no cutoffs defined, and the predicted length of the transit peptide requested. The results of TargetP 1.1 analysis of the polypeptide sequence as represented by SEQ ID NO: 251 are presented **Table G.**

**Table G:** TargetP 1.1 analysis of the polypeptide sequence as represented by SEQ ID NO: 2. Abbreviations: Len, Length; cTP, Chloroplastic transit peptide; mTP, Mitochondrial transit peptide, SP, Secretory pathway signal peptide, other, Other subcellular targeting, Loc, Predicted Location; RC, Reliability class; TPlen, Predicted transit peptide length.

| Name | Len | cTP | mTP | SP | other | Loc | RC | TPlen |
|---|---|---|---|---|---|---|---|---|
| Sequence | 95 | 0.549 | 0.095 | 0.116 | 0.519 | C | 5 | 23 |
| cutoff | | 0.000 | 0.000 | 0.000 | 0.000 | | | |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

Results of the PSORT algorithm given in **Table H** indicate for instance the following:

**Table H**

| chloroplast thylakoid space | Certainty= 0.950(Affirmative) < succ> |
|---|---|
| chloroplast stroma | Certainty= 0.727(Affirmative) < succ> |
| Chloroplast thylakoid membrane | Certainty= 0.504(Affirmative) < succ> |

Example 16: Cloning of a nucleic acid sequence encoding a GRP according to the invention

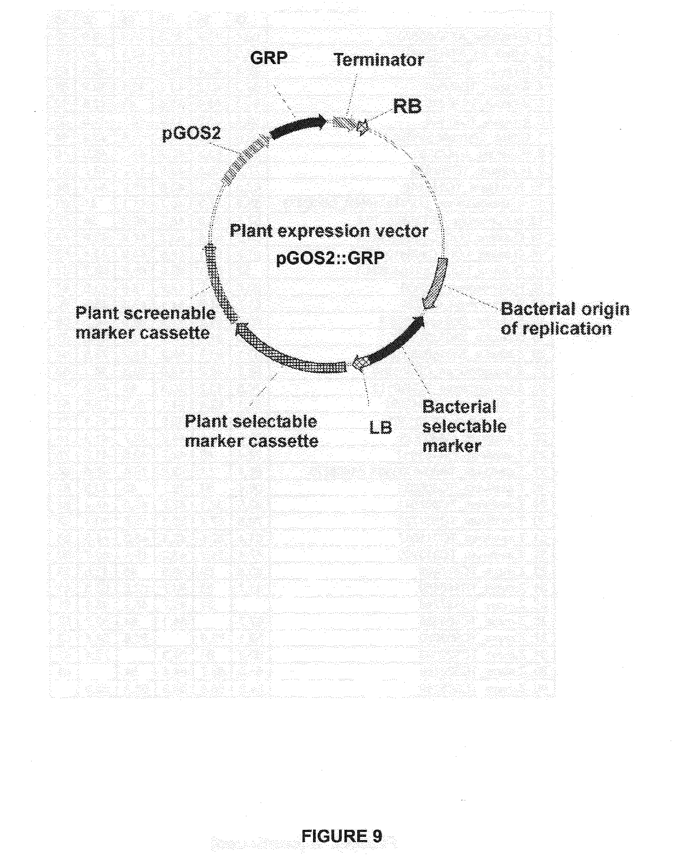

The nucleic acid sequence was amplified by PCR using as template a custom-made *Oryza sativa* seedlings cDNA library. PCR was performed using Hifi Taq DNA polymerase in standard conditions, using 200 ng of template in a 50 μl PCR mix. The primers used were prm14300 (SEQ ID NO: 740; sense): 5'-ggggacaagtttgtacaaaaaagcaggcttaaaca**atg**gaat ccattaagacacaa-3' ; and prm14301 (SEQ ID NO: 741; reverse, complementary): 5'-ggggaccactttgtacaagaaagctgggtcagaaggcaaacaagctg-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pGRP. Plasmid pDONR201 was purchased from Invitrogen, as part of the Gateway® technology. The entry clone comprising SEQ ID NO: 250 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 744) for constitutive specific expression was located upstream of this Gateway cassette. After the LR recombination step, the resulting expression vector pGOS2::GRP (Figure 9) was transformed into *Agrobacterium* strain LBA4044 according to methods well known in the art.

Example 17 Plant *transformation*

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Example 18: Transformation of other crops

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 $\mu$m acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 $\mu$g/ml cefotaxime. The seeds are then transferred to SH-medium with 50$\mu$g/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 $\mu$g/ml MgCL2, and with 50 to 100 $\mu$g/ml cefotaxime and 400-500 $\mu$g/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

*Sugarbeet transformation*

Seeds of sugarbeet (Beta vulgaris L.) are sterilized in 70% ethanol for one minute followed by 20 min. shaking in 20% Hypochlorite bleach e.g. Clorox® regular bleach (commercially available from Clorox, 1221 Broadway, Oakland, CA 94612, USA). Seeds are rinsed with sterile water and air dried followed by plating onto germinating medium (Murashige and Skoog (MS) based medium (Murashige, T., and Skoog, ., 1962. Physiol. Plant, vol. 15, 473-497) including B5 vitamins (Gamborg et al.; Exp. Cell Res., vol. 50, 151-8.) supplemented with 10 g/l sucrose and 0,8% agar). Hypocotyl tissue is used essentially for the initiation of shoot cultures according to Hussey and Hepher (Hussey, G., and Hepher, A., 1978. Annals of Botany, 42, 477-9) and are maintained on MS based medium supplemented with 30g/l sucrose plus 0,25mg/l benzylamino purine and 0,75% agar, pH 5,8 at 23-25°C with a 16-hour photoperiod. Agrobacterium tumefaciens strain carrying a binary plasmid harbouring a selectable marker gene, for example nptII, is used in transformation experiments. One day before transformation, a liquid LB culture including antibiotics is grown on a shaker (28°C, 150rpm) until an optical density (O.D.) at 600 nm of ~1 is reached. Overnight-grown bacterial cultures are centrifuged and resuspended in inoculation medium (O.D. ~1) including Acetosyringone, pH 5,5. Shoot base tissue is cut into slices (1.0 cm x 1.0 cm x 2.0 mm approximately). Tissue is immersed for 30s in liquid bacterial inoculation medium. Excess liquid is removed by filter paper blotting. Co-cultivation occurred for 24-72 hours on MS based medium incl. 30g/l sucrose followed by a non-selective period including MS based medium, 30g/l sucrose with 1 mg/l BAP to induce shoot development and cefotaxim for eliminating the Agrobacterium. After 3-10 days explants are transferred to similar selective medium harbouring for example kanamycin or G418 (50-100 mg/l genotype dependent). Tissues are transferred to fresh medium every 2-3 weeks to maintain selection pressure. The very rapid initiation of shoots (after 3-4 days) indicates regeneration of existing meristems rather than organogenesis of newly developed transgenic meristems. Small shoots are transferred after several rounds of subculture to root induction medium containing 5 mg/l NAA and kanamycin or G418. Additional steps are taken to reduce the potential of generating transformed plants that are chimeric (partially transgenic). Tissue samples from regenerated shoots are used for DNA analysis. Other transformation methods for sugarbeet are known in the art, for example those by Linsey & Gallois (Linsey, K., and Gallois, P., 1990. Journal of Experimental Botany; vol. 41, No. 226; 529-36) or the methods published in the international application published as WO9623891A.

*Sugarcane transformation*

Spindles are isolated from 6-month-old field grown sugarcane plants (see Arencibia et al., 1998. Transgenic Research, vol. 7, 213-22; Enriquez-Obregon et al., 1998. Planta, vol. 206, 20-27). Material is sterilized by immersion in a 20% Hypochlorite bleach e.g. Clorox® regular bleach (commercially available from Clorox, 1221 Broadway, Oakland, CA 94612, USA) for 20 minutes. Transverse sections around 0,5cm are placed on the medium in the top-up direction. Plant material is cultivated for 4 weeks on MS (Murashige, T., and Skoog, ., 1962. Physiol. Plant, vol. 15, 473-497) based medium incl. B5 vitamins (Gamborg, O., et al., 1968. Exp. Cell Res., vol. 50, 151-8) supplemented with 20g/l sucrose, 500 mg/l casein hydrolysate, 0,8% agar and 5mg/l 2,4-D at 23°C in the dark. Cultures are transferred after 4 weeks onto identical fresh medium. Agrobacterium tumefaciens strain carrying a binary plasmid harbouring a selectable marker gene, for example hpt, is used in transformation experiments. One day before transformation, a liquid LB culture including antibiotics is grown on a shaker (28°C, 150rpm) until an optical density (O.D.) at 600 nm of ~0,6 is reached. Overnight-grown bacterial cultures are centrifuged and resuspended in MS based inoculation medium (O.D. ~0,4) including acetosyringone, pH 5,5. Sugarcane embryogenic callus pieces (2-4 mm) are isolated based on morphological characteristics as compact structure and yellow colour and dried for 20 min. in the flow hood followed by immersion in a liquid bacterial inoculation medium for 10-20 minutes. Excess liquid is removed by filter paper blotting. Co-cultivation occurred for 3-5 days in the dark on filter paper which is placed on top of MS based medium incl. B5 vitamins containing 1 mg/l 2,4-D. After co-cultivation calli are washed with sterile water followed by a non-selective cultivation period on similar medium containing 500 mg/l cefotaxime for eliminating remaining Agrobacterium cells. After 3-10 days explants are transferred to MS based selective medium incl. B5 vitamins containing 1 mg/l 2,4-D for another 3 weeks harbouring 25 mg/l of hygromycin (genotype dependent). All treatments are made at 23°C under dark conditions. Resistant calli are further cultivated on medium lacking 2,4-D including 1 mg/l BA and 25 mg/l hygromycin under 16 h light photoperiod resulting in the development of shoot structures. Shoots are isolated and cultivated on selective rooting medium (MS based including, 20g/l sucrose, 20 mg/l hygromycin and 500 mg/l cefotaxime). Tissue samples from regenerated shoots are used for DNA analysis. Other transformation methods for sugarcane are known in the art, for example from the international application published as WO2010/151634A and the granted European patent EP1831378.

Example 19: Phenotypic evaluation procedure

*19.1 Evaluation setup*

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development.

From the stage of sowing until the stage of maturity the plants were passed several times through a digital imaging cabinet. At each time point digital images (2048x1536 pixels, 16 million colours) were taken of each plant from at least 6 different angles.

T1 events can be further evaluated in the T2 generation following the same evaluation procedure as for the T1 generation, e.g. with less events and/or with more individuals per event.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

*19.2 Statistical analysis: F test*

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

*19.3 Parameters measured*

From the stage of sowing until the stage of maturity the plants were passed several times through a digital imaging cabinet. At each time point digital images (2048x1536 pixels, 16 million colours) were taken of each plant from at least 6 different angles as described in WO2010/031780. These measurements were used to determine different parameters.

*Biomass-related parameter measurement*

The plant aboveground area, also named leafy biomass, was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

Increase in root biomass can be expressed as an increase in total root biomass (measured as maximum biomass of roots observed during the lifespan of a plant); or as an increase in the root/shoot index (measured as the ratio between root mass and shoot mass in the period of active growth of root and shoot). Root biomass can be determined using a method as described in WO 2006/029987.

*Parameters related to development time*

The early vigour is the plant (seedling) aboveground area three weeks post-germination. Early vigour was determined by counting the total number of pixels from aboveground plant parts discriminated from the background. This value was averaged for the pictures taken on the same time point from different angles and was converted to a physical surface value expressed in square mm by calibration.

The "flowering time" of a plant can be determined using the method as described in WO 2007/093444.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

Example 20: Results of the phenotypic evaluation of the transgenic plants

The results of the evaluation of transgenic rice plants in the T1 generation and expressing a nucleic acid encoding the polypeptide of SEQ ID NO: 251 under non-stress conditions are presented below in **Table I.** When grown under non-stress conditions, an increase of 5% or more was observed for parameters related to increased aboveground biomass.

In addition, when grown under non-stress conditions, an increase of 5% or more was also observed for parameters related to seed yield including total weight of the seeds, thousand kernel weight (TKW), number of filled seeds, number of flowers per panicle, and harvest index.

**Table I:** Data summary for transgenic rice plants; for each parameter, the overall percent increase is shown for the T1 generation of plants, for each parameter the p-value is <0.05.

| Parameter | Overall increase |
|---|---|
| totalwgseeds | 19.1 |

(continued)

| Parameter | Overall increase |
|---|---|
| harvestindex | 17.8 |
| nrfilledseed | 15.0 |
| flowerperpan | 17.1 |
| TKW | 5.0 |
| HeightMax | 7.6 |
| GravityYMax | 6.8 |

Example 21: Identification of sequences related to SEQ ID NO: 746 and SEQ ID NO: 747

Sequences (full length cDNA, ESTs or genomic) related to SEQ ID NO: 746 and SEQ ID NO: 747 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 746 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table J refers to SEQ ID NO: 746 and SEQ ID NO: 747 and provides a list of nucleic acid sequences related to SEQ ID NO: 746 and SEQ ID NO: 747.

**Table J:** Examples of ZPR nucleic acids and polypeptides

| Plant Source | Nucleic acid SEQ ID NO: | Protein SEQ ID NO: |
|---|---|---|
| S.lycopersicum_TC205388#1 | 746 | 747 |
| Pt_ZPR_small_Lzipper | 748 | 749 |
| A.hypogaea_EG373732#1 | 750 | 751 |
| A.lyrata_322199#1 | 752 | 753 |
| A.lyrata_899056#1 | 754 | 755 |
| A.thaliana_AT2G45450.1 | 756 | 757 |
| A.thaliana_AT3G60890.1#1 | 758 | 759 |
| A.trichopoda_CK765457#1 | 760 | 761 |
| B.napus_CD822359#1 | 762 | 763 |
| C.annuum_TC13916#1 | 764 | 765 |
| C.intybus_EH709619#1 | 766 | 767 |
| C.melo_EB715099#1 | 768 | 769 |
| C.sinensis_TC13047#1 | 770 | 771 |
| C.tinctorius_EL400918#1 | 772 | 773 |
| E.tef_DN482990#1 | 774 | 775 |
| E.tirucalli_BP956571#1 | 776 | 777 |

(continued)

| Plant Source | Nucleic acid SEQ ID NO: | Protein SEQ ID NO: |
|---|---|---|
| G.hirsutum_DW501389#1 | 778 | 779 |
| G.hirsutum_DW518216#1 | 780 | 781 |
| G.hirsutum_TC163217#1 | 782 | 783 |
| G.hybrid_AJ765000#1 | 784 | 785 |
| G.max_Glyma01g34620.1#1 | 786 | 787 |
| G.max_Glyma09g40450.1#1 | 788 | 789 |
| G.max_GM06MC39606_50673886@38431#1 | 790 | 791 |
| G.max_GM06MSsu40h07.r_46819511@70598#1 | 792 | 793 |
| G.max_TC281725#1 | 794 | 795 |
| G.max_TC286014#1 | 796 | 797 |
| G.max_TC305219#1 | 798 | 799 |
| L.perennis_TA2167_43195#1 | 800 | 801 |
| L.sativa_TC25720#1 | 802 | 803 |
| L.serriola_DW114794#1 | 804 | 805 |
| L.serriola_DW122307#1 | 806 | 807 |
| L.virosa_DW156764#1 | 808 | 809 |
| M.domestica_TC51427#1 | 810 | 811 |
| N.tabacum_TC60414#1 | 812 | 813 |
| O.sativa_LOC_Os09g34890.1#1 | 814 | 815 |
| Os_ZPRa_Os09g0520500 | 816 | 817 |
| P.abies_AM172974#1 | 818 | 819 |
| P.deltoides_TA3399_3696#1 | 820 | 821 |
| P.sitchensis_TA14065_3332#1 | 822 | 823 |
| P.tremula_TA8085_113636#1 | 824 | 825 |
| P.trichocarpa_554264#1 | 826 | 827 |
| P.trichocarpa_823907#1 | 828 | 829 |
| P.vulgaris_FE900059#1 | 830 | 831 |
| R.chinensis_BI978399#1 | 832 | 833 |
| S.bicolor_Sb02g030180.1#1 | 834 | 835 |
| S.hybrid_CF573336#1 | 836 | 837 |
| S.hybrid_CF575621#1 | 838 | 839 |
| S.lycopersicum_TC207895#1 | 840 | 841 |
| S.propinquum_TA5427_132711#1 | 842 | 843 |
| S.tuberosum_EG011112#1 | 844 | 845 |
| S.tuberosum_TC176950#1 | 846 | 847 |
| T.cacao_CU522069#1 | 848 | 849 |
| T.cacao_TC3649#1 | 850 | 851 |
| T.cryptomerioides_DN975845#1 | 852 | 853 |

(continued)

| Plant Source | Nucleic acid SEQ ID NO: | Protein SEQ ID NO: |
| --- | --- | --- |
| Triphysaria_sp_TC4136#1 | 854 | 855 |
| V.vinifera_GSVIVT00001026001#1 | 856 | 857 |
| V.vinifera_GSVIVT00026366001#1 | 858 | 859 |
| Z.mays_BG842570#1 | 860 | 861 |
| Z.mays_c62174110gm030403@15401#1 | 862 | 863 |
| Z.mays_c62219732gm030403@9841#1 | 864 | 865 |
| Z.mays_TC477977#1 | 866 | 867 |
| Z.mays_TC529546#1 | 868 | 869 |
| Z.mays_ZM07MC28025_BFb0123H02@27941#1 | 870 | 871 |
| Zeama_ZmPC0129214_ZPRB | 872 | 873 |
| A.thaliana_AT2G36307.1 | 874 | 875 |
| A.thaliana_AT3G52770.1 | 876 | 877 |
| B.napus_BN06MC24768_49494820@24676#1 | 878 | 879 |
| H.annuus_CD854488#1 | 880 | 881 |
| H.vulgare_TC169924#1 | 882 | 883 |
| L.perennis_DW077975#1 | 884 | 885 |
| M.truncatula_AC154034_39.5#1 | 886 | 887 |
| N.tabacum_AM841801#1 | 888 | 889 |
| O.sativa_LOC_Os04g33560.1#1 | 890 | 891 |
| Os_ZPRc_Os02g0530500 | 892 | 893 |
| Os_ZPRd_Os12g05560 | 894 | 895 |
| Os_ZPRe_Os11g05430 | 896 | 897 |
| P.trichocarpa_548562#1 | 898 | 899 |
| P.trichocarpa_560740#1 | 900 | 901 |
| Pt_ZPR3 | 902 | 903 |
| R.hybrid_EC587934#1 | 904 | 905 |
| S.bicolor_Sb04g021620.1#1 | 906 | 907 |
| S.bicolor_Sb06g015480.1#1 | 908 | 909 |
| V.vinifera_GSVIVT00021631001#1 | 910 | 911 |
| Z.mays_c62080453gm030403@7498#1 | 912 | 913 |
| Z.mays_TC487620#1 | 914 | 915 |
| Z.mays_TC492042#1 | 916 | 917 |
| Z.mays_ZM07MC37474_BFb0118006@37348#1 | 918 | 919 |
| Zea_mays_AC217051.3_FGT005#1 | 920 | 921 |
| Zea_mays_GRMZM2G062504_T01#1 | 922 | 923 |
| Zeama_Zmcl15605_1_ZPRA | 924 | 925 |

Sequences have been tentatively assembled and publicly disclosed by research institutions, such as The Institute for Genomic Research (TIGR; beginning with TA). For instance, the Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, e.g. for certain prokaryotic organisms, such as by the Joint Genome Institute. Furthermore, access to proprietary databases, has allowed the identification of novel nucleic acid and polypeptide sequences.

Example 22: Alignment of ZPR polypeptide sequences

Alignment of polypeptide sequences was performed using MAFFT (version 6.624, L-INS-I method - Katoh and Toh (2008) - Briefings in Bioinformatics 9:286-298). Minor manual editing was done to further optimize the alignment. A representative number of ZPR polypeptides are aligned in Figure 13. The represented ZPR polypeptides are characterized in that the Leucine Zipper domain is located in the central to C-terminal region of said ZPR polypeptide.

Example 23: Calculation of global percentage identity between polypeptide sequences

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix.

Results of the analysis are shown in Figure 14 for the global similarity and identity over the full length of a representative number of polypeptide sequences. Sequence similarity is shown in the bottom half of the dividing line and sequence identity is shown in the top half of the diagonal dividing line. Parameters used in the comparison were: Scoring matrix: Blosum62, First Gap: 12, Extending Gap: 2. The sequence identity (in %) between the ZPR polypeptide sequences useful in performing the methods of the invention is generally higher than 20% compared to SEQ ID NO: 747.

Example 24: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

Example 25: Topology prediction of the ZPR polypeptide sequences

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters can be selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:

• ChloroP 1.1 hosted on the server of the Technical University of Denmark;
• Protein Prowler Subcellular Localisation Predictor version 1.2 hosted on the server of the Institute for Molecular Bioscience, University of Queensland, Brisbane, Australia;

- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

Example 26: Functional assay for a ZPR polypeptide

LITTLE ZIPPER (ZPR) genes have HD-ZIPIII-suppressing activity and are transcriptionally upregulated by HD-ZIPIII activity (see Wenkel et al. (2007). Wenkel et al. (2007) described that the structural features of ZPR proteins, and their induction by HD-ZIPIII activity, suggest a model for the action of the ZPR genes as part of a negative feedback loop on HD-ZIPIII function. In the regulatory module proposed by Wenkel et al. (2007), HD-ZIPIII proteins activate transcription of the ZPR genes. The ZPR proteins then form heterodimers with the HD-ZIPIII proteins, preventing or altering their DNA binding.

Kim et al. (2008) demonstrated that a small ZIP protein, ZPR3, and its functionally redundant homolog, ZPR4, negatively regulate the HD-ZIP III activity in Shoot apical meristem (SAM) development. ZPR3 directly interacts with PHABULOSA (PHB) and other HD-ZIP III proteins via the ZIP motifs and forms nonfunctional heterodimers. In view thereof, Kim et al. (2008) proposed that HD-ZIPIII activity in regulating SAM is modulated by, among other things, a feedback loop involving the competitive inhibitors ZPR3 and ZPR4.

Example 27: Cloning of the ZPR encoding nucleic acid sequence

The nucleic acid sequence was amplified by PCR using as template a custom-made *Solanum lycopersicum* seedlings cDNA library. PCR was performed using a commercially available proofreading Taq DNA polymerase in standard conditions, using 200 ng of template in a 50 $\mu$l PCR mix. The primers used were prm15860 (SEQ ID NO: 926; sense): prm15860: 5' ggggacaagtttgtacaaaaaagcaggcttaaaca**atg**tgtcatgcaatttcagatcat 3' and prm15859 (SEQ ID NO: 927; reverse, complementary): 5'-ggggaccactttgtacaaga aagctgggtctaattgtgattattagagggttgagaa 3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pZPR. Plasmid pDONR201 was purchased from Invitrogen, as part of the Gateway® technology.

The entry clone comprising SEQ ID NO: 746 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 928) for constitutive expression was located upstream of this Gateway cassette.

After the LR recombination step, the resulting expression vector pGOS2::ZPR (Figure 15) was transformed into *Agrobacterium* strain LBA4044 according to methods well known in the art.

Example 28: Plant transformation

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% HgCl$_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD$_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Example 29: Transformation of other crops

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 $\mu$m acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 $\mu$g/ml cefotaxime. The seeds are then transferred to SH-medium with 50$\mu$g/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 $\mu$g/ml MgCL2, and with 50 to 100 $\mu$g/ml cefotaxime and 400-500 $\mu$g/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

*Sugarbeet transformation*

Seeds of sugarbeet (Beta vulgaris L.) are sterilized in 70% ethanol for one minute followed by 20 min. shaking in 20% Hypochlorite bleach e.g. Clorox® regular bleach (commercially available from Clorox, 1221 Broadway, Oakland, CA 94612, USA). Seeds are rinsed with sterile water and air dried followed by plating onto germinating medium (Murashige and Skoog (MS) based medium (Murashige, T., and Skoog, ., 1962. Physiol. Plant, vol. 15, 473-497) including B5 vitamins (Gamborg et al.; Exp. Cell Res., vol. 50, 151-8.) supplemented with 10 g/l sucrose and 0,8% agar). Hypocotyl tissue is used essentially for the initiation of shoot cultures according to Hussey and Hepher (Hussey, G., and Hepher, A., 1978. Annals of Botany, 42, 477-9) and are maintained on MS based medium supplemented with 30g/l sucrose plus 0,25mg/l benzylamino purine and 0,75% agar, pH 5,8 at 23-25°C with a 16-hour photoperiod. Agrobacterium tumefaciens strain carrying a binary plasmid harbouring a selectable marker gene, for example nptII, is used in transformation experiments. One day before transformation, a liquid LB culture including antibiotics is grown on a shaker (28°C, 150rpm) until an optical density (O.D.) at 600 nm of ~1 is reached. Overnight-grown bacterial cultures are centrifuged and resuspended in inoculation medium (O.D. ~1) including Acetosyringone, pH 5,5. Shoot base tissue is cut into slices (1.0 cm x 1.0 cm x 2.0 mm approximately). Tissue is immersed for 30s in liquid bacterial inoculation medium. Excess liquid is removed by filter paper blotting. Co-cultivation occurred for 24-72 hours on MS based medium incl. 30g/l sucrose followed by a non-selective period including MS based medium, 30g/l sucrose with 1 mg/l BAP to induce shoot development and cefotaxim for eliminating the Agrobacterium. After 3-10 days explants are transferred to similar selective medium harbouring for example kanamycin or G418 (50-100 mg/l genotype dependent). Tissues are transferred to fresh medium every 2-3 weeks to maintain selection pressure. The very rapid initiation of shoots (after 3-4 days) indicates regeneration of existing meristems rather than organogenesis of newly developed transgenic meristems. Small shoots are transferred after several rounds of subculture to root induction medium containing 5 mg/l NAA and kanamycin or G418. Additional steps are taken to reduce the potential of generating transformed plants that are chimeric (partially transgenic). Tissue samples from regenerated shoots are used for DNA analysis. Other transformation methods for sugarbeet are known in the art, for example those by Linsey & Gallois (Linsey, K., and Gallois, P., 1990. Journal of Experimental Botany; vol. 41, No. 226; 529-36) or the methods published in the international application published as WO9623891A.

*Sugarcane transformation*

Spindles are isolated from 6-month-old field grown sugarcane plants (see Arencibia et al., 1998. Transgenic Research, vol. 7, 213-22; Enriquez-Obregon et al., 1998. Planta, vol. 206, 20-27). Material is sterilized by immersion in a 20% Hypochlorite bleach e.g. Clorox® regular bleach (commercially available from Clorox, 1221 Broadway, Oakland, CA 94612, USA) for 20 minutes. Transverse sections around 0,5cm are placed on the medium in the top-up direction. Plant material is cultivated for 4 weeks on MS (Murashige, T., and Skoog, ., 1962. Physiol. Plant, vol. 15, 473-497) based medium incl. B5 vitamins (Gamborg, O., et al., 1968. Exp. Cell Res., vol. 50, 151-8) supplemented with 20g/l sucrose, 500 mg/l casein hydrolysate, 0,8% agar and 5mg/l 2,4-D at 23°C in the dark. Cultures are transferred after 4 weeks onto identical fresh medium. Agrobacterium tumefaciens strain carrying a binary plasmid harbouring a selectable marker gene, for example hpt, is used in transformation experiments. One day before transformation, a liquid LB culture including antibiotics is grown on a shaker (28°C, 150rpm) until an optical density (O.D.) at 600 nm of ~0,6 is reached. Overnight-grown bacterial cultures are centrifuged and resuspended in MS based inoculation medium (O.D. ~0,4) including acetosyringone, pH 5,5. Sugarcane embryogenic callus pieces (2-4 mm) are isolated based on morphological characteristics as compact structure and yellow colour and dried for 20 min. in the flow hood followed by immersion in a liquid bacterial inoculation medium for 10-20 minutes. Excess liquid is removed by filter paper blotting. Co-cultivation occurred for 3-5 days in the dark on filter paper which is placed on top of MS based medium incl. B5 vitamins containing 1 mg/l 2,4-D. After co-cultivation calli are washed with sterile water followed by a non-selective cultivation period on similar medium containing 500 mg/l cefotaxime for eliminating remaining Agrobacterium cells. After 3-10 days explants are transferred to MS based selective medium incl. B5 vitamins containing 1 mg/l 2,4-D for another 3 weeks harbouring 25 mg/l of hygromycin (genotype dependent). All treatments are made at 23°C under dark conditions. Resistant calli are further cultivated on medium lacking 2,4-D including 1 mg/l BA and 25 mg/l hygromycin under 16 h light photoperiod resulting in the development of shoot structures. Shoots are isolated and cultivated on selective rooting medium (MS based including, 20g/l sucrose, 20 mg/l hygromycin and 500 mg/l cefotaxime). Tissue samples from regenerated shoots are used for DNA analysis. Other transformation methods for sugarcane are known in the art, for example from the international application published as WO2010/151634A and the granted European patent EP1831378.

Example 30: Phenotypic evaluation procedure

*30.1 Evaluation setup*

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development, unless they were used in a stress screen.

From the stage of sowing until the stage of maturity the plants were passed several times through a digital imaging cabinet. At each time point digital images (2048x1536 pixels, 16 million colours) were taken of each plant from at least 6 different angles.

T1 events can be further evaluated in the T2 generation following the same evaluation procedure as for the T1 generation, e.g. with less events and/or with more individuals per event.

*Drought screen*

T1 or T2 plants are grown in potting soil under normal conditions until they approached the heading stage. They are then transferred to a "dry" section where irrigation is withheld. Soil moisture probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

T1 or T2 plants are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

T1 or T2 plants are grown on a substrate made of coco fibers and particles of baked clay (Argex) (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Growth and yield parameters are recorded as detailed for growth under normal conditions.

*30.2 Statistical analysis: F test*

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

*30.3 Parameters measured*

From the stage of sowing until the stage of maturity the plants were passed several times through a digital imaging cabinet. At each time point digital images (2048x1536 pixels, 16 million colours) were taken of each plant from at least 6 different angles as described in WO2010/031780. These measurements were used to determine different parameters.

*Biomass-related parameter measurement*

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

Increase in root biomass is expressed as an increase in total root biomass (measured as maximum biomass of roots observed during the lifespan of a plant); or as an increase in the root/shoot index, measured as the ratio between root mass and shoot mass in the period of active growth of root and shoot. In other words, the root/shoot index is defined as the ratio of the rapidity of root growth to the rapidity of shoot growth in the period of active growth of root and shoot. Root biomass can be determined using a method as described in WO 2006/029987.

*Parameters related to development time*

The early vigour is the plant aboveground area three weeks post-germination. Early vigour was determined by counting the total number of pixels from aboveground plant parts discriminated from the background. This value was averaged for the pictures taken on the same time point from different angles and was converted to a physical surface value expressed in square mm by calibration.

AreaEmer is an indication of quick early development when this value is decreased compared to control plants. It is the ratio (expressed in %) between the time a plant needs to make 30 % of the final biomass and the time needs to make 90 % of its final biomass.

The "time to flower" or "flowering time" of the plant can be determined using the method as described in WO 2007/093444.

*Seed-related parameter measurements*

The mature primary panicles were harvested, counted, bagged, barcode-labelled and then dried for three days in an oven at 37°C. The panicles were then threshed and all the seeds were collected and counted. The seeds are usually covered by a dry outer covering, the husk. The filled husks (herein also named filled florets) were separated from the empty ones using an air-blowing device. The empty husks were discarded and the remaining fraction was counted again. The filled husks were weighed on an analytical balance.

The total number of seeds was determined by counting the number of filled husks that remained after the separation step. The total seed weight was measured by weighing all filled husks harvested from a plant.

The total number of seeds (or florets) per plant was determined by counting the number of husks (whether filled or not) harvested from a plant.

Thousand Kernel Weight (TKW) is extrapolated from the number of seeds counted and their total weight.

The Harvest Index (HI) in the present invention is defined as the ratio between the total seed weight and the above ground area ($mm^2$), multiplied by a factor $10^6$.

The number of flowers per panicle as defined in the present invention is the ratio between the total number of seeds over the number of mature primary panicles.

The "seed fill rate" or "seed filling rate" as defined in the present invention is the proportion (expressed as a %) of the number of filled seeds (i.e. florets containing seeds) over the total number of seeds (i.e. total number of florets). In other words, the seed filling rate is the percentage of florets that are filled with seed.

Example 31: Results of the phenotypic evaluation of the transgenic plants

The results of the evaluation of transgenic rice plants in the T1 generation and expressing a nucleic acid encoding the ZPR polypeptide of SEQ ID NO: 747 under non-stress conditions are presented below in Table C. When grown under non-stress conditions, an increase of at least 5 % was observed for seed yield, including total weight of seeds, fill rate, number of filled seeds, harvest index, and number of flowers per panicle.

**Table K**: Data summary for transgenic rice plants; for each parameter, the overall percent increase is shown for the confirmation (T2 generation), for each parameter the p-value is <0.05.

| Parameter | Overall increase |
| --- | --- |
| totalwgseeds | 21.5 |
| fillrate | 21.1 |
| harvestindex | 18.4 |
| nrfilledseed | 19.8 |
| flowerperpan | 5.7 |

**Claims**

1. A method for enhancing yield-related traits in plants relative to control plants, comprising modulating expression in a plant of a nucleic acid selected from the group consisting of:

   a. a nucleic acid encoding a growth-related protein (GRP) having at least 25% sequence identity to the amino acid sequence represented by SEQ ID NO: 251; or

b. a nucleic acid encoding a ZPR polypeptide, wherein said ZPR polypeptide comprises a Leucine Zipper (ZIP) domain that is six heptads in length.

**2.** Method according to claim 1 (a), wherein said GRP has a motif having at least 70% sequence identity to motif 7: [NHPS][SC][CR][RK]K[NK]    [VST][PD][GD][TAV][ST]F[VL][SE]DL[RK]DH[IM][HD]EFI[NH]AS[ASM]DEH[MRK][TH]CF[TK][KN]T[IL][KQ][KR]MF[GD]MS[KM][TAV]V[AT] (SEQ ID NO: 742); or

a method according claim 1(b), wherein said ZPR polypeptide comprises one or more of the following motifs:

(i) Motif 8: II[EK]ENE[KR]LR[KE][KR]A[LS][LA]L[HR][QR]EN (SEQ ID NO: 929),
(ii) Motif 9: [EI]M[EK][MI]KNLKLY[EML]EN[KQ][SCI] (SEQ ID NO: 930),
(iii) Motif 10: [QKL][AD]L[LF][ST][QE][LI][QI][KQ][KQ][LI]SxPx (SEQ ID NO: 931);
and/or preferably wherein said Leucine Zipper domain is located in the central to C-terminal region of said ZPR polypeptide; and/or preferably wherein said ZPR polypeptide further comprises one or more of the following motifs:
(iv) Motif 11:

K[VL][KA]K[EI]M[EK]MKNLKLY[MEL]EN[QKR][SIC]I[IL]EENE[KR]LR[KE]
[KRQ]A[LS][LA]L[HR][QR]EN[LKQ][AD]L[LF][STQ][QI][LI][QI][KQN][KQ][IF]S (SEQ ID NO: 932),

(v) Motif 12: MC[HS][AG][SI]Sx[HS][LS][ES]S (SEQ ID NO: 933),
(vi) Motif 13: [VI][HS][RV]L[NK][LR]RR (SEQ ID NO: 934).

**3.** Method according to any preceding claim, wherein said modulated expression is effected by introducing and expressing said nucleic acid encoding a GRP in a plant; or
wherein said modulated expression is effected by introducing and expressing in a plant said nucleic acid encoding said ZPR polypeptide.

**4.** Method according to any preceding claim, wherein said nucleic acid encoding a GRP encodes any one of the polypeptides listed in Table F or is a portion of such a nucleic acid, or a nucleic acid capable of hybridising with such a nucleic acid or wherein said nucleic acid encoding a GRP encodes an orthologue or paralogue of any of the polypeptides given in Table F; or
wherein said nucleic acid encoding a ZPR polypeptide encodes any one of the polypeptides listed in Table J or is a portion of such a nucleic acid, or a nucleic acid capable of hybridising with such a nucleic acid, or wherein said nucleic acid sequence encoding a ZPR polypeptide encodes an orthologue or paralogue of any of the polypeptides given in Table J.

**5.** Method according to any preceding claim, wherein said nucleic acid encoding a GRP encodes the polypeptide as represented by SEQ ID NO: 251; or wherein said nucleic acid encoding a ZPR polypeptide encodes the polypeptide represented by SEQ ID NO: 747 or a homologue thereof.

**6.** Method according to any preceding claim, wherein said enhanced yield-related trait obtained by modulating expression of a nucleic acid encoding a GRP comprises increased yield relative to control plants; or
wherein said enhanced yield-related traits obtained by modulating expression of a nucleic acid encoding a ZPR polypeptide comprises increased yield relative to control plants, and preferably increased seed yield relative to control plants.

**7.** Method according to claim 6, wherein said increased yield obtained by modulating expression of a nucleic acid encoding a GRP, comprises increased seed yield relative to control plants, and preferably said increased seed yield is selected from any one or more of:

(i) increased harvest index;
(ii) increased total seed weight;
(iii) increased number of filled seeds; and
(iv) increased thousand kernel weight, and/or wherein said enhanced yield-related trait obtained by modulating expression of a nucleic acid encoding a GRP comprises increased biomass, and preferably increased above-ground biomass, relative to control plants.

8. Method according to any preceding claim, wherein said enhanced yield-related traits are obtained under non-stress conditions; or
wherein said enhanced yield-related traits obtained by modulating expression in a plant of a nucleic acid encoding a GRP are obtained under conditions of drought stress, salt stress or nitrogen deficiency; or
wherein said enhanced yield-related traits obtained by modulating expression in a plant of a nucleic acid encoding a ZPR polypeptide are obtained under conditions of drought stress, salt stress or nitrogen deficiency.

9. Method according to any one of claims 3 to 8, wherein said nucleic acid encoding a GRP is operably linked to a constitutive promoter, preferably to a medium strength constitutive promoter, preferably to a plant promoter, more preferably to a GOS2 promoter, most preferably to a GOS2 promoter from rice; or
wherein said nucleic acid encoding a ZPR polypeptide is operably linked to a constitutive promoter, preferably to a medium strength constitutive promoter, preferably to a plant promoter, more preferably to a GOS2 promoter, most preferably to a GOS2 promoter from rice.

10. Method according to any preceding claim, wherein said nucleic acid encoding said GRP is of plant origin, preferably from a monocotyledonous plant, further preferably from the family Poaceae, more preferably from the genus Oryza, most preferably from *Oryza sativa*; or
wherein said nucleic acid encoding a ZPR polypeptide is of plant origin, and preferably from a dicotyledonous plant, preferably wherein said nucleic acid encoding a ZPR polypeptide is from the family Solanaceae, more preferably from the genus Solanum, most preferably from *Solanum lycopersicum.*

11. Construct comprising:

(i) a nucleic acid encoding a GRP as defined in any preceding claim; or
(ii) a nucleic acid encoding a ZPR as defined in any preceding claim; and
(iii) one or more control sequences capable of driving expression of the nucleic acid sequence of (i) or (ii), preferably wherein at least one of said control sequences capable of driving expression of the nucleic acid sequence of (i) or (ii) is a constitutive promoter, preferably a medium strength constitutive promoter, preferably a plant promoter, more preferably a GOS2 promoter, most preferably a GOS2 promoter from rice; and optionally
(iv) a transcription termination sequence.

12. Method for the production of a transgenic plant having enhanced yield-related traits or increased yield, particularly increased biomass and/or increased seed yield relative to control plants, comprising:

(i) introducing and expressing in a plant cell or plant a nucleic acid encoding a GRP as defined in any preceding claim; or
(ii) introducing and expressing in a plant cell or plant a nucleic acid encoding a ZPR polypeptide as defined in any preceding claim; or a genetic construct comprising a ZPR polypeptide-encoding nucleic acid as defined in claim 11; and
(iii) cultivating the plant cell under conditions promoting plant growth and development.

13. Plant, plant part thereof, including seeds, or plant cell, obtainable by a method according to any one of claims 1 to 10, wherein said plant, plant part or plant cell comprises a recombinant nucleic acid encoding a GRP as defined in any preceding claim; or plant, plant part thereof, including seeds, or plant cell, obtainable by a method according to any one of claims 1 to 10, wherein said plant, plant part or plant cell comprises a recombinant nucleic acid encoding a ZPR polypeptide as defined in any of preceding claim; or
plant, plant part or plant cell transformed with a construct according to claim 11; or
a transgenic plant having enhanced yield-related traits relative to control plants, preferably as defined in claim 7 or 8, resulting from modulated expression of a nucleic acid encoding a GRP polypeptide as defined in any preceding claim, or a transgenic plant cell derived from said transgenic plant; or
a transgenic plant having enhanced yield-related traits relative to control plants, preferably increased yield relative to control plants, and more preferably increased seed yield relative to control plants, resulting from modulated expression of a nucleic acid encoding a ZPR polypeptide as defined in any preceding claim, or a transgenic plant cell derived from said transgenic plant, preferably wherein said plant is a crop plant, such as beet or sugarbeet or alfalfa, or a monocot such as sugarcane, or a cereal, such as rice, maize, wheat, barley, millet, rye, triticale, sorghum emmer, spelt, secale, einkorn, teff, milo and oats; or harvestable parts of said plant, wherein said harvestable parts are preferably shoot biomass and/or seeds and/or products derived from said plant and/or from harvestable parts thereof.

14. Use of a nucleic acid encoding a GRP as defined in any preceding claim for enhancing yield-related traits in plants, and preferably wherein said yield-related traits are as defined in claim 7 or 8, relative to control plants; or
use of a nucleic acid encoding a ZPR polypeptide as defined in any of claims 1(b), 2, 4 or 5 for enhancing yield-related traits in plants relative to control plants, preferably for increasing yield in plants relative to control plants, and more preferably for increasing seed yield in plants relative to control plants; or
use of a construct according to claim 11, comprising the nucleic acid sequence of (i) in a method for making plants having increased yield, particularly increased biomass and/or increased seed yield relative to control plants; or
use of a construct according to claim 11, comprising the nucleic acid sequence of (ii) in a method for making plants having enhanced yield-related traits.

15. An isolated nucleic acid or polypeptide selected from:

(i) a nucleic acid represented by any one of SEQ ID NO: 266, 286, 316, 374, 376, 380, 424, 456, 458, 460, 462, 648, 650, 670, and 736;
(ii) the complement of a nucleic acid represented by any one of SEQ ID NO: 266, 286, 316, 374, 376, 380, 424, 456, 458, 460, 462, 648, 650, 670, and 736;
(iii) a nucleic acid encoding the polypeptide as represented by any one of SEQ ID NO: 267, 287, 317, 375, 377, 381, 425, 457, 459, 461, 463, 649, 651, 671 and 737, preferably as a result of the degeneracy of the genetic code, said isolated nucleic acid can be derived from a polypeptide sequence as represented by any one of SEQ ID NO: 267, 287, 317, 375, 377, 381, 425, 457, 459, 461, 463, 649, 651, 671 and 737 and further preferably confers enhanced yield-related traits relative to control plants;
(iv) a nucleic acid having, in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences represented by any one of SEQ ID NO: 266, 286, 316, 374, 376, 380, 424, 456, 458, 460, 462, 648, 650, 670, and 736; and any of the other nucleic acid sequences in Table F and further preferably conferring enhanced yield-related traits relative to control plants;
(v) a nucleic acid molecule which hybridizes with a nucleic acid molecule of (i) to (iv) under stringent hybridization conditions and preferably confers enhanced yield-related traits relative to control plants;
(vi) a nucleic acid encoding a polypeptide having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 267, 287, 317, 375, 377, 381, 425, 457, 549, 461, 463, 649, 651, 671 and 737 and any of the other amino acid sequences in Table F and preferably conferring enhanced yield-related traits relative to control plants; or

An isolated nucleic molecule selected from:

(i) a nucleic acid represented by any one of SEQ ID NO: 748, 790, 792, 862, 864, 870, 878, 902, 912, and 918;
(ii) the complement of a nucleic acid represented by any one of SEQ ID NO: 748, 790, 792, 862, 864, 870, 878, 902, 912, and 918;
(iii) a nucleic acid encoding a ZPR polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented any one of SEQ ID NO: 749, 791, 793, 863, 865, and 871; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 1 to 6 as given in SEQ ID NO: 929 to SEQ ID NO: 934, and further preferably conferring enhanced yield-related traits relative to control plants;
(iv) a nucleic acid encoding a ZPR polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented any one of SEQ ID NO: 878, 902, 912, and 918; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 10 as given in SEQ ID NO: 929 to SEQ ID NO: 931, and further preferably conferring enhanced yield-related traits relative to control plants.

(v) a nucleic acid molecule which hybridizes with a nucleic acid molecule of (i) to (iv) above under high stringency hybridization conditions and preferably confers enhanced yield-related traits relative to control plants; or

(i) an amino acid sequence represented by any one of SEQ ID NO: 267, 287, 317, 375, 377, 381, 425, 457, 459, 461, 463, 649, 651, 671 and 737;

(ii) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 267, 287, 317, 375, 377, 381, 425, 457, 459, 461, 463, 649, 651, 671 and 737, and any of the other amino acid sequences in Table F and preferably conferring enhanced yield-related traits relative to control plants, and

(iii) derivatives of any of the amino acid sequences given in (i) or (ii) above; or

(iv) an amino acid sequence represented by any one of SEQ ID NO: 749, 791, 793, 863, 865, 871, 879, 903, 913, and 919;

(v) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 749, 791, 793, 863, 865, and 871; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 13 given in SEQ ID NO: 929 to SEQ ID NO: 934, and further preferably conferring enhanced yield-related traits relative to control plants;

(vi) an amino acid sequence having, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 879, 903, 913, and 919; and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs 8 to 10 given in SEQ ID NO: 929 to SEQ ID NO: 931, and further preferably conferring enhanced yield-related traits relative to control plants;

(vii) derivatives of any of the amino acid sequences given in (iv) to (vi) above.

Two Zinc Knuckles (Plant specific)
RSZ36, RSZ37a, RSZ37b, RSZ39

| RRM | Zn Zn | RS |

**FIGURE 1 A**

MPRYDDRYGS**TRLYVGRLSSRTRSRDLEYHFSRYGRIREVELKRDYAFIEFSDPRD**

3         2

---4---       ---5---

**AEEARYNLDGRDVDGSRILVEFAKG**VPRGPGGSREYMGRGP*PPGTGRCFNCGIDGH*

2         1

*WARDCKA*GDWKN*KCYRCGERGHIERNCQNS*PRNLRRERSYSCSPSPRRGRGRSRSY

-6-

SRSRSRSRSYSRSRSRSLSGSPRARRELERSRSLSYSRSPRRSISPAANEKKRSPT

-6-  ---6--

PDGSRSPRSPQDQVSPPPKDNAERNGSDHGDSPRGRENSRSPSDGYRSPAAANGRS

PSPRNNGSPSPMDNGSRSPRDGNGDGGSRGGSRSPRPSESPEA

**FIGURE 1 B**

**FIGURE 2**

CLUSTAL 2.0.11 multiple sequence alignment

```
T.aestivum_TC300145        MPRYDDRYGNARLYVGRLSSRTRSRDLEYLFSKYGRIREVELKRDYAFIE
Triae_RSZ38                MPRYDDRYGNARLYVGRLSSRTRSRDLEYLFSKYGRIREVELKRDYAFIE
H.vulgare_@6248            MPRYDDRYGNARLYVGRLSSRTRSRDLEYLFSKYGRIREVELKRDYAFIE
T.aestivum_TC286992        MPRYDDRYGNARLYVGRLSSRTRSRDLEYLFSKYGRIREVELKRDYAFIE
T.turgidum_AJ612509        MPRYDDRYGNARLYVGRLSSRTRSRDLEYLFSKYGRIREVELKRDYAFIE
O.sativa_LOC_Os03g17710.1  MPRYDDHYGSTRLYVGRLSSRTRSRDLEYLFGRYGRIREVELKRDYAFIE
O.sativa_LOC_Os03g17710.2  MPRYDDHYGSTRLYVGRLSSRTRSRDLEYLFGRYGRIREVELKRDYAFIE
O.glaberrima_Og014036      MPHYDDHYGSTRLYVGRLSSRTRSRDLEYLFGRYGRIREVELKRDYAFIE
O.sativa_LOC_Os05g02880    MPRYDDRYGSTRLYVGRLSSRTRSRDLEYHFSRYGRIREVELKRDYAFIE
Z.mays_@11244              MPRYDDRYGGTRLYVGRLAPRTRSRDLEYLFGKYGRIREVELKRDYAFIE
Z.mays_TC490304            MPRYDDRYGGTRLYVGRLAPRTRSRDLEYLFGKYGRIREVELKRDYAFIE
Z.mays_@13012              MPRYDDRYGGTRLYVGRLAPRTRSRDLEYLFGKYGRIREVELKRDYAFIE
S.bicolor_Sb09g001910      MPRYDDRYGGTRLYVGRLAPRTRSRDLEYLFGRYGRIREVELKRDYAFIE
Z.mays_TC510523            MPRYDDRYGGTRLYVGRLATRTRSRDLEHLFGRYGRIREVELKRDYAFIE
Z.mays_@35142              MPRYDDRYGGTRLYVGRLATRTRSRDLEHLFGRYGRIREVELKRDYAFIE
S.officinarum_TC76505      MPRYDDRYGGTRLYVGRLAPRTRSRDLENLFGRYGRIREVELKRDYAFIE
S.officinarum_TC80184      MPRYDDRYGGTRLYVGRLAPRTRSRDLENLFGRYGRIREVELKRDYAFIE
S.officinarum_CA282538     MPRYDDRYGGTRLYVGRLAPRTRSRDLENLFGRYGRIREVELKRDYAFIE
P.virgatum_TC27619         MPRYDDRYGGTRLYVGRLASRTRSRDLEYLFSRYGRIREVELKRDYAFIE
S.bicolor_Sb09g001920      MPRYDDRYGNTRLYVGRLAPRTRSRDLEYLFSKYGRIREVELKRDYAFIE
S.officinarum_TC107276     MPRYDDRYGNTRLYVGRLAPRTRSRDLEYLFSKYGRIREVELKRDYAFIE
Z.mays_@11445              MPRYDDRHGNTRLYVGRLAPRTRSRDLEYLFSKYGRIREVELKRDYAFIE
Z.mays_TC527608            MPRNDDRYGNTRLYVGRLSPRTRSRDLEYLFSKYGRIREVELKRDYAFIE
Z.mays_TC518048            MPRYDDRHGNTRLYVGRLAPRTRSRDLEYLFSKYGRIREVELKRDYAFIE
P.virgatum_TC32128         MPRYDDRYGNTRLYVGRLSSRTRSRDLEYLFSKHGRIREVELKRDYAFIE
S.hybrid_CF570698          MPRYDDRYGNTRLYVGRLAPRTRSRDLEYLFSKYGRIREVELKRDYAFIE
S.officinarum_TC86028      MPRYDDRYGNTRLYVGRLAPRTRSRDLEYLFSKYGRIREVELKRDYAFIE
S.officinarum_CA177377     MPRYDDRYGNTRLYVGRLAPRTRSRDLEYLFSKYGRIREVELKRDYAFIE
P.virgatum_TC2491          MPRYDDRYGNTRLYVGRLSSRTRTRDLEYLFSKYGRIREVELKRDYAFIE
                           **: **::*.:*******:.***:****  *.::***************
```

**FIGURE 2 (continued)**

```
T.aestivum_TC300145         YSDPRDADDARYNLDGRDVDGSRIIVEFAKG-----VPRGS-GGSREREY
Triae_RSZ38                 YSDPRDADEARYNLDGRDVDGSRIIVEFAKG-----VPRGS-GGSREREY
H.vulgare_@6248             FSDSRDADEARYNLDGRDVDGSRIIVEFAKG-----VPRGS-GGSREREY
T.aestivum_TC286992         YSDPRDADEARYNLDGRDVDGSRIIVEFAKG-----VPRGS-GGSREREY
T.turgidum_AJ612509         YSDPRDADEARYNLDGRDVDGSRIIVEFAKG-----VPRGS-GGSREREY
O.sativa_LOC_Os03g17710.1   FSDPRDADEARYNLDGRDVDGSRILVEFAKGVSSSLVPRGAAGGSRE--Y
O.sativa_LOC_Os03g17710.2   FSDPRDADEARYNLDGRDVDGSRILVEFAKG-----VPRGAAGGSRE--Y
O.glaberrima_Og014036       FSDPRDADEARYNLDGRDVDGSRILVEFAKG-----VPRGAAGGSRE--Y
O.sativa_LOC_Os05g02880     FSDPRDAEEARYNLDGRDVDGSRILVEFAKG-----VPRGP-GGSRE--Y
Z.mays_@11244               FSEHRDADEARYQLDGRDVDGSRIVVEFAKG-----VPRGP-GGSRE--Y
Z.mays_TC490304             FSEHRDADEARYQLDGRDVDGSRIVVEFAKG-----VPRGP-GGSRE--Y
Z.mays_@13012               FSEHRDADEARYQLDGRDVDGSRIVVEFAKG-----VPRGP-GGSRE--Y
S.bicolor_Sb09g001910       FSDHRDADEARYQLDGRDVDGSRIVVEFAKG-----VPRGP-GGSRE--Y
Z.mays_TC510523             FSDHRDADEARYQLDGRDVDGSRIVVEFAKG-----VPRGS-GGSRE--Y
Z.mays_@35142               FSDHRDADEARYQLDGRDVDGSRIVVEFAKG-----VPRGS-GGSRE--Y
S.officinarum_TC76505       FSDHRDADEARYQLDGRDVDGSRIVVEFAKG-----VPRGP-AGSRE--Y
S.officinarum_TC80184       FSDHRDADEARYQLDGRDVDGSRIVVEFAKG-----VPRGP-GGSRE--Y
S.officinarum_CA282538      FSDHRDADEARYQLDGRDVDGSRIVVEFAKG-----VPRGP-AGSRE--Y
P.virgatum_TC27619          FSDPRDADDARYNLDGRDVDGSRIIVEFAKG-----VPRGP-GGSRE--S
S.bicolor_Sb09g001920       FSDPHDADDAQYNLDGREVDGSRIIVEFAKG-----VPRGS-GGSRE--Y
S.officinarum_TC107276      FSDPQDADDAQYNLDGREVDGSRIIVEFAKG-----VPRGS-SGSRE--Y
Z.mays_@11445               FSDPRDADDAQYNLDGREVDGSRIIVEFAKG-----VPRGS-GGSRE--Y
Z.mays_TC527608             FSDPRDADDAQYNLDGRDVDGSRIIVEFAKG-----VPRGS-GGSRD--Y
Z.mays_TC518048             FSDPRDADDAQYNLDGREVDGSRIIVEFAKG-----VPRGS-GGSRE--Y
P.virgatum_TC32128          FSNPRDADDAQYNLDGREVDGSRIIVEFAKG-----VPRGS-GGSRE--Y
S.hybrid_CF570698           FSDPQDADDAQYNLDGREVDGSRIIVEFAKG-----VPRGS-SGSRE--Y
S.officinarum_TC86028       FSDPQDADDAQYNLDGREVDGSRIIVEFAKG-----VPRAS-SGSRE--Y
S.officinarum_CA177377      FSDPQDADDAQYNLDGREVDGSRIIVEFAKG-----VPRGS-SGSRE--Y
P.virgatum_TC2491           FSDPRDADDAQYNLDGREVDGSRIIVEFAKG-----VPRGS-GSSRE--Y
                            :*:  :**::*:*:****:******:******     ***..  ..**:
```

**FIGURE 2 (continued)**

```
T.aestivum_TC300145        VGRGPPPGTGRCFNCGIDGHWARDCKAGDWKNKCYRCGERGHIERNCQNS
Triae_RSZ38                VGRGPPPGTGRCFNCGIDGHWARDCKAGDWKNKCYRCGERGHIERNCQNS
H.vulgare_@6248            VGRGPPPGTGRCFNCGIDGHWARDCKAGDWKNKCYRCGERGHIERNCQNS
T.aestivum_TC286992        VGRGPPPGTGRCFNCGIDGHWARDCKAGDWKNKCYRCGERGHIERNCQNS
T.turgidum_AJ612509        VGRGPPPGTGRCFNCGIDGHWARDCKAGDWKNKCYRCGERGHIERNCQNS
O.sativa_LOC_Os03g17710.1  MGRGPPPGTGRCFNCGIDGHWARDCKAGDWKNKCYRCGERGHIERNCQNS
O.sativa_LOC_Os03g17710.2  MGRGPPPGTGRCFNCGIDGHWARDCKAGDWKNKCYRCGERGHIERNCQNS
O.glaberrima_Og014036      MGRGPPPGTGRCFNCGIDGHWARDCKAGDWKNKCYRCGERGHIERNCQNS
O.sativa_LOC_Os05g02880    MGRGPPPGTGRCFNCGIDGHWARDCKAGDWKNKCYRCGERGHIERNCQNS
Z.mays_@11244              MGRGPPPGTGRCFNCGMDGHWARDCKAGDWKNKCYRCGERGHIERNCQNS
Z.mays_TC490304            MGRGPPPGTGRCFNCGMDGHWARDCKAGDWKNKCYRCGERGHIERNCQNS
Z.mays_@13012              MGRGPPPGTGRCFNCGMDGHWARDCKAGDWKNKCYRCGERGHIERNCQNS
S.bicolor_Sb09g001910      MGRGPPPGTGRCFNCGIDGHWARDCKAGDWKNKCYRCGERGHIERNCQNS
Z.mays_TC510523            MGRGPPPGTGRCFNCGVDGHWARDCKAGDWKNKCYRCGERGHIERNCQNS
Z.mays_@35142              MGRGPPPGTGRCFNCGVDGHWARDCKAGDWKNKCYRCGERGHIERNCQNS
S.officinarum_TC76505      MGRGPPPGTGRCFNCGIDGHWARDCKAGDWKNKCYRCGERGHIERNCQNS
S.officinarum_TC80184      MGRGPPPGTGRCFNCGIDGHWARDCKAGDWKNKCYRCGERGHIERNCQNS
S.officinarum_CA282538     MGRGPPPGTGRCFNCGIDGHWARDCKAGDWKNKCYRCGERGHIERNCQK-
P.virgatum_TC27619         MGRGPPPGTGRCFNCGIDGHWARDCKAGDWKNKCYRCGERGHIERNCQNS
S.bicolor_Sb09g001920      MGRGPPPGTGRCFNCGIDGHWARDCKAGDWKNKCYRCGERGHIERNCQNS
S.officinarum_TC107276     MGRGPPPGTGRCFNCGIDGHWARDCKAGDWKNKCYRCGERGHIERNCQNS
Z.mays_@11445              MGRGPPPGTGRCFNCGMDGHWARDCKAGDWKNKCYRCGESGHIERNCQNS
Z.mays_TC527608            NGRGPPPGTGRCFNCGVDGHWARDCQAGDWKNKCYRCGERGHIERNCQNS
Z.mays_TC518048            MGRGPPPGTGRCFNCGMDGHWARDCKAGDWKNKCYRCGESGHIERNCQNS
P.virgatum_TC32128         MGRGPPPGTGRCFNCGIDGHWARDCKAGDWKNKCYRCGERGHIERNCQNS
S.hybrid_CF570698          MGRGPPPGTGRCFNCGIDGHWARDCKAGDWKNKMLPCGERGHIERNCQNS
S.officinarum_TC86028      MGRGPPPGTGRCFNCGIDGHWARDCKAGDWKNKCYRCGERGHIERNCQNS
S.officinarum_CA177377     MGRGAPPGTGRCFNCGIDGHWARDCYAGDWKNKCYRCGERGHIERNCQNS
P.virgatum_TC2491          MGRGPPPGTGRCFNCGIDGHWARDCKAGDWKNKCYRCGERGHIERNCQNS
                           *** .***********:******* ******   *** *******:
```

**FIGURE 2 (continued)**

```
T.aestivum_TC300145        PRSLRRERSYSRSPSPRRGRARSRSYS----RSRSYSRSRSRSYSESPRG
Triae_RSZ38                PRSLRRERSYSRSPSPRRGRARSRSYS----RSRSYSRSRSRSYSESPRG
H.vulgare_@6248            PRSLRRERSYSRSPSPRRGRARSRSYS----RSRSYSRSRSRSYSESPRG
T.aestivum_TC286992        PRSLRRERSYSRSPSPRRGRARSRSYS----RSRSYSRSRSRSYSESPRG
T.turgidum_AJ612509        PRSLRRERSYSRSPSPRRGRARSRSYS----RSRSYSRSRSRSYSESPRG
O.sativa_LOC_Os03g17710.1  PRNLRRERSYSRSPSPRRGRGHGRSRSYSRSRSRSYSRSRSRSLSGSPRG
O.sativa_LOC_Os03g17710.2  PRNLRRERSYSRSPSPRRGRGHGRSRSYSRSRSRSYSRSRSRSLSGSPRG
O.glaberrima_Og014036      PRNLRRERSYSRSPSPRRGRGHGRSRSYSRSRSRSYSRSRSRSLSGSPRG
O.sativa_LOC_Os05g02880    PRNLRRERSYCSPSPRRGRGRSRSYSRSRSRSRSYSRSRSRSLSGSPRA
Z.mays_@11244              PRSVRRERSYSRSPSPRRGRGRSPSYSRSRSRSRSRSYSRSRSLSGSPRG
Z.mays_TC490304            PRSVRRERSYSRSPSPRRGRGRSPSYSRSRSRSRSRSYSRSRSLSGSPRG
Z.mays_@13012              PRSVRRERSYSRSPSPRRGRGRSPSYSRSRSRSRSRSYSRSRSLSGSPRG
S.bicolor_Sb09g001910      PRSVRRERSYSRSPSPRRGRGRSPSYSRSRSRSRSRSYSRSRSLSGSPRG
Z.mays_TC510523            PRSLRRERSYSRSPSPRRGRGRSRSYGRSRSRSR--SYSRSRSLSRSPRG
Z.mays_@35142              PRSLRRERSYSRSPSPRRGRGRSRSYSRSRSRSR--SYSRSRSLSRSPRG
S.officinarum_TC76505      PRSVRRERSYSRSPTPRRGRGRSPSYSRSRSRSR--SYSRSRSLSGSPRG
S.officinarum_TC80184      PRSVRRERSYSRSPTPRRGRGRSPSYSRSRSRS----YSRSRSLSGSPRG
S.officinarum_CA282538     --------------------------------------------------PKG
P.virgatum_TC27619         PRSIRRERSYSRSPSPRHGRGRSRSYSRSRSRSR--SNSRSRSLSGSPRG
S.bicolor_Sb09g001920      PRNLRRERSYSRSPSPRRGRGRSRSYS----RSR--SYSRSRSPSGSPRG
S.officinarum_TC107276     PRNLRRERSYSRSPSPRRGRGRSRSYS----RSR--SYSRSRSPSGSPRG
Z.mays_@11445              PRNLRRDKSYSRSPSPRGGRGRSRSYS----RSR--SYSRSRSPSGSPRG
Z.mays_TC527608            PRNLRRERSYSRSPSPRRGRGRSRSYS----RSR--SYSRSRSPSGSPRG
Z.mays_TC518048            PRNLRRDKSYSRSPSPRGGRGRSRSYS----RSR--SYSRSRSPSGSPRG
P.virgatum_TC32128         PQNLRRERSYSRSPSPHRGRGRSRSYS----RSR--SYSRSRSPSGSPRG
S.hybrid_CF570698          PRNLRRERSYSRSPSPRRGRG----------------PQPGATSRSPE-
S.officinarum_TC86028      PRNLRRERSYSRSPSPRRGRGRSRSYS----RSQ--SYSRSRAPSGSPEG
S.officinarum_CA177377     PMNLRRERSYSRSPSPRRGRG----------------RQRSYSRSRSY
P.virgatum_TC2491          PRNLRRERSYSQSPSPRRGRG----------------RSRSYSRSREL
```

100

```
T.aestivum_TC300145          RRTER--DERRSRSISYSR----SPRRSLSPEAKEMDRSPTPDRSRSPRRS
Triae_RSZ38                  RRTER--DERRSRSISYSR----SPRRSLSPGGKEMDRSPTPDRSRSPRRS
H.vulgare_@6248              RRTER--DERRSRSISYSR----SPRRSLSPEAKEMDRSPTPDRSRSPRRS
T.aestivum_TC286992          RRTER--DERRSRSISYSR----SPRRSLSPGG--------
T.turgidum_AJ612509          RRTER--DERRSREH--------
O.sativa_LOC_Os03g17710.1    RR-DR--DDRRSRSLSYSR----SPRRSISPAANGKERNPSPNGRRSPRSP
O.sativa_LOC_Os03g17710.2    RR-DR--DDRRSRSLSYSR----SPRRSISPAANGKERNPSPNGRRSPRSP
O.glaberrima_Og014036        RR-DR--DDRRSRSLSYSR----SPRRSISRAANGKERSPSPNGRRSPRSP
O.sativa_LOC_Os05g02880      RR-EL--E--RSRSLSYSR----SPRRSISPAANEKKRSPTPDGSRSPRSP
Z.mays_@11244                GRRDR----DDRRSRSLSYSR--SPVR--SPPAKEKERSPTPGGSRSPRSR
Z.mays_TC490304              GRRDR----DDRRSRSLSYSR--SPMR--SPPAKEKERSPTPGGSRSPRSR
Z.mays_@13012                GRRDR----DDRRSRSLSYSR--SPVR--SPPAKEKERSPTPGGSRSPRSR
S.bicolor_Sb09g001910        GRRDR----DDRRSRSLSYSR--SPMRSASPPPKEKERSPTPDGSRSPRSR
Z.mays_TC510523              GRRDR----DDRRSRSRPSYSR--SPMRSASPPVKEKERSPAPDGSSSPRSR
Z.mays_@35142                GRRDR----DDRRSRSRPSYSR--SPMRSASPPVKEKERSPAPDGSRSPRSR
S.officinarum_TC76505        GRRDRNRDDDRRSRSLSYSR--SPMRSASPPPKERERSPTPNGSRXPRSR
S.officinarum_TC80184        GRRDR----DDRRSRSLSYSR--SPMRSASPPPKERERSPTPDGSRSPRSR
S.officinarum_CA282538       ----------------FPPPF--
P.virgatum_TC27619           GRRDR----GERRSRSLSYSR--SPMRSASPPAKEHSR--TPDGSRSPRSP
S.bicolor_Sb09g001920        GRRER----DERRSRSLSYSRSA-SPRRSVSP-AKEKERSRTPDGSRSPRSP
S.officinarum_TC107276       GRRDR----DERRSRSRSVSYSRSA-SPRRSVSP-AKEKERSRTPDGSRSPRSP
Z.mays_@11445                GRRER----DERRSRSLSYSRSA-SPRRSVSP-VKEKERSRTPDGSRSP---
Z.mays_TC527608              GRRDR----DERRSRSLSYSRSA-SPRRSVSP-VKEKERSRTPDGSRSP---
Z.mays_TC518048              GRRER----DERRSRSLSYSRSA-SPRRSVSP-AKEKERSRTPNGSRSPRSP
Z.mays_TC32128               GRRDR----DERRSRSLSYSR---SPRRSASPSAKEKERSPTPNGSRSPRSP
S.hybrid_CF570698            L---------------
S.officinarum_TC86028        LHAGDRDQEENQGVVSFQQEPQALGGSVLPCKGKRTAPAHLVAARNPKEP
S.officinarum_CA177377       RYAQERKPSCYFSISSLDS----LG--VX--YSLLILHFWLVNQLVLTIL
P.virgatum_TC2491            --------------
```

**FIGURE 2 (continued)**

EP 2 998 401 A2

FIGURE 2 (continued)

```
T.aestivum_TC300145        ------ISPVAKDNG--------DSPRGRDKS----------RSPSDGYR
Triae_RSZ38                ------ISPVAKDNG--------DSPRGRETS----------RSPSDGYR
H.vulgare_@6248            ------ISPVAKDNG--------DSPRGRRTS----------RSPSDGYR
T.aestivum_TC286992        --------------------------------------------------
T.turgidum_AJ612509        --------------------------------------------------
O.sativa_LOC_Os03g17710.1  QDR---VSPPPKDNDERNG----DSPWGRENS----------RSPSDGYR
O.sativa_LOC_Os03g17710.2  QDR---VSPPPKDNDERNG----DSPWGRENS----------RSPSDGYR
O.glaberrima_Og014036      QDR---VSPPPKDNDERNG----DSPRGRENS----------RSPSDGYR
O.sativa_LOC_Os05g02880    QDQ---VSPPPKDNAERNGSDHGDSPRGRENS----------RSPSDGYR
Z.mays_@11244              SPQDQVMSPPPKDNGDRNGSDRGDSPRGRENS----RSRSRSRSPSGGNR
Z.mays_TC490304            SPQDQVMSPPPKDNGDRNGSDRGDSPRGRENS----RSRSRSRSPSGGNR
Z.mays_@13012              SPQDQVMSPPPKDNGDRNGSDRGDSPRGREDS----RSRSRS--PSGGNR
S.bicolor_Sb09g001910      SPQDQVMSPPPKDNGEGNGSDRGESPRGRETS----RSRSRSRSPSGDNR
Z.mays_TC510523            SPQDQVMSPPPKDNGEGNGSDRGGSPRGRENS----RSCSRSRSPSGSNR
Z.mays_@35142              SPQDQVMSPPPKDNGEGNGSDRGGSPRGRENS----RSCSRSRSPSGSNR
S.officinarum_TC76505      SPQDQVMSPPPKDNGEGNGSDRGDSPRGREN------SRSRSRSPSRGNR
S.officinarum_TC80184      SPQDQVMSPPPKDNDEGNGSDRGDSPRGRENSMSRSRSRSRSPSGGNR
S.officinarum_CA282538     --------------------------------------------------
P.virgatum_TC27619         SPRGQVS-PPPKDNGERNGSDRGDSPGGMEKE----NSRSRSRSPSDGNR
S.bicolor_Sb09g001920      SPRDD-VSPPPKDNGAHNGSDREDSPGARENSK-------RSRSPSDGYR
S.officinarum_TC107276     SPRDD-VSPPPKDNGARNGSDREDSPGARENSK-------RSRSPSDGYR
Z.mays_@11445              SPRDK-VSPPPKDNGAHNGSDREDSPGGREMSK-------RSRSPSDGYR
Z.mays_TC527608            SPRDK-VSPPPKDNGAHNGSDREDSPGGREMSK-------RSRSPSDGYR
Z.mays_TC518048            SPRAE-VSPPLKDNGARNGSDHEGNPGGGENSK-------RSRSPSDGYR
P.virgatum_TC32128         SPRDQ-VSPPPKDNGERNGSDRGDSPAKRENSRS----RSRSRSPSDGYR
S.hybrid_CF570698          --------------------------------------------------
S.officinarum_TC86028      PPQGMDVKPPTQGLWCPATVYNLEKKP--------------RGPGKTAR
S.officinarum_CA177377     LLQSGESSP------------HLDFR---------------RG-GRRDR
P.virgatum_TC2491          --------------------------------------------------
```

102

**FIGURE 2 (continued)**

```
T.aestivum_TC300145          SPVA-NGRSP--------SPANN------GNPSP------TRDNRGSPSP
Triae_RSZ38                  SPVA-NGRSPR-------SPVNN------GSPSP------TRDNRASPSL
H.vulgare_@6248              SPVA-NGRSPR-------SPANN------GSPSP------ARDNKGSPSP
T.aestivum_TC286992          --------------------------------------------------
T.turgidum_AJ612509          --------------------------------------------------
O.sativa_LOC_Os03g17710.1    SPVAANGRSP--------SPRNN------GSPSP------MDNN--SRSP
O.sativa_LOC_Os03g17710.2    SPVAANGRSP--------SPRNN------GSPSP------MDNN--SRSP
O.glaberrima_Og014036        SPVAANGRSP--------SPRNN------GSPSP------MDNN--SRSP
O.sativa_LOC_Os05g02880      SPAAANGRSP--------SPRNN------GSPSP------MDNG--SRSP
Z.mays_@11244                SPAA-NGRS--------PSPRGD------RTPSP-------MADRSPSP
Z.mays_TC490304              SPAA-NGRS--------PSPRGD------RTPSP-------MADRSPSP
Z.mays_@13012                SPAA-NGRS--------PSPRGD------RGPSP-------MADRSPSP
S.bicolor_Sb09g001910        SPAA-NGRS--------PSPRGD------RSPSPRADRSPSPKADRSPSP
Z.mays_TC510523              SPAA-NGRSRSPGGERSPSPGGE------RSPSPRGERSPSPRADRSPSP
Z.mays_@35142                SPAA-NGRSRSPGGERSPSPGGE------RSPSPRGERSPSPRADRSPSP
S.officinarum_TC76505        SPAA-XGSS--------PSPRGD------RSPSP-------RADRSPSP
S.officinarum_TC80184        SPAA-NGRS--------PSPKVT------GIQAP--------GLTVAQAP
S.officinarum_CA282538       --------------------------------------------------
P.virgatum_TC27619           SPAA-NGRS--------PSPRDD------RSPSP------TRGDRSPSP
S.bicolor_Sb09g001920        SPAA-NGRSP--------SPR--------DDRSP-------------SP
S.officinarum_TC107276       SPAA-NGRSP--------SPR--------DDRSP-------------SP
Z.mays_@11445                SPVG-NGRSP--------SRSPSPKVNNGDDPSPK------GNNGDDPSP
Z.mays_TC527608              SPVG-NGRSP--------SRSPSPKVNNGDDPSPK------GNNGDDPSP
Z.mays_TC518048              SPVA-NGRSP--------SPR--------DDRSP-------------SP
P.virgatum_TC32128           SPAA-NGRSP--------SPR--------DDRSP-------------SP
S.hybrid_CF570698            --------------------------------------------------
S.officinarum_TC86028        RSSGALLFGL--------P-------------------------------
S.officinarum_CA177377       V-------------------------------------------------
P.virgatum_TC2491            --------------------------------------------------
```

**FIGURE 2 (continued)**

```
T.aestivum_TC300145        RGNNGSPSPKGNGDGGSPSPRGNGDDDGRRGSGSPRGRSVTP--------
Triae_RSZ38                RGNNGSPSPKGNGNGGSPSPRGNGDDDGRRGSGSPRGRSVSP--------
H.vulgare_@6248            RGNNGSPSPKGNGNGGSPSPRGNGDGDGLRGSGSPRGRSASP--------
T.aestivum_TC286992        --------------------------------------------------
T.turgidum_AJ612509        --------------------------------------------------
O.sativa_LOC_Os03g17710.1  RDNGSPSPRDGNGDGGS-----RGGSRSPRASESPEA-------------
O.sativa_LOC_Os03g17710.2  RDNGSPSPRDGNGDGGS-----RGGSRSPRASESPEA-------------
O.glaberrima_Og014036      RDNGSPSPRDGNGDGGS-----RGGSRSPRASESPEA-------------
O.sativa_LOC_Os05g02880    RD--------GNGDGGS-----RGGSRSPRPSESPEA-------------
Z.mays_@11244              KGNGNNDGG----DDDTG------RGGSPTGSKSP---------------
Z.mays_TC490304            KGNGNNDGG----DDDTG------RGGSPTGSKSP---------------
Z.mays_@13012              KGNGNNDGG----DDDTG------RGGSPTGSKSP---------------
S.bicolor_Sb09g001910      KGNGNND------DDDAA------RGASPAGSKSP---------------
Z.mays_TC510523            NGNGNNDAAADVDADAAA------RSGSPTGSKSP---------------
Z.mays_@35142              NGNGNNDAAADVDADAAA------RSGSPTGSKSP---------------
S.officinarum_TC76505      KGNVTRTMMMLPPVVPQQ------EASHP---------------------
S.officinarum_TC80184      RAVVTTTMLMLPPVVPLQ------EARSPLSLNAAPSHLWGFLMQGANFL
S.officinarum_CA282538     --------------------------------------------------
P.virgatum_TC27619         KGNGNND-----DDHDDD------RQASPTGSKSP---------------
S.bicolor_Sb09g001920      KGNNGDDE----------------RRGSPRGSQSP---------------
S.officinarum_TC107276     KGDNGDDE----------------RRGSPRGSQSP---------------
Z.mays_@11445              KGNNGDDE----------------HRGSPRGSQSP---------------
Z.mays_TC527608            KGNNGDDE----------------HRGSPRGSQSP---------------
Z.mays_TC518048            KGNNEDDE----------------RRGSPRGSQSPLN-------------
P.virgatum_TC32128         KGNAGDDD----------------DRGSPRGSQSP---------------
S.hybrid_CF570698          --------------------------------------------------
S.officinarum_TC86028      --------------------------------------------------
S.officinarum_CA177377     --------------------------------------------------
P.virgatum_TC2491          --------------------------------------------------
```

**FIGURE 2 (continued)**

```
T.aestivum_TC300145          ------------
Triae_RSZ38                  ------------
H.vulgare_@6248              ------------
T.aestivum_TC286992          ------------
T.turgidum_AJ612509          ------------
O.sativa_LOC_Os03g17710.1    ------------
O.sativa_LOC_Os03g17710.2    ------------
O.glaberrima_Og014036        ------------
O.sativa_LOC_Os05g02880      ------------
Z.mays_@11244                ------------
Z.mays_TC490304              ------------
Z.mays_@13012                ------------
S.bicolor_Sb09g001910        ------------
Z.mays_TC510523              ------------
Z.mays_@35142                ------------
S.officinarum_TC76505        ------------
S.officinarum_TC80184        CITIFSSPFLKN
S.officinarum_CA282538       ------------
P.virgatum_TC27619           ------------
S.bicolor_Sb09g001920        ------------
S.officinarum_TC107276       ------------
Z.mays_@11445                ------------
Z.mays_TC527608              ------------
Z.mays_TC518048              ------------
P.virgatum_TC32128           ------------
S.hybrid_CF570698            ------------
S.officinarum_TC86028        ------------
S.officinarum_CA177377       ------------
P.virgatum_TC2491            ------------
```

EP 2 998 401 A2

FIGURE 3

106

OsRSZ33 RRM

Terminator

pGOS2

RB

Plant expression
vector
pGOS2::OsRSZ33
RRM

Plant screenable
marker cassette

Bacterial origin of
replication

Plant selectable
marker cassette

LB

Bacterial selectable
marker

FIGURE 4

EP 2 998 401 A2

**FIGURE 5**

SM00360

```
PT54_YEAST      SVLVWNI-PTKLR-SHDILNYF-----WFYNIRSSFKIYWDD--------------EMKR
CONSENSUS/80%   .shl.sh.s.t...pcsh.p.h.....hhhphtp..hhhhst...............t.tt
CONSENSUS/65%   hlhl.sh.sst.h.p-sh.p.ht....hahphtch.thhhsp.t............sttt
CONSENSUS/50%   lVlVssl.sschs.-Eslpshhs...caaGphs+shKlhhsc.s...........spps

PT54_YEAST      NL--RFISF-ENSHDAYRFKRNYHGLLAKELLTLSE
CONSENSUS/80%   s...halpa.tp.c-uh+hh.phpshhhttp.hhsp
CONSENSUS/65%   st..lalpa.ppp--Ah+shhphsGthhss+hlpsp
CONSENSUS/50%   sp..lYlsF.pcsEDAt+AlsslsGphhsGRsl+As
```

SM00343

```
63|046363/12-173 TCYNCGQT-GHLSRECPS
CONSENSUS/80%     .ChtCsp..GHhtppC.p
CONSENSUS/65%     tCapCGp..GHhupcC.p
CONSENSUS/50%     sCapCGcs.GHhu+-Css
```

Symbols used:

| Class | Key | Residues |
|---|---|---|
| Alcohol | o | S,T |
| Aliphatic | l | I,L,V |
| Any | . | A,C,D,E,F,G,H,I,K,L,M,N,P,Q,R,S,T,V,W,Y |
| Aromatic | a | F,H,W,Y |
| Charged | c | D,E,H,K,R |
| Hydrophobic | h | A,C,F,G,H,I,K,L,M,R,T,V,W,Y |
| Negative | - | D,E |
| Polar | p | C,D,E,H,K,N,Q,R,S,T |
| Positive | + | H,K,R |
| Small | s | A,C,D,G,N,P,S,T,V |
| Tiny | u | A,G,S |
| Turnlike | t | A,C,D,E,G,H,K,N,Q,R,S,T |

MESIKTQSQGSASSVPKNPAMSSCRKKKSDDAT

FLEDLKDHIDEFIHASMDEHKHCFKNTIQKMFG

MSKVVAERSAEAKEAEVESALPLQTSVSQ

**FIGURE 6**

```
B.napus_TC90230                     --------------METKNTQAENPPKP-----AMSSCRKIVK-DDANF
B.napus_TC97660                     --------------METQKTQAENQPKP-----AMSSCRKMVK-DDANF
A.thaliana_AT3G05570.1              --------------METQKTQAENPPKP-----ATSSCRKKTK-DDANF
B.napus_TC83237                     --------METPPKDETQNPPANPNPKPAMASSCRRKVK-EDATF
B.napus_TC89650                     ------------METPPKDETQNPPPRP-----AMASCRRKVK-EDATF
A.thaliana_AT4G39235                ---------------METQTNQSPKP-----AMTSCRKKVK-DDATF
G.max_Glyma06g46580.1               -------------MDTQKSQPEKQSSSTATAPSVTSCRKKKN-EEAAF
M.truncatula_AC135566_19.5          --------------MDTQKNQAEKP---TSSAAVATSCRKKKH-EEASF
P.trichocarpa_643359                -------------METQKTQPEKQQS-----PAVTSCRRKKK-DDATF
P.trichocarpa_831254                ------------METQKSQPAKQQSPAALDPAITSCRRKKK-DDATF
S.lycopersicum_TC200119             --------------MAAENVQNEKTSSAVTPNPAMTSCRKKKS-EQATF
S.tuberosum_TC181351                --------------MAAENVQNEKAPSPVTPNPAMTSCRKKKS-EQANF
L.usitatissimum_LU04MC10588_62      -------------------MEASKGSEEKQQQEQPKPVESCRKYKK-DDASF
S.lycopersicum_DB694810             --------------------MSTEKSPLEQTSPAVASSCRKKKS-ESATF
T.aestivum_CA603660                 -------------MDSGNTQTDGSQ-PAAPKNPAMTSCRKKKTDDDVTF
T.aestivum_CK151611                 -------------MDSGNTPTDGSQ-PAAPKNPAMTSCRKKKTDDDVTF
T.aestivum_TC300853                 -------------MDSGNTQTDGSQ-PAAPKNPAMTSCRKKKTDDDVTF
T.aestivum_TC311647                 -------------MDSGNTQTEGSQ-PLP-KNPAMTSCRKKKTDDDVTF
T.aestivum_TC307541                 -------------MDSGNTQTEGSQ-PPVPKNPAMTSCRKKKTDDDVTF
H.vulgare_TC173149                  -------------MDSGNTQTEGSQ-PPVPKNPAMTSCRKKKTDDDVTF
T.aestivum_TA06MC03363_5449537      -------------MDSSTTQSEGSQ-PPVPKNPAMTSCRKKKTDDDVTF
H.vulgare_TC157023                  -------------MDSSNTQSEGSQPHVP-KNPSMSSCRKKKTDDDATL
T.aestivum_TC307720                 -------------MDSSNAQSEGSQPPVPPKNPTMSSCRKKKTDDDATF
S.bicolor_Sb01g009660.1             ------------MASSTSQTEAPA-PSVPKNPAMASCRKKKT-DDATF
Z.mays_TC535140                     -------------MASSDGQTEAAA-PSVPKNPAMASCRKKKT-DDATF
Z.mays_TC477409                     -------------MASSNGQTEAPA-PSVPKNPAMASCRKKKT-DDATF
Z.mays_TC487740                     -------------MASTNGQTEAPA-PSVPKNPAMASCRKKKT-DDVTF
O.sativa_LOC_Os03g51459.1           -------------MESIKTQS-QGSASSVPKNPAMSSCRKKKS-DDATF
O.sativa_LOC_Os08g10400.1           -------------MESSKTQS-QGSAS-VPKNPAMSSCRKKKS-DDVTF
H.vulgare_AJ473991                  ------------MSGGVQSSVPQPSSSSSFVKPASTSCRKKNSGNTSF
T.aestivum_TC313329                 -------------MSGGIKSEVPQPSSSP-PVKPTSTSCRKKNSG-TSF
S.bicolor_Sb01g035820.1             -------------MSGHTQPEGPHQLDS----KRAVNSCRKN-VPGTSF
S.officinarum_CA142694              -----------MSGHTQPEGPHQLDS----KRAVNSCRKN-VPGTSF
Z.mays_TC490320                     -------------MSGHTQPEGPHQLDS----KHAANSCRKN-VPGTSF
Z.mays_TC522093                     MAYICLHHLQSDVTMSGHTQPEGPPQLDS----KHAVNSCRKN-VPGTSF
Z.mays_TC522166                     -------------MSGHTQPEGPRQLDS----KHAVNSCRKN-VPGTSF
S.bicolor_Sb01g029160.1             -----------MTMSGRTEPEGSHQLDS----KRAVHSCRKN-VPGSSF
Z.mays_TC486459                     ---------------MTTEPQGSHQHGS----KRDLPSCRKN-VPGTSF
Z.mays_TC502630                     ---------------MTTEPQGSHQHGS----KRDLHSCRKN-VPGTSF
O.sativa_LOC_Os10g40824.1           -----------MSMPGDAQPEGSHQSASS--AKPALSSCRRNKSENTSF
                                                                      . :            :
```

# FIGURE 7

```
B.napus_TC90230                      LEDVKDHIDEFINASMDDHKNCFNKTIKKMFGLSKAVAEK-QQAEEAKGV
B.napus_TC97660                      LEDVKDHIDEFINASMDDHKNCFNKTIKKMFGLSKAVAEK-QQAEEAKGV
A.thaliana_AT3G05570.1               LEDVKDHIDDFINASMDDHKNCFNKTIKKMFGLSKAVADK-QQSEAKGGV
B.napus_TC83237                      FEDVKDHIDEFIHASMDEHKTCFQKTISKMFGLSKAVAEK-QAEEAKGGV
B.napus_TC89650                      FEDVKDHIDEFIHASMDEHKTCFQKTISKMFGLSKAVAEK-QAEG---GV
A.thaliana_AT4G39235                 FEDVKDHIDDFIHASMDEHKSCFQKTIKKMFGLSKAVAEK-QAVEAK-GV
G.max_Glyma06g46580.1                LDDLKDHIDEFINASMDEHKTCFKKTIQKMFGMSKAVAEG-QSNASK-EV
M.truncatula_AC135566_19.5           LEDVKDHIDEFINASMDEHKTCFQKTIKKMFGLSKVVAEA-NSKATK-EV
P.trichocarpa_643359                 LVDVKDHIDEFIHASMDEHKDCFKKTIKKMFGMSKIVAE--RSADAK-EV
P.trichocarpa_831254                 LEDLKDHIDEFIHASMDEHKDCFTKTIKKMFGMSKIVAE--RSGDAK-EV
S.lycopersicum_TC200119              LEDIKDHMDEFIHASMDEHKTCFKKTIQKMFGMSKIVAE--RNAEAK-EV
S.tuberosum_TC181351                 LEDIKDHMDEFIHASMDEHKTCFKKTIQKMFGMSKIVAE--RNAEAK-EV
L.usitatissimum_LU04MC10588_62       LEDVKDHIDEFINASMDEHKSCFKKTINKMFSMTRIVEK--KQAEAE-GV
S.lycopersicum_DB694810              LEDVKDHIDEFIHASRDEHASCFKKTINKMFGMSKIVAE--KNAEPK-EV
T.aestivum_CA603660                  LEDVKEHIDEFIHASMDEHKTCFKKTIQKMFGMSKVVAEV-RCS-KGSEV
T.aestivum_CK151611                  LEDVKEHIDEFIHASMDEHKTCFKKTIQKMFGMSKVVAER-SAASKEAEV
T.aestivum_TC300853                  LEDVKEHIDEFIHASMDEHKTCFKKTIQKMFGMSKVVAER-SAAAKEAEV
T.aestivum_TC311647                  LEDVKEHIDEFIHASMDEHKTCFKKTIQKMFGMSKVVAER-SAAAKEAEV
T.aestivum_TC307541                  LEDVKEHIDEFIHASMDEHKTCFKKTIQKMFGMSKVVAER-SAAAKEAEV
H.vulgare_TC173149                   LEDVKEHIDEFINASMDEHKTCFKKTIQKMFGMSKAVAER-SAAAKEAEV
T.aestivum_TA06MC03363_5449537       LEDVKEHIDEFIHASMDEHKTCFKKTIQKMFGMSKVVAER-SAAAKEAEV
H.vulgare_TC157023                   LEDVKEHIDEFINASMDEHKTCFKKTIQKMFGMSKAVAER-SAAAKEAEV
T.aestivum_TC307720                  LEDVKEHIDEFIHASMDEHKTCFKKTIQKMFGMSKVVAER-SAAAKEAEV
S.bicolor_Sb01g009660.1              LEDLKDHIDEFIHASMDEHKTCFTKTIQKMFGMSKAVAERSAAEAKEAEV
Z.mays_TC535140                      LEDLKDHIDEFIHASMDEHKTCFKKTIQKMFGMSKAVAERSAAEAKEAGV
Z.mays_TC477409                      LEDLKDHMDEFIHASMDEHKSCFKKTIQKMFGMSKAVAERSAAEANKAEV
Z.mays_TC487740                      LEDLKDHMDEFIHASMDEHKTCFKKTIQKMFGMSKAVAERSAVEANKAEV
O.sativa_LOC_Os03g51459.1            LEDLKDHIDEFIHASMDEHKHCFKNTIQKMFGMSKVVAER-SAEAKEAEV
O.sativa_LOC_Os08g10400.1            LEDLKDHIDEFIHASMDEHKHCFKNTIQKMFGMSKVVAER-SAEAKEAEV
H.vulgare_AJ473991                   VTQLRDHFHEFVHASMDEHRTCLTNTVKKLYAMSKAAAER-TAGAKEAGA
T.aestivum_TC313329                  VTQLRDHFHEFIHASMGEHRTCLTNTVKKLFAMSKAVAEG-TAGAKEAGA
S.bicolor_Sb01g035820.1              VSDLRDHIHEFINASADEHRTCFTKTIKRMFQMSKTVTER-SSEAEEAGS
S.officinarum_CA142694               VSDLRDHIHEFINASADEHRTCFTKTIKRMFEMSKIVTER-SSEAEEAGS
Z.mays_TC490320                      VSDLRDHIHEFINASADEHRTCFTKTLKRMFDMSKTVTDR-SSEAEEAGP
Z.mays_TC522093                      VSDLRDHIHEFITASADEHRTCFTKTLKRMFDMSKTVTDR-SSEAEEAGP
Z.mays_TC522166                      VSDLRDHIHEFITASADEHRTCFTKTLKRMFDMSKTVTDR-SSEAEEAGP
S.bicolor_Sb01g029160.1              VSDLKDHIHEFINASADEHRTCFTKTIKKMFGMSKTVAQR-SSEAEEAGP
Z.mays_TC486459                      VSDLRDHIHEFINASSDEHMTCFTKTIKKMFGMSMTVAHK-SSKAEEAGP
Z.mays_TC502630                      VSDLRDHIHEFINASSDEHMTCFTKTIKKMFGMSKTVAHK-SSEAEQAGP
O.sativa_LOC_Os10g40824.1            VSDLRDHIQEFIHASPNEHRTCFTKTIKRMFGMSKVVAER-STEAKEPGA
                                     . :::*:.:*: ** .:*  *: :*:.::: ::   . .
```

FIGURE 7 (continued)

```
B.napus_TC90230                      ESYLPLQTTLSD------------
B.napus_TC97660                      ESYLPLQTTLSD------------
A.thaliana_AT3G05570.1               ESYLPLQTTVSD------------
B.napus_TC83237                      ESQLPLQTTVSD------------
B.napus_TC89650                      ESQLPLQTTVSD------------
A.thaliana_AT4G39235                 ESQLPLQTTVSE------------
G.max_Glyma06g46580.1                ESSLPLQTTVQD------------
M.truncatula_AC135566_19.5           ESSLPLQTVLKE------------
P.trichocarpa_643359                 ESALPLRTTVAE------------
P.trichocarpa_831254                 ESSLPLQTTVAK------------
S.lycopersicum_TC200119              ESSLPLQTTLAK------------
S.tuberosum_TC181351                 ESSLPLQTTLAK------------
L.usitatissimum_LU04MC10588_62       ESVLPLQTSVSK------------
S.lycopersicum_DB694810              ESSLPLQTVVSE------------
T.aestivum_CA603660                  ESALPLQTSVSQ------------
T.aestivum_CK151611                  ESALPLQTSVSQ------------
T.aestivum_TC300853                  ESALPLQTSVSQ------------
T.aestivum_TC311647                  ESALPLQTSVSQ------------
T.aestivum_TC307541                  ESALPLQTSVSQ------------
H.vulgare_TC173149                   ESALPLQTSVSQ------------
T.aestivum_TA06MC03363_5449537       ESALPLQTSVSQ------------
H.vulgare_TC157023                   ESALPLETSVSQ------------
T.aestivum_TC307720                  ESALPLKTSVSQ------------
S.bicolor_Sb01g009660.1              ESALPLQTSVSQ------------
Z.mays_TC535140                      ESALPLQTSVSQ------------
Z.mays_TC477409                      ESALPLQTSVSQ------------
Z.mays_TC487740                      ESALPLQTSVSQ------------
O.sativa_LOC_Os03g51459.1            ESALPLQTSVSQ------------
O.sativa_LOC_Os08g10400.1            ESALPLQTSVSQ------------
H.vulgare_AJ473991                   ESVLPLESEVSR------------
T.aestivum_TC313329                  ESGLPLQSEVSR------------
S.bicolor_Sb01g035820.1              ESVLPLQTTVSR------------
S.officinarum_CA142694               ESVLPLQTTVSR------------
Z.mays_TC490320                      ESVLPLQTTVSR------------
Z.mays_TC522093                      ESVLPLQTTVSR------------
Z.mays_TC522166                      ESVLPLQTTAPRYKLRHFILPLEK
S.bicolor_Sb01g029160.1              ASVLPLETTVSR------------
Z.mays_TC486459                      A-----------------------
Z.mays_TC502630                      S-----------------------
O.sativa_LOC_Os10g40824.1            ESVLPLQTTVSR------------
```

# FIGURE 7 (continued)

| | 1 | 2 | 3 | 4 | 5 | 6 | 7 | 8 | 9 | 10 |
|---|---|---|---|---|---|---|---|---|---|---|
| 1. A.thaliana_AT3G05570.1 | | 77 | 71 | 71 | 87 | 88 | 61 | 37 | 58 | 59 |
| 2. A.thaliana_AT4G39235 | 84 | | 76 | 77 | 77 | 78 | 60 | 42 | 59 | 65 |
| 3. B.napus_TC83237 | 79 | 84 | | 91 | 69 | 69 | 58 | 38 | 58 | 58 |
| 4. B.napus_TC89650 | 84 | 85 | 92 | | 72 | 72 | 59 | 41 | 59 | 60 |
| 5. B.napus_TC90230 | 92 | 87 | 76 | 82 | | 96 | 58 | 36 | 60 | 62 |
| 6. B.napus_TC97660 | 92 | 87 | 74 | 83 | 98 | | 61 | 36 | 60 | 62 |
| 7. G.max_Glyma06g46580.1 | 76 | 78 | 75 | 75 | 76 | 78 | | 44 | 62 | 63 |
| 8. H.vulgare_AJ473991 | 62 | 62 | 60 | 61 | 60 | 59 | 63 | | 47 | 49 |
| 9. H.vulgare_TC157023 | 74 | 76 | 74 | 74 | 74 | 75 | 77 | 67 | | 92 |
| 10. H.vulgare_TC173149 | 73 | 77 | 72 | 72 | 73 | 74 | 77 | 69 | 96 | |
| 11. L.usitatissimum_LU04MC10588_62332478 | 78 | 78 | 69 | 78 | 80 | 80 | 70 | 59 | 72 | 71 |
| 12. M.truncatula_AC135566_19.5 | 77 | 76 | 71 | 76 | 75 | 75 | 87 | 60 | 76 | 75 |
| 13. O.sativa_LOC_Os03g51459.1 | 74 | 77 | 74 | 72 | 73 | 75 | 80 | 68 | 89 | 89 |
| 14. O.sativa_LOC_Os08g10400.1 | 73 | 77 | 71 | 73 | 72 | 75 | 80 | 66 | 89 | 90 |
| 15. O.sativa_LOC_Os10g40824.1 | 64 | 68 | 63 | 66 | 65 | 65 | 70 | 69 | 72 | 74 |
| 16. P.trichocarpa_643359 | 82 | 83 | 72 | 75 | 81 | 83 | 84 | 61 | 79 | 80 |
| 17. P.trichocarpa_831254 | 77 | 79 | 70 | 71 | 81 | 80 | 83 | 64 | 78 | 79 |
| 18. S.bicolor_Sb01g009660.1 | 76 | 78 | 75 | 74 | 75 | 75 | 76 | 69 | 91 | 89 |
| 19. S.bicolor_Sb01g029160.1 | 62 | 63 | 62 | 64 | 64 | 65 | 63 | 67 | 70 | 71 |
| 20. S.bicolor_Sb01g035820.1 | 64 | 64 | 63 | 64 | 67 | 65 | 62 | 68 | 71 | 70 |
| 21. S.lycopersicum_DB694810 | 71 | 76 | 69 | 71 | 68 | 69 | 76 | 59 | 73 | 73 |
| 22. S.lycopersicum_TC200119 | 75 | 77 | 71 | 74 | 73 | 74 | 81 | 66 | 83 | 82 |
| 23. S.officinarum_CA142694 | 64 | 64 | 63 | 64 | 67 | 65 | 62 | 68 | 71 | 70 |
| 24. S.tuberosum_TC181351 | 74 | 76 | 71 | 73 | 74 | 73 | 79 | 63 | 82 | 82 |
| 25. T.aestivum_CA603660 | 73 | 77 | 69 | 73 | 72 | 73 | 77 | 66 | 89 | 92 |
| 26. T.aestivum_CK151611 | 71 | 76 | 70 | 74 | 71 | 72 | 78 | 68 | 93 | 96 |
| 27. T.aestivum_TA06MC03363_54495378 | 73 | 76 | 71 | 74 | 72 | 74 | 76 | 65 | 95 | 97 |
| 28. T.aestivum_TC300853 | 72 | 77 | 70 | 73 | 72 | 73 | 77 | 70 | 94 | 97 |
| 29. T.aestivum_TC307541 | 72 | 76 | 71 | 71 | 72 | 73 | 76 | 68 | 95 | 99 |
| 30. T.aestivum_TC307720 | 74 | 75 | 71 | 72 | 74 | 73 | 78 | 67 | 96 | 95 |
| 31. T.aestivum_TC311647 | 73 | 77 | 72 | 73 | 73 | 74 | 78 | 68 | 95 | 98 |
| 32. T.aestivum_TC313329 | 59 | 59 | 60 | 59 | 56 | 59 | 64 | 91 | 67 | 68 |
| 33. Z.mays_TC477409 | 76 | 76 | 76 | 75 | 76 | 75 | 77 | 67 | 91 | 89 |
| 34. Z.mays_TC486459 | 51 | 56 | 50 | 55 | 53 | 53 | 52 | 52 | 54 | 54 |
| 35. Z.mays_TC487740 | 73 | 76 | 75 | 73 | 73 | 72 | 76 | 67 | 89 | 89 |
| 36. Z.mays_TC490320 | 64 | 63 | 63 | 62 | 66 | 64 | 61 | 68 | 70 | 70 |
| 37. Z.mays_TC502630 | 52 | 56 | 50 | 56 | 54 | 54 | 53 | 53 | 55 | 55 |
| 38. Z.mays_TC522093 | 56 | 55 | 56 | 56 | 59 | 57 | 55 | 63 | 62 | 63 |
| 39. Z.mays_TC522166 | 55 | 56 | 57 | 55 | 58 | 56 | 56 | 61 | 64 | 64 |
| 40. Z.mays_TC535140 | 74 | 78 | 75 | 75 | 74 | 75 | 76 | 68 | 92 | 90 |

FIGURE 8

| | 11 | 12 | 13 | 14 | 15 | 16 | 17 | 18 |
|---|---|---|---|---|---|---|---|---|
| 1. A.thaliana_AT3G05570.1 | 60,6 | 64,1 | 58,9 | 58,5 | 44,3 | 66,7 | 62,1 | 62,5 |
| 2. A.thaliana_AT4G39235 | 62,4 | 64,8 | 65,6 | 65,3 | 50 | 71,9 | 67 | 66,7 |
| 3. B.napus_TC83237 | 55,7 | 58,8 | 60,8 | 58,8 | 48,5 | 60,2 | 60,2 | 64,3 |
| 4. B.napus_TC89650 | 58,7 | 62 | 61,1 | 61,1 | 51,5 | 63,3 | 62,4 | 63,9 |
| 5. B.napus_TC90230 | 61,7 | 63 | 58,9 | 58,5 | 45,4 | 64,4 | 61,1 | 62,5 |
| 6. B.napus_TC97660 | 63,8 | 64,1 | 61,1 | 60,6 | 45,4 | 67,8 | 63,2 | 61,5 |
| 7. G.max_Glyma06g46580.1 | 56,4 | 73,4 | 62,1 | 61,1 | 51,5 | 69,1 | 71,3 | 63,5 |
| 8. H.vulgare_AJ473991 | 37,1 | 39,2 | 51,5 | 49,5 | 54,6 | 44,3 | 46,4 | 50,5 |
| 9. H.vulgare_TC157023 | 58,3 | 57,3 | 80,2 | 80,2 | 51 | 65,6 | 60,4 | 81,4 |
| 10. H.vulgare_TC173149 | 58,3 | 59,4 | 80,2 | 81,3 | 54,1 | 67,7 | 64,6 | 83,5 |
| 11. L.usitatissimum_LU04MC10588_62332478 | | 54,3 | 58,9 | 58,5 | 44,3 | 62,6 | 59,1 | 59,4 |
| 12. M.truncatula_AC135566_19.5 | 71,4 | | 57,9 | 56,4 | 50,5 | 67 | 66 | 58,3 |
| 13. O.sativa_LOC_Os03g51459.1 | 72,6 | 78,9 | | 96,8 | 57,7 | 69,5 | 68,4 | 83,3 |
| 14. O.sativa_LOC_Os08g10400.1 | 72,3 | 77,7 | 96,8 | | 56,7 | 69,1 | 66,3 | 83,3 |
| 15. O.sativa_LOC_Os10g40824.1 | 68 | 68 | 72,2 | 73,2 | | 55,7 | 57,7 | 56,1 |
| 16. P.trichocarpa_643359 | 76,9 | 82,4 | 83,2 | 83 | 71,1 | | 83,9 | 65,6 |
| 17. P.trichocarpa_831254 | 73,1 | 81,7 | 82,1 | 84 | 75,3 | 90,3 | | 67,7 |
| 18. S.bicolor_Sb01g009660.1 | 70,8 | 72,9 | 88,5 | 88,5 | 74,2 | 79,2 | 78,1 | |
| 19. S.bicolor_Sb01g029160.1 | 68,1 | 63,8 | 71,6 | 71,3 | 80,4 | 67 | 64,9 | 68,8 |
| 20. S.bicolor_Sb01g035820.1 | 66,3 | 63 | 68,4 | 69,1 | 79,4 | 67,4 | 71 | 69,8 |
| 21. S.lycopersicum_DB694810 | 70,3 | 83,5 | 76,8 | 77,7 | 67 | 85,2 | 78,5 | 75 |
| 22. S.lycopersicum_TC200119 | 74,2 | 81,7 | 87,4 | 86,2 | 74,2 | 82,8 | 81,7 | 83,3 |
| 23. S.officinarum_CA142694 | 67,4 | 64,1 | 69,5 | 70,2 | 80,4 | 68,5 | 72 | 69,8 |
| 24. S.tuberosum_TC181351 | 74,2 | 80,6 | 84,2 | 84 | 75,3 | 81,7 | 81,7 | 84,4 |
| 25. T.aestivum_CA603660 | 71,6 | 72,6 | 85,3 | 86,3 | 76,3 | 78,9 | 77,9 | 82,3 |
| 26. T.aestivum_CK151611 | 72,9 | 75 | 87,5 | 88,5 | 76,3 | 81,3 | 79,2 | 86,5 |
| 27. T.aestivum_TA06MC03363_54495378 | 72,9 | 76 | 89,6 | 91,7 | 73,2 | 81,3 | 80,2 | 89,6 |
| 28. T.aestivum_TC300853 | 71,9 | 76 | 88,5 | 89,6 | 77,3 | 81,3 | 80,2 | 87,5 |
| 29. T.aestivum_TC307541 | 71,9 | 76 | 89,6 | 90,6 | 75,3 | 81,3 | 80,2 | 87,5 |
| 30. T.aestivum_TC307720 | 71,1 | 74,2 | 86,6 | 85,6 | 72,2 | 78,4 | 78,4 | 89,7 |
| 31. T.aestivum_TC311647 | 71,6 | 76,8 | 90,5 | 91,6 | 75,3 | 82,1 | 81,1 | 87,5 |
| 32. T.aestivum_TC313329 | 58,9 | 63,2 | 67,4 | 67,4 | 68 | 60 | 62,1 | 66,7 |
| 33. Z.mays_TC477409 | 71,9 | 75 | 87,5 | 87,5 | 72,2 | 78,1 | 74 | 95,8 |
| 34. Z.mays_TC486459 | 48,4 | 53,8 | 53,7 | 53,2 | 62,9 | 52,3 | 53,8 | 54,2 |
| 35. Z.mays_TC487740 | 69,8 | 74 | 86,5 | 88,5 | 72,2 | 77,1 | 72,9 | 93,8 |
| 36. Z.mays_TC490320 | 63 | 63 | 68,4 | 68,1 | 77,3 | 64,1 | 69,9 | 69,8 |
| 37. Z.mays_TC502630 | 51,6 | 54,9 | 54,7 | 54,3 | 63,9 | 54,5 | 54,8 | 55,2 |
| 38. Z.mays_TC522093 | 54,7 | 52,8 | 62,3 | 60,4 | 70,8 | 57,5 | 60,4 | 64,2 |
| 39. Z.mays_TC522166 | 55,8 | 53,8 | 61,5 | 60,6 | 69,2 | 58,7 | 61,5 | 63,5 |
| 40. Z.mays_TC535140 | 74 | 71,9 | 88,5 | 88,5 | 75,3 | 80,2 | 76 | 94,8 |

**FIGURE 8 (continued)**

| | 19 | 20 | 21 | 22 | 23 | 24 | 25 | 26 |
|---|---|---|---|---|---|---|---|---|
| 1. A.thaliana_AT3G05570.1 | 47,4 | 47,9 | 58,9 | 55,8 | 47,9 | 56,8 | 57,3 | 55,2 |
| 2. A.thaliana_AT4G39235 | 48,4 | 49,5 | 65,2 | 61,7 | 49,5 | 60,6 | 64,2 | 63,5 |
| 3. B.napus_TC83237 | 45,4 | 47,4 | 60,8 | 57,7 | 47,4 | 57,7 | 56,7 | 57,7 |
| 4. B.napus_TC89650 | 47,4 | 50 | 62,2 | 62,4 | 50 | 61,3 | 61,1 | 62,5 |
| 5. B.napus_TC90230 | 49,5 | 51,1 | 57,8 | 60 | 51,1 | 61,1 | 57,3 | 57,3 |
| 6. B.napus_TC97660 | 49,5 | 50 | 58,9 | 58,9 | 50 | 60 | 57,3 | 57,3 |
| 7. G.max_Glyma06g46580.1 | 49,5 | 51,5 | 62,8 | 68,1 | 51,5 | 67 | 58,9 | 60,8 |
| 8. H.vulgare_AJ473991 | 51 | 54,6 | 41,2 | 44,3 | 54,6 | 44,3 | 43,4 | 44,9 |
| 9. H.vulgare_TC157023 | 52,6 | 51,5 | 59,4 | 64,6 | 51,5 | 63,5 | 80,2 | 85,4 |
| 10. H.vulgare_TC173149 | 55,7 | 55,2 | 59,4 | 67,7 | 55,2 | 67,7 | 87,5 | 92,7 |
| 11. L.usitatissimum_LU04MC10588_62332478 | 49,5 | 49,5 | 61,5 | 58,1 | 50,5 | 58,1 | 55,8 | 57,7 |
| 12. M.truncatula_AC135566_19.5 | 49 | 50 | 68,1 | 64,9 | 50 | 64,9 | 57,9 | 56,3 |
| 13. O.sativa_LOC_Os03g51459.1 | 53,1 | 52,6 | 63,2 | 69,5 | 52,6 | 68,4 | 75 | 79,2 |
| 14. O.sativa_LOC_Os08g10400.1 | 53,7 | 53,2 | 63,2 | 68,4 | 53,2 | 67,4 | 76 | 80,2 |
| 15. O.sativa_LOC_Os10g40824.1 | 67 | 71,1 | 53,6 | 53,6 | 71,1 | 53,6 | 52,5 | 54,1 |
| 16. P.trichocarpa_643359 | 52,6 | 52,7 | 70,8 | 68,8 | 53,8 | 67,7 | 64,2 | 66,7 |
| 17. P.trichocarpa_831254 | 50,5 | 53,6 | 66,7 | 69,9 | 54,6 | 69,9 | 63,2 | 63,5 |
| 18. S.bicolor_Sb01g009660.1 | 53,1 | 56,3 | 60,4 | 69,8 | 56,3 | 71,1 | 74,2 | 79,4 |
| 19. S.bicolor_Sb01g029160.1 | | 84 | 49,5 | 47,4 | 83 | 46,3 | 51 | 52,6 |
| 20. S.bicolor_Sb01g035820.1 | 91,5 | | 47,3 | 47,4 | 97,8 | 46,3 | 50,5 | 51 |
| 21. S.lycopersicum_DB694810 | 62,8 | 63 | | 69,9 | 48,4 | 66,7 | 57,9 | 60,4 |
| 22. S.lycopersicum_TC200119 | 68,1 | 68,8 | 80,6 | | 46,3 | 95,7 | 65,3 | 65,6 |
| 23. S.officinarum_CA142694 | 90,4 | 98,9 | 64,1 | 67,7 | | 47,4 | 50,5 | 51 |
| 24. S.tuberosum_TC181351 | 67 | 67,7 | 77,4 | 95,7 | 68,8 | | 63,2 | 63,5 |
| 25. T.aestivum_CA603660 | 68,4 | 67,4 | 73,7 | 82,1 | 68,4 | 80 | | 92,7 |
| 26. T.aestivum_CK151611 | 69,8 | 68,8 | 75 | 82,3 | 69,8 | 80,2 | 93,8 | |
| 27. T.aestivum_TA06MC03363_54495378 | 69,8 | 67,7 | 74 | 83,3 | 68,8 | 83,3 | 90,6 | 94,8 |
| 28. T.aestivum_TC300853 | 70,8 | 69,8 | 74 | 83,3 | 70,8 | 81,3 | 94,8 | 99 |
| 29. T.aestivum_TC307541 | 70,8 | 69,8 | 74 | 83,3 | 70,8 | 83,3 | 92,7 | 96,9 |
| 30. T.aestivum_TC307720 | 68 | 67 | 73,2 | 83,5 | 67 | 83,5 | 86,6 | 90,7 |
| 31. T.aestivum_TC311647 | 71,6 | 70,5 | 73,7 | 84,2 | 71,6 | 84,2 | 93,7 | 96,9 |
| 32. T.aestivum_TC313329 | 67,4 | 67,4 | 58,9 | 66,3 | 67,4 | 61,1 | 66,3 | 67,7 |
| 33. Z.mays_TC477409 | 66,7 | 67,7 | 74 | 84,4 | 67,7 | 85,4 | 83,3 | 86,5 |
| 34. Z.mays_TC486459 | 76,6 | 72,8 | 55,7 | 54,8 | 71,7 | 54,8 | 52,6 | 52,1 |
| 35. Z.mays_TC487740 | 65,6 | 67,7 | 72,9 | 83,3 | 67,7 | 84,4 | 85,4 | 86,5 |
| 36. Z.mays_TC490320 | 89,4 | 95,7 | 64,1 | 65,6 | 95,7 | 66,7 | 67,4 | 68,8 |
| 37. Z.mays_TC502630 | 78,7 | 75 | 56,8 | 55,9 | 73,9 | 55,9 | 53,7 | 53,1 |
| 38. Z.mays_TC522093 | 80,2 | 82,1 | 53,8 | 63,2 | 82,1 | 63,2 | 59,4 | 61,3 |
| 39. Z.mays_TC522166 | 77,9 | 81,7 | 54,8 | 57,7 | 81,7 | 58,7 | 61,5 | 63,5 |
| 40. Z.mays_TC535140 | 68,8 | 69,8 | 76 | 85,4 | 69,8 | 83,3 | 83,3 | 87,5 |

FIGURE 8 (continued)

| | 27 | 28 | 29 | 30 | 31 | 32 | 33 | 34 |
|---|---|---|---|---|---|---|---|---|
| 1. A.thaliana_AT3G05570.1 | 57,3 | 56,3 | 57,3 | 58,8 | 57,9 | 39,6 | 61,5 | 37,4 |
| 2. A.thaliana_AT4G39235 | 63,5 | 64,6 | 64,6 | 60,8 | 65,3 | 45,8 | 64,6 | 41,1 |
| 3. B.napus_TC83237 | 58,2 | 57,7 | 58,2 | 56,7 | 59,2 | 45,4 | 62,2 | 38,1 |
| 4. B.napus_TC89650 | 62,5 | 61,5 | 60,4 | 59,8 | 61,1 | 45,8 | 60,8 | 42,7 |
| 5. B.napus_TC90230 | 58,3 | 58,3 | 59,4 | 60,8 | 60 | 39,6 | 63,5 | 40,7 |
| 6. B.napus_TC97660 | 59,4 | 58,3 | 59,4 | 58,8 | 60 | 39,6 | 61,5 | 40,7 |
| 7. G.max_Glyma06g46580.1 | 60,4 | 59,4 | 60,4 | 60,8 | 61,5 | 49 | 62,5 | 45,7 |
| 8. H.vulgare_AJ473991 | 45,9 | 48,5 | 48,5 | 47,5 | 48,5 | 85,6 | 47,5 | 40,2 |
| 9. H.vulgare_TC157023 | 90,6 | 87,5 | 89,6 | 92,8 | 88,5 | 46,9 | 79,4 | 41,7 |
| 10. H.vulgare_TC173149 | 94,8 | 94,8 | 97,9 | 90,7 | 95,8 | 50 | 81,4 | 41,7 |
| 11. L.usitatissimum_LU04MC10588_62332478 | 58,3 | 56,3 | 57,3 | 56,7 | 56,8 | 42,7 | 61,5 | 37,4 |
| 12. M.truncatula_AC135566_19.5 | 59,4 | 58,3 | 59,4 | 55,7 | 60 | 43,8 | 57,3 | 46,2 |
| 13. O.sativa_LOC_Os03g51459.1 | 83,3 | 81,3 | 82,3 | 80,4 | 81,3 | 52,1 | 81,3 | 42,1 |
| 14. O.sativa_LOC_Os08g10400.1 | 85,4 | 82,3 | 83,3 | 79,4 | 83,2 | 52,1 | 81,3 | 41,5 |
| 15. O.sativa_LOC_Os10g40824.1 | 55,1 | 56,1 | 56,1 | 53,1 | 55,1 | 56,1 | 51 | 51,5 |
| 16. P.trichocarpa_643359 | 68,8 | 67,7 | 68,8 | 66 | 69,5 | 45,8 | 63,5 | 40,9 |
| 17. P.trichocarpa_831254 | 65,6 | 65,6 | 65,6 | 62,9 | 65,6 | 47,9 | 62,5 | 43 |
| 18. S.bicolor_Sb01g009660.1 | 84,5 | 81,4 | 83,5 | 82,7 | 82,5 | 51,5 | 92,7 | 41,7 |
| 19. S.bicolor_Sb01g029160.1 | 53,6 | 53,6 | 54,6 | 50 | 55,2 | 50,5 | 49 | 69,1 |
| 20. S.bicolor_Sb01g035820.1 | 53,1 | 53,1 | 54,2 | 49,5 | 54,7 | 55,8 | 52,1 | 64,1 |
| 21. S.lycopersicum_DB694810 | 60,4 | 59,4 | 60,4 | 60,8 | 60 | 45,8 | 60,4 | 41,6 |
| 22. S.lycopersicum_TC200119 | 67,7 | 67,7 | 68,8 | 67 | 68,8 | 46,4 | 70,8 | 37,6 |
| 23. S.officinarum_CA142694 | 53,1 | 53,1 | 54,2 | 49,5 | 54,7 | 55,8 | 52,1 | 63 |
| 24. S.tuberosum_TC181351 | 67,7 | 65,6 | 68,8 | 67 | 67,7 | 46,9 | 72,2 | 37,6 |
| 25. T.aestivum_CA603660 | 86,5 | 92,7 | 89,6 | 80,4 | 89,6 | 44,9 | 73,2 | 42,1 |
| 26. T.aestivum_CK151611 | 91,7 | 97,9 | 94,8 | 85,6 | 94,8 | 48 | 77,3 | 39,6 |
| 27. T.aestivum_TA06MC03363_54495378 | | 93,8 | 96,9 | 91,8 | 94,8 | 50 | 80,4 | 41,7 |
| 28. T.aestivum_TC300853 | 95,8 | | 96,9 | 87,6 | 96,9 | 49 | 79,4 | 40,6 |
| 29. T.aestivum_TC307541 | 97,9 | 97,9 | | 90,7 | 97,9 | 50 | 81,4 | 40,6 |
| 30. T.aestivum_TC307720 | 93,8 | 91,8 | 93,8 | | 88,7 | 51 | 80,6 | 38,4 |
| 31. T.aestivum_TC311647 | 96,9 | 97,9 | 99 | 91,8 | | 50 | 80,4 | 40 |
| 32. T.aestivum_TC313329 | 65,6 | 68,8 | 66,7 | 68 | 67,4 | | 46,4 | 43,2 |
| 33. Z.mays_TC477409 | 87,5 | 87,5 | 87,5 | 89,7 | 87,5 | 63,5 | | 36,5 |
| 34. Z.mays_TC486459 | 55,2 | 53,1 | 54,2 | 53,6 | 53,7 | 51,6 | 50 | |
| 35. Z.mays_TC487740 | 87,5 | 87,5 | 87,5 | 87,6 | 87,5 | 63,5 | 97,9 | 52,1 |
| 36. Z.mays_TC490320 | 67,7 | 69,8 | 69,8 | 66 | 69,5 | 67,4 | 67,7 | 71,7 |
| 37. Z.mays_TC502630 | 56,3 | 54,2 | 55,2 | 55,7 | 54,7 | 52,6 | 51 | 97,5 |
| 38. Z.mays_TC522093 | 61,3 | 62,3 | 63,2 | 62,3 | 62,3 | 62,3 | 62,3 | 60,4 |
| 39. Z.mays_TC522166 | 62,5 | 64,4 | 64,4 | 60,6 | 63,5 | 58,7 | 61,5 | 62,5 |
| 40. Z.mays_TC535140 | 88,5 | 88,5 | 88,5 | 90,7 | 88,5 | 64,6 | 96,9 | 55,2 |

**FIGURE 8 (continued)**

| | 35 | 36 | 37 | 38 | 39 | 40 |
|---|---|---|---|---|---|---|
| 1. A.thaliana_AT3G05570.1 | 60,4 | 47,9 | 38,5 | 42,1 | 39,6 | 62 |
| 2. A.thaliana_AT4G39235 | 63,5 | 47,3 | 41,1 | 42,1 | 40 | 67 |
| 3. B.napus_TC83237 | 62,2 | 46,4 | 39,2 | 42,1 | 38,5 | 65 |
| 4. B.napus_TC89650 | 62,5 | 45,2 | 44,9 | 42,1 | 39,6 | 66 |
| 5. B.napus_TC90230 | 61,5 | 48,9 | 41,8 | 43 | 42,5 | 62 |
| 6. B.napus_TC97660 | 59,4 | 47,9 | 41,8 | 42,1 | 41,5 | 63 |
| 7. G.max_Glyma06g46580.1 | 62,5 | 48,5 | 43,6 | 43,5 | 42,2 | 64 |
| 8. H.vulgare_AJ473991 | 48,5 | 53,6 | 40,2 | 48,1 | 45,9 | 48 |
| 9. H.vulgare_TC157023 | 77,3 | 49,5 | 43,8 | 42,2 | 40,7 | 81 |
| 10. H.vulgare_TC173149 | 82,5 | 53,1 | 43,8 | 45,9 | 44,4 | 84 |
| 11. L.usitatissimum_LU04MC10588_62332478 | 55,2 | 47,3 | 40,7 | 41,1 | 41 | 63 |
| 12. M.truncatula_AC135566_19.5 | 57,3 | 47,9 | 46,2 | 40,2 | 39 | 57 |
| 13. O.sativa_LOC_Os03g51459.1 | 80,2 | 51,6 | 43,2 | 44,9 | 43,9 | 83 |
| 14. O.sativa_LOC_Os08g10400.1 | 81,3 | 51,1 | 42,6 | 44,9 | 43,4 | 83 |
| 15. O.sativa_LOC_Os10g40824.1 | 52 | 69,1 | 52,6 | 60,6 | 58,7 | 57 |
| 16. P.trichocarpa_643359 | 62,5 | 46,7 | 43,2 | 43,9 | 43,8 | 67 |
| 17. P.trichocarpa_831254 | 61,5 | 51,5 | 44,1 | 45,8 | 45,9 | 66 |
| 18. S.bicolor_Sb01g009660.1 | 90,6 | 54,2 | 43,8 | 48,6 | 46,3 | 95 |
| 19. S.bicolor_Sb01g029160.1 | 49 | 81,9 | 70,2 | 73,6 | 69,8 | 53 |
| 20. S.bicolor_Sb01g035820.1 | 53,1 | 93,5 | 65,2 | 80,2 | 79,8 | 54 |
| 21. S.lycopersicum_DB694810 | 58,3 | 46,2 | 43,8 | 38,3 | 38,1 | 63 |
| 22. S.lycopersicum_TC200119 | 70,8 | 43,2 | 39,8 | 43 | 40,2 | 71 |
| 23. S.officinarum_CA142694 | 53,1 | 92,4 | 64,1 | 79,2 | 78,8 | 54 |
| 24. S.tuberosum_TC181351 | 72,2 | 44,2 | 39,8 | 41,1 | 41,1 | 71 |
| 25. T.aestivum_CA603660 | 76,5 | 48,5 | 44,2 | 42,7 | 41,3 | 74 |
| 26. T.aestivum_CK151611 | 78,4 | 49 | 42,7 | 43,5 | 41,7 | 79 |
| 27. T.aestivum_TA06MC03363_54495378 | 80,4 | 51 | 43,8 | 45,4 | 43,5 | 84 |
| 28. T.aestivum_TC300853 | 80,4 | 51 | 42,7 | 45 | 43,5 | 81 |
| 29. T.aestivum_TC307541 | 82,5 | 52,1 | 42,7 | 45,9 | 44,4 | 84 |
| 30. T.aestivum_TC307720 | 78,6 | 47,4 | 38,1 | 43,5 | 40,4 | 83 |
| 31. T.aestivum_TC311647 | 81,4 | 52,6 | 42,1 | 46,3 | 44,9 | 83 |
| 32. T.aestivum_TC313329 | 47,4 | 54,7 | 43,2 | 49,1 | 46,7 | 50 |
| 33. Z.mays_TC477409 | 95,8 | 50 | 38,5 | 45 | 42,6 | 93 |
| 34. Z.mays_TC486459 | 41,7 | 63 | 93,7 | 52,8 | 53,8 | 41 |
| 35. Z.mays_TC487740 | | 51 | 42,7 | 45,9 | 43,5 | 91 |
| 36. Z.mays_TC490320 | 67,7 | | 64,1 | 84 | 83,7 | 52 |
| 37. Z.mays_TC502630 | 53,1 | 73,9 | | 53,8 | 54,8 | 42 |
| 38. Z.mays_TC522093 | 62,3 | 84 | 62,3 | | 75,4 | 47 |
| 39. Z.mays_TC522166 | 61,5 | 83,7 | 64,4 | 84 | | 44 |
| 40. Z.mays_TC535140 | 94,8 | 68,8 | 56,3 | 63,2 | 62,5 | |

FIGURE 8 (continued)

FIGURE 9

ZPR1 (93 aas)

ZPR2 (105 aas)

ZPR3 (67 aas)

ZPR4 (72 aas)

FIGURE 10

Os011g05430 (1)

AtZPR3 (4)

AtZPR4 (5)

Os04g0411200 (7)

ZmCL15605 (10)

Os02g0530500 (8)

AtZPR1 (2)

AtZPR2 (3)

Os09g0520500 (6)

ZMPCO129214 (11)

Os12g05560 (9)

0.200 expected changes per site

FIGURE 11

MCHAISDHLESFQPSCYTDLVHKSQTSKTNKIHVLKLRRRNGERLSNEA
-----12----
                                 ---13---

KKRAKLSKLKKIMKMKNLKLYEENKIIIEENEKLRKRALLLHQENKDLF
                      ----------8----------
       --------9-------
                                    -10-
----------------------11--------------------

TQIIQQISQPSNNHN
----10-----
---11---

**FIGURE 12**

```
                                            1                                                50
              A.hypogaea_EG373732     (1) --------------------------------------------------
          G.max_Glyma09g40450.1       (1) --------------------------------------------------
                 G.max_TC286014        (1) --------------------------------------------------
  G.max_GM06MC39606_50673886@38431     (1) --------------------------------------------------
                 G.max_TC305219        (1) --------------------------------------------------
G.max_GM06MSsu40h07.r_46819511@70598   (1) --------------------------------------------------
          G.max_Glyma01g34620.1        (1) --------------------------------------------------
                 G.max_TC281725        (1) --------------------------------------------------
             P.vulgaris_FE900059       (1) --------------------------------------------------
                A.lyrata_322199        (1) --------------------------------------------------
          A.thaliana_AT2G45450.1       (1) -------------------------MQREHKTHNKLSFIYITPLYISP
               B.napus_CD822359        (1) --------------------------------------------------
                A.lyrata_899056        (1) --------------------------------------------------
          A.thaliana_AT3G60890.1       (1) --------------------------------------------------
            A.trichopoda_CK765457      (1) --------------------------------------------------
                P.abies_AM172974       (1) --------------------------------------------------
         F.sitchensis_TA14065_3332     (1) --------------------------------------------------
         T.cryptomerioides_DN975845    (1) --------------------------------------------------
                  E.tef_DN482990       (1) --------------------------------------------------
          S.bicolor_Sb02g030180.1      (1) --------------------------------------------------
         S.propinquum_TA5427_132711    (1) --------------------------------------------------
              S.hybrid_CF573336        (1) --------------------------------------------------
              S.hybrid_CF575621        (1) --------------------------------------------------
                Z.mays_BG842570        (1) --------------------------------------------------
                Z.mays_TC477977        (1) --------------------------------------------------
                Z.mays_TC529546        (1) --------------------------------------------------
      Z.mays_c62174110gm030403@15401   (1) --------------------------------------------------
  Z.mays_ZM07MC28025_BFb0123H02@27941  (1) MRRARQCVHAPPNKKPKATTQALRRGEARRDLSLYIALKPLEHFISKLSR
          Zeama_ZmPC0129214_ZPRB       (1) MRRARQCVHAPPNKKPKATTQALRRGEARRDLSLYIALKPLEHFISKLSR
       Z.mays_c62219732gm03040309841   (1) --------------------------------------------------
          O.sativa_LOC_Os09g34890.1    (1) --------------------------------------------------
            Os_ZPRa_Os09g0520500       (1) --------------------------------------------------
                C.annuum_TC13916       (1) --------------------------------------------------
           S.lycopersicum_TC205388     (1) --------------------------------------------------
             S.tuberosum_TC176950      (1) --------------------------------------------------
               N.tabacum_TC60414       (1) --------------------------------------------------
             C.intybus_EH709619        (1) --------------------------------------------------
          L.perennis_TA2167_43195      (1) --------------------------------------------------
            C.tinctorius_EL400918      (1) --------------------------------------------------
                C.melo_EB715099        (1) --------------------------------------------------
       V.vinifera_GSVIVT00026366001    (1) --------------------------------------------------
             G.hirsutum_DW518216       (1) --------------------------------------------------
             G.hirsutum_TC163217       (1) --------------------------------------------------
                T.cacao_CU522069       (1) --------------------------------------------------
         P.deltoides_TA3399_3696       (1) --------------------------------------------------
            P.trichocarpa_554264       (1) --------------------------------------------------
       V.vinifera_GSVIVT00001026001    (1) --------------------------------------------------
          S.lycopersicum_TC207895      (1) --------------------------------------------------
             S.tuberosum_EG011112      (1) --------------------------------------------------
           Triphysaria_sp_TC4136       (1) --------------------------------------------------
               G.hybrid_AJ765000       (1) --------------------------------------------------
               L.sativa_TC25720        (1) --------------------------------------------------
               L.virosa_DW156764       (1) --------------------------------------------------
             L.serriola_DW114794       (1) --------------------------------------------------
             L.serriola_DW122307       (1) --------------------------------------------------
               C.sinensis_TC13047      (1) --------------------------------------------------
             E.tirucalli_BP956571      (1) --------------------------------------------------
         P.tremula_TA8085_113636       (1) --------------------------------------------------
            P.trichocarpa_823907       (1) --------------------------------------------------
            Pt_ZPR_small_Lzipper       (1) --------------------------------------------------
             G.hirsutum_DW501389       (1) --------------------------------------------------
                T.cacao_TC3649         (1) --------------------------------------------------
             M.domestica_TC51427       (1) --------------------------------------------------
             R.chinensis_BI978399      (1) --------------------------------------------------
                    Consensus          (1)
```

**FIGURE 13**

```
                                                     51                                                    100
            A.hypogaea_EG373732   (1) ------------------MCYSSSKH-----T----------------
          G.max_Glyma09g40450.1   (1) ------------------MCSISSKQ-----T----------------
               G.max_TC286014     (1) ------------------MCSFSSKL-----T----------------
   G.max_GM06MC39606_50673886838431 (1) ------------------MCSFSSKQ-----T----------------
               G.max_TC305219     (1) ------------------MCSFSSKQ-----T----------------
   G.max_GM06MSsu40h07.r_46819511070598 (1) -------------------------------------------------
          G.max_Glyma01g34620.1   (1) ------------------MCSFSSEQ-LPYLF----------------
               G.max_TC281725     (1) ------------------MCSFSSEQ-SSPYD----------------
            P.vulgaris_FE900059    (1) ------------------MCSFSSEK-TPYHP----------------
              A.lyrata_322199     (1) ------------------MCLSSSEP-FSDTP----------------
        A.thaliana_AT2G45450.1   (24) INQTRLTYLSSSFLLSSSSKMCLSSSET-FSDTP----------------
             B.napus_CD822359     (1) ------------------MCLSSSQP-FSDTP----------------
              A.lyrata_899056     (1) ------------------MCLTSSEPPFPDTH----------------
        A.thaliana_AT3G60890.1   (1) ------------------MCLTTSEPPFPDTD----------------
         A.trichopoda_CK765457    (1) ------------------MCFCNFLSPPSSLN----------------
            P.abies_AM172974      (1) -----------------MPLDNIWTVSSSSRRRPAQRLWFGREQVVL
        P.sitchensis_TA14065_3332 (1) -----------------MPLDNIWTVSSSSRRRPAQRLWFGREQVVL
      T.cryptomerioides_DN975845  (1) -----------------MWVKRIDSLSNAKRRRVGQRLWL-------
              E.tef_DN482990      (1) ------------------MCSG---SWNSKK----------------
         S.bicolor_Sb02g030180.1  (1) ------------------MCSGSSWSWSSSSS---------------
      S.propinquum_TA5427_132711  (1) ------------------MCSGSSWSWSSSSSS--------------
            S.hybrid_CF573336     (1) ------------------MCSG-SWSWSSS-----------------
            S.hybrid_CF575621     (1) ------------------MCSG-SWSWSSS-----------------
             Z.mays_BG842570      (1) ------------------MCSG-SWSWSSSKK---------------
             Z.mays_TC477977      (1) ------------------MCSG-SWSWSSSKK---------------
             Z.mays_TC529546      (1) ------------------MCSG-SWSWSSSKK---------------
   Z.mays_c62174110gm030403015401 (1) ------------------MCSG-SWFWSSSGSG--------------
 Z.mays_ZM07MC28025_BFb0123H02027941 (51) RALKNLARSRPPTPSAVHSNMCSG-SWFWSSSGSG--------------
        Zeama_ZmPC0129214_ZPRB   (51) RALKNLARSRPPTPSAVHSNMCSG-SWFWSSSGSG--------------
   Z.mays_c62219732gm030403@9841  (1) ------------------MCSG-SWSWSSSKKRSARLLLSSTSSGASC
        O.sativa_LOC_Os09g34890.1 (1) ------------------MCSG---SWNSKNKI--------------
         Os_ZPRa_Os09g0520500     (1) ------------------MCSG---SWNSKNKI--------------
            C.annuum_TC13916      (1) ------------------MCHGVSD---LESF---------------
        S.lycopersicum_TC205388   (1) ------------------MCHAISDH--LESF---------------
         S.tuberosum_TC176950     (1) ------------------MCHAVSDH--LESF---------------
           N.tabacum_TC60414      (1) ------------------MCHACATS-QLESF---------------
           C.intybus_EH709619     (1) ------------------MCLCTSKG-----Y---------------
         L.perennis_TA2167_43195  (1) ------------------MCLGTPKE-N--IY---------------
        C.tinctorius_EL400918     (1) -------------------------------------------------
             C.melo_EB715099      (1) ------------------MCHSSRFI-LTPSY---------------
     V.vinifera_GSVIVT00026366001 (1) ----------------------MLVLF--------------------
          G.hirsutum_DW518216     (1) -----------------MCKNMDWSPYPSHP----------------
          G.hirsutum_TC163217     (1) -----------------MCNNMNWSPSHPIL----------------
             T.cacao_CU522069     (1) -----------------MCNNMDWSPTIS------------------
       P.deltoides_TA3399_3696    (1) -----------------MCTNGVDRIPSPFH----------------
        P.trichocarpa_554264      (1) -----------------MCTNGVDRIPSPFH----------------
     V.vinifera_GSVIVT00001026001 (1) -----------------MCMSTARWIPSR------------------
       S.lycopersicum_TC207895    (1) -----------------MCISSTEWSNNSSR----------------
         S.tuberosum_EG011112     (1) -----------------MCISSTEWSNNSSR----------------
        Triphysaria_sp_TC4136     (1) ------------------MCQG------SN-----------------
          G.hybrid_AJ765000       (1) -----------------MCTNKSNISKCISS----------------
            L.sativa_TC25720      (1) -----------------MYTYN---SKCVSS----------------
            L.virosa_DW156764     (1) -----------------MCTYN---SKCVSS----------------
          L.serriola_DW114794     (1) -----------------MCTSN--TTECMSP----------------
          L.serriola_DW122307     (1) -----------------MCTSN--TTECMSP----------------
          C.sinensis_TC13047      (1) -----------------------MTP-SLCSG---------------
         E.tirucalli_BP956571     (1) -----------------MCFITSSSTL-CPYSS--------------
       P.tremula_TA8085_113636    (1) -----------------MCTGTCSKL-RPCSP---------------
        P.trichocarpa_823907      (1) -----------------MCTGTCSKL-SPCSP---------------
        Pt_ZPR_small_Izipper      (1) -----------------MCTGTCSKL-SPCSP---------------
          G.hirsutum_DW501389     (1) ----------------MLVNSS-------------------------
             T.cacao_TC3649       (1) -----------------MCMISSES-IPSRS----------------
         M.domestica_TC51427      (1) -----------------MCLN-SEL-SPSSP----------------
         R.chinensis_BI978399     (1) -----------------MCRNSSEM-IPYSP----------------
                  Consensus      (51)                   MC   S
```

**FIGURE 13 (continued)**

```
                                                           101                                  150
            A.hypogaea_EG373732   (10)  ------LNVPLVSSCSSK-------TQHLQLSFLRRRRAQLKEARER---
           G.max_Glyma09g40450.1  (10)  ------LYLVLPRPWPSKRHHHN--HRHLRLGMLNRRRAHLKEARQR---
                  G.max_TC286014   (10)  ------LYLVLPRPWPSKRHHHN--HRHLRLGMLNRRRAHLKEARQR---
   G.max_GM06MC39606_50673886838431 (10) ------LYLALPRPWTSKRHHHS--HRHLRLGMLNRRRANLKEARQR---
                  G.max_TC305219   (10)  ------LYLALPRPWTSKRHHHS--HRHLRLGMLNRRRAHLKEARQR---
   G.max_GM06MSsu40h07.r_46819511070598 (1) --------------------------MLTRRRAHLKEPRQR---
           G.max_Glyma01g34620.1  (14)  ------LGFALHPPWPSKRQ-----HQHLRLNIHKRRRAQLQEARRR---
                  G.max_TC281725   (14)  ------DLPLVHPPWPSKRQ-----PQHLRFSLHKRRRAQLQEARRR---
             P.vulgaris_FE900059   (14)  ------LVLALQPPCPSKR------HHHLRLSLHKRRRAQVEBRRR----
                  A.lyrata_322199   (14)  ------TRLVLY--------LKT--QSHVRIPRLSRRRRMWREEK-----
            A.thaliana_AT2G45450.1 (57)  ------TRLVLY--------LKT--QSHVRIPRLSRRRRMWREEK-----
                 B.napus_CD822359   (14)  ------TRLVLY--------LKT--QTHVRIPRLSRRRRMWRKEK-----
                  A.lyrata_899056   (15)  ------TPTMRPSSYHNKHKSKT--QSHLRTLNLTRRRRLLKEQK-----
            A.thaliana_AT3G60890.1 (15)  ------TPTMRSASYHIKHKSKT--QTHLRILNLTRRRRLLKEQK-----
            A.trichopoda_CK765457  (15)  ------PSFAKLRIKKP-----------KVRILIRRR-----------
                  P.abies_AM172974  (31)  GQFLQRRSFSIKQRIKRRPILYSLAPRAAKAWLAFRRRS----KR-----
           P.sitchensis_TA14065_3332 (31) GQFLQRRSFSFKQRIKRRPILYSLAPRAAKAWLAFRRRS----KR-----
      T.cryptomerioides_DN975845    (24)  ----------ISAVDKRHPTLS-----SVKAWVVCRRRV----KR-----
                    E.tef_DN482990  (11)  -----HQPFLVLKQQ---------RK--PHVSVVIRRKR----------
            S.bicolor_Sb02g030180.1 (15)  ---KKHPSLVVVNLER--QLRKTTTSH---VSVAIRRKR-----------
          S.propinquum_TA5427_132711 (16) --SKKHPSLVVVNLER--QLLKTTTSH---VSVAIRRKR-----------
               S.hybrid_CF573336   (12)  ---KKHPSLVVVNLKR--QLQKTPPPH---VSVAIRRKR-----------
               S.hybrid_CF575621   (12)  ---KKHPSLVVVNLKR--QLQKTQPPH---VSVAIRRKR-----------
                  Z.mays_BG842570   (14)  ---R--PSLVVVNLKRQ-QLQKTPPPTPPRASVAIRRKRYFANPPCSSFP
                  Z.mays_TC477977   (14)  ---R--PSLVVVNLKRQ-QLQKTPPPTPPRASVAIRRK-----------
                  Z.mays_TC529546   (14)  --RRRPSLVVVNLKRR-QLQKTPPPTPPRSSVAIRRK-----------
       Z.mays_c62174110gm030403015401 (15) ---KEHPSLVVVNLER--QLKKTLAVR--------RRRR---------
   Z.mays_ZM07MC28025_BFb0123H02027941 (85) ---KEHPSLVVVNLER--QLKKTLAVR-------RRRR---------
                Zeama_ZmPC0129214_ZPRB (85) ---KEHPSLVVVNLER--QLKKTLTVR-------RRRR---------
       Z.mays_c62219732gm030403@9841 (30) RRRRRRRHLVPQLLSGGRGTQLTRPARVSQASFAQVSAPSATVSCN----
           O.sativa_LOC_Os09g34890.1 (13) -----YLSLVVFRQDNQ---QQQQRKQPAHAPMALRRKR-----------
             Os_ZPRa_Os09g0520500   (13)  -----YLSLVVFRQDNQ---QQQQRKQPAHAPMALRRKR-----------
                 C.annuum_TC13916   (12)  ------QTSCYTDLVHRPDPEF---SKTAKAHVVSLRRRNGERLS-----
          S.lycopersicum_TC205388    (13)  ------QPSCYTDLVHKSQT-----SKTNKIHVLKLRRRNGERLSNEA----
            S.tuberosum_TC176950    (13)  ------QPSCYTDLVHRPQT-----SKTTKIHVLKLRRRNGERLSNEA--
                N.tabacum_TC60414   (14)  ------QPSCYTDFHSPQLS------KTAKIHVLKLR-RNGGRLSKEA--
                C.intybus_EH709619  (10)  -------DPSHLTTGEQLKR-------SNFDFHHLQ-RKRIMGMKNPRN--
            L.perennis_TA2167_43195 (12)  ------RLSYLTTHQEQLKR-----SSSFHFHHLQSRKRVMGMKNSRN--
              C.tinctorius_EL400918  (1)  --------------------------------------MGKKNPRN--
                    C.melo_EB715099 (14)  ------LACRHHRRRRPVRV-------YSLLRRMRFATEEANSAG-----
      V.vinifera_GSVIVT00026366001  (6)  ------VWMRSRRTRK-------------ES----KDKAMMVVG-----
              G.hirsutum_DW518216   (15)  ------TIASSK-RRHRLKH------SKVQVYRLSRKR-----------
              G.hirsutum_TC163217   (15)  ------VSS--K-RRHRSKQ------SKVQIYRLSRKR-----------
                  T.cacao_CU522069  (13)  ------ASS--K-RRHRSKQ------SKVQIYRLSRKR-----------
            P.deltoides_TA3399_3696 (15)  ------PLK----RRQRSKK------CKVQVIGLTCRRS----------
            P.trichocarpa_554264    (15)  ------PSK----RRQRSKK------CKVQVIGLTCRRS----------
      V.vinifera_GSVIVT00001026001  (13) --------------PRTKR------SKVQVHRLTRRR-----------
          S.lycopersicum_TC207895   (15)  ------PLYFSMQKKQRPKI------SRVQLHRLARKKR----------
             S.tuberosum_EG011112   (15)  ------PLYFSMQKKQRPKI------SRVQLHRLARKKR----------
            Triphysaria_sp_TC4136   (7)  --------TRLIQAHKLRFFPRRDKRTCVRVLGLSKR------------
              G.hybrid_AJ765000     (15)  ------TSLNFRTHKNKFK--RALIRIQIRIHRFKRAH-----------
                L.sativa_TC25720    (12)  ------PLLSSPTRKMKSN--RVRKHIQVRLHRFNRR-----------
                 L.virosa_DW156764  (12)  ------PLPSSPIRKMKSN--RLRKHIQVRLHRFNRR-----------
              L.serriola_DW114794   (13)  ------PSYYFLNQKTKSKQAKFRMRNQVRIHRLNRYIYILCFLWVLLTF
              L.serriola_DW122307   (13)  ------PSYYFLNQKTKSKQAKFRMRNQVRIHRLN--------------
               C.sinensis_TC13047   (9)  ------CLLHFAYRRPCRRH-------NFRVRRLNRGRRLSSALN-----
             E.tirucalli_BP956571   (16)  ------LILAAANTKPSRGF-------NVQYRRLSRRRRVSREAKKK---
          P.tremula_TA8085_113636   (15)  ------LYIALSNQQPSKGH-------NLRYHRLNRRRRLSKGKR-----
             P.trichocarpa_823907   (15)  ------LYIALSNQQPSKGH-------NLRYHRLNRRRRLSKGKR-----
          Pt_ZPR_small_Lzipper      (15)  ------LYIALSNQQPSKGH-------NLRYHRLNRRRRLSKGKR-----
              G.hirsutum_DW501389   (7)  --------SCCRKRQSKLRH-------NVRVPRLNRLRRLRT--------
                 T.cacao_TC3649     (14)  ------LYSFYRKRPS-KRH-------NVLVRRLNRWRRLRKGAG-----
              M.domestica_TC51427   (13)  ------LYSSPHLRRPSKQH-------SFRVRRLINRNWKRKPAGPA---
              R.chinensis_BI978399  (14)  ------LYSSFRLRRPSKHH-------SFRVHRLINR--RRS----I---
                      Consensus    (101)         L                   VRV L RRRR
```

FIGURE 13 (continued)

```
                                                          151                                          200
                      A.hypogaea_EG373732   (44)  --------------------------------RKITVVKT-QIKMRNLKLY
                    G.max_Glyma09g40450.1   (49)  --------------------------------KRIVVVKS-EIQMKNLKLY
                          G.max_TC286014    (49)  --------------------------------KRIVVVKS-EIQMKNLKLY
           G.max_GM06MC39606_50673886038431 (49)  --------------------------------K-RIVVKS-EIQMKNLKLY
                          G.max_TC305219    (49)  --------------------------------K-RIVVKS-EIQMKNLKLY
       G.max_GM06MSsu40h07.r_46819511070598 (16)  --------------------------------K-RIVVKS-EIQMKNLKLY
                    G.max_Glyma01g34620.1   (50)  --------------------------------KRMIIIKLTRIKMRNLKLY
                          G.max_TC281725    (50)  --------------------------------KRMIIIK-TEIKMRNLKLY
                     P.vulgaris_FE900059    (48)  --------------------------------KKMILKK--EMKMRNLKLY
                        A.lyrata_322199     (43)  ---------------------------------KMEMINLKLY
                   A.thaliana_AT2G45450.1   (86)  ---------------------------------KMEMINLKLY
                       B.napus_CD822359     (43)  ---------------------------------EMEMKNIRLY
                        A.lyrata_899056     (52)  ---------------------------------VMEMRNLKLF
                   A.thaliana_AT3G60890.1   (52)  ---------------------------------EMEMRNLKLF
                     A.trichopoda_CK765457  (35)  --------------------------------FEEASEMEISNLELY
                       P.abies_AM172974     (72)  --------------------------------KIDKEASRIERILNLQLF
                 P.sitchensis_TA14065_3332  (72)  --------------------------------KIDKEASRIERILNLQLF
                T.cryptomerioides_DN975845  (50)  --------------------------------TSDKEALRIQRILNLQLY
                       E.tef_DN482990       (34)  ----------------------LRLRWR------AGAETMEMVNLKLY
                   S.bicolor_Sb02g030180.1  (46)  --------------------LRLRRL---RLRAGAEAMEMILNLKLY
                S.propinquum_TA5427_132711  (48)  --------------------LRLRRL---RLRAGAEAMEIILNLKLY
                    S.hybrid_CF573336       (43)  ---------------------LRLR-----RLRGGAEAMEIILNLKLY
                    S.hybrid_CF575621       (43)  ---------------------LRLR-----RLRAGAEAMEIINLKLY
                     Z.mays_BG842570        (58)  SFLCSSFSSISDSFLQSHKYTVHVRRLRLR---RLRAGAEAMEMVNLKLY
                     Z.mays_TC477977        (46)  -------------------R-LRLR---RLRAGAEAMEMVNLKLY
                     Z.mays_TC529546        (48)  -------------------R-LRLR---RLRAGAEAMEMVNLKLY
            Z.mays_c62174110gm030403015401  (41)  --------------------LRLR-R---LRDGGAEAMEMVNLKLY
     Z.mays_ZM07MC28025_BFb0123H02027941    (111) --------------------LRLR-R---LRDGGAEAMEMVNLKLY
                 Zeama_ZmPC0129214_ZPRB     (111) --------------------LRLR-R---LRAGGAEAMEMVNLKLY
            Z.mays_c62219732gm03040309841   (76)  ----------HTSTLYTYVRRLRLR---RLRAGAEAMEMVNLKLY
               O.sativa_LOC_Os09g34890.1    (44)  -----------------LRLRRRRETMRRSDGMEMEMVNLKLY
                 Os_ZPRa_Os09g0520500       (44)  -----------------LRLRRRRETMRRSDGMEMEMVNLKLY
                      C.annuum_TC13916       (48)  -----------------KERAKLRKLKKIMKMKNLKLY
                   S.lycopersicum_TC205388  (50)  -----------------KKRAKLSKLKKIMKMKNLKLY
                   S.tuberosum_TC176950     (50)  -----------------KKRAKLRKLKKLMKMKNLKLY
                     N.tabacum_TC60414      (49)  -----------------KERTRLRKLKKAMKMKNLKLY
                    C.intybus_EH709619      (44)  -----------------KTRVLFVMLKRKMAMKNLKLY
                 L.perennis_TA2167_43195    (49)  -----------------KTRVLFVMLKRKMAMKNLKLY
                   C.tinctorius_EL400918    (9)   -----------------KKKVLFFVLKRKMAMKNLKLY
                       C.melo_EB715099      (46)  -----------------------VKVEMEIKNLKLY
         V.vinifera_GSVIVT00026366001       (27)  -----------------------VKSEMEIKNWKLY
                   G.hirsutum_DW518216      (40)  ----------------WEEN-MKKDMDLMNLKLY
                   G.hirsutum_TC163217      (38)  ----------------CEEED-VKDMELMNLKLH
                     T.cacao_CU522069       (36)  ----------------CEEN-VEKDMERILNLKLY
                P.deltoides_TA3399_3696     (38)  ----------------GYEDWEEKNMELKNLKLY
                 P.trichocarpa_554264       (38)  ----------------GYEEWEEKNMELKNLKLY
         V.vinifera_GSVIVT00001026001       (30)  ----------------RCEVQVEKDMELKNLKLY
                S.lycopersicum_TC207895     (42)  ----------------GEGKETKVDMEMRNLKLY
                  S.tuberosum_EG011112      (42)  ----------------SEGKETKVDVEMRNLKLY
                Triphysaria_sp_TC4136       (36)  --------------DEKMDKNMEEKNMKLY
                   G.hybrid_AJ765000        (45)  ------------K-----FIDEKIKREMELKNLKLY
                   L.sativa_TC25720         (41)  ------------------INEKMKQEMEFKNLKLY
                   L.virosa_DW156764        (41)  ------------------INEKMKQEMEFKNLKLY
                  L.serriola_DW114794       (57)  IFFLSIGLIILFEC------GFLSFVCFRVDKFIEEKIKQEMELKNLKLY
                  L.serriola_DW122307       (42)  --------------------RVDKFIEEKIKQEMELKNLKLY
                     C.sinensis_TC13047     (41)  ----------------------ETMELKRKNLKLY
                   E.tirucalli_EP956571     (50)  -----------------NKMGSVN-IQNEMKIKNLKLY
                 P.tremula_TA8085_113636    (47)  -----------------VALVL-VKKEMEIKNLKLF
                  P.trichocarpa_823907      (47)  -----------------VALVL-VKKEMEIKNLKLY
                 Pt_ZPR_small_Lzipper       (47)  -----------------VALVL-VKKEMEIKNLKLY
                   G.hirsutum_DW501389      (34)  -----------------KKMGAVG-IKTDMEIKNLKLY
                     T.cacao_TC3649         (45)  -----------------TKKVVVG-VQTDMQMKNLKLY
                  M.domestica_TC51427       (47)  -----------------AKEAETGGAKVEMEMKNLKLY
                  R.chinensis_BI978399      (42)  -----------------TKEAKE---KEMEIKNLKLY
                          Consensus         (151)                      EMEMKNLKLY
```

# FIGURE 13 (continued)

```
                                              20L                                    250
A.hypogaea_EG373732            (62) KENQNLLNENERLRNQAMLLYKENQDLLSKLQKKPSQQNNKNN-------
G.max_Glyma09g40450.1          (67) MENQTIIEENEKLRKQAMLLHKENQALLSQLQKKLSEQNNNTNNN-----
G.max_TC286014                 (67) MENQTIIEENEKLRKQAMLLHKENQALLSQLLKKLSEQNYYTNYN-----
G.max_GM06MC39606_50673886838431 (66) MENQTIIEENEKLRKQAMLLIHKENQALSSQLQKKLSGQNNNTNNN-----
G.max_TC305219                 (66) MENQTIIEENEKLRKQAMLLHKENQALSSQLQKKLSGQNNNTNNN-----
G.max_GM06MSsu40h07.r_46819511070598 (33) MENXPIIEENEKLRKQAMLLHKENQALSSQLQKKLSGQNNNTNNN-----
G.max_Glyma01g34620.1          (69) MENQSIIEENQKLRKQAMLLHKENDVLLFQLQKKLSEQNNSKTN------
G.max_TC281725                 (68) MENQSIIEENEKLRKQAVLLHKENEALLFQLQKKLSEQNNSKTNQIAVS-
P.vulgaris_FE900059            (65) MKNQSIIEENEKLRKQALLLHKENETLLFELQNKFSQQSNIKTK------
A.lyrata_322199                (53) VENQNIIRENEKLRKKALLLHQENKALFSLLQTKKLSSVP---------
A.thaliana_AT2G45450.1         (96) VENQNIIRENEKLRKKALLLHQENKTLFSLLQTKKLSSVHK---------
B.napus_CD822359               (53) MENQYIIQENEKLRKKALLLHQENKALFSQLQTKNVSHVP----------
A.lyrata_899056                (62) VANQSIMRENEALKKKALLLHQENNALFALLHPKPSPVATSLLL------
A.thaliana_AT3G60890.1         (62) VENQSIIRENEALKKKALLLHHENNALFALLHPKYSPVSTSLLQ------
A.trichopoda_CK765457          (50) LQNWCIIEENERLRKKALVLDQENRALLSELNKKFSGFNKVVAKP-----
P.abies_AM172974               (89) QRNQCIVEENERLRKQALLLTEENQLLQQKLQEQLRCPDKVLGA------
P.sitchensis_TA14065_3332      (89) QRNQCIVEENERLRKQALLLTEENQLLQQKLQEQLRCPDKVLGA------
T.cryptomerioides_DN975845     (67) ERNQSIVQENERLRKQALLLSEENQNLQQKLQEQISMPEQ----------
E.tef_DN482990                 (54) LENRCIIAENERLREKANALRRENLALRENLS-KTVAAELPAAGARAA--
S.bicolor_Sb02g030180.1        (69) LENRCILAENERLRERATALRRENLALLQNLS-KTAAVPEAGAGAA----
S.propinquum_TA5427_132711     (71) LENRCILAENERLRERATALRRENLALLQNLS-KTAAVAEAGAGAA----
S.hybrid_CF573336              (64) LENRCIIAENERLREKATALRRENLALLQNLS-KTAAVPEAGAGAGTA--
S.hybrid_CF575621              (64) LENRCIIAENERLREKATALRRENLALLQNLS-KTAAVPEAGAGAGTA--
Z.mays_BG842570                (105) LENRCIIAENERLRERASALRRENLALLQDLS-KTPAVPEAGAGAGAA--
Z.mays_TC477977                (68) LENRCIIAENEKLRERASALRRENLALLQDLS-KTPAVPEAGAGAGAA--
Z.mays_TC529546                (70) LENRCIIAENEKLRERASALRRENLALLQDLS-KTPAVPEAGAGAGAGAA
Z.mays_c62174110gm0304030l5401 (63) LENRCIIAENERLRERASALRRENLALLQNLSSRTAAVPEPGAGAA----
Z.mays_ZM07MC28025_BFb0123H02027941 (133) LENRCIIAENERLRERASALRRENLALLQNLSSRTAAVPEPGAGAA----
Zeama_ZmPC0129214_ZPRB         (133) LENRCIIAENERLRERASALRRENLALLQNLSSRTAAVPEPGAGAA----
Z.mays_c62219732gm03040309841  (108) LENRCIIAENEKLRERASALRRENLALLQDLS-KTPAVPEAGAGAGAGAA
O.sativa_LOC_Os09g34890.1      (70) LENRCILEENERLREKASALHRENLALRADLR-NTSSPATTAAAASSC--
Os_ZPRa_Os09g0520500           (70) LENRCILEENERLREKASALHRENLALRADLR-NTSSPATTAAAASSC--
C.annuum_TC13916               (69) MENNKTIIEENEKLRKQALLLHQENKALFSQMLIQQVSQNPN--------
S.lycopersicum_TC205388        (71) EENKIIIEENEKLRKRALLLHQENKDLFTQIIQQISQPSNNHN-------
S.tuberosum_TC176950           (71) KENKIIIEENEKLRKRALLLHQENKAMFTQIIQQISQPSNNHNY------
N.tabacum_TC60414              (70) KENKTIIEENERLRKKALLLHQENKALFSKLQQVFQSPNHD---------
C.intybus_EH709619             (65) IQNQCIIEENEKLRKKALLLHQENQVLLCQLQNVKINPQNINTDVIV---
L.perennis_TA2167_43195        (70) MQNQFIIEENEKLRRKALLLHQENQVLFYQLQNVKINKHNIDTDVVV---
C.tinctorius_EL400918          (30) MQNQCMIEENEKLRRKALVLHQENQILFSQLQNAKINQHNINNNAIV---
C.melo_EB715099                (59) MENQSIIEENERLRKKAFLLHKENQVLLSQLQNFSDYKPPQSSSDLSACS
V.vinifera_GSVIVT00026366001   (40) MLNQSIIQFNEKLRMKALLLHQENQALLSQLQNKFKTSPHNNNH------
G.hirsutum_DW518216            (57) LENQSIIEENEKLRKKASLLHQENLALMSEYQKKFPHLDRFSTTLLLLLQ
G.hirsutum_TC163217            (56) LENRSIIEENEKLRKKANLLHQENLALMSELQNKFPHLDRFSTTLLVLLQ
T.cacao_CU522069               (53) LENQSIIEENEKLRKKANLLHQENLALMSDFQKKFPHLDRFSTTLLLLLR
P.deltoides_TA3399_3696        (56) LENQSIVEENEKLRKKASLLHQENLALMSELQKKFPHLDRFSNTLLLLHK
P.trichocarpa_554264           (56) LENQSIVEENEKLRKKASLLHQENLALMSELQKKFPHLDRFSNTLLLLHK
V.vinifera_GSVIVT00001026001   (48) LENRNIMEENEKLRKKATLLHQENLALMSEFQTKFPHFNRFSTTLFLVLN
S.lycopersicum_TC207895        (60) MENMSILEENEKLRKKASLLHEENIALMFEPQKKFCKFDRVSNKETPHLP
S.tuberosum_EG011112           (60) MENMSILEENEKLRKKASLLHEENIALMTEFQKKFSKFDRVSTKETPHLP
Triphysaria_sp_TC4136          (52) MENMSILLENEKLRRQANLLHQERLALISQFQNRFPDDK----------
G.hybrid_AJ765000              (64) MENMSILKENEKLRKKASLLHQENLVLLSELKKKSSPLQESLSS------
L.sativa_TC25720               (58) MENMNILKENAELWKKAIQLREENTILLSKLVKNIKPSGS----------
L.virosa_DW156764              (58) MENMNILKENAELWKKAIQLREENTVLLSELAKKIKPSGS----------
L.serriola_DW114794            (101) MENMSILEENERLRNKATMLHQENLALLSLLENKRFNL------------
L.serriola_DW122307            (64) MENMSILEENERLRNKATMLHQENLALLSLLENKRFNL------------
C.sinensis_TC13047             (54) MENQSLIKENEKLRNKALLLHQENQALLSQSLFNLRLISSIPLPFFP---
E.tirucalli_BP956571           (70) MENKSIIQENEKLRKKALLLHQENQALLFQLKNNFSKNF-----------
P.tremula_TA8085_113636        (65) MENKSIIEENEKLRKKAFLLHQENQALLYELQKKRSDPRHDHLAHSNS--
P.trichocarpa_823907           (65) MENKSIIEENEKLRKKAFLLHQENQALLYQLQKKRSNPRHDHLAHSNN--
Pt_ZPR_small_Lzipper           (65) MENKSIIEENEKLRKKAFLLHQENQALLYQLQKKRSNPRHDHLAHSNN--
G.hirsutum_DW501389            (54) MENQIIIQENKKLRKKALLLHQENQTLLAQLQKKLCNPPNQSLKT-----
T.cacao_TC3649                 (65) MQNQIIIEENERLRKKAILLHQENQTLLAQLQKKLSNPQNSS--------
M.domestica_TC51427            (68) MENQSIIEENTKLRRKALLLVQENQALFSQLQQKQLFLNKTTAAV-----
R.chinensis_BI978399           (59) MENKSIIEENNKLRRKALLLVQENQALFSQLQQKISDHACSATMKQDH--
Consensus                      (201) MEN SIIEENEKLRKKALLLHQEN ALLSQLQ K S
```

FIGURE 13 (continued)

```
                                              251
            A.hypogaea_EG373732    (105)  --------
         G.max_Glyma09g40450.1    (112)  --------
               G.max_TC286014    (112)  --------
  G.max_GM06MC39606_50673886@38431  (111)  --------
               G.max_TC305219    (111)  --------
 G.max_GM06MSsu40h07.r_46819511@70598  (78)  --------
         G.max_Glyma01g34620.1    (113)  --------
               G.max_TC281725    (117)  --------
           P.vulgaris_FE900059    (109)  --------
             A.lyrata_322199     (93)  --------
        A.thaliana_AT2G45450.1    (137)  --------
             B.napus_CD822359     (93)  --------
             A.lyrata_899056    (106)  --------
        A.thaliana_AT3G60890.1    (106)  --------
          A.trichopoda_CK765457     (95)  --------
              P.abies_AM172974    (133)  --------
        P.sitchensis_TA14065_3332  (133)  --------
      T.cryptomerioides_DN975845    (107)  --------
               E.tef_DN482990    (101)  --------
         S.bicolor_Sb02g030180.1   (114)  --------
       S.propinquum_TA5427_132711   (116)  --------
              S.hybrid_CF573336    (111)  --------
              S.hybrid_CF575621    (111)  --------
               Z.mays_BG842570    (152)  --------
               Z.mays_TC477977    (115)  --------
               Z.mays_TC529546    (119)  --------
     Z.mays_c62174110gm030403@15401   (109)  --------
 Z.mays_ZM07MC28025_BFb0123H02@27941   (179)  --------
       Zeama_ZmPC0129214_ZPRB    (179)  --------
      Z.mays_c62219732gm030403@9841   (157)  --------
         O.sativa_LOC_Os09g34890.1   (117)  --------
             Os_ZPRa_Os09g0520500    (117)  --------
             C.annuum_TC13916    (110)  --------
           S.lycopersicum_TC205388    (114)  --------
           S.tuberosum_TC176950    (115)  --------
             N.tabacum_TC60414    (111)  --------
             C.intybus_EH709619    (112)  --------
       L.perennis_TA2167_43195    (117)  --------
          C.tinctorius_EL400918     (77)  --------
               C.melo_EB715099    (109)  WESKQNHL
      V.vinifera_GSVIVT00026366001     (84)  --------
           G.hirsutum_DW518216    (107)  QKH-----
           G.hirsutum_TC163217    (106)  KH------
             T.cacao_CU522069    (103)  NH------
        P.deltoides_TA3399_3696    (106)  H-------
          P.trichocarpa_554264    (106)  H-------
      V.vinifera_GSVIVT00001026001     (98)  KQ------
        S.lycopersicum_TC207895    (110)  TTKLTK--
          S.tuberosum_EG011112    (110)  TTKLTK--
        Triphysaria_sp_TC4136     (91)  --------
           G.hybrid_AJ765000    (108)  --------
             L.sativa_TC25720     (98)  --------
             L.virosa_DW156764     (98)  --------
           L.serriola_DW114794    (139)  --------
           L.serriola_DW122307    (102)  --------
            C.sinensis_TC13047    (101)  --------
            E.tirucalli_BP956571    (109)  --------
         P.tremula_TA8085_113636    (113)  --------
          P.trichocarpa_823907    (113)  --------
          Pt_ZPR_small_Lzipper    (113)  --------
           G.hirsutum_DW501389     (99)  --------
               T.cacao_TC3649    (107)  --------
          M.domestica_TC51427    (113)  --------
           R.chinensis_BI978399    (107)  --------
                    Consensus    (251)
```

# FIGURE 13 (continued)

| | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| 1 | | 38,9 | 38,1 | 35 | 33,8 | 35,7 | 32,2 | 38,9 | 73,3 | 38,1 | 37,2 | 36,1 | 34,2 | 33,3 | 40,7 |
| 2 | 68,1 | | 40 | 36,5 | 27,5 | 36,5 | 33 | 34,3 | 36,6 | 36 | 38,5 | 33,9 | 30 | 29,6 | 35,7 |
| 3 | 59,3 | 59,6 | | 58,1 | 64,7 | 57,1 | 45,5 | 83,7 | 47,7 | 36,6 | 39,8 | 40,8 | 34,3 | 38,8 | 44,5 |
| 4 | 56,6 | 62,9 | 70,5 | | 40,3 | 87,6 | 35,8 | 51,4 | 34,2 | 33,6 | 35,4 | 36 | 27,8 | 31,3 | 32,7 |
| 5 | 52,9 | 50,7 | 65,4 | 52,9 | | 40,3 | 31,9 | 55,1 | 34,3 | 28,9 | 29,4 | 30,3 | 25,2 | 26 | 34,5 |
| 6 | 55,8 | 61,9 | 73,3 | 93,3 | 56,6 | | 37,7 | 53,3 | 36,7 | 33,9 | 34,9 | 35,4 | 25,9 | 29,5 | 35 |
| 7 | 51,3 | 56,7 | 66 | 55,2 | 47,1 | 59 | | 43,4 | 33,3 | 38,6 | 39,5 | 33,6 | 40,6 | 38,2 | 39,5 |
| 8 | 58,4 | 58,7 | 94,6 | 69,5 | 61,8 | 73,3 | 64,9 | | 47,7 | 34,8 | 39 | 40,8 | 32,3 | 36,9 | 44 |
| 9 | 83,2 | 60,6 | 60,6 | 56 | 48,5 | 60,6 | 51,4 | 62,4 | | 38 | 38,1 | 38,8 | 34,8 | 33 | 44,8 |
| 10 | 56,6 | 57,7 | 54,1 | 59,5 | 44,9 | 60,4 | 51,4 | 57,7 | 55 | | 38,5 | 39,5 | 52,3 | 30,6 | 38,5 |
| 11 | 59,5 | 57,8 | 54,3 | 58,6 | 47,8 | 56,9 | 56,9 | 54,3 | 54,3 | 55,2 | | 37,8 | 32,8 | 33,3 | 40,6 |
| 12 | 54,9 | 49 | 60 | 56,2 | 47,8 | 61 | 58 | 59 | 59,6 | 55 | 54,3 | | 37,6 | 37,3 | 44,8 |
| 13 | 49,6 | 50 | 54,3 | 46,7 | 40,4 | 47,6 | 56,4 | 56,5 | 52,3 | 61,3 | 50 | 50 | | 29 | 30,2 |
| 14 | 48,7 | 49 | 55 | 51,4 | 41,2 | 51,4 | 57 | 54 | 51,4 | 40,5 | 51,7 | 50 | 41 | | 28,4 |
| 15 | 63,7 | 65,7 | 63 | 53,7 | 52,9 | 59,3 | 55,6 | 63 | 64,2 | 60,4 | 57,8 | 63 | 47,2 | 46,3 | |
| 16 | 54,9 | 60,6 | 67,3 | 60 | 51,5 | 61 | 61,2 | 67,3 | 56 | 56,8 | 56 | 62 | 53,1 | 51 | 62 |
| 17 | 53,1 | 54,1 | 59,6 | 61,5 | 48,5 | 63,3 | 59,6 | 56,9 | 57,8 | 52,3 | 60,3 | 56,9 | 45,9 | 49,5 | 55 |
| 18 | 57,5 | 56,1 | 58,9 | 62,6 | 50 | 65,4 | 58,9 | 57,9 | 58,7 | 55 | 62,1 | 59,8 | 48,6 | 52,3 | 59,3 |
| 19 | 56,6 | 58,9 | 57 | 58,9 | 48,5 | 59,8 | 57 | 57,9 | 56 | 59,5 | 60,3 | 61,7 | 49,5 | 51,4 | 57,4 |
| 20 | 65,5 | 71,4 | 54,5 | 54,5 | 50,7 | 56,3 | 51,8 | 57,1 | 60,7 | 64,3 | 57,8 | 52,7 | 50,9 | 53,6 | 67,9 |
| 21 | 65,5 | 74,8 | 58,6 | 57,7 | 51,5 | 57,7 | 55 | 61,3 | 64 | 64 | 62,1 | 55 | 49,5 | 53,2 | 71,2 |
| 22 | 65,5 | 73,6 | 59,1 | 57,3 | 50 | 57,3 | 57,3 | 61,8 | 65,5 | 61,3 | 60,3 | 55,5 | 50,9 | 51,8 | 67,3 |
| 23 | 50,4 | 60,6 | 59,8 | 50,5 | 41,9 | 48,6 | 58,5 | 62 | 51,4 | 53,2 | 47,4 | 49 | 68,8 | 47 | 53,7 |
| 24 | 64,7 | 73,3 | 56,9 | 54,3 | 46,3 | 55,2 | 56,9 | 57,8 | 62,1 | 62,9 | 60,3 | 51,7 | 48,3 | 51,7 | 66,4 |
| 25 | 66,4 | 71,2 | 57,7 | 56,8 | 51,5 | 56,8 | 55,9 | 59,5 | 64 | 62,2 | 61,2 | 55,9 | 50,5 | 53,2 | 70,3 |
| 26 | 65,5 | 74,5 | 59,1 | 57,3 | 50 | 57,3 | 57,3 | 61,8 | 63,6 | 61,3 | 60,3 | 55,5 | 50,9 | 52,7 | 67,3 |
| 27 | 56,9 | 54,3 | 50 | 55,2 | 47,8 | 56 | 50 | 53,4 | 57,8 | 86,2 | 55,2 | 52,6 | 57,8 | 41,4 | 59,5 |
| 28 | 56,6 | 58,7 | 59,8 | 50,5 | 44,9 | 53,3 | 54,6 | 62,9 | 56,9 | 50,5 | 48,3 | 58 | 47,4 | 52 | 50,9 |
| 29 | 46,4 | 44,9 | 47,1 | 45,7 | 52,9 | 47,8 | 44,9 | 45,7 | 44,9 | 42,8 | 46,4 | 45,7 | 38,4 | 36,2 | 46,4 |
| 30 | 54,9 | 57,7 | 62,4 | 60 | 50 | 61,9 | 62,4 | 61,4 | 58,7 | 53,2 | 55,2 | 61,4 | 50,5 | 48,5 | 60,2 |
| 31 | 57,5 | 57,7 | 59,8 | 55,2 | 47,8 | 52,4 | 53,6 | 59,8 | 56,9 | 48,6 | 49,1 | 56 | 46,4 | 51 | 49,1 |
| 32 | 57,5 | 54,5 | 56,3 | 58 | 50 | 60,7 | 58 | 57,1 | 59,8 | 58 | 62,1 | 60,7 | 46,4 | 48,2 | 62,5 |
| 33 | 83,2 | 65,5 | 60 | 54,5 | 48,5 | 57,3 | 53,6 | 63,6 | 81,8 | 60,4 | 59,5 | 50,9 | 50 | 48,2 | 65,5 |
| 34 | 50 | 50 | 49,1 | 47,4 | 46,3 | 48,3 | 50 | 49,1 | 51,7 | 50,9 | 55,2 | 51,7 | 40,5 | 66,4 | 53,4 |
| 35 | 50 | 50 | 49,1 | 47,4 | 46,3 | 48,3 | 50 | 49,1 | 51,7 | 50,9 | 55,2 | 51,7 | 40,5 | 66,4 | 53,4 |
| 36 | 47 | 43,9 | 43,9 | 40,9 | 43,4 | 41,7 | 48,5 | 47 | 49,2 | 51,5 | 48,5 | 42,4 | 40,2 | 44,7 | 47,7 |
| 37 | 59,3 | 55,7 | 55,7 | 61,3 | 47,8 | 63,2 | 60,4 | 55,7 | 61,5 | 56,8 | 58,6 | 54,7 | 50,9 | 50,9 | 53,7 |
| 38 | 47 | 43,9 | 43,9 | 40,9 | 43,4 | 40,9 | 48,5 | 47 | 50 | 52,3 | 48,5 | 42,4 | 40,2 | 44,7 | 47,7 |
| 39 | 61,9 | 64,3 | 56,3 | 58 | 52,9 | 59,8 | 55,4 | 60,7 | 62,5 | 65,2 | 60,3 | 58,9 | 51,8 | 49,1 | 71,4 |
| 40 | 59,3 | 57,5 | 56,6 | 62,3 | 48,5 | 64,2 | 62,3 | 56,6 | 61,5 | 56,8 | 57,8 | 57,5 | 50 | 50 | 53,7 |
| 41 | 61,9 | 65,2 | 59,8 | 58 | 52,9 | 59,8 | 56,3 | 63,4 | 61,6 | 63,4 | 61,2 | 58,9 | 52,7 | 47,3 | 71,4 |
| 42 | 59,3 | 73,1 | 59,3 | 55,6 | 50,7 | 57,4 | 58,3 | 59,3 | 62,4 | 62,2 | 59,5 | 52,8 | 50,9 | 52,8 | 67,6 |
| 43 | 61,1 | 65,2 | 59,8 | 58 | 52,9 | 59,8 | 56,3 | 63,4 | 61,6 | 63,4 | 61,2 | 58,9 | 52,7 | 47,3 | 71,4 |
| 44 | 59,3 | 64,2 | 63,2 | 64,2 | 50,7 | 66 | 59,4 | 65,1 | 63,3 | 59,5 | 62,9 | 58,5 | 49,1 | 50 | 67,6 |
| 45 | 47,8 | 49,6 | 55,8 | 49,6 | 44,9 | 49,6 | 50,4 | 53,1 | 54,9 | 47,8 | 50,9 | 47,8 | 40,7 | 76,1 | 51,3 |

**FIGURE 14**

|    | 1 | 2 | 3 | 4 | 5 | 6 | 7 | 8 | 9 | 10 | 11 | 12 | 13 | 14 | 15 |
|----|---|---|---|---|---|---|---|---|---|----|----|----|----|----|----|
| 46 | 46,9 | 50 | 55,5 | 46,4 | 46,3 | 48,2 | 51,8 | 52,7 | 53,6 | 45 | 50 | 46,4 | 38,2 | 74,5 | 51,8 |
| 47 | 46,9 | 50,9 | 56,4 | 46,4 | 47,1 | 50,9 | 51,8 | 55,5 | 53,6 | 47,7 | 51,7 | 50,9 | 38,2 | 75,5 | 50 |
| 48 | 53,9 | 54,8 | 58,3 | 56,5 | 50 | 57,4 | 55,7 | 60 | 54,8 | 55,7 | 57,8 | 53 | 40,9 | 52,2 | 57,4 |
| 49 | 48,7 | 44,3 | 54,8 | 48,7 | 49,3 | 49,6 | 49,6 | 53 | 55,7 | 49,6 | 51,7 | 43,5 | 40,9 | 73 | 51,3 |
| 50 | 56,5 | 55,7 | 60,9 | 56,5 | 51,5 | 57,4 | 57,4 | 61,7 | 53 | 56,5 | 61,2 | 54,8 | 41,7 | 52,2 | 55,7 |
| 51 | 95,6 | 64 | 59,6 | 56,1 | 52,2 | 55,3 | 51,8 | 61,4 | 84,2 | 55,3 | 54,3 | 55,3 | 48,2 | 46,5 | 64 |
| 52 | 54,9 | 57,7 | 58,7 | 59 | 48,5 | 67,6 | 59,6 | 56,7 | 56,9 | 50,5 | 58,6 | 59,6 | 47,1 | 51,9 | 62 |
| 53 | 63,7 | 63,2 | 58,5 | 62,3 | 47,8 | 62,3 | 58,5 | 67 | 66,1 | 60,4 | 60,3 | 60,4 | 48,1 | 56,6 | 67,6 |
| 54 | 55,8 | 52,8 | 53,8 | 50 | 46,3 | 47,2 | 50 | 52,8 | 55 | 44,1 | 47,4 | 49,1 | 45,3 | 51,9 | 52,8 |
| 55 | 45,1 | 51,9 | 57,6 | 49,5 | 41,9 | 54,3 | 56,4 | 58,7 | 48,6 | 50,5 | 49,1 | 53 | 47,8 | 49 | 51,9 |
| 56 | 57,5 | 61,5 | 64,6 | 63,8 | 47,1 | 64,8 | 60,6 | 66,7 | 56 | 57,7 | 56,9 | 54 | 50,5 | 53 | 61,1 |
| 57 | 49,6 | 60,6 | 60,9 | 49,5 | 41,2 | 51,4 | 54,3 | 62 | 53,2 | 52,3 | 51,7 | 58 | 63,9 | 39 | 58,3 |
| 58 | 41,7 | 37,7 | 40,4 | 40,4 | 49 | 37,1 | 37,1 | 39,1 | 41,1 | 38,4 | 37,1 | 37,7 | 27,2 | 55 | 39,1 |
| 59 | 50,4 | 48,1 | 55,6 | 52,8 | 42,6 | 50,9 | 52,8 | 53,7 | 57,8 | 43,2 | 49,1 | 51,9 | 40,7 | 73,1 | 55,6 |
| 60 | 38,5 | 39,1 | 38,5 | 36,5 | 41,7 | 35,9 | 37,2 | 36,5 | 39,1 | 36,5 | 42,3 | 35,9 | 28,8 | 51,9 | 38,5 |
| 61 | 48,2 | 46,5 | 53,5 | 48,2 | 44,9 | 45,6 | 49,1 | 52,6 | 50,9 | 46,5 | 47,4 | 49,1 | 36 | 72,8 | 47,4 |
| 62 | 51,7 | 46,6 | 51,7 | 46,6 | 45,6 | 46,6 | 49,2 | 50,8 | 50,8 | 46,6 | 48,3 | 46,6 | 35,6 | 72 | 45,8 |
| 63 | 39,3 | 33,7 | 36 | 36 | 46,6 | 33,7 | 34,3 | 34,8 | 38,8 | 30,9 | 35,4 | 35,4 | 24,7 | 44,4 | 34,8 |
| 64 | 39,3 | 33,7 | 36 | 36 | 46,6 | 33,1 | 34,3 | 33,1 | 38,8 | 30,9 | 35,4 | 34,8 | 24,7 | 45,5 | 32,6 |

FIGURE 14 (continued)

| | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|
| 1 | 38,3 | 35 | 33,1 | 31,9 | 39,3 | 38,8 | 39,7 | 33 | 41 | 37,6 | 39,7 | 38,5 | 31,4 | 26,9 | 34,8 |
| 2 | 36,1 | 29,6 | 29,3 | 36,4 | 53,6 | 58,6 | 57,7 | 47,2 | 50,9 | 55,9 | 57,7 | 31,9 | 35,5 | 27,6 | 37 |
| 3 | 45,3 | 43,8 | 44 | 38 | 37,2 | 40,2 | 40,5 | 39,8 | 41,9 | 39,7 | 40,5 | 37,9 | 38 | 32,9 | 42,7 |
| 4 | 37,7 | 38,6 | 38,6 | 36,1 | 32,5 | 37,1 | 36,5 | 28,3 | 34,4 | 37,1 | 36,5 | 35,3 | 31,1 | 27,6 | 36,5 |
| 5 | 34,2 | 32 | 32,4 | 31,2 | 32,6 | 29,8 | 32,4 | 27,7 | 29,7 | 29,8 | 33,1 | 33,8 | 29,7 | 27,2 | 31,2 |
| 6 | 37,2 | 38,6 | 38,6 | 36,1 | 34,2 | 37,1 | 37,4 | 32,1 | 35,3 | 37,1 | 37,4 | 34,5 | 30,2 | 28,3 | 36,4 |
| 7 | 36,3 | 42,2 | 42,1 | 40,7 | 35,4 | 35,1 | 36,4 | 37,2 | 34,5 | 36 | 36,4 | 32,2 | 35,2 | 32,7 | 44,5 |
| 8 | 45,2 | 38,7 | 39,1 | 37 | 41,1 | 41,4 | 41,8 | 39,2 | 41,4 | 39,1 | 41,8 | 37,1 | 40,6 | 29,3 | 38,8 |
| 9 | 35,7 | 34,7 | 35 | 36,6 | 39,7 | 44,5 | 42,9 | 38,1 | 44,2 | 44,5 | 46,3 | 39,2 | 31,3 | 28,2 | 34,5 |
| 10 | 38,7 | 32,5 | 31,9 | 37,9 | 38,1 | 42,6 | 40,9 | 36,9 | 37,7 | 40,9 | 40,9 | 78,4 | 31,3 | 28 | 35,3 |
| 11 | 41,5 | 41,3 | 40,5 | 40,2 | 38,5 | 42,7 | 42,7 | 37,9 | 40,5 | 41,9 | 42,7 | 35,4 | 33,3 | 30,4 | 38,9 |
| 12 | 45,2 | 40,2 | 37,6 | 41,1 | 35,6 | 41,2 | 40,7 | 36 | 37,6 | 42,1 | 40,7 | 35,9 | 35,2 | 31,3 | 39,1 |
| 13 | 38 | 32,1 | 32,7 | 32,7 | 29,6 | 34,5 | 35,7 | 47,6 | 30,2 | 32,7 | 35,7 | 50 | 27,9 | 23,8 | 31,8 |
| 14 | 32,7 | 33,6 | 36,7 | 31,8 | 30,5 | 32,5 | 32,5 | 29 | 31,7 | 32,5 | 31,6 | 28,4 | 35,5 | 24,5 | 31,8 |
| 15 | 45,6 | 35,5 | 34,7 | 41,2 | 44,8 | 44,5 | 40 | 34,2 | 42,1 | 42,9 | 40 | 39 | 33,9 | 34 | 42,3 |
| 16 | | 38,8 | 38,9 | 43,6 | 41,6 | 44,6 | 42,9 | 41,8 | 40,2 | 42,9 | 42,9 | 38,8 | 38,9 | 29 | 36,3 |
| 17 | 57,8 | | 79,1 | 40,5 | 33,9 | 35,8 | 36,1 | 34,9 | 36,7 | 35 | 36,1 | 34,4 | 34,5 | 30,3 | 39,8 |
| 18 | 57 | 90,8 | | 41,4 | 33,3 | 32,2 | 34,7 | 34,6 | 35 | 31,4 | 34,7 | 31,4 | 31,3 | 32,9 | 42,6 |
| 19 | 64,5 | 66,1 | 68,2 | | 39,1 | 39,3 | 36,4 | 36,1 | 37,3 | 39,3 | 36,4 | 34,7 | 51,4 | 43,2 | 57,8 |
| 20 | 57,1 | 59,8 | 58,9 | 60,7 | | 65,8 | 63,8 | 46 | 81,4 | 64,1 | 63,8 | 34,2 | 27,7 | 28,6 | 32,2 |
| 21 | 61,3 | 57,7 | 60,4 | 60,4 | 83,9 | | 92,8 | 64 | 63,3 | 94,6 | 93,7 | 40,3 | 33,6 | 31,7 | 36,2 |
| 22 | 60,9 | 59,1 | 61,8 | 57,3 | 79,5 | 94,6 | | 65,5 | 61,3 | 89,2 | 99,1 | 38,8 | 33,9 | 31 | 35,7 |
| 23 | 61,2 | 48,6 | 50,5 | 49,5 | 57,1 | 64 | 66,4 | | 46,6 | 60,4 | 66,4 | 35,3 | 32,4 | 24,1 | 31,5 |
| 24 | 58,6 | 56,9 | 56,9 | 55,2 | 89,7 | 83,6 | 79,3 | 58,6 | | 62,5 | 61,3 | 37 | 32,5 | 29,3 | 34,5 |
| 25 | 59,5 | 57,7 | 60,4 | 58,6 | 82,1 | 94,6 | 91 | 60,4 | 81,9 | | 90,1 | 39,5 | 33,6 | 31,7 | 36,2 |
| 26 | 60,9 | 59,1 | 61,8 | 57,3 | 80,4 | 94,6 | 100 | 66,4 | 80,2 | 91 | | 38,8 | 33 | 31 | 35,7 |
| 27 | 53,4 | 54,3 | 52,6 | 56 | 62,1 | 64,7 | 60,3 | 50,9 | 61,2 | 62,1 | 60,3 | | 27,6 | 27,2 | 32,8 |
| 28 | 63,3 | 53,2 | 57,9 | 63,6 | 54,5 | 53,2 | 52,7 | 46,4 | 51,7 | 55 | 52,7 | 51,7 | | 36,9 | 50 |
| 29 | 46,4 | 52,2 | 50,7 | 56,5 | 45,7 | 48,6 | 47,8 | 35,5 | 47,8 | 47,8 | 47,8 | 44,2 | 48,6 | | 73,2 |
| 30 | 65,3 | 66,1 | 65,4 | 72,9 | 58 | 58,6 | 58,2 | 45,5 | 56,9 | 57,7 | 58,2 | 54,3 | 66,3 | 73,2 | |
| 31 | 64,3 | 53,2 | 56,1 | 63,6 | 55,4 | 55,9 | 56,4 | 47,4 | 54,3 | 55,9 | 57,3 | 44,8 | 94,8 | 46,4 | 63,4 |
| 32 | 57,1 | 56,3 | 56,3 | 57,1 | 60,7 | 58,9 | 62,5 | 47,3 | 57,8 | 61,6 | 62,5 | 54,3 | 50 | 46,4 | 55,4 |
| 33 | 55,5 | 58,2 | 60,9 | 56,4 | 65,2 | 64 | 63,6 | 53,6 | 60,3 | 64 | 64,5 | 56 | 52,7 | 46,4 | 57,3 |
| 34 | 48,3 | 53,4 | 57,8 | 55,2 | 53,4 | 55,2 | 56 | 40,5 | 60,3 | 54,3 | 56 | 50 | 44,8 | 41,3 | 51,7 |
| 35 | 48,3 | 53,4 | 57,8 | 55,2 | 53,4 | 55,2 | 56 | 40,5 | 60,3 | 54,3 | 56 | 50 | 44,8 | 41,3 | 51,7 |
| 36 | 50 | 46,2 | 49,2 | 47,7 | 47 | 47,7 | 48,5 | 37,9 | 50,8 | 47 | 48,5 | 48,5 | 43,9 | 43,5 | 39,4 |
| 37 | 56,6 | 78,9 | 80,4 | 61,7 | 58 | 64 | 62,7 | 50,9 | 56 | 63,1 | 62,7 | 50,9 | 53,8 | 50 | 62,3 |
| 38 | 50 | 46,2 | 49,2 | 47 | 47 | 47,7 | 48,5 | 37,9 | 50,8 | 47 | 48,5 | 50 | 43,2 | 42,8 | 42,4 |
| 39 | 62,5 | 56,3 | 59,8 | 61,6 | 68,8 | 71,4 | 67,9 | 50,9 | 68,1 | 72,3 | 67,9 | 63,8 | 50,9 | 50,7 | 57,1 |
| 40 | 56,6 | 79,8 | 80,4 | 61,7 | 60,7 | 64,9 | 63,6 | 50,9 | 56,9 | 64 | 63,6 | 50,9 | 54,7 | 49,3 | 61,3 |
| 41 | 61,6 | 57,1 | 62,5 | 63,4 | 67,9 | 70,5 | 67,9 | 50,9 | 71,6 | 72,3 | 67,9 | 64,7 | 53,6 | 51,4 | 58 |
| 42 | 59,3 | 60,6 | 65,7 | 58,3 | 87,5 | 78,4 | 77,3 | 55,6 | 84,5 | 76,6 | 78,2 | 58,6 | 55,6 | 46,4 | 59,3 |
| 43 | 61,6 | 57,1 | 61,6 | 63,4 | 67,9 | 70,5 | 67,9 | 50,9 | 71,6 | 72,3 | 67,9 | 64,7 | 53,6 | 51,4 | 58 |
| 44 | 62,3 | 62,4 | 63,6 | 57,9 | 68,8 | 69,4 | 68,2 | 51,9 | 65,5 | 67,6 | 68,2 | 54,3 | 56,6 | 47,8 | 61,3 |
| 45 | 46 | 49,6 | 55,8 | 54 | 54 | 50,4 | 50,4 | 40,7 | 53,4 | 50,4 | 52,2 | 48,3 | 46,9 | 42 | 48,7 |

FIGURE 14 (continued)

| | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|
| 46 | 50 | 51,8 | 59,1 | 53,6 | 53,6 | 51,4 | 50,9 | 39,1 | 50,9 | 51,4 | 50,9 | 43,1 | 46,4 | 39,1 | 49,1 |
| 47 | 50 | 51,8 | 56,4 | 54,5 | 52,7 | 52,3 | 50 | 40 | 51,7 | 52,3 | 51,8 | 43,1 | 46,4 | 41,3 | 50,9 |
| 48 | 55,7 | 67,8 | 67,8 | 63,5 | 62,6 | 58,3 | 58,3 | 45,2 | 62,9 | 55,7 | 57,4 | 58,6 | 49,6 | 51,4 | 60,9 |
| 49 | 45,2 | 50,4 | 54,8 | 52,2 | 49,6 | 51,3 | 50,4 | 39,1 | 52,6 | 50,4 | 51,3 | 45,7 | 47,8 | 44,2 | 48,7 |
| 50 | 54,8 | 68,7 | 69,6 | 63,5 | 62,6 | 60 | 60 | 47,8 | 59,5 | 56,5 | 59,1 | 56,9 | 50,4 | 52,2 | 61,7 |
| 51 | 54,4 | 54,4 | 60,5 | 55,3 | 64,9 | 65,8 | 65,8 | 51,8 | 65,5 | 65,8 | 65,8 | 56 | 53,5 | 45,7 | 54,4 |
| 52 | 57,7 | 89 | 92,5 | 70,1 | 58 | 55,9 | 58,2 | 51,9 | 55,2 | 55,9 | 58,2 | 49,1 | 55,8 | 49,3 | 64,4 |
| 53 | 76,4 | 56,9 | 59,8 | 64,5 | 68,8 | 72,1 | 70,9 | 56,6 | 66,4 | 67,6 | 70,9 | 56,9 | 57,5 | 47,1 | 57,5 |
| 54 | 56,6 | 56,9 | 56,1 | 51,4 | 48,2 | 52,3 | 55,5 | 44,3 | 49,1 | 47,7 | 55,5 | 45,7 | 47,2 | 37,7 | 43,4 |
| 55 | 57,1 | 57,8 | 61,7 | 57,9 | 51,8 | 52,3 | 50,9 | 57,8 | 49,1 | 50,5 | 50,9 | 49,1 | 54,6 | 44,2 | 60,4 |
| 56 | 63,6 | 77,1 | 79,4 | 65,4 | 58 | 58,6 | 59,1 | 54,5 | 58,6 | 57,7 | 59,1 | 54,3 | 58,6 | 48,6 | 66,3 |
| 57 | 60,2 | 46,8 | 51,4 | 47,7 | 51,8 | 58,6 | 57,3 | 67,5 | 54,3 | 58,6 | 57,3 | 50,9 | 47,4 | 40,6 | 51,5 |
| 58 | 38,4 | 41,1 | 42,4 | 44,4 | 41,1 | 40,4 | 39,1 | 31,1 | 41,7 | 39,7 | 38,4 | 37,7 | 33,1 | 41,1 | 39,1 |
| 59 | 49,1 | 51,4 | 55,6 | 55,6 | 52,7 | 49,5 | 50 | 39,8 | 48,3 | 49,5 | 50 | 46,6 | 49,1 | 42,8 | 53,7 |
| 60 | 41 | 39,1 | 43,6 | 42,3 | 41 | 40,4 | 40,4 | 30,1 | 41 | 39,7 | 40,4 | 38,5 | 38,5 | 41 | 35,9 |
| 61 | 50,9 | 51,8 | 57 | 53,5 | 54,4 | 44,7 | 43,9 | 42,1 | 49,1 | 44,7 | 43,9 | 44,8 | 43,9 | 39,9 | 47,4 |
| 62 | 50 | 49,2 | 55,1 | 44,9 | 50 | 46,6 | 46,6 | 41,5 | 46,6 | 46,6 | 47,5 | 44,1 | 42,4 | 40,6 | 44,1 |
| 63 | 32,6 | 33,1 | 34,3 | 38,8 | 37,1 | 34,8 | 35,4 | 27,5 | 36,5 | 33,7 | 35,4 | 33,7 | 29,8 | 41,6 | 35,4 |
| 64 | 31,5 | 32,6 | 33,7 | 36,5 | 36,5 | 34,8 | 35,4 | 27,5 | 36,5 | 33,7 | 35,4 | 33,1 | 30,3 | 41,6 | 35,4 |

FIGURE 14 (continued)

| | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|
| 1 | 34,2 | 39,7 | 71,1 | 30,6 | 30,6 | 23 | 36,6 | 23 | 42,4 | 37,4 | 44,9 | 41,7 | 44,9 | 36,4 | 33,1 |
| 2 | 37,4 | 35,4 | 39,8 | 32,5 | 32,5 | 27,3 | 32,5 | 27,3 | 36,8 | 33 | 39,5 | 51,8 | 39,5 | 35,1 | 34,2 |
| 3 | 37,8 | 41,2 | 43,6 | 34,5 | 34,5 | 27,1 | 41,3 | 27,1 | 41,1 | 41,8 | 41,1 | 44 | 40,2 | 46,2 | 37,4 |
| 4 | 33,6 | 38,1 | 36,8 | 32,8 | 32,8 | 28,8 | 38,6 | 28,8 | 41 | 38,6 | 40,2 | 39,1 | 39,3 | 42,9 | 31,6 |
| 5 | 30,1 | 34,5 | 36,5 | 27,8 | 27,8 | 24,8 | 32,4 | 24,8 | 31,8 | 33,6 | 33,1 | 33,1 | 32,5 | 37 | 27,7 |
| 6 | 35,2 | 37,3 | 37,4 | 33,6 | 33,6 | 28 | 38,6 | 27,3 | 42,7 | 38,6 | 41,9 | 38,2 | 41 | 44,2 | 29,2 |
| 7 | 39,2 | 42,9 | 38,6 | 33,6 | 33,6 | 32,3 | 43,4 | 32,3 | 38,3 | 45,3 | 39,1 | 39,4 | 38,3 | 40,7 | 32,7 |
| 8 | 38,8 | 41,2 | 43,2 | 31,9 | 31,9 | 30,3 | 39,4 | 30,3 | 41,1 | 40 | 42 | 41,3 | 41,1 | 47,2 | 33,6 |
| 9 | 32,4 | 42,7 | 69,8 | 34,5 | 34,5 | 28,2 | 38 | 28,7 | 41 | 37,7 | 45,4 | 42,9 | 45,4 | 39,3 | 34,5 |
| 10 | 32,4 | 39,3 | 42,7 | 31,1 | 31,1 | 31,1 | 31,5 | 31,9 | 38,5 | 31,6 | 41 | 42,2 | 40,2 | 38,6 | 29,3 |
| 11 | 35,6 | 40,9 | 37 | 33,9 | 33,9 | 29,9 | 40,8 | 29,9 | 42,6 | 40,7 | 45,1 | 42,5 | 44,3 | 47,5 | 31,5 |
| 12 | 35,2 | 44,2 | 37,1 | 31,2 | 31,2 | 27 | 37,7 | 27 | 42,2 | 37,8 | 44 | 36 | 43,1 | 43,9 | 26,4 |
| 13 | 26,9 | 38,6 | 31 | 29,3 | 29,3 | 22,1 | 34 | 22,1 | 30,4 | 33 | 33,9 | 34,2 | 33 | 37,4 | 25 |
| 14 | 33,6 | 38,3 | 35,9 | 55,9 | 55,9 | 26,7 | 35,8 | 26,7 | 31,3 | 34,9 | 32,1 | 29,7 | 31,3 | 33 | 66,1 |
| 15 | 32,1 | 42,7 | 42 | 31,6 | 31,6 | 27,1 | 35 | 28,4 | 52,1 | 35 | 53,8 | 46,9 | 53 | 45,8 | 28,8 |
| 16 | 40,7 | 44,7 | 33,3 | 29,1 | 29,1 | 26,5 | 37,5 | 26,5 | 47,3 | 39,6 | 50 | 41,1 | 49,1 | 45,5 | 31,9 |
| 17 | 36,6 | 37,7 | 37,9 | 31,7 | 31,7 | 25 | 67 | 25 | 38,7 | 68,8 | 38,7 | 39,5 | 37,8 | 42,9 | 30,3 |
| 18 | 31,5 | 39,5 | 34,1 | 32,8 | 32,8 | 25,5 | 64,9 | 25,5 | 39,8 | 65,8 | 39,8 | 38,8 | 39,8 | 42,7 | 35,8 |
| 19 | 53,3 | 42 | 35,3 | 36,1 | 36,1 | 27,8 | 43 | 27,1 | 43,2 | 42,1 | 43,2 | 36,4 | 42,4 | 40,2 | 32,5 |
| 20 | 32,5 | 40,4 | 40,3 | 32,2 | 32,2 | 30,3 | 35 | 30,3 | 42,4 | 35,8 | 44,9 | 70,5 | 44,9 | 42,9 | 30,2 |
| 21 | 36 | 44,7 | 39,5 | 33,6 | 33,6 | 27,4 | 35,3 | 27,4 | 47,9 | 37 | 51,3 | 56,5 | 51,3 | 44 | 31,6 |
| 22 | 33,9 | 46,6 | 39,5 | 34,5 | 34,5 | 32,3 | 35,6 | 32,3 | 44,9 | 37,3 | 48,3 | 57 | 48,3 | 45,2 | 32,5 |
| 23 | 32,4 | 35,7 | 33,3 | 27,6 | 27,6 | 26,3 | 36,8 | 26,3 | 36,6 | 36,8 | 39,3 | 39,4 | 39,3 | 35,8 | 26,5 |
| 24 | 34,2 | 39,3 | 39,3 | 34,4 | 34,4 | 32,1 | 35 | 32,1 | 41,9 | 35,8 | 44,5 | 69,2 | 44,5 | 42 | 32 |
| 25 | 36 | 44,3 | 39,1 | 32,8 | 32,8 | 26,7 | 34,5 | 26,7 | 49,6 | 36,1 | 51,7 | 55,7 | 51,7 | 43,1 | 31,6 |
| 26 | 33,9 | 46,6 | 40,4 | 34,5 | 34,5 | 32,3 | 35,6 | 32,3 | 44,9 | 37,3 | 48,3 | 57 | 48,3 | 45,2 | 31,2 |
| 27 | 30,2 | 42,1 | 41,2 | 34,7 | 34,7 | 28,8 | 30 | 29,3 | 40,7 | 30 | 43,2 | 40,3 | 42,4 | 39,8 | 28,5 |
| 28 | 91,8 | 37,2 | 33 | 31 | 31 | 26,5 | 31,2 | 26,5 | 37,4 | 33,6 | 36,2 | 31,2 | 35,3 | 38,2 | 32,5 |
| 29 | 36,4 | 33,8 | 31,9 | 27,9 | 27,9 | 24,8 | 33,8 | 25,5 | 36 | 33,8 | 37,3 | 28,5 | 36,7 | 32,5 | 24,8 |
| 30 | 49,5 | 40,7 | 39,6 | 35,6 | 35,6 | 24,2 | 42,1 | 27,3 | 41,6 | 40,4 | 43,4 | 35,8 | 42,5 | 42,1 | 33,3 |
| 31 | | 38,4 | 32,7 | 31,9 | 31,9 | 26,5 | 33,9 | 26,5 | 40,9 | 36,4 | 40 | 36,7 | 39,1 | 40,2 | 35,1 |
| 32 | 52,7 | | 42 | 32,8 | 32,8 | 31,6 | 39,5 | 30,8 | 47 | 39,8 | 50,4 | 41,2 | 49,6 | 64,1 | 33,3 |
| 33 | 53,6 | 60,7 | | 34,5 | 34,5 | 30,3 | 39,5 | 29,5 | 45,4 | 40,3 | 46,2 | 38,9 | 45,4 | 42 | 38,1 |
| 34 | 45,7 | 47,4 | 49,1 | | 100 | 27,2 | 32,5 | 27,2 | 38,8 | 32,5 | 39,2 | 32,8 | 38,4 | 36,4 | 53,7 |
| 35 | 45,7 | 47,4 | 49,1 | 100 | | 27,2 | 32,5 | 27,2 | 38,8 | 32,5 | 39,2 | 32,8 | 38,4 | 36,4 | 53,7 |
| 36 | 43,9 | 47 | 49,2 | 47 | 47 | | 27,3 | 99,2 | 28,5 | 25,2 | 27 | 30,3 | 27 | 30,1 | 32,1 |
| 37 | 56,6 | 58 | 61,8 | 51,7 | 51,7 | 43,9 | | 27,3 | 42,7 | 98,1 | 42,4 | 37,6 | 41,5 | 45 | 32,5 |
| 38 | 43,2 | 47 | 49,2 | 47 | 47 | 99,2 | 43,9 | | 28,5 | 25,2 | 27 | 30,3 | 27 | 29,9 | 32,1 |
| 39 | 53,6 | 66,1 | 62,5 | 56 | 56 | 50 | 61,6 | 50 | | 42,7 | 95,5 | 46 | 94,6 | 52,2 | 32,5 |
| 40 | 56,6 | 57,1 | 60 | 51,7 | 51,7 | 41,7 | 99,1 | 41,7 | 61,6 | | 41,9 | 37,8 | 41 | 47,7 | 28,6 |
| 41 | 53,6 | 66,1 | 63,4 | 58,6 | 58,6 | 49,2 | 60,7 | 49,2 | 99,1 | 62,5 | | 46 | 99,1 | 53,1 | 34,2 |
| 42 | 55,6 | 63,4 | 62,7 | 56 | 56 | 48,5 | 60,2 | 48,5 | 65,2 | 59,3 | 66,1 | | 46 | 45,1 | 30,2 |
| 43 | 53,6 | 66,1 | 63,4 | 58,6 | 58,6 | 49,2 | 60,7 | 49,2 | 99,1 | 62,5 | 100 | 66,1 | | 52,2 | 34,2 |
| 44 | 59,4 | 75 | 62,7 | 50,9 | 50,9 | 46,2 | 67 | 44,7 | 70,5 | 67 | 71,4 | 67,6 | 71,4 | | 31 |
| 45 | 50,4 | 49,6 | 51,3 | 69 | 69 | 48,5 | 47,8 | 48,5 | 52,2 | 46 | 53,1 | 53,1 | 52,2 | 48,7 | |

FIGURE 14 (continued)

| | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|
| 46 | 51,8 | 50 | 50 | 69 | 69 | 47,7 | 51,8 | 48,5 | 53,6 | 50 | 52,7 | 53,6 | 52,7 | 48,2 | 92 |
| 47 | 51,8 | 46,4 | 50 | 69,8 | 69,8 | 49,2 | 53,6 | 49,2 | 54,5 | 50,9 | 53,6 | 53,6 | 53,6 | 44,5 | 92,9 |
| 48 | 50,4 | 60 | 56,5 | 56 | 56 | 49,2 | 64,3 | 49,2 | 61,7 | 64,3 | 62,6 | 59,1 | 62,6 | 59,1 | 53,9 |
| 49 | 48,7 | 43,5 | 49,6 | 69 | 69 | 49,2 | 47 | 49,2 | 50,4 | 46,1 | 51,3 | 51,3 | 50,4 | 47 | 96,5 |
| 50 | 53,9 | 59,1 | 58,3 | 57,8 | 57,8 | 49,2 | 63,5 | 49,2 | 60,9 | 64,3 | 61,7 | 59,1 | 61,7 | 60,9 | 53 |
| 51 | 53,5 | 56,1 | 85,1 | 51,7 | 51,7 | 47,7 | 59,6 | 47,7 | 61,4 | 59,6 | 61,4 | 59,6 | 60,5 | 62,3 | 50 |
| 52 | 58,7 | 56,3 | 56,4 | 51,7 | 51,7 | 49,2 | 79,2 | 49,2 | 59,8 | 80,2 | 59,8 | 58,3 | 59,8 | 65,1 | 50,4 |
| 53 | 57,5 | 69,6 | 60 | 56 | 56 | 50,8 | 61,3 | 50,8 | 71,4 | 62,3 | 72,3 | 67,6 | 72,3 | 69,8 | 53,1 |
| 54 | 46,2 | 52,7 | 53,6 | 44 | 44 | 62,1 | 52,8 | 61,4 | 53,6 | 52,8 | 51,8 | 57,4 | 51,8 | 54,7 | 47,8 |
| 55 | 57,7 | 46,4 | 51,8 | 48,3 | 48,3 | 43,2 | 63,2 | 42,4 | 52,7 | 60,4 | 52,7 | 54,6 | 52,7 | 51,9 | 45,1 |
| 56 | 59,6 | 56,3 | 59,1 | 50 | 50 | 43,9 | 77,4 | 43,9 | 58 | 77,4 | 58,9 | 61,1 | 58,9 | 64,2 | 50,4 |
| 57 | 46,4 | 47,3 | 51,8 | 44,8 | 44,8 | 37,1 | 50,9 | 37,1 | 53,6 | 53,8 | 54,5 | 52,8 | 54,5 | 53,8 | 37,2 |
| 58 | 39,1 | 36,4 | 40,4 | 49,7 | 49,7 | 44,4 | 39,7 | 43,7 | 43,7 | 39,7 | 41,7 | 41,1 | 41,1 | 38,4 | 66,9 |
| 59 | 50 | 52,7 | 50 | 64,7 | 64,7 | 48,5 | 47,2 | 47,7 | 50,9 | 47,2 | 50,9 | 56,5 | 50 | 46,3 | 85,8 |
| 60 | 39,7 | 37,8 | 41 | 49,4 | 49,4 | 42,3 | 37,8 | 44,2 | 42,9 | 37,8 | 41 | 39,7 | 40,4 | 36,5 | 58,3 |
| 61 | 47,4 | 43,9 | 50 | 65,5 | 65,5 | 51,5 | 50 | 51,5 | 55,3 | 50 | 52,6 | 50,9 | 51,8 | 46,5 | 89,5 |
| 62 | 45,8 | 44,1 | 50 | 66,1 | 66,1 | 51,5 | 50 | 51,5 | 53,4 | 49,2 | 50 | 50 | 49,2 | 42,4 | 86,4 |
| 63 | 30,9 | 38,2 | 38,2 | 42,1 | 42,1 | 39,3 | 33,1 | 39,9 | 37,1 | 30,3 | 36 | 37,6 | 35,4 | 33,7 | 54,5 |
| 64 | 30,3 | 38,2 | 38,2 | 42,1 | 42,1 | 38,8 | 29,8 | 39,3 | 37,1 | 30,3 | 36 | 37,6 | 35,4 | 33,1 | 55,1 |

FIGURE 14 (continued)

| | 46 | 47 | 48 | 49 | 50 | 51 | 52 | 53 | 54 | 55 | 56 | 57 | 58 | 59 | 60 |
|---|---|---|---|---|---|---|---|---|---|---|---|---|---|---|---|
| 1 | 30 | 30 | 31 | 31,7 | 30,2 | 91,2 | 32,5 | 39,8 | 27,7 | 33,6 | 32 | 31,3 | 25,8 | 30 | 24,4 |
| 2 | 32,8 | 33,6 | 36,4 | 30 | 35,5 | 37,1 | 31 | 40,2 | 30,4 | 30,8 | 33,3 | 34 | 27,8 | 31,9 | 26,9 |
| 3 | 37,2 | 38,1 | 41 | 34,8 | 41,9 | 41,2 | 46,2 | 42,7 | 34,9 | 38,7 | 45,7 | 40,4 | 29,2 | 36,7 | 28,3 |
| 4 | 28,8 | 28,8 | 37,5 | 31,6 | 36,7 | 34,7 | 41,6 | 41,4 | 26,8 | 31,1 | 40,4 | 30,5 | 27,2 | 30,4 | 25,2 |
| 5 | 27,3 | 27,9 | 30,6 | 28,5 | 30,6 | 34 | 34,7 | 30,7 | 27,9 | 27,9 | 31,1 | 25,9 | 27,7 | 28,7 | 27,4 |
| 6 | 29,5 | 31,9 | 37,5 | 28,4 | 37,5 | 35,9 | 42,1 | 39,7 | 27 | 30,5 | 40,5 | 31,4 | 25,8 | 31,9 | 25,3 |
| 7 | 37,3 | 37,3 | 36,5 | 33,9 | 36,5 | 31 | 42,9 | 38,4 | 27,7 | 33,3 | 43,4 | 38,5 | 25,2 | 35,2 | 26,9 |
| 8 | 34,2 | 34,2 | 38,5 | 32,2 | 40,2 | 41,7 | 42,1 | 42,7 | 34,9 | 40,9 | 41,9 | 43 | 26,3 | 33 | 25,5 |
| 9 | 33,1 | 33,1 | 33,9 | 33,1 | 31,5 | 74,4 | 35,8 | 43,1 | 33,9 | 38,5 | 34,2 | 35,4 | 25 | 34,4 | 26,1 |
| 10 | 29,6 | 31,9 | 32,5 | 28 | 29,4 | 36,4 | 29,3 | 44,2 | 27,4 | 29,8 | 35,3 | 38,4 | 25,2 | 28,8 | 25,6 |
| 11 | 33,1 | 33,9 | 36,6 | 31 | 37,4 | 32,8 | 41,9 | 43,2 | 27,7 | 37 | 38,5 | 39,7 | 24,5 | 31,7 | 27,2 |
| 12 | 33,3 | 34,4 | 33,6 | 30,3 | 36,5 | 33,9 | 40,2 | 44,1 | 29,2 | 40 | 37,1 | 42 | 28,3 | 35 | 26,8 |
| 13 | 27,3 | 27,3 | 27 | 23,7 | 25,2 | 32,5 | 33,7 | 36 | 23,9 | 31,6 | 35 | 47,1 | 20,5 | 28,4 | 19,2 |
| 14 | 66,4 | 67,3 | 32,8 | 61,5 | 32,2 | 29,1 | 35,1 | 37,2 | 26,5 | 34 | 31,5 | 27,7 | 49,7 | 58,2 | 45,2 |
| 15 | 32,2 | 30,8 | 34,6 | 29,2 | 37,8 | 41,2 | 40,2 | 47,7 | 33 | 38 | 40,5 | 46,9 | 23,7 | 31,9 | 24,4 |
| 16 | 33,9 | 35,7 | 39,1 | 30,4 | 39,3 | 37,4 | 39,8 | 63,1 | 33,3 | 35 | 41 | 46,9 | 24,8 | 33 | 27,7 |
| 17 | 33,1 | 33,1 | 50,4 | 31,5 | 47,8 | 35 | 79,8 | 37 | 27,6 | 38,5 | 60,4 | 35,7 | 27,2 | 31,3 | 26,7 |
| 18 | 34,7 | 35 | 47,8 | 34,4 | 47 | 34,7 | 81,3 | 37,6 | 23,7 | 40 | 61,1 | 37,3 | 28,8 | 35,1 | 28,3 |
| 19 | 31,9 | 31,9 | 42 | 31,9 | 41,2 | 29,6 | 42,6 | 43 | 29,5 | 40,2 | 39,1 | 34,6 | 25,2 | 32,1 | 25,6 |
| 20 | 29,8 | 31,5 | 36 | 29,4 | 36 | 38,5 | 33,9 | 46,4 | 33,6 | 31 | 30,8 | 33 | 27,9 | 28,6 | 25,6 |
| 21 | 30,8 | 30,8 | 34,1 | 30,4 | 34,1 | 38,8 | 33,1 | 53 | 32,8 | 31,5 | 35,6 | 44,6 | 28,9 | 30,5 | 29,5 |
| 22 | 29,2 | 30 | 32,8 | 31,2 | 32,8 | 39,7 | 36,4 | 50,4 | 30,5 | 32,7 | 37,3 | 41,1 | 27,2 | 29,7 | 29,5 |
| 23 | 26,4 | 26,4 | 32,2 | 24,3 | 31,3 | 34,8 | 36,5 | 40,4 | 27,9 | 38,5 | 37,4 | 51,2 | 21,9 | 25,9 | 23,1 |
| 24 | 35,3 | 36,1 | 39,8 | 32,8 | 39 | 41,8 | 34,5 | 47,4 | 34,7 | 30,2 | 34,7 | 36,1 | 30,1 | 33,6 | 28,8 |
| 25 | 30,8 | 30,8 | 30,3 | 31,1 | 30,3 | 37,6 | 31,4 | 50,4 | 29,7 | 32,4 | 33,9 | 41,1 | 28,9 | 30,5 | 29,5 |
| 26 | 30 | 30 | 34,1 | 30,4 | 34,1 | 39,7 | 36,4 | 50,4 | 30,5 | 32,7 | 37,3 | 41,1 | 26,5 | 29,7 | 29,5 |
| 27 | 25,2 | 25,2 | 33,9 | 27,2 | 33,1 | 37,7 | 30,6 | 40,5 | 24 | 33,6 | 32,2 | 37,6 | 22,5 | 28,3 | 25,3 |
| 28 | 27,9 | 27,9 | 36,4 | 33 | 35,6 | 29,6 | 30,9 | 37 | 27,9 | 36,7 | 32,7 | 32,7 | 23,2 | 30,4 | 25,6 |
| 29 | 26 | 27,9 | 28,5 | 26,9 | 27,8 | 26 | 32,2 | 33,1 | 22,7 | 30,7 | 31,6 | 26,4 | 29,4 | 25 | 25,5 |
| 30 | 34,5 | 35,9 | 36,4 | 33,6 | 34,7 | 34,5 | 41,7 | 39,3 | 26,6 | 42,7 | 40,9 | 35,5 | 25,8 | 32,2 | 23,7 |
| 31 | 33 | 33 | 37,1 | 33,9 | 34,2 | 33 | 32,7 | 37,4 | 27 | 35,4 | 33,9 | 32,7 | 23,8 | 31,2 | 24,4 |
| 32 | 33,6 | 33,9 | 41,3 | 31,4 | 38,1 | 40,5 | 39,5 | 51,3 | 30,3 | 36,5 | 35,8 | 37,5 | 27,9 | 34,5 | 28,7 |
| 33 | 36,8 | 36,8 | 32,8 | 35,8 | 30,4 | 73 | 35 | 41,4 | 33,9 | 34,5 | 36,4 | 35,1 | 27,8 | 34,2 | 26,8 |
| 34 | 53,4 | 54,2 | 34,6 | 51,2 | 34,1 | 31,5 | 31,1 | 37,7 | 26,1 | 34,5 | 34,4 | 26,7 | 41,7 | 50,4 | 39,1 |
| 35 | 53,4 | 54,2 | 34,6 | 51,2 | 34,1 | 31,5 | 31,1 | 37,7 | 26,1 | 34,5 | 34,4 | 26,7 | 41,7 | 50,4 | 39,1 |
| 36 | 28,1 | 32,4 | 29,5 | 32,4 | 28,8 | 23 | 28,9 | 30,6 | 50 | 28 | 24,3 | 21,2 | 27,6 | 28,9 | 27,2 |
| 37 | 35,5 | 35,5 | 47,4 | 31,1 | 45,7 | 38,2 | 65,4 | 39,1 | 27,5 | 43 | 62,6 | 38,7 | 27,5 | 30,4 | 24,1 |
| 38 | 32,4 | 32,4 | 29,5 | 32,4 | 28,8 | 23 | 28,9 | 30,6 | 49,2 | 28 | 24,3 | 22 | 27,6 | 30,4 | 25,8 |
| 39 | 38 | 38,8 | 42,1 | 31,9 | 42,1 | 43,2 | 41,2 | 50,4 | 27,5 | 34,5 | 41,5 | 42 | 28,1 | 34,2 | 27,6 |
| 40 | 33,6 | 35,3 | 45,2 | 32 | 45,7 | 39 | 67,3 | 40,9 | 27,5 | 42,5 | 62,6 | 40,2 | 27,5 | 32,5 | 24,7 |
| 41 | 38,8 | 39,7 | 43 | 33,6 | 42,1 | 44,9 | 42 | 53 | 29,2 | 35,3 | 41,5 | 43,8 | 28,1 | 34,7 | 27,6 |
| 42 | 30,4 | 29,6 | 39,3 | 28,8 | 37,7 | 41,2 | 36,4 | 45,9 | 37,1 | 36,6 | 34,5 | 37,3 | 27,8 | 32,2 | 26,9 |
| 43 | 38 | 38,8 | 42,1 | 33,6 | 41,3 | 44,9 | 41,2 | 52,2 | 29,2 | 35,3 | 40,7 | 42,9 | 28,1 | 34,7 | 27,6 |
| 44 | 33,6 | 33,3 | 41,4 | 30,5 | 42,2 | 39 | 44,1 | 49,1 | 27,4 | 40,2 | 45,5 | 39,6 | 26 | 31,3 | 23,1 |
| 45 | 86,1 | 87 | 32 | 95,7 | 32,3 | 34,7 | 33,3 | 36,4 | 29,2 | 25,7 | 30,8 | 26,3 | 61,7 | 78,3 | 50,3 |

FIGURE 14 (continued)

| | 46 | 47 | 48 | 49 | 50 | 51 | 52 | 53 | 54 | 55 | 56 | 57 | 58 | 59 | 60 |
|---|---|---|---|---|---|---|---|---|---|---|---|---|---|---|---|
| 46 | | 98,2 | 35 | 84,6 | 33,6 | 32 | 33,9 | 36,7 | 33,1 | 29,1 | 31,6 | 29,7 | 65,6 | 76,7 | 51,9 |
| 47 | 98,2 | | 33,6 | 85,5 | 34,4 | 32 | 33,9 | 35,8 | 33,1 | 28,2 | 34,2 | 29,7 | 65,6 | 75,9 | 52,6 |
| 48 | 51,3 | 50,4 | | 30,2 | 93,9 | 29,9 | 49,6 | 37,7 | 30,9 | 35,7 | 52,2 | 33 | 26,5 | 33,6 | 25,6 |
| 49 | 88,7 | 89,6 | 53 | | 31,7 | 33,1 | 32,5 | 35,2 | 28,6 | 25,2 | 32,5 | 24,3 | 58,3 | 75,9 | 50,3 |
| 50 | 50,4 | 51,3 | 96,5 | 53,9 | | 29,9 | 47 | 39 | 31,7 | 35,7 | 52,2 | 33 | 26,4 | 34,4 | 26,3 |
| 51 | 52,6 | 52,6 | 58,3 | 50,4 | 60,9 | | 32,8 | 40,4 | 26,7 | 35,1 | 35,5 | 30,4 | 27,2 | 30,6 | 25 |
| 52 | 52,7 | 54,5 | 67 | 51,3 | 67 | 55,3 | | 40,5 | 29,3 | 41,5 | 63,2 | 39,3 | 25,5 | 30,7 | 27,5 |
| 53 | 55,5 | 56,4 | 61,7 | 54,8 | 60 | 60,5 | 58,5 | | 35,1 | 34,9 | 42,2 | 41,3 | 30,2 | 34,7 | 28,1 |
| 54 | 50 | 51,8 | 53,9 | 47 | 54,8 | 53,5 | 54,7 | 54,7 | | 30,2 | 25,4 | 31,8 | 27,5 | 31,1 | 26,3 |
| 55 | 43,6 | 42,7 | 53 | 43,5 | 53,9 | 47,4 | 60,6 | 52,8 | 46,2 | | 41,5 | 38,9 | 26,3 | 31,5 | 24,2 |
| 56 | 47,3 | 49,1 | 67 | 47,8 | 68,7 | 59,6 | 81,7 | 62,3 | 51,9 | 64,6 | | 41,2 | 28 | 30,7 | 26,4 |
| 57 | 40,9 | 41,8 | 48,7 | 36,5 | 49,6 | 49,1 | 50 | 61,3 | 47,2 | 54,4 | 58,6 | | 22,4 | 27,8 | 20,4 |
| 58 | 68,9 | 68,9 | 41,7 | 64,9 | 40,4 | 43 | 39,7 | 43 | 44,4 | 35,1 | 38,4 | 29,8 | | 57,7 | 58,8 |
| 59 | 90,9 | 90 | 50,4 | 82,6 | 51,3 | 52,6 | 52,8 | 54,6 | 49,1 | 50 | 49,1 | 39,8 | 64,9 | | 50,6 |
| 60 | 60,9 | 62,2 | 40,4 | 60,3 | 41,7 | 39,1 | 41 | 40,4 | 38,5 | 35,3 | 35,9 | 27,6 | 70,5 | 55,8 | |
| 61 | 91,2 | 91,2 | 53,9 | 86,1 | 52,2 | 50 | 53,5 | 55,3 | 51,8 | 43 | 46,5 | 40,4 | 75,5 | 85,1 | 66 |
| 62 | 88,1 | 88,1 | 52,5 | 84,7 | 53,4 | 50 | 51,7 | 53,4 | 46,6 | 43,2 | 47,5 | 39,8 | 75,5 | 82,2 | 65,4 |
| 63 | 56,2 | 55,6 | 34,8 | 53,4 | 34,8 | 38,8 | 34,3 | 38,8 | 34,8 | 29,2 | 30,9 | 27 | 56,2 | 60,7 | 51,7 |
| 64 | 56,2 | 56,2 | 34,8 | 53,9 | 34,3 | 38,8 | 34,3 | 38,8 | 34,3 | 29,2 | 28,7 | 27 | 56,7 | 59,6 | 52,2 |

FIGURE 14 (continued)

|    | 61   | 62   | 63   | 64   |
|----|------|------|------|------|
| 1  | 30,3 | 30,5 | 24,2 | 24,2 |
| 2  | 30,7 | 30,3 | 20,6 | 20,6 |
| 3  | 37,6 | 36,4 | 25,6 | 25,6 |
| 4  | 30,4 | 30,5 | 22,8 | 22,8 |
| 5  | 26,1 | 27,8 | 29,8 | 29,8 |
| 6  | 32,2 | 31,4 | 24,2 | 23,6 |
| 7  | 33,3 | 32,2 | 21,9 | 21,9 |
| 8  | 33,9 | 32,8 | 22,3 | 22,3 |
| 9  | 32   | 32,6 | 25,7 | 25,7 |
| 10 | 30,5 | 28,2 | 22,9 | 22,9 |
| 11 | 30,7 | 31   | 23,2 | 23,2 |
| 12 | 31,2 | 32,3 | 26,2 | 25,7 |
| 13 | 25,4 | 23,7 | 17,3 | 17,3 |
| 14 | 65,8 | 62,7 | 35,6 | 36,9 |
| 15 | 29,7 | 30,3 | 23,3 | 20,8 |
| 16 | 33,6 | 35   | 20,1 | 20,7 |
| 17 | 30,8 | 33,6 | 21,1 | 21,1 |
| 18 | 36,1 | 37,2 | 22,8 | 22,8 |
| 19 | 30,3 | 30,3 | 22,9 | 20,2 |
| 20 | 34,7 | 30,3 | 22,5 | 22,5 |
| 21 | 30,7 | 30,5 | 22,5 | 22,5 |
| 22 | 28,9 | 30,5 | 22,5 | 22,5 |
| 23 | 27,2 | 29,7 | 18   | 18   |
| 24 | 36,1 | 34,7 | 23,6 | 24,3 |
| 25 | 30,7 | 30,5 | 22,5 | 22,5 |
| 26 | 28,9 | 31,9 | 22,5 | 22,5 |
| 27 | 26,7 | 27,4 | 21   | 21   |
| 28 | 26,3 | 27,1 | 19,6 | 18,4 |
| 29 | 25,7 | 24,4 | 22,1 | 22,1 |
| 30 | 32,5 | 31,1 | 21,7 | 21,7 |
| 31 | 30,7 | 28,8 | 20,1 | 20,1 |
| 32 | 32,8 | 32,3 | 24,3 | 24,3 |
| 33 | 33,9 | 32,8 | 28,7 | 28,7 |
| 34 | 55,8 | 53,2 | 31,9 | 33   |
| 35 | 55,8 | 53,2 | 31,9 | 33   |
| 36 | 31,4 | 30   | 26,4 | 25,3 |
| 37 | 33,3 | 34,4 | 21,9 | 20,2 |
| 38 | 31,4 | 30   | 25,6 | 24,4 |
| 39 | 36,8 | 35,2 | 24,9 | 24,9 |
| 40 | 33,3 | 34,4 | 20,2 | 20,2 |
| 41 | 36,8 | 35,4 | 26   | 26   |
| 42 | 33,3 | 33,1 | 21,9 | 21,9 |
| 43 | 36,8 | 35,4 | 26   | 26   |
| 44 | 30,4 | 32   | 23,3 | 23,3 |
| 45 | 80,7 | 77,7 | 48,6 | 49,2 |

FIGURE 14 (continued)

|     | 61   | 62   | 63   | 64   |
|-----|------|------|------|------|
| 46  | 86,8 | 83,1 | 47,8 | 48,1 |
| 47  | 86,8 | 83,1 | 47,3 | 48,1 |
| 48  | 31,7 | 31,5 | 22,3 | 22,3 |
| 49  | 76   | 74,8 | 47,3 | 47,8 |
| 50  | 31,7 | 32,3 | 21,8 | 21,3 |
| 51  | 32   | 31,2 | 25,1 | 25,1 |
| 52  | 32,5 | 36,1 | 21,2 | 21,2 |
| 53  | 35,5 | 34,4 | 24,2 | 24,2 |
| 54  | 30,6 | 26,7 | 23,6 | 23   |
| 55  | 29,9 | 29,4 | 19,1 | 19,1 |
| 56  | 31,7 | 32,8 | 19,6 | 20,1 |
| 57  | 26,1 | 26,1 | 19,1 | 19,1 |
| 58  | 75,5 | 71,6 | 47,8 | 48,3 |
| 59  | 74,2 | 71,3 | 60,7 | 59,6 |
| 60  | 57,7 | 60,3 | 41   | 41,5 |
| 61  |      | 94,1 | 46,8 | 47,1 |
| 62  | 96,6 |      | 45,3 | 45,5 |
| 63  | 54,5 | 54,5 |      | 98,9 |
| 64  | 53,4 | 53,4 | 98,9 |      |

FIGURE 14 (continued)

FIGURE 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5811238 A
- US 6395547 B
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A, Zarling
- WO 9853083 A, Grierson
- WO 9953050 A, Waterhouse
- US 4987071 A, Cech

- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- EP 1198985 A1
- WO 2007093444 A
- US 5164310 A
- US 5159135 A
- WO 9623891 A
- WO 2010151634 A
- EP 1831378 A
- WO 2010031780 A
- WO 2006029987 A

**Non-patent literature cited in the description**

- **WANG et al.** *Planta,* 2003, vol. 218, 1-14
- **BARTA et al.** *Curr. Top. Microbiol. Immunol.,* 2008, vol. 326, 83-102
- **KALYNA et al.** *Nucleic Acids Res.,* 2006, vol. 34, 4395-4405
- **KALYNA et al.** *Mol Biol. Cell,* 2003, vol. 14, 3565-3577
- **LOPATO et al.** *J. Biol. Chem.,* 2002, vol. 277, 39989-39998
- **ISSHIKI et al.** *Plant Cell,* 2006, vol. 18, 146-148
- **CHAKRABARTY et al.** *Chemosphere,* 2009, vol. 74, 688-702
- **GADJEV et al.** *Plant Physiology,* 2006, vol. 141, 436-445
- **SWEETLOVE et al.** *Plant Journal,* 2002, vol. 32, 891-904
- **HO et al.** *Plant Physiology,* 2008, vol. 147, 1858-1873
- **JUN et al.** *Plant Signaling & Behavior,* 2008, vol. 9, 615-617
- **WENKEL et al.** *The Plant Cell,* 2007, vol. 19, 3379-3390
- **KIM et al.** *The Plant Cell,* 2008, vol. 20, 920-933
- **TERPE.** *Appl. Microbiol. Biotechnol.,* 2003, vol. 60, 523-533

- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7

- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856

- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHORN.** *Plant Cell,* 2001, vol. 13 (2), 303-318
- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- *Mol Gen Genet,* vol. 208, 1-9
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38

- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- Current Protocols in Molecular Biology
- **LANDSCHULZ et al.** *Science,* 1988, vol. 240, 1759-1764
- **BAILEY ; ELKAN.** Proceedings of the Second International Conference on Intelligent Systems for Molecular Biology. AAAI Press, 1994, 28-36
- **HUELSENBECK ; RONQUIST.** *Bioinformatics,* 2001, vol. 17, 754-755
- **SAMBROOK.** Molecular Cloning: a laboratory manual. Cold Spring Harbor Laboratory Press, 2001
- **AUSUBEL et al.** Current Protocols in Molecular Biology, Current Protocols. Standard materials and methods for plant molecular work. 1994, vol. 1 and 2
- **R.D.D. CROY.** Plant Molecular Biology Labfax. BIOS Scientific Publications Ltd (UK) and Blackwell Scientific Publications, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **PARK ; KANEHISA.** *Bioinformatics,* 2003, vol. 19, 1656-1663
- **LORKOVIC et al.** *Exp. Cell Res.,* 2008, vol. 314, 3175-3186
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158
- **MURASHIGE, T. ; SKOOG, .** *Physiol. Plant,* 1962, vol. 15, 473-497
- **GAMBORG et al.** *Exp. Cell Res.,* vol. 50, 151-8
- **HUSSEY, G. ; HEPHER, A.** Annals of Botany. 1978, vol. 42, 477-9
- **LINSEY, K. ; GALLOIS, P.** *Journal of Experimental Botany,* 1990, vol. 41 (226), 529-36
- **ARENCIBIA et al.** *Transgenic Research,* 1998, vol. 7, 213-22
- **ENRIQUEZ-OBREGON et al.** *Planta,* 1998, vol. 206, 20-27
- **GAMBORG, O. et al.** *Exp. Cell Res.,* 1968, vol. 50, 151-8
- **KATOH ; TOH.** *Briefings in Bioinformatics,* 2008, vol. 9, 286-298
- *BMC Bioinformatics,* 2003, vol. 4